# EUROPEAN PATENT SPECIFICATION

(11) **EP 2 756 083 B1**
(45) Date of publication and mention of the grant of the patent: **19.08.2020**
(21) Application number: 12830932.5
(22) Date of filing: 12.09.2012
(51) Int. Cl.: C12N 15/82, A01N 27/00

(54) **METHODS AND COMPOSITIONS FOR WEED CONTROL**
VERFAHREN UND ZUSAMMENSETZUNGEN ZUR UNKRAUTBEKÄMPFUNG
PROCÉDÉS ET COMPOSITIONS DE LUTTE CONTRE LES MAUVAISES HERBES

(30) Priority: 13.09.2011 US 201161534066 P
(43) Date of publication of application: 23.07.2014
(73) Proprietor: Monsanto Technology LLC, St. Louis, MO 63167 (US)
(72) Inventor: ADER, Daniel, St. Louis Missouri 63167 (US); FINNESSY, John, J., St. Louis Missouri 63167 (US); KAPOOR, Mahak, St. Louis Missouri 63167 (US); LI, Zhaolong, St. Louis Missouri 63167 (US); MASUCCI, James, D., St. Louis Missouri 63167 (US); SHAH, Ronak, Hasmukh, St. Louis Missouri 63167 (US); TAO, Nengbing, St. Louis Missouri 63167 (US); TAYLOR, Jennifer, Chou, St. Louis Missouri 63167 (US); WANG, Dafu, St. Louis Missouri 63167 (US); YANG, Heping, St. Louis Missouri 63167 (US)
(74) Representative: Uexküll & Stolberg
(86) International application number: PCT/US2012/054842
(87) International publication number: WO 2013/040021

(56) References cited:
- EP-A1- 2 545 182
- WO-A2-02/066660
- WO-A2-03/077648
- US-A1- 2005 289 664
- US-A1- 2006 009 358
- "Agronomy Facts 37: Adjuvants for enhancing herbicide performance", , 26 January 2000 (2000-01-26), pages 1-8, XP055097433, U.S.A. Retrieved from the Internet: URL:http://pubs.cas.psu.edu/freepubs/pdfs/ uc106.pdf [retrieved on 2014-01-21]
- HOFGEN ET AL.: 'Repression of Acetolactate Synthase Activity through Antisense Inhibition 0 (Molecular and Biochemical Analysis of Transgenic Potato (Solanum tuberosum L. cv Desiree) Plants).' PLANT PHYSIOL vol. 107, no. 2, February 1995, pages 469 - 477, XP055146743
- ZHANG ET AL.: 'Agrobacterium-mediated transformation of Arabidopsis thaliana using the floral dip method.' NATURE PROTOCOLS vol. 1, no. 2, 2006, pages 1 - 6, XP055146744
- VENCILL ET AL.: 'Resistance of Weeds to Herbicides.' HERBICIDES AND ENVIRONMENT. 08 January 2011, NEW YORK, pages 585 - 594, XP055146746 ISBN: 978-953-307-4 Retrieved from the Internet: <URL:http://www.intechopen.com/books/herbic ides-and-environment/resistance-of-weeds-to - herbicides>

## Description

### FIELD

The invention relates generally to the field of weed management. More specifically, the invention relates to 4-hydroxyphenyl-pyruvate-dioxygenase genes in weedy plants and compositions containing polynucleotide molecules for modulating their expression. The invention further provides methods and compositions useful for weed control.

### BACKGROUND

Weeds are plants that compete with cultivated plants in an agronomic environment and cost farmers billions of dollars annually in crop losses and the expense of efforts to keep weeds under control. Weeds also serve as hosts for crop diseases and insect pests. The losses caused by weeds in agricultural production environments include decreases in crop yield, reduced crop quality, increased irrigation costs, increased harvesting costs, reduced land value, injury to livestock, and crop damage from insects and diseases harbored by the weeds. The principal means by which weeds cause these effects are: 1) competing with crop plants for water, nutrients, sunlight and other essentials for growth and development, 2) production of toxic or irritant chemicals that cause human or animal health problem, 3) production of immense quantities of seed or vegetative reproductive parts or both that contaminate agricultural products and perpetuate the species in agricultural lands, and 4) production on agricultural and nonagricultural lands of vast amounts of vegetation that must be disposed of. Herbicide tolerant weeds are a problem with nearly all herbicides in use, there is a need to effectively manage these weeds. There are over 365 weed biotypes currently identified as being herbicide resistant to one or more herbicides by the Herbicide Resistance Action Committee (HRAC), the North American Herbicide Resistance Action Committee (NAHRAC), and the Weed Science Society of America (WSSA).

The 4-hydroxyphenyl-pyruvate-dioxygenase (HPPD) is an Fe-containing enzyme, that catalyzes the second reaction in the catabolism of tyrosine, the conversion of 4-hydroxyphenylpyruvate to homogentisate. This enzyme is the target of many herbicides that include members of the chemical families of Triketones, Isoxazoles, and Pyrazoles.

WO 02/066660 discloses screening methods for identifying a herbicidally active substance by "selecting substances which reduce or block the expression or the activity of a number of sequences different from HPPD sequences without disclosing a novel herbicide.

### SUMMARY OF THE INVENTION

In one aspect, the invention provides method of plant control comprising: topically applying to a plant surface a composition comprising a double-stranded RNA (dsRNA) polynucleotide and a transfer agent, wherein said dsRNA polynucleotide is identical or complementary to an RNA sequence of a 4-hydroxyphenyl-pyruvate-dioxygenase (HPPD) gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide, and whereby said plant's growth, development, or reproductive ability is reduced or said plant is more sensitive to an HPPD inhibitor herbicide, relative to an untreated plant.

In another aspect of the invention, composition comprising a dsRNA polynucleotide and a transfer agent is provided, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone surfactant composition or an organosilicone compound contained therein and conditions the surface of a plant for permeation by said dsRNA polynucleotide, and whereby said plant treated with said composition has its growth, development, or reproductive ability suppressed or delayed or said plant is more sensitive to an HPPD inhibitor herbicide as a result of said dsRNA polynucleotide containing composition, relative to an untreated plant.

In another aspect, the invention also provides a method of reducing expression of an HPPD gene in a plant, which comprises: topically applying to a plant surface a composition comprising a dsRNA polynucleotide and a transfer agent, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide, and whereby said expression of said HPPD gene is reduced relative to an untreated plant. Preferably, the dsRNA polynucleotide is at least 19 contiguous nucleotides, at least 20 contiguous nucleotides, or at least 21 contiguous nucleotides in length.

In another aspect, the invention also provides a method of identifying dsRNA polynucleotides useful in modulating HPPD gene expression when topically applied to a plant surface, which comprises: a) providing a plurality of dsRNA polynucleotides that comprise a region identical or complementary to at least 18 contiguous nucleotides of an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082; b) topically applying to said plant surface a composition comprising one or more of said dsRNA polynucleotides and a transfer agent; c) analyzing said plant, or a plant extract thereof, for modulation of HPPD gene expression, wherein said transfer agent is an organosilicone surfactant composition or an organosilicone compound contained therein and conditions the surface of said plant for permeation by said dsRNA polynucleotide, and d) said plant treated with said composition has its growth, development, or reproductive ability, suppressed or delayed or said plant is more sensitive to an HPPD inhibitor herbicide as a result of said composition relative to an untreated plant.

In another aspect, the invention provides an agricultural chemical composition comprising an admixture of a dsRNA polynucleotide, a transfer agent, an HPPD inhibitor herbicide, and a co-herbicide, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone composition or an organosilicone compound contained therein and conditions a surface of a plant for permeation by said dsRNA polynucleotide, and whereby said plant treated with said composition has its growth, development, or reproductive ability suppressed or delayed or said plant is more sensitive to said HPPD inhibitor herbicide as a result of said dsRNA polynucleotide containing composition, relative to an untreated plant.

In another aspect, the invention provides an agricultural chemical composition comprising an admixture of a dsRNA polynucleotide, a transfer agent, an HPPD inhibitor herbicide, and a pesticide, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone composition or an organosilicone compound contained therein and conditions a surface of a plant for permeation by said dsRNA polynucleotide, and whereby said plant treated with said composition has its growth, development, or reproductive ability suppressed or delayed or said plant is more sensitive to said HPPD inhibitor herbicide as a result of said dsRNA polynucleotide containing composition, relative to an untreated plant.

### BRIEF DESCRIPTION OF THE FIGURES

The following drawings form part of the present specification and are included to further demonstrate certain aspects of the present invention. The invention may be better understood by reference to one or more of these drawings in combination with the detailed description of specific embodiments presented herein. The invention can be more fully understood from the following description of the figures:

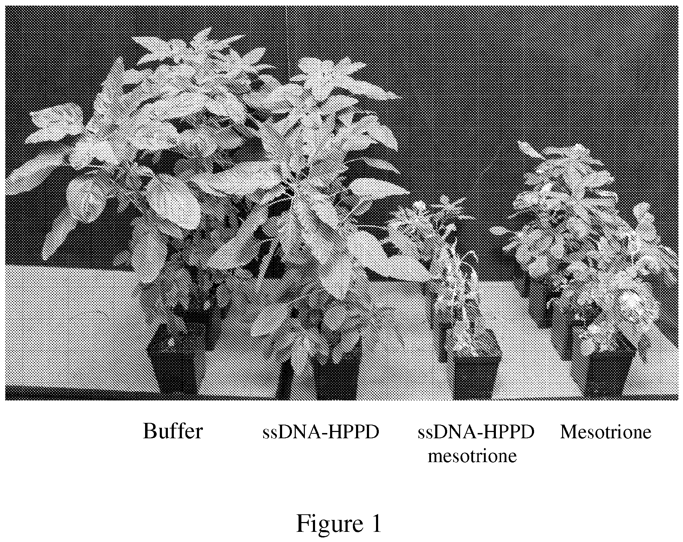

Figure 1. Treatment of *Amaranthus palmeri* plants with ssDNA trigger polynucleotides and HPPD inhibitor herbicide, Mesotrione.

### DETAILED DESCRIPTION

Provided are methods and compositions containing a polynucleotide that provide for regulation, repression or delay of HPPD (4-hydroxyphenyl-pyruvate-dioxygenase) gene expression and enhanced control of weedy plant species amd importantly HPPD inhibitor herbicide resistant weed biotypes. Aspects of the method can be applied to manage various weedy plants in agronomic and other cultivated environments.

The following definitions and methods are provided to better define the present invention and to guide those of ordinary skill in the art in the practice of the present invention. Unless otherwise noted, terms are to be understood according to conventional usage by those of ordinary skill in the relevant art. Where a term is provided in the singular, the inventors also contemplate aspects of the invention described by the plural of that term.

By "non-transcribable" polynucleotides is meant that the polynucleotides do not comprise a complete polymerase II transcription unit.

As used herein "solution" refers to homogeneous mixtures and non-homogeneous mixtures such as suspensions, colloids, micelles, and emulsions.

Weedy plants are plants that compete with cultivated plants, those of particular importance include, but are not limited to important invasive and noxious weeds and herbicide resistant biotypes in crop production, such as, *Amaranthus* species *-A. albus*, *A. blitoides*, *A. hybridus*, *A. palmeri*, *A. powellii*, *A. retroflexus*, *A. spinosus*, *A. tuberculatus*, and *A. viridis*; *Ambrosia* species - *A. trifida, A. artemisifolia; Lolium* species *-L. multiflorum, L. rigidium, L perenne; Digitaria* species *-D. insularis; Euphorbia* species *-E. heterophylla; Kochia species* - *K. scoparia; Sorghum* species *-S. halepense; Conyza* species -*C*. *bonariensis, C. canadensis, C. sumatrensis; Chloris* species *-C. truncate; Echinochola* species - *E. colona, E. crus-galli; Eleusine species -E. indica; Poa* species *-P. annua; Plantago* species *-P. lanceolata; Avena species* - *A. fatua; Chenopodium* species - *C. album; Setaria* species - *S. viridis, Abutilon theophrasti, Ipomoea* species, *Sesbania,* species, *Cassia* species, *Sida* species, *Brachiaria,* species and *Solanum* species.

Additional weedy plant species found in cultivated areas include *Alopecurus myosuroides, Avena sterilis, Avena sterilis ludoviciana, Brachiaria plantaginea, Bromus diandrus, Bromus rigidus, Cynosurus echinatus, Digitaria ciliaris, Digitaria ischaemum, Digitaria sanguinalis, Echinochloa oryzicola, Echinochloa phyllopogon, Eriochloa punctata, Hordeum glaucum, Hordeum leporinum, Ischaemum rugosum, Leptochloa chinensis, Lolium persicum, , Phalaris minor, Phalaris paradoxa, Rottboellia exalta, Setaria faberi, Setaria viridis var, robusta-alba schreiber, Setaria viridis var, robusta -purpurea, Snowdenia polystachea, Sorghum sudanese, Alisma plantago-aquatica, Amaranthus lividus, Amaranthus quitensis, Ammania auriculata, Ammania coccinea, Anthemis cotula, Apera spica-venti, Bacopa rotundifolia, Bidens pilosa, Bidens subalternans, Brassica tournefortii, Bromus tectorum, Camelina microcarpa, Chrysanthemum coronarium, Cuscuta campestris, Cyperus difformis, Damasonium minus, Descurainia sophia, Diplotaxis tenuifolia, Echium plantagineum, Elatine triandra var, pedicellata, Euphorbia heterophylla, Fallopia convolvulus, Fimbristylis miliacea, Galeopsis tetrahit, Galium spurium, Helianthus annuus, Iva xanthifolia, Ixophorus unisetus, Ipomoea indica, Ipomoea purpurea, Ipomoea sepiaria, Ipomoea aquatic, Ipomoea triloba, Lactuca serriola, Limnocharis flava, Limnophila erecta, Limnophila sessiliflora, Lindernia dubia, Lindernia dubia var, major, Lindernia micrantha, Lindernia procumbens, Mesembryanthemum crystallinum, Monochoria korsakowii, Monochoria vaginalis, Neslia paniculata, Papaver rhoeas, Parthenium hysterophorus, Pentzia suffruticosa, Phalaris minor, Raphanus raphanistrum, Raphanus sativus, Rapistrum rugosum, Rotala indica var, uliginosa, Sagittaria guyanensis, Sagittaria montevidensis, Sagittaria pygmaea, Salsola iberica, Scirpus juncoides var, ohwianus, Scirpus mucronatus, Setaria lutescens, Sida spinosa, Sinapis arvensis, Sisymbrium orientale, Sisymbrium thellungii, Solanum ptycanthtim, Sonchus asper, Sonchus oleraceus, Sorghum bicolor, Stellaria media, Thlaspi arvense, Xanthium strumarium, Aretotheca calendula, Conyza sumatrensis, Crassocephalum crepidiodes, Cuphea carthagenenis, Epilobium adenocaulon, Erigeron philadelphicus, Landoltia punctata, Lepidium virginicum, Monochoria korsakowii, Solanum americanum, Solanum nigrum, Vulpia bromoides, Youngia japonica, Hydrilla verticillata, Carduus nutans, Carduus pycnocephalus, Centaurea solstitialis, Cirsium arvense, Commelina diffusa, Convolvulus arvensis, Daucus carota, Digitaria ischaemum, Echinochloa crus-pavonis, Fimbristylis miliacea, Galeopsis tetrahit, Galium spurium, Limnophila erecta, Matricaria perforate, Papaver rhoeas, Ranunculus acris, Soliva sessilis, Sphenoclea zeylanica, Stellaria media, Nassella trichotoma, Stipa neesiana, Agrostis stolonifera, Polygonum aviculare, Alopecurus japonicus, Beckmannia syzigachne, Bromus tectorum, Chloris inflate, Echinochloa erecta, Portulaca oleracea, and Senecio vulgaris.* It is believed that all plants contain a phytoene desaturase gene in their genome, the sequence of which can be isolated and polynucleotides made
that are useful for regulation, suppressing or delaying the expression of the target HPPD gene in the plants and the growth or development of the treated plants.

A cultivated plant may also be considered a weedy plant when they occur in unwanted environments. For example, corn plants growing in a soybean field. Transgenic crops with one or more herbicide tolerances will need specialized methods of management to control weeds and volunteer crop plants. The present invention enables the targeting of a transgene for herbicide tolerance to permit the treated plants to become sensitive to the herbicide. For example, transgene HPPD DNA sequences in transgenic events that include FG72.

A "trigger" or "trigger polynucleotide" is a polynucleotide molecule that is homologous or complementary to a target gene polynucleotide. The trigger polynucleotide molecules modulate expression of the target gene when topically applied to a plant surface with a transfer agent, whereby a plant treated with said composition has its growth or development or reproductive ability regulated, suppressed or delayed or said plant is more sensitive to a HPPD inhibitor herbicide as a result of said polynucleotide containing composition relative to a plant not treated with a composition containing the trigger molecule. Trigger polynucleotides disclosed herein are generally described in relation to the target gene sequence and maybe used in the sense (homologous) or antisense (complementary) orientation as single stranded molecules or comprise both strands as double stranded molecules or nucleotide variants and modified nucleotides thereof depending on the various regions of a gene being targeted.

It is contemplated that the composition of the present invention will contain multiple polynucleotides and herbicides that include but not limited to HPPD gene trigger polynucleotides and an HPPD inhibitor herbicide and anyone or more additional herbicide target gene trigger polynucleotides and the related herbicides and anyone or more additional essential gene trigger polynucleotides. Essential genes are genes in a plant that provide key enzymes or other proteins, for example, a biosynthetic enzyme, metabolizing enzyme, receptor, signal transduction protein, structural gene product, transcription factor, or transport protein; or regulating RNAs, such as, microRNAs, that are essential to the growth or survival of the organism or cell or involved in the normal growth and development of the plant (Meinke, et al., Trends Plant Sci. 2008 Sep;13(9):483-91). The suppression of an essential gene enhances the effect of a herbicide that affects the function of a gene product different than the suppressed essential gene. The compositions of the present invention can include various trigger polynucleotides that modulate the expression of an essential gene other than HPPD.

Herbicides for which transgenes for plant tolerance have been demonstrated and the method can be applied, include but are not limited to: auxin-like herbicides, glyphosate, glufosinate, sulfonylureas, imidazolinones, bromoxynil, delapon, dicamba, cyclohezanedione, protoporphyrionogen oxidase inhibitors, 4-hydroxyphenyl-pyruvate-dioxygenase inhibitors herbicides. For example, transgenes and their polynucleotide molecules that encode proteins involved in herbicide tolerance are known in the art, and include, but are not limited to an 5-enolpyruvylshikimate-3-phosphate synthase (EPSPS), for example, as more fully described in U.S. Pat. Nos. 7,807,791 (SEQ ID NO:5); 6,248,876 B1; 5,627,061; 5,804,425; 5,633,435; 5,145,783; 4,971,908; 5,312,910; 5,188,642; 4,940,835; 5,866,775; 6,225,114 B1; 6,130,366; 5,310,667; 4,535,060; 4,769,061; 5,633,448; 5,510,471; U.S. Pat. No. Re. 36,449; U.S. Pat. Nos. RE 37,287 E; and 5,491,288; tolerance to sulfonylurea and/or imidazolinone, for example, as described more fully in U.S. Pat. Nos. 5,605,011; 5,013,659; 5,141,870; 5,767,361; 5,731,180; 5,304,732; 4,761,373; 5,331,107; 5,928,937; and 5,378,824; and international publication WO 96/33270; tolerance to hydroxyphenylpyruvatedioxygenases inhibitiong herbicides in plants are described in U.S. Pat. Nos. 6,245,968 B1; 6,268,549; and 6,069,115; US Pat.Pub. 20110191897 and US7,312,379 SEQ ID NO:3; US7,935,869; US7,304,209, SEQ ID NO:1, 3,5 and 15; aryloxyalkanoate dioxygenase polynucleotides, which confer tolerance to 2,4-D and other phenoxy auxin herbicides as well as to aryloxyphenoxypropionate herbicides as described, for example, in WO2005/107437; US7,838,733 SEQ ID NO:5;) and dicamba-tolerance polynucleotides as described, for example, in Herman et al. (2005) J. Biol. Chem. 280: 24759-24767. Other examples of herbicide-tolerance traits include those conferred by polynucleotides encoding an exogenous phosphinothricin acetyltransferase, as described in U.S. Pat. Nos. 5,969,213; 5,489,520; 5,550,318; 5,874,265; 5,919,675; 5,561,236; 5,648,477; 5,646,024; 6,177,616; and 5,879,903. Plants containing an exogenous phosphinothricin acetyltransferase can exhibit improved tolerance to glufosinate herbicides, which inhibit the enzyme glutamine synthase. Additionally, herbicide-tolerance polynucleotides include those conferred by polynucleotides conferring altered protoporphyrinogen oxidase (protox) activity, as described in U.S. Pat. Nos. 6,288,306 B1; 6,282,837 B1; and 5,767,373; and WO 01/12825. Plants containing such polynucleotides can exhibit improved tolerance to any of a variety of herbicides which target the protox enzyme (also referred to as protox inhibitors). Polynucleotides encoding a glyphosate oxidoreductase and a glyphosate-N-acetyl transferase (GOX described in U.S. Patent 5,463,175 and GAT described in U.S. Patent publication 20030083480, dicamba monooxygenase U.S. Patent publication 20030135879
); a polynucleotide molecule encoding bromoxynil nitrilase (*Bxn* described in U.S. Patent No. 4,810,648 for Bromoxynil tolerance
); a polynucleotide molecule encoding phytoene desaturase (*crtI*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:318-2193 for tolerance to sulfonylurea herbicides; and the *bar* gene described in DeBlock, et al. (1987) EMBO J. 6:2513-2519 for glufosinate and bialaphos tolerance. The transgenic coding regions and regulatory elements of the herbicide tolerance genes are targets in which polynucleotide triggers and herbicides can be included in the composition of the present invention.

The composition of the present invention include a component that is an HPPD inhibitor herbicide which includes but are not limited to Triketones, such as, mesotrione, tefuryltrione, tembotrione, and sulcotrione; Isoxazoles, such as, isoxachlortole, pyrasulfotole, and isoxaflutole; Pyrazoles, such as, benzofenap, pyrazolynate, topramezone and pyrazoxyfen. Additional HPPD inhibitors include benzobicyclon and bicyclopyrone.

Numerous herbicides with similar or different modes of action (herein referred to as co-herbicides) are available that can be added to the composition, for example, members of the herbicide families that include but are not limited to amide herbicides, aromatic acid herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides. In particular, the rates of use of the added herbicides can be reduced in compositions comprising the polynucleotides of the invention. Use rate reductions of the additional added herbicides can be 10-25 percent, 26-50 percent, 51-75 percent or more can be achieved that enhance the activity of the polynucleotides and herbicide composition and is contemplated. Representative herbicides of the families include but are not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthal-dimethyl, cinidon-ethyl, cinmethylin, cinosulfuron, cisanilide, clethodim, clodinafop, clodinafop-propargyl, clomazone, clomeprop, clopyralid, cloransulam, cloransulam-methyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, glyphosate, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuron-ethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t-etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonyl-sulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoro-methyl)-3-pyridi- nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon-yl]-bicyclo[3.2.]oct-3-en-2-one. Additionally, including herbicidal compounds of unspecified modes of action as described in CN101279950A, CN101279951A, DE10000600A1, DE10116399A1, DE102004054666A1, DE102005014638A1, DE102005014906A1, DE102007012168A1, DE102010042866A1, DE10204951A1, DE10234875A1, DE10234876A1, DE10256353A1, DE10256354A1, DE10256367A1, EP1157991A2, EP1238586A1, EP2147919A1, EP2160098A2, JP03968012B2, JP2001253874A, JP2002080454A, JP2002138075A, JP2002145707A, JP2002220389A, JP2003064059A, JP2003096059A, JP2004051628A, JP2004107228A, JP2005008583A, JP2005239675A, JP2005314407A, JP2006232824A, JP2006282552A, JP2007153847A, JP2007161701A, JP2007182404A, JP2008074840A, JP2008074841A, JP2008133207A, JP2008133218A, JP2008169121A, JP2009067739A, JP2009114128A, JP2009126792A, JP2009137851A, US20060111241A1, US20090036311A1, US20090054240A1, US20090215628A1, US20100099561A1, US20100152443A1, US20110105329A1, US20110201501A1, WO2001055066A2, WO2001056975A1, WO2001056979A1, WO2001090071A2, WO2001090080A1, WO2002002540A1, WO2002028182A1, WO2002040473A1, WO2002044173A2, WO2003000679A2, WO2003006422A1 , WO2003013247A1, WO2003016308A1, WO2003020704A1, WO2003022051A1, WO2003022831A1, WO2003022843A1, WO2003029243A2, WO2003037085A1, WO2003037878A1, WO2003045878A2, WO2003050087A2, WO2003051823A1, WO2003051824A1, WO2003051846A2, WO2003076409A1, WO2003087067A1, WO2003090539A1, WO2003091217A1, WO2003093269A2, WO2003104206A2, WO2004002947A1, WO2004002981A2, WO2004011429A1, WO2004029060A1, WO2004035545A2, WO2004035563A1, WO2004035564A1, WO2004037787A1, WO2004067518A1, WO2004067527A1, WO2004077950A1, WO2005000824A1, WO2005007627A1, WO2005040152A1, WO2005047233A1, WO2005047281A1, WO2005061443A2, WO2005061464A1, WO2005068434A1, WO2005070889A1, WO2005089551A1, WO2005095335A1, WO2006006569A1, WO2006024820A1, WO2006029828A1, WO2006029829A1, WO2006037945A1, WO2006050803A1, WO2006090792A1, WO2006123088A2, WO2006125687A1, WO2006125688A1, WO2007003294A1, WO2007026834A1, WO2007071900A1, WO2007077201A1, WO2007077247A1, WO2007096576A1, WO2007119434A1, WO2007134984A1, WO2008009908A1, WO2008029084A1, WO2008059948A1, WO2008071918A1, WO2008074991A1, WO2008084073A1, WO2008100426A2, WO2008102908A1, WO2008152072A2, WO2008152073A2, WO2009000757A1, WO2009005297A2, WO2009035150A2, WO2009063180A1, WO2009068170A2, WO2009068171A2, WO2009086041A1, WO2009090401A2, WO2009090402A2, WO2009115788A1, WO2009116558A1, WO2009152995A1, WO2009158258A1, WO2010012649A1, WO2010012649A1, WO2010026989A1, WO2010034153A1, WO2010049270A1, WO2010049369A1, WO2010049405A1, WO2010049414A1, WO2010063422A1, WO2010069802A1, WO2010078906A2, WO2010078912A1, WO2010104217A1, WO2010108611A1, WO2010112826A3, WO2010116122A3, WO2010119906A1, WO2010130970A1, WO2011003776A2, WO2011035874A1, WO2011065451A1

The trigger polynucleotide and oligonucleotide molecule compositions are useful in compositions, such as liquids that comprise the polynucleotide molecules at low concentrations, alone or in combination with other components, for example one or more herbicide molecules, either in the same solution or in separately applied liquids that also provide a transfer agent. While there is no upper limit on the concentrations and dosages of polynucleotide molecules that can useful in the methods, lower effective concentrations and dosages will generally be sought for efficiency. The concentrations can be adjusted in consideration of the volume of spray or treatment applied to plant leaves or other plant part surfaces, such as flower petals, stems, tubers, fruit, anthers, pollen, or seed. In one embodiment, a useful treatment for herbaceous plants using 25-mer oligonucleotide molecules is about 1 nanomole (nmol) of oligonucleotide molecules per plant, for example, from about 0.05 to 1 nmol per plant. Other embodiments for herbaceous plants include useful ranges of about 0.05 to about 100 nmol, or about 0.1 to about 20 nmol, or about 1 nmol to about 10 nmol of polynucleotides per plant. Very large plants, trees, or vines may require correspondingly larger amounts of polynucleotides. When using long dsRNA molecules that can be processed into multiple oligonucleotides, lower concentrations can be used. To illustrate embodiments, the factor 1X, when applied to oligonucleotide molecules is arbitrarily used to denote a treatment of 0.8 nmol of polynucleotide molecule per plant; 10X, 8 nmol of polynucleotide molecule per plant; and 100X, 80 nmol of polynucleotide molecule per plant.

The polynucleotide compositions are useful in compositions, such as liquids that comprise polynucleotide molecules, alone or in combination with other components either in the same liquid or in separately applied liquids that provide a transfer agent. As used herein, a transfer agent is an agent that, when combined with a polynucleotide in a composition that is topically applied to a target plant surface, enables the polynucleotide to enter a plant cell. In certain embodiments, a transfer agent is an agent that conditions the surface of plant tissue, e. g., leaves, stems, roots, flowers, or fruits, to permeation by the polynucleotide molecules into plant cells. The transfer of polynucleotides into plant cells can be facilitated by the prior or contemporaneous application of a polynucleotide-transferring agent to the plant tissue. In some embodiments the transferring agent is applied subsequent to the application of the polynucleotide composition. The polynucleotide transfer agent enables a pathway for polynucleotides through cuticle wax barriers, stomata and/or cell wall or membrane barriers into plant cells. Suitable transfer agents to facilitate transfer of the polynucleotide into a plant cell include agents that increase permeability of the exterior of the plant or that increase permeability of plant cells to oligonucleotides or polynucleotides. Such agents to facilitate transfer of the composition into a plant cell include a chemical agent, or a physical agent, or combinations thereof. Chemical agents for conditioning or transfer include (a) surfactants, (b) an organic solvent or an aqueous solution or aqueous mixtures of organic solvents, (c) oxidizing agents, (d) acids, (e) bases, (f) oils, (g) enzymes, or combinations thereof. Embodiments of the method can optionally include an incubation step, a neutralization step (e.g., to neutralize an acid, base, or oxidizing agent, or to inactivate an enzyme), a rinsing step, or combinations thereof. Embodiments of agents or treatments for conditioning of a plant to permeation by polynucleotides include emulsions, reverse emulsions, liposomes, and other micellar-like compositions. Embodiments of agents or treatments for conditioning of a plant to permeation by polynucleotides include counter-ions or other molecules that are known to associate with nucleic acid molecules, *e. g.*, inorganic ammonium ions, alkyl ammonium ions, lithium ions, polyamines such as spermine, spermidine, or putrescine, and other cations. Organic solvents useful in conditioning a plant to permeation by polynucleotides include DMSO, DMF, pyridine, *N*-pyrrolidine, hexamethylphosphoramide, acetonitrile, dioxane, polypropylene glycol, other solvents miscible with water or that will dissolve phosphonucleotides in non-aqueous systems (such as is used in synthetic reactions). Naturally derived or synthetic oils with or without surfactants or emulsifiers can be used, *e*. *g.*, plant-sourced oils, crop oils (such as those listed in the 9^{th} Compendium of Herbicide Adjuvants, publicly available on the worldwide web (internet) at herbicide.adjuvants.com can be used, *e. g.*, paraffinic oils, polyol fatty acid esters, or oils with short-chain molecules modified with amides or polyamines such as polyethyleneimine or *N*-pyrrolidine. Transfer agents include, but are not limited to, organosilicone preparations.

An agronomic field in need of plant control is treated by application of the composition directly to the surface of the growing plants, such as by a spray. For example, the method is applied to control weeds in a field of crop plants by spraying the field with the composition. The composition can be provided as a tank mix, a sequential treatment of components (generally the polynucleotide containing composition followed by the herbicide), or a simultaneous treatment or mixing of one or more of the components of the composition from separate containers. Treatment of the field can occur as often as needed to provide weed control and the components of the composition can be adjusted to target specific weed species or weed families through utilization of specific polynucleotides or polynucleotide compositions capable of selectively targeting the specific species or plant family to be controlled. The composition can be applied at effective use rates according to the time of application to the field, for example, preplant, at planting, post planting, post harvest. HPPD inhibitor herbicides can be applied to a field at rates of 1 to 2000 g ai/ha (active ingredient per hectare or more. The polynucleotides of the composition can be applied at rates of 1 to 30 grams per acre depending on the number of trigger molecules needed for the scope of weeds in the field.

Crop plants in which weed control is needed include but are not limited to, i) corn, soybean, cotton, canola, sugar beet, alfalfa, sugarcane, rice, and wheat; ii) vegetable plants including, but not limited to, tomato, sweet pepper, hot pepper, melon, watermelon, cucumber, eggplant, cauliflower, broccoli, lettuce, spinach, onion, peas, carrots, sweet corn, Chinese cabbage, leek, fennel, pumpkin, squash or gourd, radish, Brussels sprouts, tomatillo, garden beans, dry beans, or okra; iii) culinary plants including, but not limited to, basil, parsley, coffee, or tea; or, iv) fruit plants including but not limited to apple, pear, cherry, peach, plum, apricot, banana, plantain, table grape, wine grape, citrus, avocado, mango, or berry; v) a tree grown for ornamental or commercial use, including, but not limited to, a fruit or nut tree; or, vi) an ornamental plant (e. g., an ornamental flowering plant or shrub or turf grass). The methods and compositions provided herein can also be applied to plants produced by a cutting, cloning, or grafting process (i. e., a plant not grown from a seed) include fruit trees and plants that include, but are not limited to, citrus, apples, avocados, tomatoes, eggplant, cucumber, melons, watermelons, and grapes as well as various ornamental plants.

### Pesticidal Mixtures

The polynucleotide compositions may also be used as mixtures with various agricultural chemicals and/or insecticides, miticides and fungicides, pesticidal and biopesticidal agents. Examples include but are not limited to azinphos-methyl, acephate, isoxathion, isofenphos, ethion, etrimfos, oxydemeton-methyl, oxydeprofos, quinalphos, chlorpyrifos, chlorpyrifos-methyl, chlorfenvinphos, cyanophos, dioxabenzofos, dichlorvos, disulfoton, dimethylvinphos, dimethoate, sulprofos, diazinon, thiometon, tetrachlorvinphos, temephos, tebupirimfos, terbufos, naled, vamidothion, pyraclofos, pyridafenthion, pirimiphos-methyl, fenitrothion, fenthion, phenthoate, flupyrazophos, prothiofos, propaphos, profenofos, phoxime, phosalone, phosmet, formothion, phorate, malathion, mecarbam, mesulfenfos, methamidophos, methidathion, parathion, methyl parathion, monocrotophos, trichlorphon, EPN, isazophos, isamidofos, cadusafos, diamidaphos, dichlofenthion, thionazin, fenamiphos, fosthiazate, fosthietan, phosphocarb, DSP, ethoprophos, alanycarb, aldicarb, isoprocarb, ethiofencarb, carbaryl, carbosulfan, xylylcarb, thiodicarb, pirimicarb, fenobucarb, furathiocarb, propoxur, bendiocarb, benfuracarb, methomyl, metolcarb, XMC, carbofuran, aldoxycarb, oxamyl, acrinathrin, allethrin, esfenvalerate, empenthrin, cycloprothrin, cyhalothrin, gamma-cyhalothrin, lambda-cyhalothrin, cyfluthrin, beta-cyfluthrin, cypermethrin, alpha-cypermethrin, zeta-cypermethrin, silafluofen, tetramethrin, tefluthrin, deltamethrin, tralomethrin, bifenthrin, phenothrin, fenvalerate, fenpropathrin, furamethrin, prallethrin, flucythrinate, fluvalinate, flubrocythrinate, permethrin, resmethrin, ethofenprox, cartap, thiocyclam, bensultap, acetamiprid, imidacloprid, clothianidin, dinotefuran, thiacloprid, thiamethoxam, nitenpyram, chlorfluazuron, diflubenzuron, teflubenzuron, triflumuron, novaluron, noviflumuron, bistrifluoron, fluazuron, flucycloxuron, flufenoxuron, hexaflumuron, lufenuron, chromafenozide, tebufenozide, halofenozide, methoxyfenozide, diofenolan, cyromazine, pyriproxyfen, buprofezin, methoprene, hydroprene, kinoprene, triazamate, endosulfan, chlorfenson, chlorobenzilate, dicofol, bromopropylate, acetoprole, fipronil, ethiprole, pyrethrin, rotenone, nicotine sulphate, BT (Bacillus Thuringiensis) agent, spinosad, abamectin, acequinocyl, amidoflumet, amitraz, etoxazole, chinomethionat, clofentezine, fenbutatin oxide, dienochlor, cyhexatin, spirodiclofen, spiromesifen, tetradifon, tebufenpyrad, binapacryl, bifenazate, pyridaben, pyrimidifen, fenazaquin, fenothiocarb, fenpyroximate, fluacrypyrim, fluazinam, flufenzin, hexythiazox, propargite, benzomate, polynactin complex, milbemectin, lufenuron, mecarbam, methiocarb, mevinphos, halfenprox, azadirachtin, diafenthiuron, indoxacarb, emamectin benzoate, potassium oleate, sodium oleate, chlorfenapyr, tolfenpyrad, pymetrozine, fenoxycarb, hydramethylnon, hydroxy propyl starch, pyridalyl, flufenerim, flubendiamide, flonicamid, metaflumizole, lepimectin, TPIC, albendazole, oxibendazole, oxfendazole, trichlamide, fensulfothion, fenbendazole, levamisole hydrochloride, morantel tartrate, dazomet, metam-sodium, triadimefon, hexaconazole, propiconazole, ipconazole, prochloraz, triflumizole, tebuconazole, epoxiconazole, difenoconazole, flusilazole, triadimenol, cyproconazole, metconazole, fluquinconazole, bitertanol, tetraconazole, triticonazole, flutriafol, penconazole, diniconazole, fenbuconazole, bromuconazole, imibenconazole, simeconazole, myclobutanil, hymexazole, imazalil, furametpyr, thifluzamide, etridiazole, oxpoconazole, oxpoconazole fumarate, pefurazoate, prothioconazole, pyrifenox, fenarimol, nuarimol, bupirimate, mepanipyrim, cyprodinil, pyrimethanil, metalaxyl, mefenoxam, oxadixyl, benalaxyl, thiophanate, thiophanate-methyl, benomyl, carbendazim, fuberidazole, thiabendazole, manzeb, propineb, zineb, metiram, maneb, ziram, thiuram, chlorothalonil, ethaboxam, oxycarboxin, carboxin, flutolanil, silthiofam, mepronil, dimethomorph, fenpropidin, fenpropimorph, spiroxamine, tridemorph, dodemorph, flumorph, azoxystrobin, kresoxim-methyl, metominostrobin, orysastrobin, fluoxastrobin, trifloxystrobin, dimoxystrobin, pyraclostrobin, picoxystrobin, iprodione, procymidone, vinclozolin, chlozolinate, flusulfamide, dazomet, methyl isothiocyanate, chloropicrin, methasulfocarb, hydroxyisoxazole, potassium hydroxyisoxazole, echlomezol, D-D, carbam, basic copper chloride, basic copper sulfate, copper nonylphenolsulfonate, oxine copper, DBEDC, anhydrous copper sulfate, copper sulfate pentahydrate, cupric hydroxide, inorganic sulfur, wettable sulfur, lime sulfur, zinc sulfate, fentin, sodium hydrogen carbonate, potassium hydrogen carbonate, sodium hypochlorite, silver, edifenphos, tolclofos-methyl, fosetyl, iprobenfos, dinocap, pyrazophos, carpropamid, fthalide, tricyclazole, pyroquilon, diclocymet, fenoxanil, kasugamycin, validamycin, polyoxins, blasticiden S, oxytetracycline, mildiomycin, streptomycin, rape seed oil, machine oil, benthiavalicarbisopropyl, iprovalicarb, propamocarb, diethofencarb, fluoroimide, fludioxanil, fenpiclonil, quinoxyfen, oxolinic acid, chlorothalonil, captan, folpet, probenazole, acibenzolar-S-methyl, tiadinil, cyflufenamid, fenhexamid, diflumetorim, metrafenone, picobenzamide, proquinazid, famoxadone, cyazofamid, fenamidone, zoxamide, boscalid, cymoxanil, dithianon, fluazinam, dichlofluanide, triforine, isoprothiolane, ferimzone, diclomezine, tecloftalam, pencycuron, chinomethionat, iminoctadine acetate, iminoctadine albesilate, ambam, polycarbamate, thiadiazine, chloroneb, nickel dimethyldithiocarbamate, guazatine, dodecylguanidine-acetate, quintozene, tolylfluanid, anilazine, nitrothalisopropyl, fenitropan, dimethirimol, benthiazole, harpin protein, flumetover, mandipropamide and penthiopyrad.

### Polynucleotides

As used herein, the term "DNA", "DNA molecule", "DNA polynucleotide molecule" refers to a single-stranded DNA (ssDNA) or double-stranded DNA (dsDNA) molecule of genomic or synthetic origin, such as, a polymer of deoxyribonucleotide bases or a DNA polynucleotide molecule. As used herein, the term "DNA sequence", "DNA nucleotide sequence" or "DNA polynucleotide sequence" refers to the nucleotide sequence of a DNA molecule. As used herein, the term "RNA", "RNA molecule", "RNA polynucleotide molecule" refers to a single-stranded RNA (ssRNA) or double-stranded RNA (dsRNA) molecule of genomic or synthetic origin, such as, a polymer of ribonucleotide bases that comprise single or double stranded regions. Unless otherwise stated, nucleotide sequences in the text of this specification are given, when read from left to right, in the 5' to 3' direction. The nomenclature used herein is that required by Title 37 of the United States Code of Federal Regulations § 1.822 and set forth in the tables in WIPO Standard ST.25 (1998), Appendix 2, Tables 1 and 3.

As used herein, "polynucleotide" refers to a DNA or RNA molecule containing multiple nucleotides and generally refers both to "oligonucleotides" (a polynucleotide molecule of typically 50 or fewer nucleotides in length) and polynucleotides of 51 or more nucleotides. Embodiments of this invention include compositions including oligonucleotides having a length of 18-25 nucleotides (18-mers, 19-mers, 20-mers, 21-mers, 22-mers, 23-mers, 24-mers, or 25-mers)
or medium-length polynucleotides having a length of 26 or more nucleotides (polynucleotides of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, or about 300 nucleotides), or fragments thereof or long polynucleotides having a length greater than about 300 nucleotides (for example, polynucleotides of between about 300 to about 400 nucleotides, between about 400 to about 500 nucleotides, between about 500 to about 600 nucleotides, between about 600 to about 700 nucleotides, between about 700 to about 800 nucleotides, between about 800 to about 900 nucleotides, between about 900 to about 1000 nucleotides, between about 300 to about 500 nucleotides, between about 300 to about 600 nucleotides, between about 300 to about 700 nucleotides, between about 300 to about 800 nucleotides, between about 300 to about 900 nucleotides, or about 1000 nucleotides in length, or even greater than about 1000 nucleotides in length, for example up to the entire length of a target gene including coding or non-coding or both coding and non-coding portions of the target gene).

A target gene comprises any polynucleotide molecule in a plant cell or fragment thereof for which the modulation of the expression of the target gene is provided by the methods and compositions of the present invention. Where a polynucleotide is double-stranded, its length can be similarly described in terms of base pairs. Oligonucleotides and polynucleotides
can be made that are essentially identical or essentially complementary to adjacent genetic elements of a gene, for example, spanning the junction region of an intron and exon, the junction region of a promoter and a transcribed region, the junction region of a 5' leader and a coding sequence, the junction of a 3' untranslated region and a coding sequence.

Examples of chemically modified oligonucleotides or polynucleotides are well known in the art; see, for example, US Patent Publication 20110171287, US Patent Publication 20110171176, and US Patent Publication 20110152353, US Patent Publication, 20110152346, US Patent Publication 20110160082. For example, including but not limited to the naturally occurring phosphodiester backbone of an oligonucleotide or polynucleotide can be partially or completely modified with phosphorothioate, phosphorodithioate, or methylphosphonate internucleotide linkage modifications, modified nucleoside bases or modified sugars can be used in oligonucleotide or polynucleotide synthesis, and oligonucleotides or polynucleotides can be labeled with a fluorescent moiety (for example, fluorescein or rhodamine) or other label (for example, biotin).

The polynucleotides can be double-stranded RNA

The term "gene" refers to chromosomal DNA, plasmid DNA, cDNA, intron and exon DNA, artificial DNA polynucleotide, or other DNA that encodes a peptide, polypeptide, protein, or RNA transcript molecule, and the genetic elements flanking the coding sequence that are involved in the regulation of expression, such as, promoter regions, 5' leader regions, 3' untranslated regions.

The trigger polynucleotide molecules are designed to modulate expression by inducing regulation or suppression of an endogenous HPPD gene in a plant and are designed to have a nucleotide sequence essentially identical or essentially complementary to the nucleotide sequence of an endogenous HPPD gene of a plant or to the sequence of RNA transcribed from an endogenous HPPD gene of a plant, including a transgene in a plant that provides for a herbicide resistant HPPD enzyme, which can be coding sequence or non-coding sequence. Effective molecules that modulate expression are referred to as "a trigger molecule, or trigger polynucleotide". By "essentially identical" or "essentially complementary" is meant that the trigger polynucleotides (or at least one strand of a double-stranded polynucleotide or portion thereof, or a portion of a single strand polynucleotide) are designed to hybridize to the endogenous gene noncoding sequence or to RNA transcribed (known as messenger RNA or an RNA transcript) from the endogenous gene to effect regulation or suppression of expression of the endogenous gene. Trigger molecules are identified by "tiling" the gene targets with partially overlapping probes or non-overlapping probes of antisense or sense polynucleotides that are essentially identical or essentially complementary to the nucleotide sequence of an endogenous gene. Multiple target sequences can be aligned and sequence regions with homology in common
are identified as potential trigger molecules for the multiple targets. Multiple trigger molecules of various lengths, for example 18-25 nucleotides, 26-50 nucleotides, 51-100 nucleotides, 101-200 nucleotides, 201-300 nucleotides or more can be pooled into a few treatments in order to investigate polynucleotide molecules that cover a portion of a gene sequence (for example, a portion of a coding versus a portion of a noncoding region, or a 5' versus a 3' portion of a gene) or an entire gene sequence including coding and noncoding regions of a target gene. Polynucleotide molecules of the pooled trigger molecules can be divided into smaller pools or single molecules inorder to identify trigger molecules that provide the desired effect.

The target gene RNA and DNA polynucleotide molecules are (Table 1, SEQ ID NO: 1-32) sequenced by any number of available methods and equipment. Some of the sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Illumina/Solexa (Hayward, Calif.) and Helicos Biosciences (Cambridge, Mass.), and the sequencing-by-ligation platform from Applied Biosystems (Foster City, Calif.), as described below. In addition to the single molecule sequencing performed using sequencing-by-synthesis of Helicos Biosciences, other single molecule sequencing technologies are encompassed by the method of the invention and include the SMRT.TM. technology of Pacific Biosciences, the Ion Torrent.TM. technology, and nanopore sequencing being developed for example, by Oxford Nanopore Technologies. A HPPD target gene comprising DNA or RNA can be isolated using primers or probes essentially complementary or essentially homologous to SEQ ID NO:1-32 or a fragment thereof. A polymerase chain reaction (PCR) gene fragment can be produced using primers essentially complementary or essentially homologous to SEQ ID NO:1-32 or a fragment thereof that is useful to isolate an HPPD gene from a plant genome. SEQ ID NO: 1-32 or fragments thereof can be used in various sequence capture technologies to isolate additional target gene sequences

"Identity" refers to the degree of similarity between two polynucleic acid or protein sequences. An alignment of the two sequences is performed by a suitable computer program. A widely used and accepted computer program for performing sequence alignments is CLUSTALW vl.6 (Thompson, et al. Nucl. Acids Res., 22: 4673-4680, 1994). The number of matching bases or amino acids is divided by the total number of bases or amino acids, and multiplied by 100 to obtain a percent identity. For example, if two 580 base pair sequences had 145 matched bases, they would be 25 percent identical. If the two compared sequences are of different lengths, the number of matches is divided by the shorter of the two lengths. For example, if there are 100 matched amino acids between a 200 and a 400 amino acid protein, they are 50 percent identical with respect to the shorter sequence. If the shorter sequence is less than 150 bases or 50 amino acids in length, the number of matches are divided by 150 (for nucleic acid bases) or 50 (for amino acids), and multiplied by 100 to obtain a percent identity.

Trigger molecules for specific gene family members can be identified from coding and/or non-coding sequences of gene families of a plant or multiple plants, by aligning and selecting 200-300 polynucleotide fragments from the least homologous regions amongst the aligned sequences and evaluated using topically applied polynucleotides (as sense or anti-sense ssDNA or ssRNA, dsRNA, or dsDNA) to determine their relative effectiveness in inducing the herbicidal phenotype. The effective segments are further subdivided into 50-60 polynucleotide fragments, prioritized by least homology, and reevaluated using topically applied polynucleotides. The effective 50-60 polynucleotide fragments are subdivided into 19-30 polynucleotide fragments, prioritized by least homology, and again evaluated for induction of the yield/quality phenotype. Once relative effectiveness is determined, the fragments are utilized singly, or again evaluated in combination with one or more other fragments to determine the trigger composition or mixture of trigger polynucleotides for providing the yield/quality phenotype.

Trigger molecules for broad activity can be identified from coding and/or non-coding sequences of gene families of a plant or multiple plants, by aligning and selecting 200-300 polynucleotide fragments from the most homologous regions amongst the aligned sequences and evaluated using topically applied polynucleotides (as sense or anti-sense ssDNA or ssRNA, dsRNA, or dsDNA) to determine their relative effectiveness in inducing the yield/quality phenotype. The effective segments are subdivided into 50-60 polynucleotide fragments, prioritized by most homology, and reevaluated using topically applied polynucleotides. The effective 50-60 polynucleotide fragments are subdivided into 19-30 polynucleotide fragments, prioritized by most homology, and again evaluated for induction of the yield/quality phenotype. Once relative effectiveness is determined, the fragments may be utilized singly, or in combination with one or more other fragments to determine the trigger composition or mixture of trigger polynucleotides for providing the yield/quality phenotype.

Methods of making polynucleotides are well known in the art. Chemical synthesis, in vivo synthesis and in vitro synthesis methods and compositions are known in the art and include various viral elements, microbial cells, modified polymerases, and modified nucleotides. Commercial preparation of oligonucleotides often provides two deoxyribonucleotides on the 3' end of the sense strand. Long polynucleotide molecules can be synthesized from commercially available kits, for example, kits from Applied Biosystems/Ambion (Austin, TX) have DNA ligated on the 5' end in a microbial expression cassette that includes a bacterial T7 polymerase promoter that makes RNA strands that can be assembled into a dsRNA and kits provided by various manufacturers that include T7 RiboMax Express (Promega, Madison, WI), AmpliScribe T7-Flash (Epicentre, Madison, WI), and TranscriptAid T7 High Yield (Fermentas, Glen Burnie, MD). dsRNA molecules can be produced from microbial expression cassettes in bacterial cells (Ongvarrasopone et al. ScienceAsia 33:35-39; Yin, Appl. Microbiol. Biotechnol 84:323-333, 2009; Liu et al., BMC Biotechnology 10:85, 2010) that have regulated or deficient RNase III enzyme activity or the use of various viral vectors to produce sufficient quantities of dsRNA. In the present invention, HPPD gene fragments are inserted into the microbial expression cassettes in a position in which the fragments are express to produce dsRNA useful in the methods described herein to regulate expression on a target HPPD gene. Long polynucleotide molecules can also be assembled from multiple RNA or DNA fragments. In some embodiments design parameters such as Reynolds score (Reynolds et al. Nature Biotechnology 22, 326 - 330 (2004),Tuschl rules (Pei and Tuschl, Nature Methods 3(9): 670-676, 2006), i-score (Nucleic Acids Res 35: e123, 2007), i-Score Designer tool and associated algorithms (Nucleic Acids Res 32: 936-948, 2004. Biochem Biophys Res Commun 316: 1050-1058, 2004, Nucleic Acids Res 32: 893-901, 2004, Cell Cycle 3: 790-5, 2004, Nat Biotechnol 23: 995-1001, 2005, Nucleic Acids Res 35: e27, 2007, BMC Bioinformatics 7: 520, 2006, Nucleic Acids Res 35: e123, 2007, Nat Biotechnol 22: 326-330, 2004) are known in the art and may be used in selecting polynucleotide sequences effective in gene silencing. In some embodiments the sequence of a polynucleotide is screened against the genomic DNA of the intended plant to minimize unintentional silencing of other genes.

Ligands can be tethered to a polynucleotide, for example a dsRNA, ssRNA, dsDNA or ssDNA. Ligands in general can include modifiers, e.g., for enhancing uptake; diagnostic compounds or reporter groups e.g., for monitoring distribution; cross-linking agents; nuclease-resistance conferring moieties; and natural or unusual nucleobases. General examples include lipophiles, lipids (e.g., cholesterol, a bile acid, or a fatty acid (e.g., lithocholic-oleyl, lauroyl, docosnyl, stearoyl, palmitoyl, myristoyl oleoyl, linoleoyl), steroids (e.g., uvaol, hecigenin, diosgenin), terpenes (e.g., triterpenes, e.g., sarsasapogenin, Friedelin, epifriedelanol derivatized lithocholic acid), vitamins (e.g., folic acid, vitamin A, biotin, pyridoxal), carbohydrates, proteins, protein binding agents, integrin targeting molecules, polycationics, peptides, polyamines, and peptide mimics. The ligand may also be a recombinant or synthetic molecule, such as a synthetic polymer, e.g., polyethylene glycol (PEG), PEG-40K, PEG-20K and PEG-5K. Other examples of ligands include lipophilic molecules, e.g, cholesterol, cholic acid, adamantane acetic acid, 1-pyrene butyric acid, dihydrotestosterone, glycerol (e.g., esters and ethers thereof, e.g., C.sub.10, C.sub.11, C.sub.12, C.sub.13, C.sub.14, C.sub.15, C.sub.16, C.sub.17, C.sub.18, C.sub.19, or C.sub.20 alkyl; e.g., lauroyl, docosnyl, stearoyl, oleoyl, linoleoyl 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dodecanoyl, lithocholyl, 5.beta.-cholanyl, N,N-distearyl-lithocholamide, 1,2-di-O-stearoylglyceride, dimethoxytrityl, or phenoxazine) and PEG (e.g., PEG-5K, PEG-20K, PEG-40K). Preferred lipophilic moieties include lipid, cholesterols, oleyl, retinyl, or cholesteryl residues.

Conjugating a ligand to a dsRNA can enhance its cellular absorption, lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-radiated endocytosis. dsRNA compounds bearing folic acid would be efficiently transported into the cell via the folate-receptor-mediated endocytosis. Other ligands that have been conjugated to oligonucleotides include polyethylene glycols, carbohydrate clusters, cross-linking agents, porphyrin conjugates, delivery peptides and lipids such as cholesterol. In certain instances, conjugation of a cationic ligand to oligonucleotides results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed, throughout the oligonucleotide. See M. Manoharan Antisense & Nucleic Acid Drug Development 2002, 12, 103 and references therein.

A biologic delivery can be accomplished by a variety of methods including, without limitation, (1) loading liposomes with a dsRNA acid molecule provided herein and (2) complexing a dsRNA molecule with lipids or liposomes to form nucleic acid-lipid or nucleic acid-liposome complexes. The liposome can be composed of cationic and neutral lipids commonly used to transfect cells in vitro. Cationic lipids can complex (e.g., charge-associate) with negatively charged, nucleic acids to form liposomes. Examples of cationic liposomes include, without limitation, lipofectin, lipofectamine, lipofectace, and DOTAP. Procedures for forming liposomes are well known in the art. Liposome compositions can be formed, for example, from phosphatidylcholine, dimyristoyl phosphatidylcholine, dipalmitoyl phosphatidylcholine, dimyristoyl phosphatidyl glycerol, dioleoyl phosphatidylethanolamine or liposomes comprising dihydrosphingomyelin (DHSM) Numerous lipophilic agents are commercially available, including Lipofectin® (Invitrogen/Life Technologies, Carlsbad, Calif.) and Effectene™ (Qiagen, Valencia, Calif.), In addition, systemic delivery methods can be optimized using commercially available cationic lipids such as DDAB or DOTAP, each of which can be mixed with a neutral lipid such as DOPE or cholesterol. In some cases, liposomes such as those described by Templeton et al. (Nature Biotechnology, 15:647-652 (1997)) can be used. In other embodiments, polycations such as polyethyleneimine can be used to achieve delivery in vivo and ex vivo (Boletta et al., J. Am Soc. Nephrol. 7:1728 (1996)). Additional information regarding the use of liposomes to deliver nucleic acids can be found in U.S. Pat. No. 6,271,359, PCT Publication WO 96/40964 and Morrissey, D. et al. 2005. Nat Biotechnol. 23(8):1002-7.

In certain embodiments, an organosilicone preparation that is commercially available as Silwet® L-77 surfactant having CAS Number 27306-78-1 and EPA Number: CAL.REG.NO. 5905-50073-AA, and currently available from Momentive Performance Materials, Albany, New York can be used to prepare a polynucleotide composition. In certain embodiments where a Silwet L-77 organosilicone preparation is used as a pre-spray treatment of plant leaves or other plant surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation comprising Silwet L-77 in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

In certain embodiments, any of the commercially available organosilicone preparations provided such as the following Breakthru S 321, Breakthru S 200 Cat# 67674-67-3, Breakthru OE 441 Cat#68937-55-3, Breakthru S 278 Cat #27306-78-1, Breakthru S 243, Breakthru S 233 Cat#134180-76-0, available from manufacturer Evonik Goldschmidt (Germany), Silwet® HS 429, Silwet® HS 312, Silwet® HS 508, Silwet® HS 604 (Momentive Performance Materials, Albany, New York) can be used as transfer agents in a polynucleotide composition. In certain embodiments where an organosilicone preparation is used as a pre-spray treatment of plant leaves or other surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Organosilicone preparations used in the methods and compositions provided herein can comprise one or more effective organosilicone compounds. As used herein, the phrase "effective organosilicone compound" is used to describe any organosilicone compound that is found in an organosilicone preparation that enables a polynucleotide to enter a plant cell. In certain embodiments, an effective organosilicone compound can enable a polynucleotide to enter a plant cell in a manner permitting a polynucleotide mediated suppression of a target gene expression in the plant cell. In general, effective organosilicone compounds include, but are not limited to, compounds that can comprise: i) a trisiloxane head group that is covalently linked to, ii) an alkyl linker including, but not limited to, an n-propyl linker, that is covalently linked to, iii) a poly glycol chain, that is covalently linked to, iv) a terminal group. Trisiloxane head groups of such effective organosilicone compounds include, but are not limited to, heptamethyltrisiloxane. Alkyl linkers can include, but are not limited to, an n-propyl linker. Polyglycol chains include, but are not limited to, polyethylene glycol or polypropylene glycol. Polyglycol chains can comprise a mixture that provides an average chain length "n" of about "7.5". In certain embodiments, the average chain length "n" can vary from about 5 to about 14. Terminal groups can include, but are not limited to, alkyl groups such as a methyl group. Effective organosilicone compounds are believed to include, but are not limited to, trisiloxane ethoxylate surfactants or polyalkylene oxide modified heptamethyl trisiloxane.

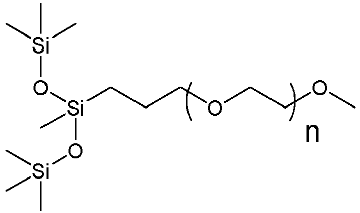

### (Compound I: polyalkyleneoxide heptamethyltrisiloxane, average n=7.5)

In certain embodiments, an organosilicone preparation that comprises an organosilicone compound comprising a trisiloxane head group is used in the methods and compositions provided herein. In certain embodiments, an organosilicone preparation that comprises an organosilicone compound comprising a heptamethyltrisiloxane head group is used in the methods and compositions provided herein. In certain embodiments, an organosilicone composition that comprises Compound I is used in the methods and compositions provided herein. In certain embodiments, an organosilicone composition that comprises Compound I is used in the methods and compositions provided herein. In certain embodiments of the methods and compositions provided herein, a composition that comprises a polynucleotide molecule and one or more effective organosilicone compound in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Compositions include but are not limited components that are one or more polynucleotides essentially identical to, or essentially complementary to an HPPD gene sequence (promoter, intron, exon, 5' untranslated region, 3' untranslated region), a transfer agent that provides for the polynucleotide to enter a plant cell, a herbicide that complements the action of the polynucleotide, one or more additional herbicides that further enhance the herbicide activity of the composition or provide an additional mode of action different from the complementing herbicide, various salts and stabilizing agents that enhance the utility of the composition as an admixture of the components of the composition.

In certain aspects, methods include one or more applications of a polynucleotide composition and one or more applications of a permeability-enhancing agent for conditioning of a plant to permeation by polynucleotides. When the agent for conditioning to permeation is an organosilicone composition or compound contained therein, embodiments of the polynucleotide molecules are double-stranded RNA oligonucleotides, single-stranded RNA oligonucleotides, double-stranded RNA polynucleotides, single-stranded RNA polynucleotides, double-stranded DNA oligonucleotides, single-stranded DNA oligonucleotides, double-stranded DNA polynucleotides, single-stranded DNA polynucleotides, chemically modified RNA or DNA oligonucleotides or polynucleotides or mixtures thereof.

Compositions and methods are useful for modulating the expression of an endogenous HPPD gene (for example, US Pat. No. 7,297,541, U.S. Patent Publ. 20110185444, and 20110185445) or transgenic HPPD gene (for example,U.S. Patent No: 7,312,379, U.S. Patent Publ. 20110191897) or HPPD inhibitor inactivating genes (US Pat. No. 6,268,549; 6,768,044; 7,312,379; 7,304,209; WO 96/38567, WO 99/24585) in a plant cell. In various embodiments, an HPPD gene includes coding (protein-coding or translatable) sequence, non-coding (non-translatable) sequence, or both coding and non-coding sequence. Compositions can include polynucleotides and oligonucleotides designed to target multiple genes, or multiple segments of one or more genes. The target gene can include multiple consecutive segments of a target gene, multiple non-consecutive segments of a target gene, multiple alleles of a target gene, or multiple target genes from one or more species.

Provided is a method for modulating expression of an HPPD gene in a plant including (a) conditioning of a plant to permeation by polynucleotides and (b) treatment of the plant with the polynucleotide molecules, wherein the polynucleotide molecules include at least one segment of 18 or more contiguous nucleotides cloned from or otherwise identified from the target HPPD gene in either anti-sense or sense orientation, whereby the polynucleotide molecules permeate the interior of the plant and induce modulation of the target gene. The conditioning and polynucleotide application can be performed separately or in a single step. When the conditioning and polynucleotide application are performed in separate steps, the conditioning can precede or can follow the polynucleotide application within minutes, hours, or days. In some embodiments more than one conditioning step or more than one polynucleotide molecule application can be performed on the same plant. In embodiments of the method, the segment can be cloned or identified from (a) coding (protein-encoding), (b) non-coding (promoter and other gene related molecules), or (c) both coding and non-coding parts of the target gene. Non-coding parts include DNA, such as promoter regions or the RNA transcribed by the DNA that provide RNA regulatory molecules, including but not limited to: introns, 5' or 3' untranslated regions, and microRNAs (miRNA), *trans*-acting siRNAs, natural anti-sense siRNAs, and other small RNAs with regulatory function or RNAs having structural or enzymatic function including but not limited to: ribozymes, ribosomal RNAs, t-RNAs, aptamers, and riboswitches.

The following examples are included to demonstrate examples of certain preferred embodiments of the invention.

### EXAMPLES

### Example 1. Polynucleotides related to the HPPD gene sequences.

The target HPPD gene polynucleotide molecules have been found that naturally occur in the genome of *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum, and Xanthium strumarium* and include molecules related to the expression of a polypeptide identified as an HPPD, that include regulatory molecules, cDNAs comprising coding and noncoding regions of an HPPD gene and fragments thereof as shown in Table 1.

Polynucleotide molecules were extracted from these plant species by methods standard in the field, for example, total RNA is extracted using Trizol Reagent (Invitrogen Corp, Carlsbad, CA Cat. No. 15596-018), following the manufacturer's protocol or modifications thereof by those skilled in the art of polynucleotide extraction that may enhance recover or purity of the extracted RNA. Briefly, start with 1 gram of ground plant tissue for extraction. Prealiquot 10 milliliters (mL) Trizol reagent to 15 mL conical tubes. Add ground powder to tubes and shake to homogenize. Incubate the homogenized samples for 5 minutes (min) at room temperature (RT) and then add 3 mL of chloroform. Shakes tubes vigorously by hand for 15-30 seconds(sec) and incubate at RT for 3 min. Centrifuge the tubes at 7,000 revolutions per minute (rpm) for 10 min at 4 degrees C. Transfer the aqueous phase to a new 1.5 mL tube and add 1 volume of cold isopropanol. Incubate the samples for 20-30 min at RT and centrifuge at 10,000 rpm for 10 min at 4 degrees C. Wash pellet with Sigma-grade 80 percent ethanol. Remove the supernatant and briefly air-dry the pellet. Dissolve the RNA pellet in approximately 200 microliters of DEPC treated water. Heat briefly at 65C to dissolve pellet and vortex or pipet to resuspend RNA pellet. Adjust RNA concentraiton to 1-2 microgram/microliter.

DNA was extracted using EZNA SP Plant DNA Mini kit (Omega Biotek, Norcross GA, Cat#D5511) and Lysing Matrix E tubes (Q-Biogen, Cat#6914), following the manufacturer's protocol or modifications thereof by those skilled in the art of polynucleotide extraction that may enhance recover or purity of the extracted DNA. Briefly, aliquot ground tissue to a Lysing Matrix E tube on dry ice, add 800µl Buffer SP1 to each sample, homogenize in a bead beater for 35-45sec, incubate on ice for 45-60 sec, centrifuge at ≥14000 rpm for 1min at RT, add 10 microliter RNase A to the lysate, incubate at 65°C for 10min, centrifuge for 1min at RT, add 280µl Buffer SP2 and vortex to mix, incubate the samples on ice for 5min, centrifuge at ≥10,000g for 10min at RT, transfer the supernatant to a homogenizer column in a 2ml collection tube, centrifuge at 10,000g for 2min at RT, transfer the cleared lysate into a 1.5ml microfuge tube, add 1.5 volumes Buffer SP3 to the cleared lysate, vortex immediately to obtain a homogeneous mixture, transfer up to 650µl supernatant to the Hi-Bind column, centrifuge at 10,000g for 1min, repeat, apply 100µl 65°C Elution Buffer to the column, centrifuge at 10,000g for 5min at RT.

Next-generation DNA sequencers, such as the 454-FLX (Roche, Branford, CT), the SOLiD (Applied Biosystems,), and the Genome Analyzer (HiSeq2000, Illumina, San Diego, CA) are used to provide polynucleotide sequence from the DNA and RNA extracted from the plant tissues. Raw sequence data is assembled into contigs. The contig sequence is used to identify trigger molecules that can be applied to the plant to enable regulation of the gene expression. The target DNA sequence isolated from genomic (gDNA) and coding DNA (cDNA) from the various weedy plant species for the HPPD gene and the assembled contigs as set forth in SEQ ID NOs 1-32 and Table 1.

### Example 2. Polynucleotides of the invention related to the trigger molecules

The gene sequences and fragments of Table 1 were divided into 200 polynucleotide (200-mer) lengths with 25 polynucleotide overlapping regions SEQ ID NO:33-596. These polynucleotides are tested to select the most efficacious trigger regions across the length of any target sequence. The trigger polynucleotides are constructed as sense or anti-sense ssDNA or ssRNA, dsRNA, or dsDNA, or dsDNA/RNA hybrids and combined with an organosilicone based transfer agent to provide a polynucleotide preparation. The polynucleotides are combined into sets of two to three polynucleotides per set, using 4-8 nmol of each polynucleotide. Each polynucleotide set is prepared with the transfer agent and applied to a plant or a field of plants in combination with a glyphosate containing herbicide, or followed by a glyphosate treatment one to three days after the polynucleotide application, to determine the effect on the plant's susceptibility to glyphosate. The effect is measured as stunting the growth and/or killing of the plant and is measured 8-14 days after treatment with the polynucleotide set and glyphosate. The most efficacious sets are identified and the individual polynucleotides are tested in the same methods as the sets are and the most efficacious single 200-mer identified. The 200-mer sequence is divided into smaller sequences of 50-70-mer regions with 10-15 polynucleotide overlapping regions and the polynucleotides tested individually. The most efficacious 50-70-mer is further divided into smaller sequences of 25-mer regions with a 12 to 13 polynucleotide overlapping region and tested for efficacy in combination with HPPD inhibitor treatment. By this method it is possible to identify an oligonucleotide or several oligonucleotides that are the most efficacious trigger molecule to effect plant sensitivity to glyphosate or modulation of HPPD gene expression. The modulation of HPPD gene expression is determined by the detection of HPPD siRNA moleclules specific to HPPD gene or by an observation of a reduction in the amount of HPPD RNA transcript produced relative to an untreated plant or by merely observing the anticipated phenotype of the application of the trigger with the glyphosate containing herbicide. Detection of siRNA can be accomplished, for example, using kits such as mirVana (Ambion, Austin TX) and mirPremier (Sigma-Aldrich, St Louis, MO).

The target DNA sequence isolated from genomic (gDNA) and coding DNA (cDNA) from the various weedy plant species for the HPPD gene and the assembled contigs as set forth in SEQ ID NOs 1-32 were divided into polynucleotide fragments as set forth in SEQ ID NO:33-596

The gene sequences and fragments of Table 1 are compared and 21-mers of contiguous polynucleotides are identified that have homology across the various HPPD gene sequences. The purpose is to identify trigger molecules that are useful as herbicidal molecules or in combination with an HPPD inhibitor herbicide across a broad range of weed species. The sequences (SEQ ID NO: 597-1082 represent the 21-mers that are present in the HPPD gene of at least six of the weed species of Table 1. It is contemplated that additional 21-mers can be selected from the sequences of Table 1 that are specific for a single weed species or a few weeds species within a genus or trigger molecules that are at least 18 contiguous nucleotides, at least 19 contiguous nucleotides, at least 20 contiguous nucleotides or at least 21 contiguous nucleotides in length and at least 85 percent identical to an HPPD gene sequence selected from the group consisting of SEQ ID NO:1-32.

By this method it is possible to identify an oligonucleotide or several oligonucleotides that are the most efficacious trigger molecule to effect plant sensitivity to glyphosate or modulation of HPPD gene expression. The modulation of HPPD gene expression is determined by the detection of HPPD siRNA moleclules specific to HPPD gene or by an observation of a reduction in the amount of HPPD RNA transcript produced relative to an untreated plant. Detection of siRNA can be accomplished, for example, using kits such as mirVana (Ambion, Austin TX) and mirPremier (Sigma-Aldrich, St Louis, MO).

The target DNA sequence isolated from genomic (gDNA) and coding DNA (cDNA) from the various weedy plant species for the HPPD gene and the assembled contigs as set forth in SEQ ID NOs 1-32 were divided into fragments as set forth in SEQ ID NO: 597-1082.

### Example 3. Methods used in the invention related to treating plants or plant parts with a topical mixture of the trigger molecules.

Glyphosate-sensitive Palmer amaranth (A. palmeri R-22) plants were grown in the greenhouse (30 / 20 C day/night T; 14 hour photoperiod) in 4 inch corresponding to 10,16 cm square pots containing Sun Gro® Redi-Earth and 3.5 kg/cubic meter Osmocote® 14-14-14 fertilizer. Palmer amaranth plants at 5 to 10 cm in height were pre-treated with a mixture of eight 8 short (21-22mer) single-strand antisense oligo DNA polynucleotides (ssDNAas) targeting HPPD shown in Table 2 as HPPD_OLIGO1-8 (SEQ ID NO: 1083-1090, respectively) at two concentrations, 16 nmol and 80 nmol, formulated in 10 millimolar sodium phosphate buffer (pH 6.8) containing 2% ammonium sulfate and 0.5% Silwet L-77. Plants were treated manually by pipetting 10 µL of polynucleotide solution on four fully expanded mature leaves, for a total of 40 microliters of solution per plant. Twenty-four and forty-eight hours later, the plants were treated with mesotrione (Callisto®, 4 lb ai per gallon corresponding to 1,81437 kg ai per 3,78451 Litre; HPPD inhibitor) at 13 g ai/ha, or atrazine (Aatrex® Nine-0®, 90% w/w ai; Photosystem II inhibitor) at 170 g ai/ha using a track-sprayer equipped with a 9501E nozzle and calibrated to deliver 93 liters of solution per hectare. Crop oil concentrate (COC) at 1 % was added to the herbicide treatments. Four replications of each treatment was conducted. Plant height was determined just before ssDNA treatment and at intervals upto twelve days after herbicide treatments to determine effect of the oligonucleotide and herbicide treatments.

**Table 2. ssDNA HPPD oligonucleotides**

| | | |
|---|---|---|
| HPPD_OLIGO1 | TCCGTAGCTTACATACCGAAG | CTTCGGTATGTAAGCTACGGA |
| HPPD_OLIGO2 | TCCAAGTGAATAGGAGAAACA | TGTTTCTCCTATTCACTTGGA |
| HPPD_OLIGO3 | AGCAGCTTCtgCGTCTTCTAC | GTAGAAGACGcaGAAGCTGCT |
| HPPD_OLIGO4 | ACAGCACGCACGCCAAGACCG | CGGTCTTGGCGTGCGTGCTGT |
| HPPD_OLIGO5 | CGaTGTAAGGAATTTGGtAAA | TTTaCCAAATTCCTTACAtCG |
| HPPD_OLIGO6 | CGAGGGGATTGCAGCAGAAGA | TCTTCTGCTGCAATCCCCTCG |
| HPPD_OLIGO7 | GTAGGAGaATacGGTGAAGTA | TACTTCACCgtATtCTCCTAC |
| HPPD_OLIGO8 | GACCCCAAGaAAATCGTCTGC | GCAGACGATTTtCTTGGGGTC |
| HPPD-T67 | ATTGAGGAGTACGAGAAGACT | AGTCTTCTCGTACTCCTCAAT |
| HPPD-T68 | CTTGAACGTAAACAGGTTCCA | TGGAACCTGTTTACGTTCAAG |

The results of the treatments demonstrated that plants treated only with 16 nmol and 80 nmol of the ssDNA oligonucleotides that targets HPPD showed growth stunting relative to the buffer control of 35 percent and 46 percent, respectively. Four days after treatment the plants treated with ssDNA followed by mesotrione or atrazine at 24 hours showed greater growth stunting than plants treated with the herbicide only. Thus, plants treated with ssDNA at 16 nmol and 80 nmol followed by mesotrione resulted in 77 and 75 percent growth reduction, respectively, relative to the buffer control. Plants treated with ssDNA at 16 nmol and 80 nmol followed by atrazine, resulted in 85 and 83 percent growth reduction, respectively, relative to the buffer control.

Twelve days after treatment the ssDNA at 16 nmol and 80 nmol provided 6 percent and 20 percent reduction in plant growth, the treatments that included mesotrione showed 91 and 89 percent growth reduction, compared to 48 percent control by mesotrione alone (Figure 1). Plants treated with ssDNA at 16 nmol and 80 nmol followed by atrazine at 24 hours showed 50 and 74 percent growth reduction, compared to 29 percent control by atrazine alone. Thus, mesotrione and atrazine efficacy in Palmer amaranth can increase significantly by treating the plants with ssDNA that targets HPPD.

In another similar test, two pools of 5 double stand DNA oligonucleotides were tested, pool 1 contained HPPD-T67 (SEQ ID NO: 1091), HPPD-T68 (SEQ ID NO: 1092) and OLIGO1-3 of Table 2. Pool 2 contained OLIGO 4-8 of Table 2. Plants were treated with 10nmoles of each oligonucleotide and sprayed with Diruon (DCMU (3-(3,4-dichlorophenyl)-1,1-dimethylurea, Bayer) and scored 14 days after treatment for effect on plant growth and development. The results indicate that the oligonucleotides increased the diuron sensitivity of the treated plants upto 22 percent.

### Example 4. A method to control weeds in a field.

A method to control weeds in a field comprises the use of trigger polynucleotides that can modulate the expression of an HPPD gene in one or more target weed plant species. An analysis of HPPD gene sequences from thirteen plant species provided a collection of 21-mer polynucleotides (SEQ ID NO:597-1082) that can be used in compositions to affect the growth or develop or sensitivity to glyphosate herbicide to control multiple weed species in a field. A composition containing 1 or 2 or 3 or 4 or more of the polynucleotides (SEQ ID NO:597-1082) would enable broad activity of the composition against the multiple weed species that occur in a field environment.

The method includes creating a composition that comprises components that include at least one polynucleotide of (SEQ ID NO:597-1082) or any other effective gene expression modulating polynucleotide essentially identical or essentially complementary to SEQ ID NO:1-32 or fragment thereof, a transfer agent that mobilizes the polynucleotide into a plant cell and a HPPD inhibiting herbicide and optionally a polynucleotide that modulates the expression of an essential gene and optionally a herbicide that has a different mode of action relative to an HPPD inhibitor. The polynucleotide of the composition includes a dsRNA, ssDNA or dsDNA or a combination thereof. A composition containing a polynucleotide can have a use rate of about 1 to 30 grams or more per acre depending on the size of the polynucleotide and the number of polynucleotides in the composition. The composition may include one or more additional herbicides as needed to provide effective multi-species weed control. A field of crop plants in need of weed plant control is treated by spray application of the composition. The composition can be provided as a tank mix, a sequential treatment of components (generally the polynucleotide followed by the herbicide), a simultaneous treatment or mixing of one or more of the components of the composition from separate containers. Treatment of the field can occur as often as needed to provide weed control and the components of the composition can be adjusted to target specific weed species or weed families.

**Table 1. HPPD gene polynucleotide sequences**

| SEQ ID NO | SPECIES | TYPE | LENGTH |
|---|---|---|---|
| 1 | Abutilon theophrasti | cDNAContig | 713 |
| 2 | Amaranthus graecizans | cDNAContig | 1234 |
| 3 | *Amaranthus* hybridus | cDNAContig | 547 |
| 4 | *Amaranthus* palmeri | cDNAContig | 1265 |
| 5 | *Amaranthus* palmeri | gDNAContig | 3245 |
| 6 | *Amaranthus* palmeri | gDNAContig | 3416 |
| 7 | Amaranthus palmeri | gDNAContig | 3818 |
| 8 | Amaranthus rudis | cDNAContig | 775 |
| 9 | Amaranthus rudis | cDNAContig | 1204 |
| 10 | Amaranthus rudis | gDNAContig | 511 |
| 11 | Amaranthus rudis | gDNAContig | 770 |
| 12 | Amaranthus rudis | gDNAContig | 1412 |
| 13 | Amaranthus thunbergii | cDNAContig | 707 |
| 14 | Amaranthus viridis | cDNAContig | 1267 |
| 15 | Ambrosia trifida | cDNAContig | 637 |
| 16 | Ambrosia trifida | cDNAContig | 718 |
| 17 | Ambrosia trifida | gDNAContig | 54 |
| 18 | Ambrosia trifida | gDNAContig | 719 |
| 19 | Ambrosia trifida | gDNAContig | 845 |
| 20 | Ambrosia trifida | gDNAContig | 1024 |
| 21 | Conyza canadensis | cDNAContig | 1610 |
| 22 | Conyza canadensis | gDNAContig | 4963 |
| 23 | Conyza canadensis | gDNAContig | 5610 |
| 24 | Euphorbia heterophylla | cDNAContig | 386 |
| 25 | Euphorbia heterophylla | gDNAContig | 2639 |
| 26 | Euphorbia heterophylla | gDNAContig | 6569 |
| 27 | Xanthium strumarium | cDNAContig | 1567 |
| 28 | Digitaria sanguinalis | cDNAContig | 892 |
| 29 | Digitaria sanguinalis | cDNAContig | 975 |
| 30 | Kochia scoparia | cDNAContig | 1266 |
| 31 | Lolium multiflorum | cDNAContig | 783 |
| 32 | Lolium multiflorum | cDNAContig | 1041 |

## Claims

1. A method of plant control comprising: topically applying to a plant surface a composition comprising a double-stranded RNA (dsRNA) polynucleotide and a transfer agent, wherein said dsRNA polynucleotide is identical or complementary to an RNA sequence of a 4-hydroxyphenyl-pyruvate-dioxygenase (HPPD) gene sequence selected from the group consisting of SEQ ID NOs:21, 27, 28, 33-36, 39-42, 87-92, 103,104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide, and whereby said plant's growth, development, or reproductive ability is reduced or said plant is more sensitive to an HPPD inhibitor herbicide, relative to an untreated plant.

2. The method as claimed in claim 1, wherein
(i) said plant is selected from the group consisting of *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum* and *Xanthium strumarium;* or
(ii) said composition further comprises said HPPD inhibitor herbicide, preferably said composition further comprises one or more herbicides different from said HPPD inhibitor herbicide.

3. The method as claimed in claim 1, wherein said dsRNA polynucleotide is at least 19 contiguous nucleotides, at least 20 contiguous nucleotides, or at least 21 contiguous nucleotides in length, preferably said composition comprises any combination of two or more of said dsRNA polynucleotides.

4. A composition comprising a dsRNA polynucleotide and a transfer agent, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone surfactant composition or an organosilicone compound contained therein and conditions the surface of a plant for permeation by said dsRNA polynucleotide, and whereby said plant treated with said composition has its growth, development, or reproductive ability suppressed or delayed or said plant is more sensitive to an HPPD inhibitor herbicide as a result of said dsRNA polynucleotide containing composition, relative to an untreated plant.

5. The composition of claim 4, wherein
(i) said dsRNA polynucleotide is at least 19 contiguous nucleotides, at least 20 contiguous nucleotides, or at least 21 contiguous nucleotides in length; or
(ii) said composition further comprises an HPPD inhibitor herbicide, preferably said HPPD inhibitor herbicide is selected from the group consisting of mesotrione, tefuryltrione, tembotrione, sulcotrione; isoxachlortole, pyrasulfotole, isoxaflutole, benzofenap, pyrazolynate, topramezone and pyrazoxyfen, or said composition further comprises a co-herbicide selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

6. A method of reducing expression of an HPPD gene in a plant comprising: topically applying to a plant surface a composition comprising a dsRNA polynucleotide and a transfer agent, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide, and whereby said expression of said HPPD gene is reduced relative to an untreated plant.

7. The method as claimed in claim 6, wherein
(i) said dsRNA polynucleotide is at least 19 contiguous nucleotides, at least 20 contiguous nucleotides, or at least 21 contiguous nucleotides in length.

8. A method of identifying dsRNA polynucleotides useful in modulating HPPD gene expression when topically applied to a plant surface comprising: a) providing a plurality of dsRNA polynucleotides that comprise a region identical or complementary to at least 18 contiguous nucleotides of an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082; b) topically applying to said plant surface a composition comprising one or more of said dsRNA polynucleotides and a transfer agent; c) analyzing said plant, or a plant extract thereof, for modulation of HPPD gene expression, wherein said transfer agent is an organosilicone surfactant composition or an organosilicone compound contained therein and conditions the surface of said plant for permeation by said dsRNA polynucleotide, and d) said plant treated with said composition has its growth, development, or reproductive ability, suppressed or delayed or said plant is more sensitive to an HPPD inhibitor herbicide as a result of said composition relative to an untreated plant.

9. The method as claimed in claim 8, wherein
(i) said plant is selected from the group consisting of *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum,* and *Xanthium strumarium;* or
(ii) said HPPD gene expression is reduced relative to a plant not treated with said composition.

10. An agricultural chemical composition comprising an admixture of a dsRNA polynucleotide, a transfer agent, an HPPD inhibitor herbicide, and a co-herbicide, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone composition or an organosilicone compound contained therein and conditions a surface of a plant for permeation by said dsRNA polynucleotide, and whereby said plant treated with said composition has its growth, development, or reproductive ability suppressed or delayed or said plant is more sensitive to said HPPD inhibitor herbicide as a result of said dsRNA polynucleotide containing composition, relative to an untreated plant.

11. The agricultural chemical composition of claim 10, wherein said co-herbicide is selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

12. An agricultural chemical composition comprising an admixture of a dsRNA polynucleotide, a transfer agent, an HPPD inhibitor herbicide, and a pesticide, wherein said dsRNA polynucleotide is identical or complementary to an RNA sequence of an HPPD gene sequence selected from the group consisting of SEQ ID NOs:21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092, and an at least 18 contiguous nucleotides polynucleotide fragment thereof, wherein said transfer agent is an organosilicone composition or an organosilicone compound contained therein and conditions a surface of a plant for permeation by said dsRNA polynucleotide, and whereby said plant treated with said composition has its growth, development, or reproductive ability suppressed or delayed or said plant is more sensitive to said HPPD inhibitor herbicide as a result of said dsRNA polynucleotide containing composition, relative to an untreated plant.

13. The agricultural chemical composition of claim 12, wherein said pesticide is selected from the group consisting of insecticides, fungicides, nematicides, bactericides, acaricides, growth regulators, chemosterilants, semiochemicals, repellents, attractants, pheromones, feeding stimulants, and biopesticides.

## Patentansprüche

1. Verfahren zur Kontrolle von Pflanzen, Schritte umfassend, bei denen man: eine Zusammensetzung, die ein doppelsträngiges RNA (dsRNA)-Polynukleotid und ein Übertragungsmittel umfasst, topisch auf eine Oberfläche einer Pflanze aufbringt, wobei das dsRNA-Polynukleotid identisch oder komplementär zu einer RNA-Sequenz einer 4-Hydroxyphenyl-Pyruvat-Dioxygenase (HPPD) -Gensequenz ist, die der Gruppe bestehend aus SEQ ID NOs: 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092 und einem mindestens 18 aufeinanderfolgenden Nukleotide langem Polynukleotid-Fragment davon augewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, wodurch das Wachstum, die Entwicklung oder die Reproduktionsfähigkeit der Pflanze verringert wird oder die Pflanze empfindlicher auf ein HPPD-Inhibitor-Herbizid im Vergleich zu einer unbehandelten Pflanze reagiert.

2. Verfahren gemäß Anspruch 1, bei dem
(i) die Pflanze aus der Gruppe bestehend aus *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum* und *Xanthium strumarium;* oder
(ii) die Zusammensetzung ferner das HPPD-Inhibitor-Herbizid umfasst, vorzugsweise ferner ein oder mehrere Herbizide, die sich von dem HPPD-Inhibitor-Herbizid unterscheiden.

3. Verfahren gemäß Anspruch 1, bei dem das dsRNA-Polynukleotid mindestens 19 aufeinanderfolgende Nukleotide, mindestens 20 aufeinanderfolgende Nukleotide oder mindestens 21 aufeinanderfolgende Nukleotide lang ist, vorzugsweise umfasst die Zusammensetzung eine beliebige Kombination von zwei oder mehren der dsRNA-Polynukleotide.

4. Zusammensetzung, umfassend ein dsRNA-Polynukleotid und ein Übertragungsmittel, wobei das dsRNA-Polynukleotid einer RNA-Sequenz aufweist, die identisch oder komplementär zu einer HPPD-Gensequenz ist, die aus der Gruppe bestehend aus SEQ ID NOs: 21, 27, 28, 33-36, 39-42, 87-92, 103, 104, 163-166, 169, 170, 201-204, 225, 226, 237-242, 313-322, 369-378, 381-384, 407-424, 435- 442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092 und ein einem Polynukleotid-Fragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, wodurch das Wachstum, die Entwicklung oder die Reproduktionsfähigkeit der Pflanze verringert wird oder die Pflanze empfindlicher auf ein HPPD-Inhibitor-Herbizid im Vergleich zu einer unbehandelten Pflanze reagiert.

5. Zusammensetzung gemäß Anspruch 4, bei der
(i) das dsRNA-Polynukleotid mindestens 19 aufeinanderfolgende Nukleotide, mindestens 20 aufeinanderfolgende Nukleotide oder mindestens 21 aufeinanderfolgende Nukleotide lang ist; oder
(ii) die Zusammensetzung ferner ein HPPD-Inhibitor-Herbizid umfasst, vorzugsweise ein HPPD-Inhibitor-Herbizid, das aus der Gruppe bestehend aus Mesotrion, Tefuryltrion, Tembotrion, Sulcotrion; Isoxachlortol, Pyrasulfotol, Isoxaflutol, Benzofenap, Pyrazolynat, Topramezone und Pyrazoxyfen ausgewählt wurde oder die Zusammensetzung ferner ein Co-Herbizid umfasst, das aus der Gruppe bestehend aus Amidherbiziden, Arsenherbiziden, Benzothiazol-herbiziden, Benzoylcyclohexandion-Herbizide, Benzofuranylalkylsulfonat-Herbiziden, Cyclohexenoxim-Herbiziden, Cyclopropylisoxazol-Herbiziden, Dicarboximid-Herbiziden, Dinitroanilin-Herbiziden, Dinitrophenol-Herbiziden, Diphenylether-Herbiziden, Dithiocarbamat-Herbiziden, Glycin-Herbiziden, halogenierten aliphatischen Herbiziden, Imidazolinon-Herbiziden, anorganischen Herbiziden, Nitril-Herbiziden, phosphororganischen Herbiziden, Oxadiazolon-Herbiziden, Oxazol-Herbiziden, Phenoxyherbiziden, Phenylen-diaminherbiziden, Pyrazolherbiziden, Pyridazinherbiziden, Pyridazinonherbiziden, Pyridinherbiziden, Pyrimidindiaminherbiziden, Pyrimidinyloxybenzylaminherbiziden, quaternären Ammoniumherbiziden, Thiocarbamat-Herbiziden, Thiocarbonat-Herbiziden, Thioharnstoff-Herbiziden, Triazin-Herbiziden, Triazinon-Herbiziden, Triazolon-Herbiziden, Triazolopyrimidin-Herbiziden, Uracil-Herbiziden und Harnstoff-Herbiziden ausgewählt wurde.

6. Verfahren zur Verringerung der Expression eines HPPD-Gens in einer Pflanze, Schritte umfassend, bei denen man: eine Zusammensetzung, die ein doppelsträngiges RNA (dsRNA)-Polynukleotid und ein Übertragungsmittel umfasst, topisch auf eine Oberfläche einer Pflanze aufbringt, wobei das dsRNA-Polynukleotid identisch oder komplementär zu einer RNA-Sequenz einer 4-Hydroxyphenyl-Pyruvat-Dioxygenase (HPPD) -Gensequenz ist, die der Gruppe bestehend aus SEQ ID NOs: 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092 und einem mindestens 18 aufeinanderfolgenden Nukleotide langem Polynukleotid-Fragment davon augewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, wodurch die Expression eines HPPD-Gens in einer Pflanze im Vergleich zu einer unbehandelten Pflanze verringert wird.

7. Verfahren gemäß Anspruch 6, bei dem
(i) das dsRNA-Polynukleotid mindestens 19 aufeinanderfolgende Nukleotide, mindestens 20 aufeinanderfolgende Nukleotide oder mindestens 21 aufeinanderfolgende Nukleotide lang ist.

8. Verfahren zur Identifizierung von dsRNA-Polynukleotiden, die zur Modulation der HPPD-Genexpression nützlich sind, wenn sie topisch auf eine Pflanzenoberfläche aufgebracht werden, Schritte umfassend bei denen man: a) eine Vielzahl an dsRNA-Polynukleotiden bereitstellt, die einen Bereich aus mindestens 18 aufeinanderfolgenden Nukleotiden umfassen, der identisch oder komplementär zu einer RNA-Sequenz einer HPPD-Gensequenz ist, die aus der Gruppe bestehend aus SEQ ID NOs: 21, 27, 28, 33-36, 39-42, 87-92, 103, 104, 163-166, 169, 170, 201-204, 225, 226 237-242, 313-322, 369-378, 381-384, 407-424, 435-442, 581-596, 643, 648, 652, 665-667, 682, 683, 688, 690, 695, 711 737-739, 751, 758-760, 769, 774, 776, 777, 779, 787, 792, 793, 804, 808, 823, 828, 837, 839, 842, 848, 849, 851, 887, 909 911, 913, 915, 918, 919, 926, 928, 930, 935-937, 940, 947, 954, 958, 963, 972, 981, 984, 988, 991, 992, 998-1000, 1010, 1012 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092 ausgewählt wurde; b) eine Zusammensetzung auf die Pflanzenoberfläche topisch aufträgt, die eines oder mehrere der dsRNA-Polynukleotide und ein Übertragungsmittel umfasst; c) die Pflanze oder einen Pflanzenextrakt davon auf Modulation der HPPD-Genexpression hin analysiert, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und d) die Wachstums-, Entwicklungs- oder Fortpflanzungsfähigkeit der mit der Zusammensetzung behandelten Pflanze unterdrückt oder verzögert wird, oder die Pflanze aufgrund der Zusammensetzung im Vergleich zu einer unbehandelten Pflanze empfindlicher auf einem HPPD-Inhibitor-Herbizid reagiert.

9. Verfahren gemäß Anspruch 8, wobei
(i) Die Pflanze ist ausgewählt aus der Gruppe bestehend aus *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum* und *Xanthium strumarium*; oder
(ii) die HPPD-Genexpression im Vergleich zu einer Pflanze, die nicht mit der Zusammensetzung behandelt wurde, verringert ist.

10. Agrochemische Zusammensetzung, umfassend eine Mischung eines dsRNA-Polynukleotids, eines Übertragungsmittels, eines HPPD-Inhibitor-Herbizids und eines Co-Herbizids, wobei das dsRNA-Polynukleotid identisch oder komplementär zu einer RNA-Sequenz einer HPPD-Gensequenz ist, die aus der Gruppe bestehend aus SEQ ID NOs: 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758- 760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092 und einem Polynukleotid-Fragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und das Wachstum, die Entwicklung oder die Fortpflanzungsfähigkeit der mit der Zusammensetzung behandelten Pflanze als Ergebnis der Zusammensetzung des dsRNA-Polynukleotids unterdrückt oder verzögert ist oder die Pflanze im Vergleich zu einer unbehandelten Pflanze empfindlicher auf das HPPD-Inhibitor-Herbizid reagiert.

11. Agrochemische Zusammensetzung gemäß Anspruch 10, wobei das Co-Herbizid aus der Gruppe bestehend aus Amidherbiziden, Arsenherbiziden, Benzothiazol-herbiziden, Benzoylcyclohexandion-Herbizide, Benzofuranylalkylsulfonat-Herbiziden, Cyclohexenoxim-Herbiziden, Cyclopropylisoxazol-Herbiziden, Dicarboximid-Herbiziden, Dinitroanilin-Herbiziden, Dinitrophenol-Herbiziden, Diphenylether-Herbiziden, Dithiocarbamat-Herbiziden, Glycin-Herbiziden, halogenierten aliphatischen Herbiziden, Imidazolinon-Herbiziden, anorganischen Herbiziden, Nitril-Herbiziden, phosphororganischen Herbiziden, Oxadiazolon-Herbiziden, Oxazol-Herbiziden, Phenoxyherbiziden, Phenylen-diaminherbiziden, Pyrazolherbiziden, Pyridazinherbiziden, Pyridazinonherbiziden, Pyridinherbiziden, Pyrimidindiaminherbiziden, Pyrimidinyloxybenzylaminherbiziden, quaternären Ammoniumherbiziden, Thiocarbamat-Herbiziden, Thiocarbonat-Herbiziden, Thioharnstoff-Herbiziden, Triazin-Herbiziden, Triazinon-Herbiziden, Triazolon-Herbiziden, Triazolopyrimidin-Herbiziden, Uracil-Herbiziden und Harnstoff-Herbiziden ausgewählt wurde.

12. Agrochemische Zusammensetzung, umfassend eine Mischung einem dsRNA-Polynukleotids, einem Übertragungsmittel, einem HPPD-Inhibitor-Herbizid und einem Pestizid, wobei das dsRNA-Polynukleotid identisch oder komplementär zu einer RNA-Sequenz einer HPPD-Gensequenz ist, die aus der Gruppe bestehend aus SEQ ID NOs: 21, 27, 28, 33-36, 39-42, 87-92, 103, 104, 163-166, 169, 170, 201-204, 225, 226, 237-242, 313-322, 369- 378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, 1084-1092 und einem Polynukleotid-Fragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und das Wachstum, die Entwicklung oder die Fortpflanzungsfähigkeit der mit der Zusammensetzung behandelten Pflanze als Ergebnis der Zusammensetzung des dsRNA-Polynukleotids unterdrückt oder verzögert ist oder die Pflanze im Vergleich zu einer unbehandelten Pflanze empfindlicher auf das HPPD-Inhibitor-Herbizid reagiert.

13. Agrochemische Zusammensetzung gemäß Anspruch 12, wobei das Pestizid aus der Gruppe bestehend aus Insektiziden, Fungiziden, Nematiziden, Bakteriziden, Akariziden, Wachstumsregulatoren, Chemosterilantien, Semiochemikalien, Repellentien, Lockstoffen, Pheromonen, Fütterungsstimulanzien und Biopestiziden ausgewählt wurde.

## Revendications

1. Procédé de lutte contre des végétaux, qui comporte l'application locale, à la surface d'un végétal, d'une composition comprenant un polynucléotide d'ARN double brin (ARNdb) et un agent de transfert, étant entendu que ledit polynucléotide d'ARNdb est identique ou complémentaire à une séquence d'ARN appartenant à une séquence de gène de 4-hydroxyphényl-pyruvate dioxygénase (HPPD) choisie dans l'ensemble constitué par les séquences de numéros SEQ ID NO : 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082 et 1084-1092, et un fragment polynucléotidique de celles-ci constitué d'au moins 18 nucléotides contigus, étant entendu que ledit agent de transfert est une composition de tensioactif du type organosilicone ou un composé du type organosilicone contenu en son sein, et prépare la surface dudit végétal à la perméation dudit polynucléotide d'ARNdb, moyennant quoi la croissance, le développement ou la capacité de reproduction dudit végétal est réduit(e), ou bien ledit végétal est plus sensible à un herbicide inhibiteur de HPPD qu'un végétal non traité.

2. Procédé selon la revendication 1, dans lequel :
(i) ledit végétal est choisi dans le groupe constitué par les végétaux *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum* et *Xanthium strumarium,*
(ii) ou ladite composition comprend par ailleurs ledit herbicide inhibiteur de HPPD, et de préférence ladite composition comprend en outre un ou plusieurs herbicide(s) différent(s) dudit herbicide inhibiteur de HPPD.

3. Procédé selon la revendication 1, dans lequel ledit polynucléotide d'ARNdb est d'une longueur d'au moins 19 nucléotides contigus, d'au moins 20 nucléotides contigus ou d'au moins 21 nucléotides contigus, et de préférence ladite composition comprend n'importe quelle combinaison de deux ou plus desdits polynucléotides d'ARNdb.

4. Composition comprenant un polynucléotide d'ARNdb et un agent de transfert, dans laquelle ledit polynucléotide d'ARNdb est identique ou complémentaire à une séquence d'ARN appartenant à une séquence de gène de HPPD choisie dans l'ensemble constitué par les séquences de numéros SEQ ID NO : 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082 et 1084-1092, et un fragment polynucléotidique de celles-ci constitué d'au moins 18 nucléotides contigus, étant entendu que ledit agent de transfert est une composition de tensioactif du type organosilicone ou un composé du type organosilicone contenu en son sein, et prépare la surface d'un végétal à la perméation dudit polynucléotide d'ARNdb, moyennant quoi ledit végétal traité avec ladite composition voit sa croissance, son développement ou sa capacité de reproduction supprimé(e) ou retardé(e), ou bien ledit végétal, sous l'effet de ladite composition contenant ledit polynucléotide d'ARNdb, est plus sensible à un herbicide inhibiteur de HPPD qu'un végétal non traité.

5. Composition selon la revendication 4, dans laquelle :
(i) ledit polynucléotide d'ARNdb est d'une longueur d'au moins 19 nucléotides contigus, d'au moins 20 nucléotides contigus ou d'au moins 21 nucléotides contigus,
(ii) ou ladite composition comprend en outre un herbicide inhibiteur de HPPD, et de préférence ledit herbicide inhibiteur de HPPD est choisi dans l'ensemble constitué par les herbicides mésotrione, téfuryltrione, tembotrione, sulcotrione, isoxachlortole, pyrasulfotole, isoxaflutole, benzofénap, pyrazolynate, topramézone et pyrazoxyfène, ou ladite composition comprend en outre un co-herbicide choisi dans l'ensemble constitué par les herbicides de type amide, herbicides arsenicaux, herbicides de type benzothiazole, herbicides de type benzoyl-cyclohexanedione, herbicides de type alkylsulfonate de benzofuranyle, herbicides de type cyclohexène-oxime, herbicides de type cyclopropyl-isoxazole, herbicides de type dicarboximide, herbicides de type dinitroaniline, herbicides de type dinitrophénol, herbicides de type éther diphénylique, herbicides de type dithiocarbamate, herbicides de type glycine, herbicides aliphatiques halogénés, herbicides de type imidazolinone, herbicides minéraux, herbicides de type nitrile, herbicides organophosphorés, herbicides de type oxadiazolone, herbicides de type oxazole, herbicides phénoxyliques, herbicides de type phénylène-diamine, herbicides de type pyrazole, herbicides de type pyridazine, herbicides de type pyridazinone, herbicides de type pyridine, herbicides de type pyrimidine-diamine, herbicides de type pyrimidinyloxy-benzylamine, herbicides de type ammonium quaternaire, herbicides de type thiocarbamate, herbicides de type thiocarbonate, herbicides de type thiourée, herbicides de type triazine, herbicides de type triazinone, herbicides de type triazole, herbicides de type triazolone, herbicides de type triazolo-pyrimidine, herbicides de type uracile et herbicides de type urée.

6. Procédé de réduction de l'expression d'un gène de HPPD dans un végétal, qui comporte l'application locale, à la surface d'un végétal, d'une composition comprenant un polynucléotide d'ARNdb et un agent de transfert, étant entendu que ledit polynucléotide d'ARNdb est identique ou complémentaire à une séquence d'ARN appartenant à une séquence de gène de HPPD choisie dans l'ensemble constitué par les séquences de numéros SEQ ID NO : 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082 et 1084-1092, et un fragment polynucléotidique de celles-ci constitué d'au moins 18 nucléotides contigus, étant entendu que ledit agent de transfert est une composition de tensioactif du type organosilicone ou un composé du type organosilicone contenu en son sein, et prépare la surface dudit végétal à la perméation dudit polynucléotide d'ARNdb, moyennant quoi ladite expression dudit gène de HPPD est moins importante que dans un végétal non traité.

7. Procédé selon la revendication 6, dans lequel :
(i) ledit polynucléotide d'ARNdb est d'une longueur d'au moins 19 nucléotides contigus, d'au moins 20 nucléotides contigus ou d'au moins 21 nucléotides contigus.

8. Procédé d'identification de polynucléotides d'ARNdb utilisables pour moduler l'expression du gène de HPPD lorsqu'ils sont appliqués localement à la surface d'un végétal, lequel procédé comprend le fait de :
a) se procurer plusieurs polynucléotides d'ARNdb qui comportent une région identique ou complémentaire à au moins 18 nucléotides contigus d'une séquence d'ARN appartenant à une séquence de gène de HPPD choisie dans l'ensemble constitué par les séquences de numéros SEQ ID NO : 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082, et 1084-1092
b) appliquer localement, à la surface dudit végétal, une composition comprenant un ou plusieurs desdits polynucléotides d'ARNdb et un agent de transfert,
c) analyser la modulation de l'expression du gène de HPPD dans ledit végétal ou un extrait végétal de celui-ci, ledit agent de transfert étant une composition de tensioactif du type organosilicone ou un composé du type organosilicone contenu en son sein, et préparant la surface dudit végétal à la perméation dudit polynucléotide d'ARNdb,
d) et vérifier si ledit végétal traité avec ladite composition voit sa croissance, son développement ou sa capacité de reproduction supprimé(e) ou retardé(e), ou bien si ledit végétal, sous l'effet de ladite composition, est plus sensible à un herbicide inhibiteur de HPPD qu'un végétal non traité.

9. Procédé selon la revendication 8, dans lequel :
(i) ledit végétal est choisi dans le groupe constitué par les végétaux *Amaranthus palmeri, Amaranthus rudis, Amaranthus thunbergii, Amaranthus graecizans, Amaranthus hybridus, Amaranthus viridis, Ambrosia trifida, Kochia scoparia, Abutilon theophrasti, Conyza canadensis, Digitaria sanguinalis, Euphorbia heterophylla, Lolium multiflorum* et *Xanthium strumarium,*
(ii) ou ladite expression du gène de HPPD est moins importante que dans un végétal non traité avec ladite composition.

10. Composition agrochimique comprenant un mélange constitué par un polynucléotide d'ARNdb, un agent de transfert, un herbicide inhibiteur de HPPD et un co-herbicide, dans laquelle ledit polynucléotide d'ARNdb est identique ou complémentaire à une séquence d'ARN appartenant à une séquence de gène de HPPD choisie dans l'ensemble constitué par les séquences de numéros SEQ ID NO : 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082 et 1084-1092, et un fragment polynucléotidique de celles-ci constitué d'au moins 18 nucléotides contigus, étant entendu que ledit agent de transfert est une composition à base d'organosilicone ou un composé du type organosilicone contenu en son sein, et prépare la surface d'un végétal à la perméation dudit polynucléotide d'ARNdb, moyennant quoi ledit végétal traité avec ladite composition voit sa croissance, son développement ou sa capacité de reproduction supprimé(e) ou retardé(e), ou bien ledit végétal, sous l'effet de ladite composition contenant ledit polynucléotide d'ARNdb, est plus sensible audit herbicide inhibiteur de HPPD qu'un végétal non traité.

11. Composition agrochimique selon la revendication 10, dans laquelle ledit co-herbicide est choisi dans l'ensemble constitué par les herbicides de type amide, herbicides arsenicaux, herbicides de type benzothiazole, herbicides de type benzoyl-cyclohexanedione, herbicides de type alkylsulfonate de benzofuranyle, herbicides de type cyclohexène-oxime, herbicides de type cyclopropyl-isoxazole, herbicides de type dicarboximide, herbicides de type dinitroaniline, herbicides de type dinitrophénol, herbicides de type éther diphénylique, herbicides de type dithiocarbamate, herbicides de type glycine, herbicides aliphatiques halogénés, herbicides de type imidazolinone, herbicides minéraux, herbicides de type nitrile, herbicides organophosphorés, herbicides de type oxadiazolone, herbicides de type oxazole, herbicides phénoxyliques, herbicides de type phénylène-diamine, herbicides de type pyrazole, herbicides de type pyridazine, herbicides de type pyridazinone, herbicides de type pyridine, herbicides de type pyrimidine-diamine, herbicides de type pyrimidinyloxy-benzylamine, herbicides de type ammonium quaternaire, herbicides de type thiocarbamate, herbicides de type thiocarbonate, herbicides de type thiourée, herbicides de type triazine, herbicides de type triazinone, herbicides de type triazole, herbicides de type triazolone, herbicides de type triazolo-pyrimidine, herbicides de type uracile et herbicides de type urée.

12. Composition agrochimique comprenant un mélange constitué par un polynucléotide d'ARNdb, un agent de transfert, un herbicide inhibiteur de HPPD et un pesticide, dans laquelle ledit polynucléotide d'ARNdb est identique ou complémentaire à une séquence d'ARN appartenant à une séquence de gène de HPPD choisie dans l'ensemble constitué par les séquences de numéros SEQ ID NO : 21, 27, 28, 33-36, 39-42, 87-92, 103, 104, 163-166, 169, 170, 201-204, 225, 226, 237-242, 313-322, 369-378, 381-384, 407-424, 435-442, 581-596, 643, 648, 652, 665-667, 682, 683, 688, 690, 695, 711, 737-739, 751, 758-760, 769, 774, 776, 777, 779, 787, 792, 793, 804, 808, 823, 828, 837, 839, 842, 848, 849, 851, 887, 909, 911, 913, 915, 918, 919, 926, 928, 930, 935-937, 940, 947, 954, 958, 963, 972, 981, 984, 988, 991, 992, 998-1000, 1010, 1012, 1013, 1015, 1017, 1019, 1020, 1023, 1030, 1034, 1035, 1041, 1045, 1051, 1053-1056, 1058, 1059, 1062, 1063, 1065-1070, 1073-1082 et 1084-1092, et un fragment polynucléotidique de celles-ci constitué d'au moins 18 nucléotides contigus, étant entendu que ledit agent de transfert est une composition à base d'organosilicone ou un composé du type organosilicone contenu en son sein, et prépare la surface d'un végétal à la perméation dudit polynucléotide d'ARNdb, moyennant quoi ledit végétal traité avec ladite composition voit sa croissance, son développement ou sa capacité de reproduction supprimé(e) ou retardé(e), ou bien ledit végétal, sous l'effet de ladite composition contenant ledit polynucléotide d'ARNdb, est plus sensible audit herbicide inhibiteur de HPPD qu'un végétal non traité.

13. Composition agrochimique selon la revendication 12, dans laquelle ledit pesticide est choisi dans le groupe constitué par les insecticides, fongicides, nématocides, bactéricides, acaricides, régulateurs de croissance, chimiostérilisants, substances sémiochimiques, répulsifs, attractifs, phéromones, phagostimulants et biopesticides.


<110> Ader, Daniel Finnessy, John J Kapoor, Mahak Li, Zhaolong Masucci, James D Shah, Ronak Hasmukh Tao, Nengbing Taylor, Jennifer Chou Wang, Dafu Yang, Heping
<120> Methods and Compositions for Weed Control
<130> 40-21(58636)
<150> 61/534066
   <151> 2011-09-13
<160> 1092
<210> 1
   <211> 713
   <212> DNA
   <213> Abutilon theophrasti
<400> 1
   tccataatcc aacgcagaca tttcagagat tcatccccga agaaatcaaa aatccctcaa    60
   caaaatgatt cagatatgct taaacaaaat atgaattgag cacaatatca tatttgacat   120
   ttatgttgaa gtagcaaagg ttcgtatagt ttatgtatgt gcaattaaaa ctaacagtaa   180
   cggaattcat tctgacactc cctatatgct gctagcaact acctcgatct ctcaagcctc   240
   gacaacggtg gtggattctg tagattgttt ggcttcaaga gacttctcgt attcttcaat   300
   ggatttgaag agctcggaaa agttgccttt cccgaaacct ccgcatcctc ctttctggta   360
   ttgctttcct tcttcatcct tcaccatgca ccctaatctc tgtattattt ctatgaatat   420
   cgtaggccta tctccaatgg gcttagtgaa aatctgaagc agagtgcctt gatcatctct   480
   gtcaaccaaa atccccaact cttcacactc cttaatctgc tcatcgctca aaatgtcccc   540
   tgccctttgc ttcaatttct tatagtaagt gggcggcggc gatggcatga actcgaaacc   600
   acccaccaaa cttctcttcc tcatttctct cagcgttctg aatatatctt cactcaccag   660
   agccaaatgt tgaaccccgg caccttcgtt gtgttctaaa tacgtttgga ttt          713
<210> 2
   <211> 1234
   <212> DNA
   <213> Amaranthus graecizans
<400> 2
   cttcttcccc gtctgagttt ttattacttc actttctctc tcatcatcta acatgggaac    60
   tttgaaaccc gaaactcaac ccgactccga attcaaactc gtgggttact ccaacttcat   120
   tcgggttaac cccaaatctg accgttttac tgttaagcgt ttccatcata tagagttctg  180
   gtgtggcgat gcaaccaatg ttagcagacg cttttcttgg ggtcttggaa tgcctaccgt  240
   tgctaaatct gacctttcta ctggaaactc tgttcacgct tcttttcttc ttcgttccgg  300
   tgacctttct ttcctcttta cttcacctta ctctcctacc atgtccatcc cttcttctgc  360
   tgcaatcccc tcgtttgatt tcaatcattt taccaaattt gttacatcgc acggtcttgg  420
   cgtgcgtgct gttgccgtcg aagtagaaga cgcagaagct gcttttaata tcagcgtttc  480
   gcacggggct atcccctgtg tttctcctat tcacttggaa aacggtgtcg ttttatctga  540
   ggttcattta tatggggatg ttgtgcttcg ttatgtaagc tacggaaatg aatgtgggga  600
   tgtgtttttt cttcctgggt ttgagcaaat gcccgaggaa tcctcgtttc gaggacttga  660
   tttcggcctt cgaaggttgg atcatgctgt agggaatgtc cctgagttgg ctcctgcaat  720
   tgcttatttg aagaagttta ctgggtttca tgagtttgct gagtttacag ctgaagatgt  780
   tggaacgagt gaaagtgggt tgaattcagc cgtattggca aataatgatg aaatggtgtt  840
   gtttccaatg aatgaacctg tgtatgggac aaaaaggaag agtcaaattc aaacttattt  900
   ggagcataat gaaggagctg gtgtacaaca tttggctttg atgagtgaag atatattttg  960
   gactttaagg gagatgagga agagaagtgg tcttggtggg tttgagttta tgccgtcgcc 1020
   gcctccgact tattaccgga atttgaggaa cagagctgct gatgtattga gtgaggagca 1080
   gatgaaggag tgtgaagagt tggggatttt ggtggataaa gatgatcagg gtactttgct 1140
   tcaaatcttc actaagccta ttggtgacag gccaaccata tttatcgaga ttatacaaag 1200
   aatcggttgc atgatgaaag atgaagacgg caag                              1234
<210> 3
   <211> 547
   <212> DNA
   <213> Amaranthus hybridus
<400> 3
   tcttcctggg tttgaggaaa tgccggagga atcgtcgttt cgaggacttg atttcggcct   60
   tcgaaggttg gatcatgctg tagggaatgt tcccgagttg gctcctgcaa ttgcttattt  120
   gaagaagttt actgggtttc atgagtttgc tgagtttaca gctgaagatg ttgggacgag  180
   tgaaagtggg ttgaattcag ccgtattggc aaataatgat gaaatggtgt tgtttccaat  240
   gaatgaacct gtgtatggga caaaaaggaa gagccaaatt caaacttatt tggagcataa  300
   tgaaggagct ggtgtacaac atttggcttt gatgagtgaa gatatatttt ggactttaag  360
   ggagatgagg aagagaagtg gtcttggtgg gtttgagttt atgccgtcgc cgcctccgac  420
   ttattaccgg aatttgagga atagagctgc tgatgtattg agtgaggagc agatgaagga  480
   gtgtgaagag ttggggattt tggtggataa agatgatcag ggtactttgc ttcaaatctt 540
   cactaag 547
<210> 4
   <211> 1265
   <212> DNA
   <213> Amaranthus palmeri
<400> 4
   agatgtgtat aagagacagg tcttggcgtg cgtgctgttg ccgtcgaagt agaagacgcc 60
   gaagctgctt ttaatatcag cgtttcgcac ggggctatcc cctgtgtttc tcctattcac 120
   ttggaaaacg gtgtcgtttt atctgaggtt catttatatg gggatgttgt gcttcggtat 180
   gtaagctacg gaaatgaatg tggggatgtg tttttcttcc tgggtttgag gaaatgccgg 240
   aggaatcatc gtttcgagga cttgattttg gcattcgaag gttggatcat gctgtaggga 300
   atgtccctga gttggctcct gcaattgctt atttgaagaa gtttactggg tttcatgagt 360
   ttgctgagtt tacagctgaa gatgttggga cgagtgaaag tggattgaat tcagccgtat 420
   tggcaaacaa tgatgaaatg gtgttgtttc caatgaatga acctgtgtat gggacaaaaa 480
   ggaagagcca aattcaaact tatttggagc ataatgaagg agctggtgta cagcatttgg 540
   ctttgatgag tgaagacata ttttggactt taagggagat gaggaagaga agtggtcttg 600
   gtgggtttga gtttatgccg tcgccgcctc cgacttatta ccggaatttg aggaacagag 660
   ctgctgatgt attgagtgag gagcagatga aggagtgtga agagttgggg attttggtgg 720
   ataaagatga tcagggtact ttgcttcaaa tcttcaccaa acctattgga gacaggccaa 780
   ccatattcat cgagattatc caaagaattg gttgcatgat gaaagatgaa gacggcaaga 840
   tgtaccaaaa gggtggttgc ggaggattcg gaaagggaaa cttttcagag ctgttcaaat 900
   caatagagga gtacgagaag actcttgaac gtaaacaggt tccagataca gctgctgcat 960
   gatgagcaga ctaaaatatt gttgtcttgc tgatgaaatg atagaaaagg tttgtttctt 1020
   ggtacaatgc tcaacttcaa aattttcttt attaaataat gaagtgtaaa cttatacaaa 1080
   ctgtgtcata tatggtgatt ggtgatcatg gtttatgtag aatgtataac ataattgata 1140
   atctgtgtat atgctgaata cttacatact gtgaaatcat tctgatggaa acatacaatt 1200
   ggtcagtagc tgaggctggt agctcctcag attagttttt ttcagtcaaa atcgtagatg 1260
   tatat 1265
<210> 5
   <211> 3245
   <212> DNA
   <213> Amaranthus palmeri
<400> 5
   ttttctcttt tatttttatc caaaaatatc atgggatgtc tagattggaa taattaggga 60
   gtaaaaagta ccccttgatt atgcagagca aaaataaatt gtctgaattt gaaataaata 120
   tctacaagag taaatttttc catcttattc aaaggtaaat gtttgatcca cctacctcta 180
   tagatatatt tcagggaact aaattgtctt caccattaaa tttggttact tggtctttaa 240
   aacccaaatc atagaaatta atgataaaat caaaataaaa aagatattta aattcaaatt 300
   caaactaact aattttaaat tacaaaatga atatctgtaa tttacaaaag aaagtatcaa 360
   aaacatatga aaatctcaac atctgaaaat tacaaacaag tattctgttt tttcattttt 420
   tttttctttt tcgctatttc ctttcaaaaa taaaagtaaa taaaaatatt caaaagcaat 480
   tccataaaca aaatcttaga tatgtaaatc acaaaaacat tagatctaga aaaaaaaaat 540
   ttcttccatt gcaaaccctt tttcaacctt cataacttcc actaccataa tgaggccagt 600
   aaagagacaa aagtcattga gttgttgttg tgcagttgat gataaatgat gatagaaggg 660
   tttatttttt ttttgaaatg aatggttaga ttttctgact ttttatttac cctataatga 720
   atatcaaaca attaactcta taaattattt aatacattaa aatgtttcat gtaatatgtc 780
   tcctatatta tttacccttt aatttttaag tgggaaccaa gtatgtctta attatcttta 840
   ttttaatcaa atacgcggta tacatgaaat aatcaacaaa tgcaattact atgctcggac 900
   gagagtaaat ataatgggag gaagttgtac atacaattac gaaatagtct aaataaataa 960
   cgataatttg taatataaac aaacaaatca cacttatata aacagatttt atagggtgga 1020
   atcattagga ttctaattta tcttttttct tcttttgttt actttgctga tatttatttt 1080
   gtatttttcc tatttctcaa aaggaagact aacactcaaa taaaatgata ttgaaataca 1140
   aagcatcacc ggccaagccg agatgacgaa actattttga ataattatga tgatttacaa 1200
   ctccaaatag aagtaattga tcaagacttt aggacttgga agtgttgggc aaaatcttcc 1260
   agagtccagg ataagtgata agtgacgtat ttccgttact cttaagtgtt aacagctttt 1320
   tgtcacgcaa ggaaaagaag accgtggacg tcaacgatga cgttgaatgt tcatctttac 1380
   agtcgcagtc aatcaatctc tttttagatc gatcttccac ctcaattctc cgttacaaat 1440
   caaattccat ctagaacttc ttttttatta ttttgactca taaattcccc caaaaatact 1500
   tctattttat tataaataaa ttccaatttc tatgttctcc attcattacc acccattact 1560
   ccgttttcca aaccaccatt ttctctctcc tcctttaccg ctaacgctac caccattttc 1620
   gcttcttccc cgtctgaatt ttattacttc gctttctcta tcatcatctg acatgggtac 1680
   tttgaaaccc gaaactcaac ccgattccga attcaaactc gtgggttact ccaacttcgt 1740
   tcgggttaac cccaaatctg accgttttgc tgttaagcgt ttccaccata tagagttttg 1800
   gtgtggcgat gcaaccaatg ttagcagacg attttcttgg ggtcttggaa tgcctaccgt 1860
   tgctaaatct gacctttcta caggaaactc tgttcacgct tcttttcttc ttcgttccgg 1920
   tgacctttct tttctcttta cttcacctta ctctcctacc atgtccatcc cttcttctgc 1980
   tgcaatcccc tcgtttgatt tcaatcattt taccaaattc cttacatcgc acggtcttgg 2040
   cgtgcgtgct gttgccgtcg aagtagaaga cgcggaagct gcttttaaca tcagcgtttc 2100
   gcatggggct attccctgtg tttctcctat tcaattggaa aacggtgtcg ttttatctga 2160
   ggttcattta tatggggatg ttgtgcttcg gtatgtaagc tacggaaatg aatgtgggga 2220
   tgtgtttttt cttcctgggt ttgaggaaat gccggaggaa tcatcgtttc gaggacttga 2280
   ttttggcatt cgaaggttgg atcatgctgt agggaatgtc cctgagttgg ctcctgcaat 2340
   tgcttatttg aagaagttta ctgggtttca tgagtttgct gagtttacag ctgaagatgt 2400
   tgggacgagt gaaagtggat tgaattcagc cgtattggca aacaatgatg aaatggtgtt 2460
   gtttccaatg aatgaacctg tgtatgggac aaaaaggaag agccaaattc aaacttattt 2520
   ggagcataat gaaggagctg gtgtacagca tttggctttg atgagtgaag acatattttg 2580
   gactttaagg gagatgagga agagaagtgg tcttggtggg tttgaattta tgccgtcgcc 2640
   gcctccgact tattaccgga atttgaggag cagagctgct gatgtattga gtgaggagca 2700
   gatgaaggag tgtgaagagt tggggatttt ggtggataaa gatgatcagg gcactttgct 2760
   tcaaatcttc accaaaccta ttggagacag gccaaccata ttcatcgaga ttatccaaag 2820
   aattggttgc atgatgaaag atgaagacgg caagatgtac caaaagggtg gttgcggagg 2880
   atttggaaag ggaaactttt cagagttgtt caaatcaatt gaggagtacg agaagactct 2940
   tgaacgtaaa caggttccag atacagctgc tgcatgatga gcagactgaa atattgctgt 3000
   cttgctggtg gaagccatat aatggtaata tgatagaaaa ggtttgttgc tcaaaatttt 3060
   ctttattaaa taatgaagtg taaacttata caaactgtgt catatatggt gattgatgat 3120
   catgcatggt tatgtagaat gtataacata attgataatc tgtgtatatg ctgaaaactt 3180
   acatactgtg aaatcattct gatagaaaca tacaattggt gagtagctgt ctcttataca 3240
   catct 3245
<210> 6
   <211> 3416
   <212> DNA
   <213> Amaranthus palmeri
<400> 6
   ggaggaattt ttttgtgcat gtaaaatgtt ttctctctat tttttgattt atgctatttt   60
   ttctctttta tttttatcca aaaatatcat gggatgtcta gattggaata attagggagt  120
   aaaaagtacc ccttgattat gcagagcaaa aataaattgt ctgaatttga aataaatatc  180
   tacaagagta aatttttcca tcttattcaa aggtaaatgt ttgatccacc tacctctata  240
   gatatatttc agggaactaa aattgtcttc accattatat ttagttactt ggtctttaaa  300
   acccaaatca tagaaattaa cgataaaatc aaaatataaa gatatttaaa ttccaattca  360
   aactaactaa ttttaaatta caaaatgaat atctgtaatt tacaaaagaa agtatcaaaa  420
   acatatgaaa atctcaacat ctgaaaatta aaaaacaagt attttgtttc ttcatttttt  480
   tctttttcgc tatttccttt caaaaataaa agtaaataaa aatattcaaa agcaattcca  540
   taaacaaaat cttagatatg taaatcacaa aaacattaga tctagaaaaa aaaaatttct  600
   tccattgcaa accctttttc aaccttcata acttccacta ccataatgag gccagtaaag  660
   agacaaaagt cattgagttg ttgttgtgca gttgatgata aatgatgata gaagggttta  720
   tttttttttt gaaatgaatg gttagatttt ctgacttttt atttacccta tatagaatat  780
   caaacaatta actctataaa ttatttaata cattaaaatg tttcatgtaa tatgtctcct  840
   atattattta ccctttaatt tttaagtggg aaccaagtat gtcttaatta tctttatttt  900
   aatcaaatac gcggtataca tgaaataatc aacaaatgca attactatgc tcggacgaga  960
   gtaaatataa tgggaggaag ttgtacatac aattacgaaa tagtctaaat aaataacgat 1020
   aatttgtaat ataaacaaac aaatcacact tatataaaca gattttatag ggtggaatca 1080
   ttaggattct aatttatctt ttttcttctt ttgtttactt tgctgatatt tattttgtat 1140
   ttttcctatt tctcaaaagg aagactaaca ctcaaataaa atgatattga aatacaaagc 1200
   atcaccagcc aagccgagat gacaaaacta ttggctaagt gataactgat aagtgacgta 1260
   tttccgttac tctcaagtct taacagcttt ttgtcacgca aggaaaagaa gaccgtggac 1320
   gtcaacggtg acgttgaatg ttcatcttta cagtcgcagt caatcaatct ctttttagat 1380
   cgatcttcca cctcaattct ccgttacaaa tcaaattcca tctagaactt ctttttaatt 1440
   attttgactc ataaattccc ccaaaaatac ttctatttta ttataaataa attccaattt 1500
   ctatgttctc cattcatcac cacccattac tccgttttcc aaaccaccat tttctctctc 1560
   ctccattacc cctaacacaa ctaccatttt cgcttcttcc ccgtctgagt cttattacat 1620
   cgctttctct ctcatcatct gacatgggaa ctttgaaacc cgaaactcaa cccgattccg 1680
   aattcaaact cgtgggttac tccaacttcg ttcgggttaa ccccaaatct gaccgttttg 1740
   ctgttaagcg tttccaccat atagagtttt ggtgtggcga tgcaaccaat gttagcagac 1800
   gattttcttg ggtcttggaa atgcctatcg tcgctaaatc tgacctgtct acaggaaact 1860
   ctgttcacgc ttcttttctt cttcgttccg gtgacctttc ttttctcttt acttcaccgt 1920
   attctcctac catgtccatc ccttcttctg ctgcaatccc ctcgtttgat ttcaatcatt 1980
   ttaccaaatt ccttacatcg cacggtcttg gcgtgcgtgc tgttgccgtc gaagtagaag 2040
   acgccgaagc tgcttttaat atcagcgttt cgcacggggc tatcccctgt gtttctccta 2100
   ttcacttgga aaacggtgtc gttttatctg aggttcattt atatggggat gttgtgcttc 2160
   ggtatgtaag ctacggaaat gaatgtgggg atgtgttttt tcttcctggg tttgaggaaa 2220
   tgccggagga atcatcgttt cgaggacttg attttggcat tcgaaggttg gatcatgctg 2280
   tagggaatgt ccctgagttg gctcctgcaa ttgcttattt gaagaagttt actgggtttc 2340
   atgagtttgc tgagtttaca gctgaagatg ttgggacgag tgaaagtgga ttgaattcag 2400
   ccgtattggc aaacaatgat gaaatggtgt tgtttccaat gaatgaacct gtgtatggga 2460
   caaaaaggaa gagccaaatt caaacttatt tggagcataa tgaaggagct ggtgtacagc 2520
   atttggcttt gatgagtgaa gacatatttt ggactttaag ggagatgagg aagagaagtg 2580
   gtcttggtgg gtttgaattt atgccgtcgc cgcctccgac ttattaccgg aatttgagga 2640
   gcagagctgc tgatgtattg agtgaggagc agatgaagga gtgtgaagag ttggggattt 2700
   tggtggataa agatgatcag ggcactttgc ttcaaatctt caccaaacct attggagaca 2760
   ggtagatttt aatcttgctt tcaattgctt ttgcttgatt gattgactag ccaattttga 2820
   ttgcattttg ttgcttatat gacttgatga taatagatgg tttacctttc tcagctgttc 2880
   atttgtagcc agtatagatt cgttctaaaa tatttgcaac tgattatgac atgtagtagc 2940
   agaaaatgtc cctatattgg atgtttggca taaattaagc ttggttttgc acttatcctc 3000
   atttatttat aaattctaaa acttgttagt tgtaattaag ttaatgagaa caaagcctta 3060
   atattccttc aaggtgattg tagttggggc actagttcta acaatggaaa tttggaaatc 3120
   tattccaact ggtcccaagt taatctttgt ttgcaagcct gattggttca aattaaggtt 3180
   attgtattct tgtatgaatt cgactcaatg taaatttgtt taatggagca tcaattttta 3240
   atagttttcg accaagcagt attagatata ttcgggattg aaataatgca tctatgagtg 3300
   tataaaacca aatggcacat ttgattagaa taaaagagag tataaggcta atttcgttta 3360
   cctaatattt aaagcgaccc ctaaaattca attggcctaa acccataaag ttcaaa 3416
<210> 7
   <211> 3818
   <212> DNA
   <213> Amaranthus palmeri
<400> 7
   gagttcaaga ttaaaagtta aattacattt atgtgggttt tatataggca atgattgcat   60
   tatattgttt ttcttttggt gggaagattt tcctttttaa aaaattttaa tttccctaca  120
   ttttcaaatg atggaggaat ttttttgtgc atgtaaaatg ttttctctct attttttgat  180
   ttatgctatt ttttctcttt tatttttatc caaaaatatc atgggatgtc tagattggaa  240
   taattaggga gtaaaaagta ccccttgatt atgcagagca aaaataaatt gtctgaattt  300
   gaaataaata tctacaagag taaatttttc catcttattc aaaggtaaat gtttgatcca  360
   cctacctcta tagatatatt tcagggaact aaattgtctt caccattaaa tttggttact 420
   tggtctttaa aacccaaatc atagaaatta atgataaaat caaaataaaa aagatattta 480
   aattcaaatt caaactaact aattttaaat tacaaaatga atatctgtaa tttacaaaag 540
   aaagtatcaa aaacatatga aaatctcaac atctgaaaat taaaaaacaa gtattttgtt 600
   tcttcatttt tttctttttc gctatttcct ttcaaaaata aaagtaaata aaaatattca 660
   aaagcaattc cataaacaaa atcttagata tgtaaatcac aaaaacatta gatctagaaa 720
   aaaaaaattt cttccattgc aaaccctttt tcaaccttca taacttccac taccataatg 780
   aggccagtaa agagacaaaa gtcattgagt tgttgttgtg cagttgatga taaatgatga 840
   tagaagggtt tatttttttt ttgaaatgaa tggttagatt ttctgacttt ttatttaccc 900
   tatatagaat atcaaacaat taactctata aattatttaa tacattaaaa tgtttcatgt 960
   aatatgtctc ctatattatt taccctttaa tttttaagtg ggaaccaagt atgtcttaat 1020
   tatctttatt ttaatcaaat acgcggtata catgaaataa tcaacaaatg caattactat 1080
   gctcggacga gagtaaatat aatgggagga agttgtacat acaattacga aatagtctaa 1140
   ataaataacg ataatttgta atataaacaa acaaatcaca cttatataaa cagattttat 1200
   agggtggaat cattaggatt ctaatttatc ttttttcttc ttttgtttac tttgctgata 1260
   tttattttgt atttttccta tttctcaaaa ggaagactaa cactcaaata aaatgatatt 1320
   gaaatacaaa gcatcaccag ccaagccgag atgacaaaac tattggctaa gtgataactg 1380
   ataagtgacg tatttccgtt actctcaagt cttaacagct ttttgtcacg caaggaaaag 1440
   aagaccgtgg acgtcaacgg tgacgttgaa tgttcatctt tacagtcgca gtcaatcaat 1500
   ctctttttag atcgatcttc cacctcaatt ctccgttaca aatcaaattc catctagaac 1560
   ttctttttta ttattttgac tcataaattc ccccaaaaat acttctattt tattataaat 1620
   aaattccaat ttctatgttc tccattcatt accacccatt actccgtttt ccaaaccacc 1680
   attttctctc tcctccttta ccgctaacgc taccaccatt ttcgcttctt ccccgtctga 1740
   attttattac ttcgctttct ctatcatcat ctgacatggg aactttaaaa cccgaaactc 1800
   aacccgattc cgaattcaaa ctcgtgggtt actccaactt cgtccgggtt aaccccaaat 1860
   ctgaccgttt tgctgttaag cgtttccacc atatagagtt ttggtgtggc gatgcaacca 1920
   atgttagcag acgattttct tggggtcttg gaatgcctac cgtcgctaaa tctgacctgt 1980
   ctacaggaaa ctctgttcac gcttcttttc ttcttagttc cggtgacctt tcttttctct 2040
   ttacttcacc ttactctcct accatgtcca tcccttcttc tgctgcaatc ccctcgtttg 2100
   atttcaatca ttttaccaaa ttccttacat cgcacggtct tggcgtgcgt gctgttgccg 2160
   tcgaagtaga agacgcagaa gctgctttta atatcagcgt ttcgcacggg gctatcccct 2220
   gtgtttctcc tattcacttg gaaaacggtg tcgttttatc tgaggttcat ttatatgggg 2280
   atgttgtgct tcggtatgta agctacggaa atgaatgtgg ggatgtgttt tttcttcctg 2340
   ggtttgagga aatgccggag gaatcatcgt ttcgaggact tgattttggc attcgaaggt 2400
   tggatcatgc tgtagggaat gtccctgagt tggctcctgc aattgcttat ttgaagaagt 2460
   ttactgggtt tcatgagttt gctgagttta cagctgaaga tgttgggacg agtgaaagtg 2520
   ggttgaattc agccgtattg gcaaacaatg atgaaatggt gttgtttccg atgaatgaac 2580
   ctgtgtatgg gacaaaaagg aagagccaaa ttcaaactta tttggagcat aatgaaggag 2640
   ctggtgtaca gcatttggct ttgatgagtg aagacatatt ttggacttta agggagatga 2700
   ggaagagaag tggtcttggt gggtttgagt ttatgccgtc gccgcctccg acttattacc 2760
   ggaatttgag gaacagagct gctgatgtat tgagtgagga gcagatgaag gagtgtgaag 2820
   agttggggat tttggtggat aaagatgatc agggcacttt gcttcaaatc ttcaccaaac 2880
   ctattggaga caggtagatt ttaatcttgc tttcaattgc ttttgcttga tggattgact 2940
   agccaatttg attgcatttt gttgcttata tgacttgatg ctagatagtt tacctttctc 3000
   agctgttaag ttgtagcaag tatctaatac attcgttctg aaatatctga aatatttgca 3060
   actgattatg acatgtagcg ggagaaaatg tccgtttggc ataaattaag cttggttttg 3120
   cacttatcct catttattta taaattctaa aacttgttag ttgtaagctc ctttcagttg 3180
   tcctgaattt aattaagtta atgagaacaa agccttaata ttccttcaag gtgattgtag 3240
   ttggggcact agttctaaca atggaaattt ggaaatctat tccaactggt cccaagttaa 3300
   tctttgtttg caagcctgat tggttcaaat taaggttatt gtattcttgt atgaattcga 3360
   ctcaatgtaa atttgtttaa tggagtatca atttttaata gttttcaacc aagtagtatt 3420
   agatatattc gggattgaaa taatgcatct atgagtgtac aaaaccaaat ggcacatttg 3480
   attagaataa aagagagtat aaggctaatt tcgtttacct aatatttaaa gcgaccccta 3540
   aaattcaatt ggcctaaacc cataaagttc aaaagcagag aagaacatag aatagtgcag 3600
   gtccattggt aatgcactag gagttggagc ttttatgggt acaagtgtgt ggctagttgg 3660
   ggatgactgt ctagcattgt ctaggtgaaa agctgaagcc ttaagccatg aaggttttga 3720
   gtagaggtgt tcatttgggt catcgggttg atttcgggtc agatgtttcg ggtcggttta 3780
   aaatcgggtt ttgtgttcac attggttttt acgtaatt 3818
<210> 8
   <211> 775
   <212> DNA
   <213> Amaranthus rudis
<400> 8
   aaatgaatgt ggggatgtgt tttttcttcc tgggtttgag gaaatgccgg aggaatcatc   60
   gtttcgagga cttgatttcg gccttcgaag gttggatcat gctgtaggga atgtccctga  120
   gttggctcct gcaattgctt atttgaagaa gtttactggg tttcatgagt ttgctgagtt  180
   tacagctgaa gatgttggga cgagtgaaag tgggttgaat tcagccgtat tggcaaataa  240
   tgatgaaatg gtgttgtttc caatgaatga accagtgtat gggacaaaaa ggaagagtca  300
   aattcaaact tatttggagc ataatgaagg agctggtgta caacatttgg ctttgatgag  360
   tgaagatata ttctggactt taagggagat gaggaagaga agtggtcttg gtgggtttga  420
   gtttatgccg tcgccgcctc cgacttatta ccggaatttg aggaacagag ctgctgatgt  480
   attgagtgag gagcagatga aggagtgtga agagttgggg attttggtgg ataaagatga  540
   tcagggtact ttgcttcaaa tcttcaccaa acctattgga gacaggccaa ctatatttat  600
   cgagatcatc caaagaattg gttgcatgat gaaagatgaa gacggcaaga tgtaccaaaa  660
   gggtggttgc ggaggatttg gaaagggaaa cttttcggag cttttcaaat caattgagga  720
   gtacgagaag actcttgaac gtaaacaggt tccagataca gctgctgcat gagtt        775
<210> 9
   <211> 1204
   <212> DNA
   <213> Amaranthus rudis
<400> 9
   cttacatcgc acggtcttgg tgtgcgtgct gttgctgtcg aagtagagga cgcagaagct 60
   gcttttaata tcagcgtttc caacggggct attccctgtg tttctcctat tcaattggaa 120
   aacggtgtcg ttttatctga ggttcattta tatggggatg ttgtgcttcg ctatgtaagc 180
   tacggaaatg aacgtgggga tgtgtttttt cttcctgggt ttgaggaaat gccggaggaa 240
   tcgtcgtttc gaggacttga tttcggcctt cgaaggttgg atcatgctgt agggaatgtt 300
   cccgagttgg ctcctgcaat tgcttatttg aagaagttta ctgggtttca tgagtttgct 360
   gagtttacag ctgaagatgt tgggacgagt gaaagtgggt tgaattcagc cgtattggca 420
   aataatgatg aaatggtgtt gtttccaatg aatgaacctg tgtatgggac aaaaaggaag 480
   agtcaaattc aaacttattt ggagcataat gaaggagctg gtgtacaaca tttggctttg 540
   atgagtgaag atatattttg gactttaagg gagatgagga agagaagtgg tcttggtggg 600
   tttgagttta tgccgtcgcc gcctccgact tattaccgga atttgaggaa tagagctgct 660
   gatgtattga gtgaggagca gatgaaggag tgtgaagagt tggggatttt ggtggataaa 720
   gatgatcagg gtactttgct tcaaatcttc actaagccta ttggtgacag gccaactata 780
   tttatcgaga tcatccaaag aattggttgc atgatgaaag atgaagacgg caagatgtac 840
   caaaagggtg gttgcggagg atttggaaag ggaaactttt cggagctttt caaatcaatt 900
   gaggagtatg agaagactct tgaacgtaaa caggttccag atacagctgc tgcatgagca 960
   gactaaaata ttgctgaaac gcaggctgca gccatatgtt agaacagtca ttctgatgga 1020
   aacactcaag cggtgagtag ctgaggttgg tgatgctgaa gtcgagtcgg tatttggatc 1080
   atcttacaat tacagtgcaa ggatagtaat gaagcatgta aacagctcct cagattagtt 1140
   ttttccagtc ataatcgtag atgtatatga gaaaatttaa attgctcttt taagttaatg 1200
   aaaa 1204
<210> 10
   <211> 511
   <212> DNA
   <213> Amaranthus rudis
<400> 10
   tcatcaacaa caaaagagtt gaaattctaa taataatccc agtaagcagt gagtaagatc   60
   aaatggagag tcagttagta gctaatcata caaaaccatt aaagctacaa agttactcta  120
   atttcttaag atcaaaccca aaatctgatc gtttcaaagt gaaaaggttc caccacattg  180
   agttctggtg tggtgatgca accaacacta gccttttatt ttcgttaggg cttggcatgc  240
   ctatggttgc caaatccgat ctctccacag gcaaccttat acatgcctcc tacgtcttac  300
   gtagtggcga actttgtttc gtattcacgg ctccttactc tccgtcatcc atgctcacaa  360
   ctgcatcaat tccttcgttt gactacagtg cgcacgcctc ttttgtgtcc tgtcatggcc  420
   ttggtgttcg cgctgtggcc cttgaggtgg agaacgctga atccgccttt aggattagtg  480
   ttgcagcagg agctcatccg tcagccccac c                                 511
<210> 11
   <211> 770
   <212> DNA
   <213> Amaranthus rudis
<400> 11
   aatcatttta ccaaattcct tacatcgcac ggtcttggcg tgcgtgctgt tgctgtcgaa   60
   gtagaagacg cagaagctgc ttttaatatc agcgtttcca acggggctat tccctgtgtt  120
   tctcctattc acttggaaaa cggtgtcgtt ttatctgagg ttcatttata tggggatgtt  180
   gtgcttcggt atgtaagcta cggaaatgaa tgtggggatg tgttttttct tcctgggttt  240
   gaggaaatgc cggaggaatc atcgtttcga ggactagatt tcggccttcg aaggttggat  300
   catgctgtag ggaatgtccc tgagttggct cctgcaattg cttatttgaa gaagtttact  360
   gggtttcatg agtttgctga gtttacagct gaagatgttg ggacgagtga aagtgggttg  420
   aattcagccg tattggcaaa taatgatgaa atggtgttgt ttccaatgaa tgaaccagtg  480
   tatgggacaa aaaggaagag tcaaattcaa acttatttgg agcataatga aggagctggt  540
   gtacaacatt tggctttgat gagtgaagat atattctgga ctttaaggga gatgaggaag  600
   agaagtggtc ttggtgggtt tgagtttatg ccgtcgccgc ctccgactta ttaccggaat  660
   ttgaggaaca gagctgctga tgtattgagt gaggagcaga tgaaggagtg tgaagagttg  720
   gggattttgg tggataaaga tgatcagggt actttgcttc aaatcttcac             770
<210> 12
   <211> 1412
   <212> DNA
   <213> Amaranthus rudis
<400> 12
   accaccattt tcgtttcttc cccgtctgag ttttattact tcactttctc tctcatcatc   60
   tgacatggga actttgaaac ccgaaaatca acccgattcc gaattcaaac tcgtgggtta  120
   ctccaacttt gttcgggtta accccaaatc tgaccgtttt actgttaagc gtttccatca  180
   tatagagttt tggtgtggcg acgcaaccaa tgttagcaga cgattttctt ggggtcttgg  240
   aatgcctacc gtcgctaaat ctgacctttc tacgggaaac tctgttcacg cttcttttct  300
   tcttcgttcc ggtgaccttt ctttccttac ttcaccttac tcccctacca tgtccatccc  360
   ttcttctgct gcaatcccct cgtttgattt caatcatttt accaaattcc ttacatcgca  420
   cggtcttggc gtgcgtgctg ttgctgtcga agtagaagac gcagaagctg cttttaatat  480
   cagcgtttcc aacggggcta ttccctgtgt ttctcctatt cacttggaaa acggtgtcgt  540
   tttatctgag gttcatttat atggggatgt tgtgcttcgg tatgtaagct acggaaatga  600
   atgtggggat gtgttttttc ttcctgggtt tgaggaaatg ccggaggaat cttcgtttcg  660
   aggacttgat ttcggccttc gaaggttgga tcatgctgta gggaatgtcc ctgagttggc  720
   tcctgcaatt gcttatttga agaagtttac tgggtttcat gagtttgctg agtttacagc  780
   tgaagatgtt gggacgagtg aaagtgggtt gaattcagcc gtattggcaa ataatgatga  840
   aatggtgttg tttccaatga atgaaccagt gtatgggaca aaaaggaaga gtcaaattca  900
   aacttatttg gagcataatg aaggagctgg tgtacaacat ttggctttga tgagtgaaga  960
   tatattctgg actttaaggg agatgaggaa gagaagtggt cttggtgggt ttgagtttat 1020
   gccgtcgccg cctccgactt attaccggaa tttgaggaac agagctgctg atgtattgag 1080
   tgaggagcag atgaaggagt gtgaagagtt ggggattttg gtggataaag atgatcaggg 1140
   tactttgctt caaatcttca ccaaacctat tggagacagg tagtttttaa tcttgctttc 1200
   aattgctttt gattaattga ttgattagcc aatttgatga ttgcattttg ttgcttgtat 1260
   gacttgatga tatatggttt accttttctc agctgttcag ttgtagcaag tatttctaat 1320
   ccgttctgaa atactccatt cgcaactgat tgtgacatgt tgtgcagaaa attatggaaa 1380
   atgagaaaat gtccctatat tggaagattg gt                               1412
<210> 13
   <211> 707
   <212> DNA
   <213> Amaranthus thunbergii
<400> 13
   cccgagttgg ctcctgcaat tgcttatttg aagaagttta ctgggtttca tgagtttgct 60
   gagtttacag ctgaagatgt tgggacgagt gaaagtgggt tgaattcagc cgtattggca 120
   aataatgatg aaatggtgtt gtttccaatg aatgaacctg tgtatgggac aaaaaggaag 180
   agccaaattc aaacttattt ggagcataat gaaggagctg gtgtacaaca tttggctttg 240
   atgagtgaag atatattttg gactttaagg gagatgagga agagaagtgg tcttggtggg 300
   tttgagttta tgccgtcgcc gcctccgact tattaccgga atttgaggaa tagagctgct 360
   gatgtattga gtgaggagca gatgaaggag tgtgaagagt tggggatttt ggtggataaa 420
   gatgatcagg gtactttgct tcaaatcttc actaagccta ttggtgacag gccaaccata 480
   tttatcgaga ttatccaaag aattggatgc atgatgaaag atgaggacgg caagatgtac 540
   caaaagggtg gttgtggagg atttggaaag ggaaactttt cggagctgtt caaatcaatt 600
   gaggagtatg agaagactct tgaacgtaaa caggttccag atacagctgc tgcatgagca 660
   gactaaaata ttgctgaaac gcaggctgca gccatatgtt agaacag 707
<210> 14
   <211> 1267
   <212> DNA
   <213> Amaranthus viridis
<400> 14
   aaccaccatt ttctctctcc tccattacca ctaacactcc caccattttc gtttcttccc 60
   cgtctgagtc ttattacttc gctttctctc acatcatctg acatgggaac tttgaaaccc 120
   gaaactcagc ccgattccga attcaaactc gtgggatact ccaacttcgt tcgggttaac 180
   cccaaatctg accgttttac tgttaagcgt tttcatcata tagagttttg gtgtggcgat 240
   gcaaccaatg ttagcagacg attttcttgg ggtcttggaa tgcctaccgt tgctaaatct 300
   gacctttcta ctggaaactc tgttcacgct tcttttcttc ttcgttccgg tgacctttct 360
   tttctcttta cttcacctta ctctcctacc atgtccgtcc cttcttctgc tgcaatcccc 420
   tcgtttgatt tcaatcattt taccaaattc cttacatcgc acggtcttgg tgtgcgtgct 480
   gttgctgtcg aagtagaaga cgcagaagct gcttttaata tcagcgtttc caacggggct 540
   attccctgtg tttctcctat tcaattggaa aacggtgtcg ttttatctga ggttcattta 600
   tatggggatg ttgtgcttcg gtatgtaagc tacggaaatg aatgtgggga tgtgtttttc 660
   ttcctgggtt tgaggaaatg ccggaggaat cgtcgtttcg aggacttgat ttcggccttc 720
   gaaggttgga tcatgctgta gggaatgttc ccgagttggc tcctgcaatt gcttatttga 780
   aaaagtttac tgggtttcat gagtttgctg agtttacagc tgaagatgtt gggacgagtg 840
   aaagtgggtt gaattcagcc gtattggcaa ataatgatga aatggtgttg tttccaatga 900
   atgaacctgt gtatgggaca aaaaggaaga gccaaattca aacttatttg gagcataatg 960
   aaggagctgg tgtacaacat ttggctttga tgagtgaaga tatattttgg actttaaggg 1020
   agatgaggaa gagaagtggt cttggtgggt ttgagtttat gccgtcgccg cctccgactt 1080
   attaccggaa tttgaggaat agagctgctg atgtattgag tgaggagcag atgaaggagt 1140
   gtgaagagtt ggggattttg gttgacagag atgatcaagg cactctgctt caaattttca 1200
   ctaagcccat tggagatagg cctacgatat tcatagaaat aatacagaga ttagggtgca 1260
   tggtgaa 1267
<210> 15
   <211> 637
   <212> DNA
   <213> Ambrosia trifida
<400> 15
   ccggtttacg ggacgaagag gaagagtcag atacagacat atttggaaca taatgaaggg 60
   gcaggggtgc agcatttggc gttggcgagt gaggatatat ttaggacatt gagggagatg 120
   aggaaaagga gtggggtggg tgggtttgag tttatgccat ctccgccgcc tacttattat 180
   cggaatttga agaacagggc gggcgatgtg ttgtcggatg agcagattaa ggagtgtgag 240
   gagttgggga tattggtgga taaggatgat caggggactt tgcttcagat ttttaccaag 300
   cctgttggtg ataggccgac gatattcata gagataattc agagagtagg atgtatgatg 360
   aaggatgaag aaggaaaggt gcagcagaag gcgggctgtg gaggatttgg taaagggaac 420
   ttttcggagc tttttaaatc gattgaggaa tatgaaaaga cactggaagc aagaacctga 480
   agccacatga aaaccacaca caaataatct ttcatgagat tttaaaatct aatgattatg 540
   catctgtgga ttctatacaa caaacagatc ctgaaaatat aggatatcaa ctttacaata 600
   agttatgtaa tatgcatctc atgattatca atgtatt 637
<210> 16
   <211> 718
   <212> DNA
   <213> Ambrosia trifida
<400> 16
   tacctcacaa acccaatcct ccatacttcc gtgttctcta ctactgttcc actttattaa 60
   tcttacccaa ccccttctca tctatttccc ccctacctca caaacataac ccaaataaaa 120
   aataaaatta aaataaaaaa taaaatggaa ctgaacccac cctcgttgac cccgtcgtcg 180
   ccggagacaa ctccgaccac tccaaccacc ccatctccgc cttcaagcta gtcggcttca 240
   aaaacttcat ccgtaacaac cctaagtccg acaaattctc cgtcaaatcc ttccaccaca 300
   tcgagttctg gtgctccgac gccaccaacg ccgcccgtcg cttctcatgg ggtctcggca 360
   tgcctattac ttacaaatcc gacctttcta ccggtaacca tacccacgcc tcttatctcc 420
   ttaactccgg ccaccttaac ttcctcttca ccgcccctta ttcccccacc atctccacca 480
   ccaccaccac cgcctccatc ccgtcgttct cccactccgc ctgccgtcac ttcaccgcct 540
   cccacggcct tgctgtccgt gccatcgccg ttgaagttca agacgctgaa atcgctttct 600
   ccgttagtgt cgctaacggc gctaaaccct catctccacc tatcaccctt ggccacgacg 660
   acgtcgtttt gtcagaagtt caactctacg gtgacgtcgt tttacgttat attagtta 718
<210> 17
   <211> 54
   <212> DNA
   <213> Ambrosia trifida
<400> 17
   ctccgaggcg ttttgggtcc aaaatccgaa gcgcggggct taaagcgcga ggcg 54
<210> 18
   <211> 719
   <212> DNA
   <213> Ambrosia trifida
<400> 18
   cttcatgtag ttcagcgctt tttggcaaaa aaagcgtttt tcttggagcc ttgtgcctca 60
   aacgcttaaa gtgcgcccca ggcgagcttt tttaaaccaa ggaacacgca caagcactcc 120
   accaatagtt caatgccgtg ctaaccagtg ggattaacta gtggaagctg gattcaaaca 180
   tgctagagtg gaaatatatt ttttttaatg tttggatatg catatttaac cattatagca 240
   taatattaat gacaagggtt gtaaattggt atgcaaattg tttgagcagg ccgacgatat 300
   tcatagagat aattcagaga gtaggatgta tgatgaagga tgatgaagga aaggtgcagc 360
   agaaggcggg ttgtggagga tttggtaaag ggaacttttc ggagcttttt aaatcgattg 420
   aggaatatga aaagacactg gaagcaagaa cctgaagcca catgaaaacc acacaaataa 480
   tctttaatga gatcttaaaa tctaatgatt atgcatctgt ggattctata caacaaacag 540
   atcctgaaaa tataggatat caactttaca ataagttatg taatatgcat ctcatgatta 600
   tcaatgtatt aatttatttt tatgttgctg ttttcggttt agtttttgtt cgttggtgtt 660
   gggccggctc ttcttctatt tcgttcttga tccatttgaa gttgaaggcg aaatttagg 719
<210> 19
   <211> 845
   <212> DNA
   <213> Ambrosia trifida
<400> 19
   gcagttgagc tctacggctc actgaaacag acctttaaac catgaacaag gtttatgtgt   60
   aggtaaacta actttggacc aaaaagctgt ttttttaacc ttcacaattt gtttcatgga  120
   aggttttcct ttttgttcag ttgacatgtt tctaaacggg acaaataaac atacatctat  180
   gcctattgca catatagcat caaccacctc actcccatgt ggccctcaca acaaggttgt  240
   aaaaatcggg actagtcgcc gactagtccc cgataaatcg gtgatcggag gtctactgat  300
   taatttttca ttaatcactc aattagtcgg aatcggccca agccggtcca agccggccaa  360
   agtcggccga agtcggagct agtcgccaaa gaattccgtt tgaagttggc ttaaaaacat  420
   gctttcatcc cacaaactga tccattaccc caaaaaacca aagttaaaaa cctgacctgg  480
   atccctaaat gcttcataat ccggcaaacc tttctgttcc ggcgaacctt tctgtttccg  540
   gcgacccatt tggagcaggt ggagttggtt ttgttcagtc ttttgcggcg tataatgaaa  600
   gtggtagtga gtagggtttt taagtttgga tctttttata tttaacccct tatagtttta  660
   ctagtttata ttttgtccct taacttatat atttatacat aataagcata aagttaatgt  720
   tatatatata caaattaacc ctctacatct ttactagtct atatttggtc ctctaactta  780
   tacatctata caaaaaaagc ataaaattaa tgttatatat atataaattt ataaaattat  840
   acata                                                              845
<210> 20
   <211> 1024
   <212> DNA
   <213> Ambrosia trifida
<400> 20
   tcgtcgtttc aggagctgga ttacggtatc cggcggctag atcacgctgt ggggaacgta   60
   ccggagttag caccagcagt ggaatatata aaatcgttta ccgggtttca cgagtttgct  120
   gagtttacag cagaggatgt gggaacgagt gagagtggac tcaactcggt ggtgttggct  180
   tgcaatagtg agatggtatt aatacctctg aatgagccgg tttacgggac gaagaggaag  240
   agtcagatac agacatattt ggaacataat gaaggggcag gggtgcagca tttggcgttg  300
   gcgagtgagg atatatttag gacattgagg gagatgagga aaaggagtgg ggtgggtggg  360
   tttgagttta tgccgtctcc gccgcctact tattatcgga atttgaagaa tagggcgggc  420
   gatgtgttgt cggatgagca gattaaggag tgtgaggagt tggggatatt ggtggataag  480
   gatgatcagg ggactttgct tcagattttt accaagcctg ttggtgatag gtattgtttt 540
   tcgttaatgt agcgttactt gaattcgtaa ttatgtgttt aaactttaaa ctagatagta 600
   ttaaggctta tgcaatatgt attgttcccc tagaggacga gggttctaga ctctagttcc 660
   gtaatgtagt gataaaggtg tagattaaga gtaggattag cgggtgtgtt agttagcggt 720
   tttaacaaga aaaacacata atatgttgaa agaacaataa caagaatggc aaacataaaa 780
   aaggaaaagc cataaaggtt gtcattctat aatttgcttg cattcaagat tcttactagg  840
   attgtattat tatgtggtta ctaaactcta ttcaagtata aaaacatgaa accccttcac  900
   ataaggaaaa agaaatatgt atgttttaaa atggatcatc ttttcccttg tgtcacgatt 960
   gccaatgttt tttttgttga ccatgagaac cgttggtgaa cacgccttgg tttaaaaaag 1020
   cgcg 1024
<210> 21
   <211> 1610
   <212> DNA
   <213> Conyza canadensis
<400> 21
   tatggtattg cattgtttca ttcaattgta cataatccac aaatacatat ggtgataatc   60
   atgttctttg agtcaagaag atataaacat tatacatagg cagttttcat gcagtggcag  120
   ttggttcagt ggtgtttcgt gcttccagcg tcttctcgta ttcttcaata gatttgaaga  180
   gctctgagaa attgcccttg ccaaatcctc cacagcctgg cttctgttgc accttgcctt  240
   catcatcttt cagcatacac cctactctct gaataatctc tatgaatata gttggcctat  300
   caccaacagg cttggtgaaa atctgaagca aagttccctg atcatctcta tcaaccaaaa  360
   tccccaattc ctcacattcc ttaatctgtt cgtcactcaa cacgtccccg accctattct  420
   tcaaattctt ataataagta ggcggtggag acggcataaa ctcaaaacca ccgacactac  480
   tccgttttct catctctctc aaagtcctaa aaatatcctc actcgccaac gccaaatgct  540
   gcacaccagc tccctcatta tgttccaaat acgtctgtat ctggctcttc cttttcgttc  600
   cataaactgg ctcgttcatc ggtatcaaaa ccgtttcact attacacgct aatacaaccg  660
   aattaagccc gctctcactc gttcctacat cctctgccgt aaattcagca aattcgtgaa  720
   atccagtgaa ggacttcacg tagtctactg ccggagctag ctccggcacg tttcctacgg  780
   cgtgatctaa cttatgaatt ccgtaattaa gttcatgaaa cgacgacgtt gcgtccacag  840
   gctcaaaacc cggtaggaaa ttagtagtaa catcataatt agtgttttta aaactaacat  900
   atctcaaaac gacatcgccg tacagtttaa cttcagccaa aacgacgtcg ttatttccta  960
   gagtgaccgg agctgacgac ggtttagcac cgtgagcgat actaacagaa taagcgattt 1020
   cagcgtcttc aacttcaacg gcgatagacc gtacagcgag gccgtgagtg gcggtgaagt 1080
   tccggcaagc agtgtgagag aaagtaggaa tagacgatgt ggaaccggtg gtggagatgg 1140
   tggtggaata aggtgcggtg aagaggaagt tgagctggcc ggagttaata aggtaagaag 1200
   cgtgagtgtt gttaccggtg gagagatcgg atttgtaaag aagcggcatg ccgagacccc 1260
   aagagaagcg gcgggcggtg ttggtggcgt cggaacacca gaattcgacg tggtggaatt 1320
   ttttgacggt gaatttgtcg gatttagggt tttgacggat gaagtttttg aagccgacga 1380
   gtttgtaagt ggcggaggtg gtggtagtgg tttgttgttg tccggtgagt gtgtttccgt 1440
   ttgctgcttc agttaccatt ttgtttttat taatttgaat gtatagatag aaatatgaag 1500
   tagatatata gaatgataga tatagataca gtacaagtag ttgaggggtt ggatgatgga 1560
   gatgaagaag aacacggaag tggtttattt tctttgggtg ctattaattg            1610
<210> 22
   <211> 4963
   <212> DNA
   <213> Conyza canadensis
<400> 22
   aacacgaatg ggctagctag ctgatgaatt taatgtatat atgtctttaa aaaccacttt   60
   catgggctac ttgtgttttt gatgagtttt tcaacatcat tctcatctta ccttcttaca  120
   atggttcacc atattgcttc gtttgtcgtc tcctgcatgg ttatcgaatt tattttttca  180
   aattatatta ctattatctt tttgttttct gatttctcac atgtaattta cattatgagt  240
   ttcaccatcc gtatgtaaat tgtaaaataa atcacaaatt tggtttttaa ccatcaacta  300
   aattcattga aatccttaag ttataaccca tttgaggtta tagattatga gagtagattc  360
   ctattttctc tttggcttat gtatccttaa ttatgtttga attgattcta actttattat  420
   attttaggaa ttacttaact tcaaaaatct caacacgtca tataatgctt tttgtggatg  480
   gatgcttttg acccatttcg ttgaaaactt atgtcgacac acatatctga caaacttgta  540
   ctcgtatatc ctttatcgca tatgctatag tataatattc gaaatttaat ctcaagataa  600
   ttttcccaaa aggtcatatt aattgaattg agattaattc ctacctaaca tatatagata  660
   tattgtgttg gtgttatcct ctttggagtt ttagtcgcgc atgtattcta agaaaatggt  720
   aattttgaac ttatattgat ggtctaaatt ttatcctatc actaaaattt cagtcatgtt  780
   tcctagctaa ttaatttggg ttcaatccag gtattctaaa taagttttgt aaatacatgt  840
   ctattatcat aatgaaacat agaaaaaccc gtaaagtaaa aatgtctata tttttagtgg  900
   caaattatta atgttgctat aatgaaaaga gaagattcaa ccttttgtac atcataaaaa  960
   atgttattct acattatgct aattttacat tagacttcat agttttgaaa actttcaaat 1020
   gaagggactt aaattttaaa tttaaagatg atgtcataaa ttagttagat aaaagttaaa 1080
   taattaataa gaaaaggtct aatgatacca aataaaattt tcttaaaaaa gtattaatgt 1140
   cattacaatt ttattttatt tatataagag aagataatag attttcttgt ataaaaaact 1200
   tacctgtaaa cttctatata aactttttag aaaaataatt taaaacggtc tcaaatatcg 1260
   ttatttccga acatacaaaa acccaaaacc taatccattt aattatattg taagacctcg 1320
   attagcttaa tttattttga aacctattta caaatgagtt tggcatgata tgaacttttt 1380
   tgcatttaca tatggctgta aacgaactga acagttcacg aacaattcat gaaccgttcg 1440
   gcgggaggtt cgttcgtgtt agttcgttta gttaaatgaa tgaacatgaa caaagctctc 1500
   gttcgttcat ttacgttcat gaacgttcga tagtctgttc gtgaacttta gttcgtctat 1560
   gttcatgaac tgtcgttcgt gaacaataat ttgattatgt tcatttacac acacatacat 1620
   aatcaactgt actataaaaa tagtttaaat ataaatattt ttttatataa tatatattat 1680
   taatatctaa ttatttaaga aaaagtttaa ataaaatact taatttataa atatttcgtt 1740
   cgtttgtgtt cgtgtacatt tgttcatgaa cacaaatgaa cgaacatgaa caagtcatta 1800
   tgttcgttta tctgttcgtt aagttaaatg aacgaacgaa cacgaacaag gtctcgtttg 1860
   tgttcgttcg gttcgtttac ggcccttaat tacatgtgaa gttaataaat aaaaataaag 1920
   ttcatcaaaa ttaaacctat ggttggagat aaaaccagtc agccttacgt gtggaaacta 1980
   tcctactcta caattaatag cacccaaaga aaataaacca cttccgtgtt cttcttcatc 2040
   tccatcatcc aacccctcaa ctacttgtac tgtatctata tctatcattc tatatatcta 2100
   cttcatattt ctatctatac attcaaatta ataaaaacaa aatggtaact gaagcagcaa 2160
   acggaaacac actcaccgga caacaacaaa ccactaccac cacctccgcc acttacaaac 2220
   tcgtcggctt caaaaacttc atccgtcaaa accctaaatc cgacaaattc accgtcaaaa 2280
   aattccacca cgtcgaattc tggtgttccg acgccaccaa caccgcccgc cgcttctctt 2340
   ggggtctcgg catgccgctt ctttacaaat ccgatctctc caccggtaac aacactcacg 2400
   cttcttacct tattaactcc ggccagctca acttcctctt caccgcacct tattccacca 2460
   ccatctccac caccggttcc acatcgtcta ttcctacttt ctctcacact gcttgccgga 2520
   acttcaccgc cactcacggc ctcgctgtac ggtctatcgc cgttgaagtt gaagacgctg 2580
   aaatcgctta ttctgttagt atcgctcacg gtgctaaacc gtcgtcagct ccggtcactc 2640
   taggaaataa cgacgtcgtt ttggctgaag ttaaactgta cggcgatgtc gttttgagat 2700
   atgttagttt taaaaacact aattatgatg ttactactaa tttcctaccg ggttttgagc 2760
   ctgtggacgc aacgtcgtcg tttcatgaac ttaattacgg aattcataag ttagatcacg 2820
   ccgtaggaaa cgtgccggag ctagctccgg cagtagacta cgtgaagtcc ttcactggat 2880
   ttcacgaatt tgctgaattt acggcagagg atgtaggaac gagtgagagc gggcttaatt 2940
   cggttgtatt agcgtgtaat agtgaaacgg ttttgatacc gatgaacgag ccagtttatg 3000
   gaacgaaaag gaagagccag atacagacgt atttggaaca taatgaggga gctggtgtgc 3060
   agcatttggc gttggcgagt gaggatattt ttaggacttt gagagagatg agaaaacgga 3120
   gtagtatcgg tggttttgag tttatgccgt ctccaccgcc tacttattat aagaatttga 3180
   agaatagggt cggggacgtg ttgagtgacg aacagattaa ggaatgtgag gaattgggga 3240
   ttttggttga tagagatgat cagggaactt tgcttcagat tttcaccaag cctgttggtg 3300
   ataggtatgt ctatgttaac tttatcagtg attgtgcatt tgtccatttt gttgaattcg 3360
   taaaacatga gattaaatac ctgcaatatg ttttgcttac attgaatcta ggccaggttt 3420
   tcagtgaaca agtaccataa atgtataatg taggtctagt cttgttaatg atcagtgatc 3480
   aatagtgcat acattgaatg acgaattgac aactagtttc taatagaatg tgaaatataa 3540
   agtgaatgtc acaaaagctg ttatcttatc tattttagtt gtaatggacc aatggttgga 3600
   ctgtttgaaa gtgtgcttac aatcaagatt cgagattgtt actaggatgc tagcgctagg 3660
   tatatatcat atggtgtctg agctctatgg tagtataaat tgtgaataca ctaaacatct 3720
   cccaaaaata tttgagtaac cgatcaatct actagttatt gcaatgccaa acatctcttt 3780
   aaacaactac ttattgtaaa gccatactac atcaacacac caaaaatcta tctcccacaa 3840
   cccccgtttt acttgggtta tgaacttagt atagttattc aaaataatca ctttcaaaat 3900
   ggatccaaca catctgaaac tggtcacatt tgagttctta tctttcaaat aatttgtaat 3960
   tcatatcagg ctcagcttca tggggtctgg cctttgtctg tgtgtttgtg ttgtgtcttt 4020
   tctggtgtga tctaattcgg tatgcatatt gtttgagcag gccaactata ttcatagaga 4080
   ttattcagag agtagggtgt atgctgaaag atgatgaagg caaggtgcaa cagaagccag 4140
   gctgtggagg atttggcaag ggcaatttct cagagctctt caaatctatt gaagaatacg 4200
   agaagacgct ggaagcacga aacaccactg aaccaactgc cactgcatga aaactgccta 4260
   tgtataatgt ttatatcttc ttgactcaaa gaacatgatt atcaccatat gtatttgtgg 4320
   attatgtaca attgaatgaa acaatgcaat accatatgtg atgtgattta tatagaataa 4380
   caatagatgt cattcatata tgccgttcct gttttagtaa ttgtgtgttg gtggtgtgtt 4440
   gcaagttcca atcagctgta tataatgcct aaatattcaa tttgacctcc attaaggatc 4500
   cattgtgcag attctttgtt tctgtcttaa aagtgtggga gacttgaaac acatctgtaa 4560
   aactgaacca gatctgaatc gagctgaatg gaagcggcgc aggtctgctc tgtggttcca 4620
   cttacaacaa acaattgttc atggtacgtc gcctcttcat agccagtgtt tattgtaaga 4680
   aagcattgtt taggcgaaaa aggaaaaggt gatttatgta agaaaaggta gtgctattaa 4740
   ggttatgttc cccatgaaca aacagggtaa attcatacag gatcaacttt taaatgatac 4800
   tattaaaggt catctacttc tgtttcccat gcttgtgcca agctcaaatt tagacgaaac 4860
   caaaacagga tgagaaaact aattaacaca tattttagta agcaattaca gctatatatc 4920
   atactatcat taacaattac atcaacaatc tgaaaagttg cat 4963
<210> 23
   <211> 5610
   <212> DNA
   <213> Conyza canadensis
<400> 23
   attaaaaaca cgaatgggct agctagctga tgaatttaat gtatatatgt ctttaaaaac   60
   cactttcatg ggctacttgt gtttttgatg agtttttcaa catcattctc atcttacctt  120
   cttacaatgg ttcaccatat tgcttcgttt gtcgtctcct gcatggttat cgaatttatt  180
   ttttcaaatt atattactat tatctttttg ttttctgatt tctcacatgt aatttacatt  240
   atgagtttca ccatccgtat gtaaattgta aaataaatca caaatttggt ttttaaccat  300
   caactaaatt cattgaaatc cttaagttat aacccatttg aggttataga ttatgagagt  360
   agattcctat tttctctttg gcttatgtat ccttaattat gtttgaattg attctaactt  420
   tattatattt taggaattac ttaacttcaa aaatctcaac acgtcatata atgctttttg  480
   tggatggatg cttttgaccc atttcgttga aaacttatgt cgacacacat atctgacaaa  540
   cttgtactcg tatatccttt atcgcatatg ctatagtata atattcgaaa tttaatctca  600
   agataatttt cccaaaaggt catattaatt gaattgagat taattcctac ctaacatata  660
   tagatatatt gtgttggtgt tatcctcttt ggagttttag tcgcgcatgt attctaagaa  720
   aatggtaatt ttgaacttat attgatggtc taaattttat cctatcacta aaatttcagt  780
   catgtttcct agctaattaa tttgggttca atccaggtat tctaaataag ttttgtaaat  840
   acatgtctat tatcataatg aaacatagaa aaacccgtaa agtaaaaatg tctatatttt  900
   tagtggcaaa ttattaatgt tgctataatg aaaagagaag attcaacctt ttgtacatca  960
   taaaaaatgt tattctacat tatgctaatt ttacattaga cttcatagtt ttgaaaactt 1020
   tcaaatgaag ggacttaaat tttaaattta aagatgatgt cataaattag ttagataaaa 1080
   gttaaataat taataagaaa aggtctaatg ataccaaata aaattttctt aaaaaagtat 1140
   taatgtcatt acaattttat tttatttata taagagaaga taatagattt tcttgtataa 1200
   aaaacttacc tgtaaacttc tatataaact ttttagaaaa ataatttaaa acggtctcaa 1260
   atatcgttat ttccgaacat acaaaaaccc aaaacctaat ccatttaatt atattgtaag 1320
   acctcgatta gcttaattta ttttgaaacc tatttacaaa tgagtttggc atgatatgaa 1380
   cttttttgca tttacatatg gctgtaaacg aactgaacag ttcacgaaca attcatgaac 1440
   cgttcggcgg gaggttcgtt cgtgttagtt cgtttagtta aatgaatgaa catgaacaaa 1500
   gctctcgttc gttcatttac gttcatgaac gttcgatagt ctgttcgtga actttagttc 1560
   gtctatgttc atgaactgtc gttcgtgaac aataatttga ttatgttcat ttacacacac 1620
   atacataatc aactgtacta taaaaatagt ttaaatataa atattttttt atataatata 1680
   tattattaat atctaattat ttaagaaaaa gtttaaataa aatacttaac ttataaatat 1740
   ttcgttcgtt tgtgttcgtg tacatttgtt catgaacaca aatgaacgaa catgaacaag 1800
   tcattatgtt cgtttatctg tttgttaagt taaatgaacg aacgaacacg aacaaggtct 1860
   cgtttgtgtt cgttcggttc gtttacggcc cttaattaca tgtgaagtta ataaataaaa 1920
   ataaagttca tcaaaattaa acctatggtt ggagataaaa ccagtcagcc ttacgtgtgg 1980
   aaactatcct actctacaat taatagcacc caaagaaaat aaaccacttc cgtgttcttc 2040
   ttcatctcca tcatccaacc cctcaactac ttgtactgta tctatatcta tcattctata 2100
   tatctacttc atatttctat ctatacattc aaattaataa aaacaaaatg gtaactgaag 2160
   cagcaaacgg aaacacactc accggacaac aacaaaccac taccaccacc tccgccactt 2220
   acaaactcgt cggcttcaaa aacttcatcc gtcaaaaccc taaatccgac aaattcaccg 2280
   tcaaaaaatt ccaccacgtc gaattctggt gttccgacgc caccaacacc gcccgccgct 2340
   tctcttgggg tctcggcatg ccgcttcttt acaaatccga tctctccacc ggtaacaaca 2400
   ctcacgcttc ttaccttatt aactccggcc agctcaactt cctcttcacc gcaccttatt 2460
   ccaccaccat ctccaccacc ggttccacat cgtctattcc tactttctct cacactgctt 2520
   gccggaactt caccgccact cacggcctcg ctgtacggtc tatcgccgtt gaagttgaag 2580
   acgctgaaat cgcttattct gttagtatcg ctcacggtgc taaaccgtcg tcagctccgg 2640
   tcactctagg aaataacgac gtcgttttgg ctgaagttaa actgtacggc gatgtcgttt 2700
   tgagatatgt tagttttaaa aacactaatt atgatgttac tactaatttc ctaccgggtt 2760
   ttgagcctgt ggacgcaacg tcgtcgtttc atgaacttaa ttacggaatt cataagttag 2820
   atcacgccgt aggaaacgtg ccggagctag ctccggcagt agactacgtg aagtccttca 2880
   ctggatttca cgaatttgct gaatttacgg cagaggatgt aggaacgagt gagagcgggc 2940
   ttaattcggt tgtattagcg tgtaatagtg aaacggtttt gataccgatg aacgagccag 3000
   tttatggaac gaaaaggaag agccagatac agacgtattt ggaacataat gagggagctg 3060
   gtgtgcagca tttggcgttg gcgagtgagg atatttttag gactttgaga gagatgagaa 3120
   aacggagtag tatcggtggt tttgagttta tgccgtctcc accgcctact tattataaga 3180
   atttgaagaa tagggtcggg gacgtgttga gtgacgaaca gattaaggaa tgtgaggaat 3240
   tggggatttt ggttgataga gatgatcagg gaactttgct tcagattttc accaagcctg 3300
   ttggtgatag gaggccaact atattcatag agattattca gagagtaggg tgtatgctga 3360
   aagatgatga aggcaaggtg caacagaagc caggctgtgg aggatttggc aagggcaatt 3420
   tctcagagct cttcaaatct attgaagaat acgagaagac gctggaagca cgaaacacca 3480
   ctgaaccaac tgccactgca tgaaaactgc ctatgtataa tgtttatatc ttcttgactc 3540
   aaagaacatg attatcacca tatgtatttg tggattatgt acaattgaat gaaacaatgc 3600
   aataccatat gtgatgtgat ttatatagaa taacaataga tgtcattcat atatgccgtt 3660
   cctgttttag taattgtgtg ttggtggtgt gttgcaagtt ccaatcagct gtatataatg 3720
   cctaaatatt caatttgacc tccattaagg atccattgtg cagattcttt gtttctgtct 3780
   taaaagtgtg ggagacttga aacacatctg taaaactgaa ccagatctga atcgagctga 3840
   atggaagcgg cgcaggtctg ctctttggtt ccacttacaa caaacaattg ttcatggtac 3900
   gtcgcctctt catggccagt gtttattgta agaaagcatt gtttaggcga aaaaggaaaa 3960
   ggtgatttat gtaagaaaag gtagtgctat taaggttatg ttccccatga acaaacaggg 4020
   taaattcata caggatcaac ttttaaatga tactattaaa ggtcatctac ttctgtttcc 4080
   catgcttgtg ccaagctcaa atttagacga aaccaaaaca ggatgaaaaa actaattaac 4140
   acatatttta gtaagcaatt acagctatat atcatactat catcaacaat ctgaaaagtt 4200
   gcatacaatt tatcgtttat tcctgtcaag tgatgacatg aaaaatacca tttagtttac 4260
   acgtactcag atatgccaat aatagaggga ctttttctat atgagaacat acatgatgtt 4320
   cataattcat aatttacttg atacggcaag gtacttggta tggcgtcctt gtgcaattgt 4380
   ttttccggtc tttttgttcc ttaattcaac ggtcacaaca gcaatagcct tcccaacacg 4440
   caatgttttt gcctctatct caatctcgtc ctgtaagaaa attgcatcct taacaaaatt 4500
   ttactcaaag acgcatctat ggtacgaagt acaaaccaaa gattttcagt aagtcgtgca 4560
   aaacctttat gcatactgca actactcaaa cttcacatct tatgcctaaa atcaaagacc 4620
   ccaaagaagc cttgagttca aaatccaaga atttctgtca gctacagggg acaggttcaa 4680
   cccatttaca tacaaatggg tcaaaatggg ttatgttatt ctgtgtttct gccaaaccag 4740
   acctggcacg tactaaaact cacccattac ccaacccacc tacccagccc ccctttttac 4800
   caacatgcat tttgcttttg caaccctgtt gcttcaagca ttcaactatt caagtagcaa 4860
   gatcgaaaga acttatacaa aaagttgcca tttatgatta agtgaagcct taatgacacc 4920
   aaatcagtta ttatagcatt acaatgggag agcttcaaca gaaaagttat caagtcttct 4980
   ccaacaagat catgagctca ttttcaattt tatatcaaac ggtagcaata acgaaagtaa 5040
   atctggtggc tcaaggtaaa acctcataac ctttgatggc ataataactt ccagaccaac 5100
   taaacaccaa ggtggaagtt attactttgt gctaatgata gcagttacaa acataaaaaa 5160
   tctggtcggc ttcgttcagt tttctgaaat accaatatcg tctgacaagg tgccatcttc 5220
   atattcagag tcacttcatg atcaactctt gtggggtttc aaactcaaga acgttttaaa 5280
   tacattcatt taatattttt atctccagaa tactactcaa cactttcctg taaggtcttc 5340
   aatttcaggg ggaaaaaaac ccaaaattca aggaatcctg ctattagtgt atcgtttctt 5400
   gatagcaaag ctttcattta caatctctaa caacattttt acttcaaaat tttatcataa 5460
   tgctctttag gaaaatgttg acggtatgaa tatgagatca ggatatcaaa agaacaaaat 5520
   atttattttg ttcttaagtt gcaaaccgca caagagttga tcatgcagtg ttcgcgaatt 5580
   tgaagatggc accttgttag gcaacattgg 5610
<210> 24
   <211> 386
   <212> DNA
   <213> Euphorbia heterophylla
<400> 24
   ttgtttctgc cgaaattcga gccggtagat gaggcgtcgt cgtttccgtt ggattacggg 60
   attcgacggc tagatcacgc ggttggaaat gtaccggatc ttgctccggc ggtttcgtat 120
   gtgaagaagt tcaccggatt ccacgagttc gctgagttca cggcggagga tgtagggacg 180
   agtgagagcg gattgaactc ggtggtgttg gctaacaacg aagaaacggt actgctgccg 240
   atgaatgaac cggtgtttgg gacgaagagg aaaagtcaga tacagacgta tttggagcac 300
   aatgaaggag ctggagtaca gcatttggcg cttgtgagtg ctgatatttt caatactttg 360
   agagagatga ggaggaggag tgcgat 386
<210> 25
   <211> 2639
   <212> DNA
   <213> Euphorbia heterophylla
<400> 25
   gacaattaat aaaaaaaacg taaagactac ctttaattgg aggagaggaa aacaccacgt 60
   ttaaaaatcc cgttgttatc cgattgatga aaaaaaagat taacacgtta cgacttctcc 120
   attcaataat ccattttctt tatcttataa ataatttgaa atcccatcct cctcgttctc 180
   cgttcaccag aaaaaacaga aatgggaaaa gacacatcag ctgccgccga agcattcaag 240
   ctcgtcggat tttccaattt cgtcagaatc aatccccggt ccgaccgttt cccggtcaag 300
   cgcttccacc acgtcgagtt ctggtgctcc gacgcaacca acaccgctcg ccgcttctca 360
   tggggcctcg ggatgccgtt cgtcgccaaa tcggatctct ccaccggcaa cgtcacccac 420
   gcctcctacc tcctccgctc cggcgacctc aatttcctct tcacagctcc ctactctccc 480
   tccatagccg ccatggagaa tctctccgat actgctaccg catcaatccc tactttctcc 540
   cgcgacgttt tcctcgattt ctccgccaaa cacggcctcg ccgtccgagc tatagcaatc 600
   gaagtggagg acgctgcagt tgccttcaat actagtgttg ctcaaggcgc ggttccggtg 660
   gccggacctg tagtgctcga taatcgcgct tcggtagcgg aggttcactt gtacggcgac 720
   gtcgttttgc ggtacgtcag ttacctaaac tctgatgact gtttgtttct gccgaaattt 780
   gaggcggtag atgaggaggc gtcgtttccg ttggattacg ggatccggcg gctagatcac 840
   gcggttggaa atgtaccgga tcttgctccg gcggtttcgt atgtgaagaa gttcaccgga 900
   ttccacgagt tcgctgagtt cacggcggag gatgtaggga cgagtgagag cggattgaac 960
   tcggtggtgt tggctaacaa cgaagaaacg gtactgctgc cgatgaatga accggtgttt 1020
   gggacgaaga ggaaaagtca gatacagacg tatttggagc acaatgaagg agctggagta 1080
   cagcatttgg cgcttgtgag tgctgatatt ttcaatactt tgagagagat gaggaggagg 1140
   agtgcgattg ggggatttga gtttatgccg tctcctccgc cgacatatta ccggaatttg 1200
   aagaagagag ttggagatat tttgagtgat gaacagatta aggagtgtga agaattaggg 1260
   attctggtgg acagggatga tcaagggacc ttgcttcaga ttttcactaa acctgtggga 1320
   gataggtatt tctatcttct tcttcattgt tttcacttac aatacctttt tccattgaaa 1380
   attctttgtt tcttgtgttt ctcttatatg tgttggctgc tatactatgg ttgatagaga 1440
   attagaattt agcgtgttca aaactcaatt ctttatcttt atctttagcc ttaggtcatt 1500
   ttgggattgc tttcaaactg atttgattat gttactaatt aattagtacg aaggactaac 1560
   atgtgttgtt ctaagctaaa atatattcta taaagctaaa atatcttaat aagcaatacc 1620
   aaactagccc ttaaatgcat agttagaccc taaatttgac aacttttata acttggcgcc 1680
   tctaatttac aaatgtcttc gccaacctct tgaactggca aaagttttca ctgcaacccc 1740
   taggtgccac gtgtcacctt gtgattgatt tgacttttca aagatgtcaa aattttttat 1800
   tttcctccct ctaatcaaaa ggtgacaagt gatattgagt aggggtctgt agtgaatact 1860
   ttccccaagt tgagacatcc agaaatcgga ggcgtcatgt gataaaagtt gcttagttaa 1920
   gggagcttaa tatgtattaa gccttttcgc taccttgcca tttaaatcct tagtccaagc 1980
   aaaatataca aaaattagtt agaactttat gattcagaac ctacgtaact ttaatttgac 2040
   cagtagttct atcttatgca tgccagccat gttgactggt gaacttatat tcaattttgc 2100
   tattcaagta aatccttatc catcaatccg aatcaaaaga ttttaatgtt aggttaaact 2160
   tgtacgattc aaaacttagg ttgtactttt tgactagtag ttctattatg tatgtgtata 2220
   tataatgggg ttttttttct gtaggccaac gattttcgta gagataatac agagagtagg 2280
   gtgtatgcta aaggacgaag cagggagaga ataccagaag ggtggatgtg gcggttttgg 2340
   taagggaaac ttttcggagt tattcaaatc cattgaagaa tacgagaaaa cactggaagc 2400
   caaacgaact gcacaagcat agaggttagt agggaatttg atgataccca taaataatgg 2460
   ttgttacttg ttgccttgta ataagtttag ttaaatgatg actgtgcctg caaattggaa 2520
   tgttctctaa ttgttggttg atattgttgt aaaatgtaaa ctatgatgtt actataaagt 2580
   atagttgtgg ttatcgtgtc tcttcttttg gaatctgaag tttcgatatt tttttgggg 2639
<210> 26
   <211> 6569
   <212> DNA
   <213> Euphorbia heterophylla
<400> 26
   tgactgaaat taaattttaa ttatttgcaa agcttacggt gaaattaaac tctaaccatt 60
   aaaattttaa tgagtcaaaa ttgaccgaaa tgttcatgtc ataaatttag ataatccttt 120
   cagatatgta cgtgaaacca tgtacctaaa ttgtagtcag attgctaaac ccccttcgaa 180
   aatgaatgaa atgatatgat atgctatgct atgtatttta aggattattt tatagtaact 240
   ttgtttataa tttactttat atgatcatca tccaattaac tttcacctca ctaattcaat 300
   gattgaaatg gactaagtaa ttttacttaa taaaaaaata aaatcactat aacctacaca 360
   tatttttaaa aataacacca taatttatta aaggacattt aattgaagta gaaaattata 420
   taattactcg ctaataaatt ttcaattgga ggagaggaaa acaccacgtt taaaaatccc 480
   gttgttatcc gattgacgaa aaaaaagatt aacacgtcac gacttctcca ttcaataatc 540
   cattttcttt atcttataaa taatttgaaa tcccatcctc ctcgttctcc gttcaccaga 600
   aaaaacagaa atgggaaaag acacgtcagc cgctgccgaa gcattcaagc tcgtcggatt 660
   ttccaatttc gtcagaatca atccccggtc cgaccgtttc ccggtcaagc gcttccacca 720
   cgtcgagttc aggtgctccg acgcaaccaa caccgctcgc cgcttctcat ggggcctcgg 780
   gatgccgttc gtcgccaaat cggatctctc caccggcaac gtcacccacg cctcctacct 840
   cctccgctcc ggcgacctca atttcctctt cacagctccc tactctccct ccatagccgc 900
   catggagaat ctctccgata ctgccaccgc atcaatccct actttctccc gcgacgtttt 960
   cctcgatttc tccgccaaac acggcctcgc cgtccgagct atagccattg aagtggaaga 1020
   tgctgcgatt gctttcacta ccagcgttgc tcaaggcgcg attccggtgg ccggacctat 1080
   tgtgctcgat aatcgtgctt cagttgcgga ggtccacttg tacggcgacg tcgttttgcg 1140
   gtacgtcagt tatctaaact ccgatagttg cttgtttctg ccgaaattcg agccggtaga 1200
   tgaggcgtcg tctttcccgt tggattacgg gattcgacgg ctagatcacg cggttggaaa 1260
   tgtgccggaa ttatctccgg cggtttcgta tgtgaaacag ttcaccggat tccatgagtt 1320
   cgccgagttc acggcggagg atgtggggac gagtgagagc ggattgaact cggtggtgtt 1380
   ggctaataac gaagaaacgg ttttactacc gttgaatgaa ccggtgtatg gcacaaagag 1440
   gaaaagtcag atacaaacgt atttggagca caacgaaggg gctggagtac agcatttagc 1500
   acttgtgagt gcggatatat ttaacacttt gagagagatg aggaagagga gtggcgttgg 1560
   gggatttgag tttatgccgt ctcctccgcc cacatattac cggaatttga agaagagagt 1620
   cggggatatt ttgagcgatg aacagattaa ggaatgtgaa gaattaggga ttttggtgga 1680
   tagggatgat caagggacct tgcttcagat tttcactaaa cctgtgggag ataggtatgt 1740
   caatcaatct tgtgaaatga ttagttgttc tataattact tatgtgattt acattcttag 1800
   agtctgtaaa ttcttgtgat cataaatttg ttggcttgga taccatatta atattatcct 1860
   taatttagaa tgtcaaagtt atgttctgtc tttacaatgc cgttgaaatc gttatttatc 1920
   aagtccaagc aaacggtcct aaaatgtcct ttaacttact ggttcggaat ctcatctgtt 1980
   caatttgatt agtagttcca tcttttagta tatacgccag gcgccagaca tgttgatttg 2040
   tgagtttttg ttatttggaa tctcaaagtt gagttttttc tatctataaa aagtagctca 2100
   aatccttagt tatcaagtca aaacccaaag atcctattat taacttggat ctgaaattat 2160
   atcgctttct ggtttatttc taaatttggt ggtcacttga tttctcatct tgacaagtga 2220
   attcatctgg acatagttgg tcttttatgg gatgaatgga tgtgctgttt ttgcaggcca 2280
   accattttcg ttgaaataat tcagcgagtc gggtgtatgg tcaaggatga agcagggaga 2340
   gaataccaga agggtgggtg tggcggtttc ggtaagggaa acttctcgga gttattcaaa 2400
   tcaatcgagg aatatgagaa aacattggag gccaaacgaa ctgcagaagc agctcgagca 2460
   taaaataaag gttagtatgg aagatgatat gcatacataa ataaaggttg gctgttgttt 2520
   attacgttgt aatattctac tccaatgatg ctgttattgt ctgaaaatac cagtgcctgc 2580
   gaattggaat gttctctaat tgttaggaac aactggaatc attgtatatc gtaaactatg 2640
   gttcattaaa agttaaaaac atatatatga tatgattaat atagaattac tccctttatt 2700
   tggaatctaa agattcattt ctcagtttct ttctgggcga ttttgagcat ttctgtactt 2760
   aaattctgcg cgaatacacc caccaagccc tagctagctt ggtggttacc ttttaagggt 2820
   gttggaaacc taaattagaa aagatttcag tttaatttat gtcattttat atataaaagt 2880
   ttgaaacttt tttattcaaa ataagttgat tgataaattg aaatcaatta gctttgtagc 2940
   agatgttcac ccaaatctaa tataaatata ttataaatct aatatatttc tgttaacaaa 3000
   cttataatat attaataaat acaattgcaa agacaaagag acttatatta gaattataac 3060
   ataatttttt ataaatatat tagatacgta tatcttataa ttgtaacata taaaagattt 3120
   tatagtaaat atgtgttata agtctaaaat aagccttttt aagtttaata caagtctctt 3180
   tgtgtaacac cccgggttcg accgattgac ccccacaaac caacacaagt ctttctagta 3240
   agctttgcct tgagattgtt ccaagtcaag gacgctcaac tttggagttc tcccatataa 3300
   gctcctgaaa agaaggtgta acttgttggt ataggcagca gtaccaatca aaccttttta 3360
   agtccctttc cactttaatg tccctttcta ttgatataat tttattgact ttatgtaatc 3420
   aaatgtttat tcatcccgtg tttttttatt cattatgttt taatttttta attagtcaaa 3480
   atataatatc taaaaaatct atcaaatttg ggctcggctc ggcttgactc attaacatcc 3540
   gactctctac ctcttacttt tattaaatac atttgtgttt ttcagttgta cgttccttct 3600
   catatttaca tttccctttt ttcttttctt tttaatttat tttttattta tttcgcaaat 3660
   cgttgatttt ctcggtatgc tgctttataa ttttttgttc gtctcgtttt tatcttcgaa 3720
   agtatattca tgttgatata ctttttcaag ttcttatttt caaagatatg taacaaatga 3780
   cataaacatt atccagcaaa aatcagtaaa ttactagcat ttcaccaaca aaatactagc 3840
   attttacccg tgacgaaaag tttgaaaaaa atatataaaa aaaatcaaac aaaaatcatt 3900
   tttataccaa cattatacca tgtatgaaaa aataaaactg atcataaaca taaagaaatc 3960
   aaacaaaatt caccttatct taatatttta ccaagagtga aggaccaaac ataaacatgt 4020
   tttacgaaaa aaatcaaaca aaaatcattt cacactagca ttttattaac aaaatctcga 4080
   tatattatca gtaaaatacc aacattttat caacaaaata ccatcatttg agaggaggtt 4140
   cgagtgcgcc ttcgccgttc tgcggcggct tctttgacgg tggagaaagc atagctgcgt 4200
   gatggaggca gaagaggggt tgatgaaatt gggttggatt gaagtggaat tgagtcaatg 4260
   aggctaggtg atagggtgag atgatgcatg actcgaattg gtggttgatc gcagaggtgg 4320
   aggtggcgat tggccggcgg agaagacggc aaatagatgt ttttgggcgg agtacaaagc 4380
   gccttaaatc ctagcaaagg ctgatgaaaa taaaaaaggg taatgctgca cttttatcta 4440
   ctttgtgcaa tatctatata cagtatttat tatcatctta ttccttttat ttatttcttt 4500
   catcttttat gggagttgaa attctttcta gatattcttt tctttttgct aaatttttct 4560
   gctcattgtc atacacttgc ttggtaaact gaatctcaaa ccaaataatg aaatctgaag 4620
   tatgtatcca cagaatcgca atgctcatcg acggaagtag aaggggaaaa ctgctgttgg 4680
   agttgaggta tataatctgc agttgaaatc gacgttgtgt atgtaatttt gaattgaaca 4740
   agagcgaaac ctagttttct atgatctgta cctggcgtat aagaattcca tcaatatcgg 4800
   cagggcgatg cgttaatatt gcattaacac cggtggtgag aagggagcta attgcttgaa 4860
   atccgctggc atgccgcaat gttgatatgt cacataaatg cgactaagtg tgacttatct 4920
   tgaagaggag tgaaattggg gaaacgacta agagtgactt atcaagaagt gtgattacca 4980
   tgcaactaag tgcgacacgg tgaaacgact cgactcctgc agccaccacc aaatttcaca 5040
   atttcatata ggtaaaaggg agacgatcag aaagagggga aacaaaacac aaagagaggg 5100
   aaaagaaatg ttggagggac tgaaattata gaaggggtaa aattagaaac aaaaaaaatt 5160
   ataaggtgtt gtaagtagaa attaaattac gagcaaaagt tggttgaaaa taaaaatgaa 5220
   gatattgtga taaattattt cttttttttt atatttgacc aacttacatt gtaaagcctg 5280
   taatttttat taatttcgca ttatagccat aaaaagttat tccaatctat gaagttgtga 5340
   ctttgcggaa catattttcc gaaaaactta tttttcaagt tttttaaata gtaagaaaaa 5400
   ggaaatttta aattataata acataatagg tagtaaatat aaatagtata tatttataat 5460
   tcaaataatt atcaggaaat cttatttatt ttatgaaatt tctaggtaaa attaatttag 5520
   tcatgactaa gtgttgtttc tcacaaaatt gtaatcataa tcaaactaaa ttttgatatc 5580
   gagaaacaca aaatgagcat ttgttgaaag agacttatag gtcgagtctt aacaaacttg 5640
   ataagattat aaaattgtct agcataaact cctcatgcag cgaaacaata atgtatgatt 5700
   acaatcataa gccaatgttc atgttggata aaccacacga taactaattc tagaatctct 5760
   caagagtcta aataatttct atgcacactt ttaaatttat ttaagcttca tagtgaaata 5820
   ttagcttgtg ctacactaat ttacattgta cgttattttc aatggaatga caactttatt 5880
   ttgtgcccat agtgcataac agtaaaattt cagttcaatg atttcatata ttatttttta 5940
   tattacgaag ttttaggttt ttggatttta gattacaatc gagaccatag caagagttga 6000
   aacatgcaaa tgagatttaa gcttaaaaaa atgttataaa attattaaca tttagcactt 6060
   atagtagcta caaaaatttg aaggattgaa tatttctaaa tattacaaag tataaactta 6120
   aatatcaaca aatatgattc atggagtttg agttaagtat aatttaacaa tatatcaatg 6180
   atcgagtttt ttgcaaaaat cataaatatt agctcaattt gaagaactta acaaagcact 6240
   caaaacttta attgaaaaca aaaatttaag gttaagggct tatatatata aaaaaaaatt 6300
   caaagtatga aagcaatttc ggacttcaat tttataattt tttgattcat aacgaactat 6360
   taaattattg ataaatatac ttctgcatag agtccaaaaa attataaggc cggcctaata 6420
   aagcctatga tagaatccta tagaaacatt gttgacccaa gcacaggccg agtgaactcg 6480
   tggctgagtt tctatgctaa gagttttgat catgtgtaag aataaactaa gtaagaataa 6540
   cattaaataa ataattgaat ctttacata 6569
<210> 27
   <211> 1567
   <212> DNA
   <213> Xanthium strumarium
<400> 27
   agaaataaaa gacacatatg ttcgtgaggt tatgacacct ttggttgatg ttgtagccag 60
   tgattctagt gcaacactaa tcgatttcca caccttgtgg gtaagtcatc aatattcaag 120
   ggtacctgtt tttgaacaac gtgttgataa tatagtcggt attgcatatg caatggatct 180
   attagactat gttcaaaagg gagacctttt agaaagtacc acggtggggg atatggctca 240
   taaacctgct tattttgtgc ctgattcaat gtcggtatgg aatcttctta gagaatttcg 300
   cattaggaag gtacacatgg ctgttgtcct gaacgaatac ggtgggacag ttggaattgt 360
   aacactagaa gacgtggttg aagaaattgt tggtgaaatt tcttcaaccc acgcctctta 420
   tctccttaaa tccggccaac ttaacttcct cttcaccgcc ccttattccc cttccatctc 480
   atccaccaca accaccgcct ccatcccttc cttttctcac tccacctgtc gccacttcac 540
   ctcctcccac ggcctcgctg ttcgtgccat cgccgttgaa gtccatgacg ccgaactcgc 600
   tttctccgtt agcgtcgctc acggcgctaa agcctccgga taccataatc cagctcttca 660
   aacgacgacg tttctccaca gcttcaaacc caggcaaaac atattgttat tattattatt 720
   gtttgtaact aatataacgc aaaacgacgt cgccgtagag ttgaacttct gataagacga 780
   cgtcgttatg accgagggtg ataatccgga gggttgacca cgcagtgggg aacgtgccgg 840
   agttagcacc ggcagtggaa tatataaaat catttactgg atttcacgag tttgctgagt 900
   ttacggcgga ggatgtggga acgagtgaga gtggactaaa ctcggtggtt ttggcttgca 960
   atagtgagat ggtattgata ccgatgaatg aaccggttta cgggacgaag aggaagagtc 1020
   agatacagac gtatttggaa cataatgaag gggcgggggt tcagcatttg gcgttggcta 1080
   gtgaggatat atttaggacg ttaagggaga tgaggaaaag gagtggggtt ggtggctttg 1140
   agtttatgcc gtctccccct cctacttatt ataggaattt gaagaatagg gtgggcgatg 1200
   tgttgtctga tgaacagatt aaggagtgtg aagaattggg gatattggtt gatagagatg 1260
   atcaggggac tttgcttcag atttttacca agcctgttgg tgacaggccg acgatattca 1320
   tagagataat tcagagagta gggtgtatgg tgaaggataa tgaaggaaag gagcagcaga 1380
   aggcagggtg tggagggttt ggcaaaggga acttctcaga gctttttaaa tccattgagg 1440
   aatatgagaa gacattggaa gcaagagcca ccactgaagc cactgctgct gcatgaaaac 1500
   cacccatgaa taatcttcat gagattttat aatctaatga ttatgtatct gtggattcta 1560
   tacgaac 1567
<210> 28
   <211> 892
   <212> DNA
   <213> Digitaria sanguinalis
<400> 28
   ttgtgaagtt ggtgtcatat atttcaagat ctgttcgtat agaatccaca gatacataat   60
   cattagatta taaaatctca tgaagattat tcatgggtgg ttttcatgca gcagcagtgg  120
   cttcagtggt ggctcttgct tccaatgtct tctcatattc ctcaatggat ttaaaaagct  180
   ctgagaagtt ccctttgcca aaccctccac accctgcctt ctgctgctcc tttccttcat  240
   tatccttcac catacaccct actctctgaa ttatctctat gaatatcgtc ggcctgtcac  300
   caacaggctt ggtaaaaatc tgaagcaaag tcccctgatc atctctatca accaatatcc  360
   ccaattcttc acactcctta atctgttcat cagacaacac atcgcccacc ctattcttca  420
   aattcctata ataagtagga gggggagacg gcataaactc aaagccacca accccactcc  480
   ttttcctcat ctcccttaac gtcctaaata tatcctcact agccaacgcc aaatgctgaa  540
   cccccgcccc ttcattatgt tccaaatacg tctgtatctg actcttcctc ttcgtcccgt  600
   aaaccggttc attcatcggt atcaatacca tctcactatt gcaagccaaa accaccgagt  660
   ttagtccact ctcactcgtt cccacatcct ccgccgtaaa ctcagcaaac tcgtgaaatc  720
   cagtaaatga ttttatatat tccactgccg gtgctaactc cggcacgttc cccactgcgt  780
   ggtcaagcct ccggatacca taatccagct cttcaaacga cgacgttttc tccacagctt  840
   caaacccagg caaaaacata ttgttattat tattattgtt tttgtaacta at          892
<210> 29
   <211> 975
   <212> DNA
   <213> Digitaria sanguinalis
<400> 29
   gccgccaccg cctccctccc ctccttctcc gcccccgccg cgcgccgctt cgcctccgac 60
   cacggcctcg ccgtgcgcgc cgtagcgctc cgcgtcgccg acgccgagga cgccttccgc 120
   gccagcgtcg ccaacggggc gcgcccggcg ttcgagcccg tcgagctcgg cctcggcttc 180
   cgcctcgccg aggtcgagct ctacggcgac gtcgtgctcc gctacgtcag ctacccggac 240
   gccgcggatt tgcccttcct gccgggcttc gaggacgtcg tcatcagcaa cccaggggcg 300
   gtggactacg ggctgaggcg cttcgaccac atcgtcggca acgtctcgga gctggcgccg 360
   gtggccgcgt acgtcgccgg attcacgggg ttccacgagt tcgccgagtt cacggcggag 420
   gacgtgggca cggcggagag cgggctcaat tccgtggtgc tcgccaacaa ctccgagaac 480
   gtgctgctcc cgctcaacga gccggtgcac ggcaccaagc gccgcagcca gatacagaca 540
   tacctggacc accacggcgg ccctggagtg cagcacatcg cgctggctag cgacgacgtg 600
   ctcaggacac tgcgggagat gcaggcgcgc tccgccatgg gtgggtttga gttcatggag 660
   gctccgccac ccacttacta tgagggtgtg aggcggcgcg ccggggacgt gctctcggag 720
   gagcagataa aggagtgcca gaaactgggg gtgctggtgg acagggatga ccagggagtg 780
   ttgctccaaa tcttcaccaa gccagtgggg gacccaacgt ttttcttgga gataatccaa 840
   aggattgggt gcatggagaa ggatgagcag ggaaaggact accagaaggg cggctgtggc 900
   gggtttggca agggaaactt ctcacagctg ttcaagtcta ttgaggagta tgagaagtcc 960
   cttgaagcga agcaa 975
<210> 30
   <211> 1266
   <212> DNA
   <213> Kochia scoparia
<400> 30
   accaccgcct caaccgagtt caagctggtg ggttactcca acttcgtccg agttaacccc   60
   atgtccgacc tcttctccgt caaaaaattc caccacatcg agttctggtg cggcgacgcg  120
   accaacgtca gccgtcgctt ctcatggggt ctaggcatgc cggcggtcgc taaatccgac  180
   ctctccaccg gaaactccgt acacgcttcg tacctccttc gctcaggcga cctctccttc  240
   ctcttcacct ccccgtactc cccttctctc tcctccccct cttccgctgc aatacccacg  300
   tttgatttct ctctcttctc ctcttttatc acctcccacg gcatcggggt tcgcgccgtt  360
   gcactcgagg tcgacgatgc cgaggttgct ttcaatacga gcgtctccca cggcgcaatc  420
   cctggttctc ccccgattaa gctcggaaac ggcgtcgttt tgtccgaggt cagcctctac  480
   ggcgacgtcg ttcttcgcta cgtgagctac ggaggtgaga cagagaaagc ggatacgaat  540
   tcgaattcat ggtttcttcc tgggttcgag gaaatgccgg aggaatcgtc gtttcgaggg  600
   ctcgatttcg ggctccggag actagaccat gcggtgggga atgtcccgga gctagggaaa  660
   gcaatcgagt atgtgaagaa gttcactggg tttcacgaat ttgctgagtt tacagctgac  720
   gatgttggga cgagtgaaag tgggctgaat tcggctgtgc tggcgaacaa ctcggagacg  780
   gtgttgattc cgatgaacga gccggtttac gggacgaaga ggaagagtca aattcagaca  840
   tacttggagc acaatgaagg agctggggtt cagcatttgg cattgatgag tgaggatata  900
   ttctggactc ttagggagat gaggaagcgg agcgggctcg gtgggttcga gtttatgcca  960
   gcaccgccgc ctacgtatta tcggaacctg aggaatcgtg tcggggatgt gttgagtgaa 1020
   gaacagatga aggagtgtga agagttgggg atattggttg ataaggatga tcagggcact 1080
   ctgctccaga tttttactaa gcatattggt gacccaacta tgttcattga gattatccaa 1140
   agaattggct gcatgatgaa agatgaagaa ggcaaggtgt accaaaaggg aggctgtgga 1200
   ggatttggga agggaaactt ttcagagctt ttcaaatcta tcgaagagta tgagaagaca 1260
   cttgaa                                                            1266
<210> 31
   <211> 783
   <212> DNA
   <213> Lolium multiflorum
<400> 31
   ctcggccacg ggtttggctt cgcggaggtg gagctagccg gggacagcgt tctccgcttc   60
   gtgagctacc cggacggcac cgacgtgtcc ttcctgccgg ggttccagga cgtggcgagc  120
   gccggcgggg cgccggactt cgggctcacc cggtttgacc acgtcgacgt taacatcccg  180
   gagctggcac ccgtcgccgc caatgttgcc ggcttcaccg ggttccacaa attctgggag  240
   ttcaccgcgg acgacgtgtg cccggaagag agcggggtga acggcgtggt gatcgccaac 300
   aactcagaga acgtgctgct cagtatcttg gagccggtgt tcggcaccaa gctgcggagc 360
   catgtcgaga cgttcctgga ccaccacggt ggcccgggca tacagcacct ggcaatgacc 420
   agccacgaca tccttggcgc gctcaggaaa atccgagctc ggtcctccat gggcgggttt 480
   gagctcctgc cgccgccgcc ggccagctac tatgacggtg taaggcagcg cgccggggac 540
   gtgctgtcgg aagaacagat caaggagtgc caagagctgg gcgtgcgggt ggacagaggg 600
   tatgaggacg gagttgtgct ccaagtcttc accaaaccgg cgggagaccc aaccttactg 660
   ttagagttta tccaaagaat cgggtgcatg gtcaaggacg agaaccagca ggaataccag 720
   agaggtggat gtggcgggtt tgccaaaggg aacgtttctg aactcatcaa ggacattgag 780
   gac 783
<210> 32
   <211> 1041
   <212> DNA
   <213> Lolium multiflorum
<400> 32
   accgatcgct tccacgtgat ggatttccac cacgtcgagt tttggtgcgc cgatgccgcc 60
   tcggccgccg cacggttctc cttcgggctc ggcgtgccac tcgccgcgca gtccgacctc 120
   tccacgggga acactgcgca cgcctcacgc ctactacgcg cacgctcggg ctctctctcg 180
   ttcctcttca ccgcgccgta cgcgccgcac gtcgccgact cggcgaccac cgcgtccctg 240
   ccctccttct cggcggacgc cgcgcggcgc ttcacgggca cccacggcgg cctggccgtg 300
   cgtgccgtgg ccgtccgcgt cgctgacgcg gccgaggcct tcgtcgcgag cgtggacgcc 360
   ggagcgcggc cagcctgcgc cccgactgat ctcggccacg ggtttggctt cgcggaggtg 420
   gagctagccg gggacagcgt tctccgcttc gtgagctacc cggacggcac cgacgtgtcc 480
   ttcctgccgg ggttccagga cgtggcgagc gccggcgggg cgccggactt cgggctcacc 540
   cggtttgacc acgtcgacgt taacatcccg gagctggcac ccgtcgccgc caatgttgcc 600
   ggcttcaccg ggttccacaa attctgggag ttcaccgcgg acgacgtgtg cccggaagag 660
   agcggggtga acggcgtggt gatcgccaac aactcagaga acgtgctgct cagtatcttg 720
   gagccggtgt tcggcaccaa gctgcggagc catgtcgaga cgttcctgga ccaccacggt 780
   ggcccgggca tacagcacct ggcaatgacc agccacgaca tccttggcgc gctcaggaaa 840
   atccgagctc ggtcctccat gggcgggttt gagctcctgc cgccgccgcc ggccagctac 900
   tatgacggtg taaggcagcg cgccggggac gtgctgtcgg aagaacagat caaggagtgc 960
   caagagctgg gcgtgcgggt ggacagaggg tatgaggacg gagttgtgct ccaagtcttc 1020
   accaaaccgg cgggagacag g 1041
<210> 33
   <211> 200
   <212> DNA
   <213> Abutilon theophrasti
<400> 33
   tccataatcc aacgcagaca tttcagagat tcatccccga agaaatcaaa aatccctcaa 60
   caaaatgatt cagatatgct taaacaaaat atgaattgag cacaatatca tatttgacat 120
   ttatgttgaa gtagcaaagg ttcgtatagt ttatgtatgt gcaattaaaa ctaacagtaa 180
   cggaattcat tctgacactc 200
<210> 34
   <211> 200
   <212> DNA
   <213> Abutilon theophrasti
<400> 34
   gagtgtcaga atgaattccg ttactgttag ttttaattgc acatacataa actatacgaa 60
   cctttgctac ttcaacataa atgtcaaata tgatattgtg ctcaattcat attttgttta 120
   agcatatctg aatcattttg ttgagggatt tttgatttct tcggggatga atctctgaaa 180
   tgtctgcgtt ggattatgga 200
<210> 35
   <211> 200
   <212> DNA
   <213> Abutilon theophrasti
<400> 35
   agtaacggaa ttcattctga cactccctat atgctgctag caactacctc gatctctcaa 60
   gcctcgacaa cggtggtgga ttctgtagat tgtttggctt caagagactt ctcgtattct 120
   tcaatggatt tgaagagctc ggaaaagttg cctttcccga aacctccgca tcctcctttc 180
   tggtattgct ttccttcttc 200
<210> 36
   <211> 200
   <212> DNA
   <213> Abutilon theophrasti
<400> 36
   gaagaaggaa agcaatacca gaaaggagga tgcggaggtt tcgggaaagg caacttttcc 60
   gagctcttca aatccattga agaatacgag aagtctcttg aagccaaaca atctacagaa 120
   tccaccaccg ttgtcgaggc ttgagagatc gaggtagttg ctagcagcat atagggagtg 180
   tcagaatgaa ttccgttact 200
<210> 37
   <211> 200
   <212> DNA
   <213> Abutilon theophrasti
<400> 37
   ctttctggta ttgctttcct tcttcatcct tcaccatgca ccctaatctc tgtattattt 60
   ctatgaatat cgtaggccta tctccaatgg gcttagtgaa aatctgaagc agagtgcctt 120
   gatcatctct gtcaaccaaa atccccaact cttcacactc cttaatctgc tcatcgctca 180
   aaatgtcccc tgccctttgc 200
<210> 38
   <211> 200
   <212> DNA
   <213> Abutilon theophrasti
<400> 38
   gcaaagggca ggggacattt tgagcgatga gcagattaag gagtgtgaag agttggggat   60
   tttggttgac agagatgatc aaggcactct gcttcagatt ttcactaagc ccattggaga  120
   taggcctacg atattcatag aaataataca gagattaggg tgcatggtga aggatgaaga  180
   aggaaagcaa taccagaaag                                              200
<210> 39
   <211> 200
   <212> DNA
   <213> Amaranthus graecizans
<400> 39
   cttcttcccc gtctgagttt ttattacttc actttctctc tcatcatcta acatgggaac   60
   tttgaaaccc gaaactcaac ccgactccga attcaaactc gtgggttact ccaacttcat  120
   tcgggttaac cccaaatctg accgttttac tgttaagcgt ttccatcata tagagttctg  180
   gtgtggcgat gcaaccaatg                                              200
<210> 40
   <211> 200
   <212> DNA
   <213> Amaranthus graecizans
<400> 40
   cattggttgc atcgccacac cagaactcta tatgatggaa acgcttaaca gtaaaacggt   60
   cagatttggg gttaacccga atgaagttgg agtaacccac gagtttgaat tcggagtcgg  120
   gttgagtttc gggtttcaaa gttcccatgt tagatgatga gagagaaagt gaagtaataa  180
   aaactcagac ggggaagaag                                              200
<210> 41
   <211> 200
   <212> DNA
   <213> Amaranthus graecizans
<400> 41
   ttctggtgtg gcgatgcaac caatgttagc agacgctttt cttggggtct tggaatgcct   60
   accgttgcta aatctgacct ttctactgga aactctgttc acgcttcttt tcttcttcgt  120
   tccggtgacc tttctttcct ctttacttca ccttactctc ctaccatgtc catcccttct  180
   tctgctgcaa tcccctcgtt                                              200
<210> 42
   <211> 200
   <212> DNA
   <213> Amaranthus graecizans
<400> 42
   aacgagggga ttgcagcaga agaagggatg gacatggtag gagagtaagg tgaagtaaag    60
   aggaaagaaa ggtcaccgga acgaagaaga aaagaagcgt gaacagagtt tccagtagaa   120
   aggtcagatt tagcaacggt aggcattcca agaccccaag aaaagcgtct gctaacattg   180
   gttgcatcgc cacaccagaa                                               200
<210> 43
   <211> 200
   <212> DNA
   <213> Amaranthus graecizans
<400> 43
   cttcttctgc tgcaatcccc tcgtttgatt tcaatcattt taccaaattt gttacatcgc    60
   acggtcttgg cgtgcgtgct gttgccgtcg aagtagaaga cgcagaagct gcttttaata   120
   tcagcgtttc gcacggggct atcccctgtg tttctcctat tcacttggaa aacggtgtcg   180
   ttttatctga ggttcattta                                               200
<210> 44
   <211> 200
   <212> DNA
   <213> Amaranthus graecizans
<400> 44
   taaatgaacc tcagataaaa cgacaccgtt ttccaagtga ataggagaaa cacaggggat    60
   agccccgtgc gaaacgctga tattaaaagc agcttctgcg tcttctactt cgacggcaac   120
   agcacgcacg ccaagaccgt gcgatgtaac aaatttggta aaatgattga aatcaaacga   180
   ggggattgca gcagaagaag                                               200
<210> 45
   <211> 200
   <212> DNA
   <213> Amaranthus graecizans
<400> 45
   tgtcgtttta tctgaggttc atttatatgg ggatgttgtg cttcgttatg taagctacgg 60
   aaatgaatgt ggggatgtgt tttttcttcc tgggtttgag caaatgcccg aggaatcctc 120
   gtttcgagga cttgatttcg gccttcgaag gttggatcat gctgtaggga atgtccctga 180
   gttggctcct gcaattgctt 200
<210> 46
   <211> 200
   <212> DNA
   <213> Amaranthus graecizans
<400> 46
   aagcaattgc aggagccaac tcagggacat tccctacagc atgatccaac cttcgaaggc 60
   cgaaatcaag tcctcgaaac gaggattcct cgggcatttg ctcaaaccca ggaagaaaaa 120
   acacatcccc acattcattt ccgtagctta cataacgaag cacaacatcc ccatataaat 180
   gaacctcaga taaaacgaca 200
<210> 47
   <211> 200
   <212> DNA
   <213> Amaranthus graecizans
<400> 47
   cctgagttgg ctcctgcaat tgcttatttg aagaagttta ctgggtttca tgagtttgct 60
   gagtttacag ctgaagatgt tggaacgagt gaaagtgggt tgaattcagc cgtattggca 120
   aataatgatg aaatggtgtt gtttccaatg aatgaacctg tgtatgggac aaaaaggaag 180
   agtcaaattc aaacttattt 200
<210> 48
   <211> 200
   <212> DNA
   <213> Amaranthus graecizans
<400> 48
   aaataagttt gaatttgact cttccttttt gtcccataca caggttcatt cattggaaac 60
   aacaccattt catcattatt tgccaatacg gctgaattca acccactttc actcgttcca 120
   acatcttcag ctgtaaactc agcaaactca tgaaacccag taaacttctt caaataagca 180
   attgcaggag ccaactcagg 200
<210> 49
   <211> 200
   <212> DNA
   <213> Amaranthus graecizans
<400> 49
   ggaagagtca aattcaaact tatttggagc ataatgaagg agctggtgta caacatttgg 60
   ctttgatgag tgaagatata ttttggactt taagggagat gaggaagaga agtggtcttg 120
   gtgggtttga gtttatgccg tcgccgcctc cgacttatta ccggaatttg aggaacagag 180
   ctgctgatgt attgagtgag 200
<210> 50
   <211> 200
   <212> DNA
   <213> Amaranthus graecizans
<400> 50
   ctcactcaat acatcagcag ctctgttcct caaattccgg taataagtcg gaggcggcga 60
   cggcataaac tcaaacccac caagaccact tctcttcctc atctccctta aagtccaaaa 120
   tatatcttca ctcatcaaag ccaaatgttg tacaccagct ccttcattat gctccaaata 180
   agtttgaatt tgactcttcc 200
<210> 51
   <211> 200
   <212> DNA
   <213> Amaranthus hybridus
<400> 51
   tcttcctggg tttgaggaaa tgccggagga atcgtcgttt cgaggacttg atttcggcct 60
   tcgaaggttg gatcatgctg tagggaatgt tcccgagttg gctcctgcaa ttgcttattt 120
   gaagaagttt actgggtttc atgagtttgc tgagtttaca gctgaagatg ttgggacgag 180
   tgaaagtggg ttgaattcag 200
<210> 52
   <211> 200
   <212> DNA
   <213> Amaranthus hybridus
<400> 52
   ctgaattcaa cccactttca ctcgtcccaa catcttcagc tgtaaactca gcaaactcat 60
   gaaacccagt aaacttcttc aaataagcaa ttgcaggagc caactcggga acattcccta 120
   cagcatgatc caaccttcga aggccgaaat caagtcctcg aaacgacgat tcctccggca 180
   tttcctcaaa cccaggaaga 200
<210> 53
   <211> 200
   <212> DNA
   <213> Amaranthus hybridus
<400> 53
   acgagtgaaa gtgggttgaa ttcagccgta ttggcaaata atgatgaaat ggtgttgttt 60
   ccaatgaatg aacctgtgta tgggacaaaa aggaagagcc aaattcaaac ttatttggag 120
   cataatgaag gagctggtgt acaacatttg gctttgatga gtgaagatat attttggact 180
   ttaagggaga tgaggaagag 200
<210> 54
   <211> 200
   <212> DNA
   <213> Amaranthus hybridus
<400> 54
   ctcttcctca tctcccttaa agtccaaaat atatcttcac tcatcaaagc caaatgttgt 60
   acaccagctc cttcattatg ctccaaataa gtttgaattt ggctcttcct ttttgtccca 120
   tacacaggtt cattcattgg aaacaacacc atttcatcat tatttgccaa tacggctgaa 180
   ttcaacccac tttcactcgt 200
<210> 55
   <211> 200
   <212> DNA
   <213> Amaranthus palmeri
<400> 55
   agatgtgtat aagagacagg tcttggcgtg cgtgctgttg ccgtcgaagt agaagacgcc 60
   gaagctgctt ttaatatcag cgtttcgcac ggggctatcc cctgtgtttc tcctattcac 120
   ttggaaaacg gtgtcgtttt atctgaggtt catttatatg gggatgttgt gcttcggtat 180
   gtaagctacg gaaatgaatg 200
<210> 56
   <211> 200
   <212> DNA
   <213> Amaranthus palmeri
<400> 56
   cattcatttc cgtagcttac ataccgaagc acaacatccc catataaatg aacctcagat 60
   aaaacgacac cgttttccaa gtgaatagga gaaacacagg ggatagcccc gtgcgaaacg 120
   ctgatattaa aagcagcttc ggcgtcttct acttcgacgg caacagcacg cacgccaaga 180
   cctgtctctt atacacatct 200
<210> 57
   <211> 200
   <212> DNA
   <213> Amaranthus palmeri
<400> 57
   ggtatgtaag ctacggaaat gaatgtgggg atgtgttttt cttcctgggt ttgaggaaat 60
   gccggaggaa tcatcgtttc gaggacttga ttttggcatt cgaaggttgg atcatgctgt 120
   agggaatgtc cctgagttgg ctcctgcaat tgcttatttg aagaagttta ctgggtttca 180
   tgagtttgct gagtttacag 200
<210> 58
   <211> 200
   <212> DNA
   <213> Amaranthus palmeri
<400> 58
   ctgtaaactc agcaaactca tgaaacccag taaacttctt caaataagca attgcaggag 60
   ccaactcagg gacattccct acagcatgat ccaaccttcg aatgccaaaa tcaagtcctc 120
   gaaacgatga ttcctccggc atttcctcaa acccaggaag aaaaacacat ccccacattc 180
   atttccgtag cttacatacc 200
<210> 59
   <211> 200
   <212> DNA
   <213> Amaranthus palmeri
<400> 59
   tttcatgagt ttgctgagtt tacagctgaa gatgttggga cgagtgaaag tggattgaat 60
   tcagccgtat tggcaaacaa tgatgaaatg gtgttgtttc caatgaatga acctgtgtat 120
   gggacaaaaa ggaagagcca aattcaaact tatttggagc ataatgaagg agctggtgta 180
   cagcatttgg ctttgatgag 200
<210> 60
   <211> 200
   <212> DNA
   <213> Amaranthus palmeri
<400> 60
   ctcatcaaag ccaaatgctg tacaccagct ccttcattat gctccaaata agtttgaatt 60
   tggctcttcc tttttgtccc atacacaggt tcattcattg gaaacaacac catttcatca 120
   ttgtttgcca atacggctga attcaatcca ctttcactcg tcccaacatc ttcagctgta 180
   aactcagcaa actcatgaaa 200
<210> 61
   <211> 200
   <212> DNA
   <213> Amaranthus palmeri
<400> 61
   gtgtacagca tttggctttg atgagtgaag acatattttg gactttaagg gagatgagga 60
   agagaagtgg tcttggtggg tttgagttta tgccgtcgcc gcctccgact tattaccgga 120
   atttgaggaa cagagctgct gatgtattga gtgaggagca gatgaaggag tgtgaagagt 180
   tggggatttt ggtggataaa 200
<210> 62
   <211> 200
   <212> DNA
   <213> Amaranthus palmeri
<400> 62
   tttatccacc aaaatcccca actcttcaca ctccttcatc tgctcctcac tcaatacatc 60
   agcagctctg ttcctcaaat tccggtaata agtcggaggc ggcgacggca taaactcaaa 120
   cccaccaaga ccacttctct tcctcatctc ccttaaagtc caaaatatgt cttcactcat 180
   caaagccaaa tgctgtacac 200
<210> 63
   <211> 200
   <212> DNA
   <213> Amaranthus palmeri
<400> 63
   agagttgggg attttggtgg ataaagatga tcagggtact ttgcttcaaa tcttcaccaa 60
   acctattgga gacaggccaa ccatattcat cgagattatc caaagaattg gttgcatgat 120
   gaaagatgaa gacggcaaga tgtaccaaaa gggtggttgc ggaggattcg gaaagggaaa 180
   cttttcagag ctgttcaaat 200
<210> 64
   <211> 200
   <212> DNA
   <213> Amaranthus palmeri
<400> 64
   atttgaacag ctctgaaaag tttccctttc cgaatcctcc gcaaccaccc ttttggtaca   60
   tcttgccgtc ttcatctttc atcatgcaac caattctttg gataatctcg atgaatatgg  120
   ttggcctgtc tccaataggt ttggtgaaga tttgaagcaa agtaccctga tcatctttat  180
   ccaccaaaat ccccaactct                                               200
<210> 65
   <211> 200
   <212> DNA
   <213> Amaranthus palmeri
<400> 65
   ggaaactttt cagagctgtt caaatcaata gaggagtacg agaagactct tgaacgtaaa   60
   caggttccag atacagctgc tgcatgatga gcagactaaa atattgttgt cttgctgatg  120
   aaatgataga aaaggtttgt ttcttggtac aatgctcaac ttcaaaattt tctttattaa  180
   ataatgaagt gtaaacttat                                               200
<210> 66
   <211> 200
   <212> DNA
   <213> Amaranthus palmeri
<400> 66
   ataagtttac acttcattat ttaataaaga aaattttgaa gttgagcatt gtaccaagaa   60
   acaaaccttt tctatcattt catcagcaag acaacaatat tttagtctgc tcatcatgca  120
   gcagctgtat ctggaacctg tttacgttca agagtcttct cgtactcctc tattgatttg  180
   aacagctctg aaaagtttcc                                               200
<210> 67
   <211> 200
   <212> DNA
   <213> Amaranthus palmeri
<400> 67
   attaaataat gaagtgtaaa cttatacaaa ctgtgtcata tatggtgatt ggtgatcatg   60
   gtttatgtag aatgtataac ataattgata atctgtgtat atgctgaata cttacatact  120
   gtgaaatcat tctgatggaa acatacaatt ggtcagtagc tgaggctggt agctcctcag  180
   attagttttt ttcagtcaaa                                               200
<210> 68
   <211> 200
   <212> DNA
   <213> Amaranthus palmeri
<400> 68
   tttgactgaa aaaaactaat ctgaggagct accagcctca gctactgacc aattgtatgt 60
   ttccatcaga atgatttcac agtatgtaag tattcagcat atacacagat tatcaattat 120
   gttatacatt ctacataaac catgatcacc aatcaccata tatgacacag tttgtataag 180
   tttacacttc attatttaat 200
<210> 69
   <211> 200
   <212> DNA
   <213> Amaranthus palmeri
<400> 69
   gagttcaaga ttaaaagtta aattacattt atgtgggttt tatataggca atgattgcat 60
   tatattgttt ttcttttggt gggaagattt tcctttttaa aaaattttaa tttccctaca 120
   ttttcaaatg atggaggaat ttttttgtgc atgtaaaatg ttttctctct attttttgat 180
   ttatgctatt ttttctcttt 200
<210> 70
   <211> 200
   <212> DNA
   <213> Amaranthus palmeri
<400> 70
   aaagagaaaa aatagcataa atcaaaaaat agagagaaaa cattttacat gcacaaaaaa 60
   attcctccat catttgaaaa tgtagggaaa ttaaaatttt ttaaaaagga aaatcttccc 120
   accaaaagaa aaacaatata atgcaatcat tgcctatata aaacccacat aaatgtaatt 180
   taacttttaa tcttgaactc 200
<210> 71
   <211> 200
   <212> DNA
   <213> Amaranthus palmeri
<400> 71
   ttgatttatg ctattttttc tcttttattt ttatccaaaa atatcatggg atgtctagat 60
   tggaataatt agggagtaaa aagtacccct tgattatgca gagcaaaaat aaattgtctg 120
   aatttgaaat aaatatctac aagagtaaat ttttccatct tattcaaagg taaatgtttg 180
   atccacctac ctctatagat 200
<210> 72
   <211> 200
   <212> DNA
   <213> Amaranthus palmeri
<400> 72
   atctatagag gtaggtggat caaacattta cctttgaata agatggaaaa atttactctt 60
   gtagatattt atttcaaatt cagacaattt atttttgctc tgcataatca aggggtactt 120
   tttactccct aattattcca atctagacat cccatgatat ttttggataa aaataaaaga 180
   gaaaaaatag cataaatcaa 200
<210> 73
   <211> 200
   <212> DNA
   <213> Amaranthus palmeri
<400> 73
   gtttgatcca cctacctcta tagatatatt tcagggaact aaattgtctt caccattaaa 60
   tttggttact tggtctttaa aacccaaatc atagaaatta atgataaaat caaaataaaa 120
   aagatattta aattcaaatt caaactaact aattttaaat tacaaaatga atatctgtaa 180
   tttacaaaag aaagtatcaa 200
<210> 74
   <211> 200
   <212> DNA
   <213> Amaranthus palmeri
<400> 74
   ttgatacttt cttttgtaaa ttacagatat tcattttgta atttaaaatt agttagtttg 60
   aatttgaatt taaatatctt ttttattttg attttatcat taatttctat gatttgggtt 120
   ttaaagacca agtaaccaaa tttaatggtg aagacaattt agttccctga aatatatcta 180
   tagaggtagg tggatcaaac 200
<210> 75
   <211> 200
   <212> DNA
   <213> Amaranthus palmeri
<400> 75
   tgtaatttac aaaagaaagt atcaaaaaca tatgaaaatc tcaacatctg aaaattaaaa 60
   aacaagtatt ttgtttcttc attttttctt ttttcgctat ttcctttcaa aaataaaagt 120
   aaataaaaat attcaaaagc aattccataa acaaaatctt agatatgtaa atcacaaaaa 180
   cattagatct agaaaaaaaa 200
<210> 76
   <211> 200
   <212> DNA
   <213> Amaranthus palmeri
<400> 76
   ttttttttct agatctaatg tttttgtgat ttacatatct aagattttgt ttatggaatt   60
   gcttttgaat atttttattt acttttattt ttgaaaggaa atagcgaaaa agaaaaaaat  120
   gaagaaacaa aatacttgtt ttttaatttt cagatgttga gattttcata tgtttttgat  180
   actttctttt gtaaattaca                                              200
<210> 77
   <211> 200
   <212> DNA
   <213> Amaranthus palmeri
<400> 77
   aaaaacatta gatctagaaa aaaaaaattt cttccattgc aaaccctttt tcaaccttca   60
   taacttccac taccataatg aggccagtaa agagacaaaa gtcattgagt tgttgttgtg  120
   cagttgatga taaatgatga tagaagggtt tatttttttt ttgaaatgaa tggttagatt  180
   ttctgacttt ttatttaccc                                              200
<210> 78
   <211> 200
   <212> DNA
   <213> Amaranthus palmeri
<400> 78
   gggtaaataa aaagtcagaa aatctaacca ttcatttcaa aaaaaaaata aacccttcta   60
   tcatcattta tcatcaactg cacaacaaca actcaatgac ttttgtctct ttactggcct  120
   cattatggta gtggaagtta tgaaggttga aaaagggttt gcaatggaag aaattttttt  180
   tttctagatc taatgttttt                                              200
<210> 79
   <211> 200
   <212> DNA
   <213> Amaranthus palmeri
<400> 79
   agattttctg actttttatt taccctatat agaatatcaa acaattaact ctataaatta 60
   tttaatacat taaaatgttt catgtaatat gtctcctata ttatttaccc tttaattttt 120
   aagtgggaac caagtatgtc ttaattatct ttattttaat caaatacgcg gtatacatga 180
   aataatcaac aaatgcaatt 200
<210> 80
   <211> 200
   <212> DNA
   <213> Amaranthus palmeri
<400> 80
   aattgcattt gttgattatt tcatgtatac cgcgtatttg attaaaataa agataattaa 60
   gacatacttg gttcccactt aaaaattaaa gggtaaataa tataggagac atattacatg 120
   aaacatttta atgtattaaa taatttatag agttaattgt ttgatattct atatagggta 180
   aataaaaagt cagaaaatct 200
<210> 81
   <211> 200
   <212> DNA
   <213> Amaranthus palmeri
<400> 81
   catgaaataa tcaacaaatg caattactat gctcggacga gagtaaatat aatgggagga 60
   agttgtacat acaattacga aatagtctaa ataaataacg ataatttgta atataaacaa 120
   acaaatcaca cttatataaa cagattttat agggtggaat cattaggatt ctaatttatc 180
   ttttttcttc ttttgtttac 200
<210> 82
   <211> 200
   <212> DNA
   <213> Amaranthus palmeri
<400> 82
   gtaaacaaaa gaagaaaaaa gataaattag aatcctaatg attccaccct ataaaatctg 60
   tttatataag tgtgatttgt ttgtttatat tacaaattat cgttatttat ttagactatt 120
   tcgtaattgt atgtacaact tcctcccatt atatttactc tcgtccgagc atagtaattg 180
   catttgttga ttatttcatg 200
<210> 83
   <211> 200
   <212> DNA
   <213> Amaranthus palmeri
<400> 83
   ttatcttttt tcttcttttg tttactttgc tgatatttat tttgtatttt tcctatttct   60
   caaaaggaag actaacactc aaataaaatg atattgaaat acaaagcatc accagccaag  120
   ccgagatgac aaaactattg gctaagtgat aactgataag tgacgtattt ccgttactct  180
   caagtcttaa cagctttttg                                              200
<210> 84
   <211> 200
   <212> DNA
   <213> Amaranthus palmeri
<400> 84
   caaaaagctg ttaagacttg agagtaacgg aaatacgtca cttatcagtt atcacttagc   60
   caatagtttt gtcatctcgg cttggctggt gatgctttgt atttcaatat cattttattt  120
   gagtgttagt cttccttttg agaaatagga aaaatacaaa ataaatatca gcaaagtaaa  180
   caaaagaaga aaaaagataa                                              200
<210> 85
   <211> 200
   <212> DNA
   <213> Amaranthus palmeri
<400> 85
   actctcaagt cttaacagct ttttgtcacg caaggaaaag aagaccgtgg acgtcaacgg   60
   tgacgttgaa tgttcatctt tacagtcgca gtcaatcaat ctctttttag atcgatcttc  120
   cacctcaatt ctccgttaca aatcaaattc catctagaac ttctttttta ttattttgac   180
   tcataaattc ccccaaaaat                                              200
<210> 86
   <211> 200
   <212> DNA
   <213> Amaranthus palmeri
<400> 86
   atttttgggg gaatttatga gtcaaaataa taaaaaagaa gttctagatg gaatttgatt   60
   tgtaacggag aattgaggtg gaagatcgat ctaaaaagag attgattgac tgcgactgta  120
   aagatgaaca ttcaacgtca ccgttgacgt ccacggtctt cttttccttg cgtgacaaaa  180
   agctgttaag acttgagagt                                              200
<210> 87
   <211> 200
   <212> DNA
   <213> Amaranthus palmeri
<400> 87
   ttgactcata aattccccca aaaatacttc tattttatta taaataaatt ccaatttcta   60
   tgttctccat tcattaccac ccattactcc gttttccaaa ccaccatttt ctctctcctc  120
   ctttaccgct aacgctacca ccattttcgc ttcttccccg tctgaatttt attacttcgc  180
   tttctctatc atcatctgac                                              200
<210> 88
   <211> 200
   <212> DNA
   <213> Amaranthus palmeri
<400> 88
   gtcagatgat gatagagaaa gcgaagtaat aaaattcaga cggggaagaa gcgaaaatgg   60
   tggtagcgtt agcggtaaag gaggagagag aaaatggtgg tttggaaaac ggagtaatgg  120
   gtggtaatga atggagaaca tagaaattgg aatttattta taataaaata gaagtatttt  180
   tgggggaatt tatgagtcaa                                              200
<210> 89
   <211> 200
   <212> DNA
   <213> Amaranthus palmeri
<400> 89
   ttcgctttct ctatcatcat ctgacatggg aactttaaaa cccgaaactc aacccgattc   60
   cgaattcaaa ctcgtgggtt actccaactt cgtccgggtt aaccccaaat ctgaccgttt  120
   tgctgttaag cgtttccacc atatagagtt ttggtgtggc gatgcaacca atgttagcag  180
   acgattttct tggggtcttg                                              200
<210> 90
   <211> 200
   <212> DNA
   <213> Amaranthus palmeri
<400> 90
   caagacccca agaaaatcgt ctgctaacat tggttgcatc gccacaccaa aactctatat   60
   ggtggaaacg cttaacagca aaacggtcag atttggggtt aacccggacg aagttggagt  120
   aacccacgag tttgaattcg gaatcgggtt gagtttcggg ttttaaagtt cccatgtcag  180
   atgatgatag agaaagcgaa                                              200
<210> 91
   <211> 200
   <212> DNA
   <213> Amaranthus palmeri
<400> 91
   agcagacgat tttcttgggg tcttggaatg cctaccgtcg ctaaatctga cctgtctaca 60
   ggaaactctg ttcacgcttc ttttcttctt agttccggtg acctttcttt tctctttact 120
   tcaccttact ctcctaccat gtccatccct tcttctgctg caatcccctc gtttgatttc 180
   aatcatttta ccaaattcct 200
<210> 92
   <211> 200
   <212> DNA
   <213> Amaranthus palmeri
<400> 92
   aggaatttgg taaaatgatt gaaatcaaac gaggggattg cagcagaaga agggatggac 60
   atggtaggag agtaaggtga agtaaagaga aaagaaaggt caccggaact aagaagaaaa 120
   gaagcgtgaa cagagtttcc tgtagacagg tcagatttag cgacggtagg cattccaaga 180
   ccccaagaaa atcgtctgct 200
<210> 93
   <211> 200
   <212> DNA
   <213> Amaranthus palmeri
<400> 93
   atttcaatca ttttaccaaa ttccttacat cgcacggtct tggcgtgcgt gctgttgccg 60
   tcgaagtaga agacgcagaa gctgctttta atatcagcgt ttcgcacggg gctatcccct 120
   gtgtttctcc tattcacttg gaaaacggtg tcgttttatc tgaggttcat ttatatgggg 180
   atgttgtgct tcggtatgta 200
<210> 94
   <211> 200
   <212> DNA
   <213> Amaranthus palmeri
<400> 94
   tacataccga agcacaacat ccccatataa atgaacctca gataaaacga caccgttttc 60
   caagtgaata ggagaaacac aggggatagc cccgtgcgaa acgctgatat taaaagcagc 120
   ttctgcgtct tctacttcga cggcaacagc acgcacgcca agaccgtgcg atgtaaggaa 180
   tttggtaaaa tgattgaaat 200
<210> 95
   <211> 200
   <212> DNA
   <213> Amaranthus palmeri
<400> 95
   tggggatgtt gtgcttcggt atgtaagcta cggaaatgaa tgtggggatg tgttttttct    60
   tcctgggttt gaggaaatgc cggaggaatc atcgtttcga ggacttgatt ttggcattcg  120
   aaggttggat catgctgtag ggaatgtccc tgagttggct cctgcaattg cttatttgaa  180
   gaagtttact gggtttcatg                                              200
<210> 96
   <211> 200
   <212> DNA
   <213> Amaranthus palmeri
<400> 96
   catgaaaccc agtaaacttc ttcaaataag caattgcagg agccaactca gggacattcc   60
   ctacagcatg atccaacctt cgaatgccaa aatcaagtcc tcgaaacgat gattcctccg  120
   gcatttcctc aaacccagga agaaaaaaca catccccaca ttcatttccg tagcttacat  180
   accgaagcac aacatcccca                                              200
<210> 97
   <211> 200
   <212> DNA
   <213> Amaranthus palmeri
<400> 97
   ttgaagaagt ttactgggtt tcatgagttt gctgagttta cagctgaaga tgttgggacg   60
   agtgaaagtg ggttgaattc agccgtattg gcaaacaatg atgaaatggt gttgtttccg  120
   atgaatgaac ctgtgtatgg gacaaaaagg aagagccaaa ttcaaactta tttggagcat  180
   aatgaaggag ctggtgtaca                                              200
<210> 98
   <211> 200
   <212> DNA
   <213> Amaranthus palmeri
<400> 98
   tgtacaccag ctccttcatt atgctccaaa taagtttgaa tttggctctt cctttttgtc 60
   ccatacacag gttcattcat cggaaacaac accatttcat cattgtttgc caatacggct 120
   gaattcaacc cactttcact cgtcccaaca tcttcagctg taaactcagc aaactcatga 180
   aacccagtaa acttcttcaa 200
<210> 99
   <211> 200
   <212> DNA
   <213> Amaranthus palmeri
<400> 99
   agcataatga aggagctggt gtacagcatt tggctttgat gagtgaagac atattttgga 60
   ctttaaggga gatgaggaag agaagtggtc ttggtgggtt tgagtttatg ccgtcgccgc 120
   ctccgactta ttaccggaat ttgaggaaca gagctgctga tgtattgagt gaggagcaga 180
   tgaaggagtg tgaagagttg 200
<210> 100
   <211> 200
   <212> DNA
   <213> Amaranthus palmeri
<400> 100
   caactcttca cactccttca tctgctcctc actcaataca tcagcagctc tgttcctcaa 60
   attccggtaa taagtcggag gcggcgacgg cataaactca aacccaccaa gaccacttct 120
   cttcctcatc tcccttaaag tccaaaatat gtcttcactc atcaaagcca aatgctgtac 180
   accagctcct tcattatgct 200
<210> 101
   <211> 200
   <212> DNA
   <213> Amaranthus palmeri
<400> 101
   gcagatgaag gagtgtgaag agttggggat tttggtggat aaagatgatc agggcacttt 60
   gcttcaaatc ttcaccaaac ctattggaga caggtagatt ttaatcttgc tttcaattgc 120
   ttttgcttga tggattgact agccaatttg attgcatttt gttgcttata tgacttgatg 180
   ctagatagtt tacctttctc 200
<210> 102
   <211> 200
   <212> DNA
   <213> Amaranthus palmeri
<400> 102
   gagaaaggta aactatctag catcaagtca tataagcaac aaaatgcaat caaattggct   60
   agtcaatcca tcaagcaaaa gcaattgaaa gcaagattaa aatctacctg tctccaatag  120
   gtttggtgaa gatttgaagc aaagtgccct gatcatcttt atccaccaaa atccccaact  180
   cttcacactc cttcatctgc                                              200
<210> 103
   <211> 200
   <212> DNA
   <213> Amaranthus palmeri
<400> 103
   tgatgctaga tagtttacct ttctcagctg ttaagttgta gcaagtatct aatacattcg   60
   ttctgaaata tctgaaatat ttgcaactga ttatgacatg tagcgggaga aaatgtccgt  120
   ttggcataaa ttaagcttgg ttttgcactt atcctcattt atttataaat tctaaaactt  180
   gttagttgta agctcctttc                                              200
<210> 104
   <211> 200
   <212> DNA
   <213> Amaranthus palmeri
<400> 104
   gaaaggagct tacaactaac aagttttaga atttataaat aaatgaggat aagtgcaaaa   60
   ccaagcttaa tttatgccaa acggacattt tctcccgcta catgtcataa tcagttgcaa  120
   atatttcaga tatttcagaa cgaatgtatt agatacttgc tacaacttaa cagctgagaa  180
   aggtaaacta tctagcatca                                              200
<210> 105
   <211> 200
   <212> DNA
   <213> Amaranthus palmeri
<400> 105
   aacttgttag ttgtaagctc ctttcagttg tcctgaattt aattaagtta atgagaacaa   60
   agccttaata ttccttcaag gtgattgtag ttggggcact agttctaaca atggaaattt  120
   ggaaatctat tccaactggt cccaagttaa tctttgtttg caagcctgat tggttcaaat  180
   taaggttatt gtattcttgt                                              200
<210> 106
   <211> 200
   <212> DNA
   <213> Amaranthus palmeri
<400> 106
   acaagaatac aataacctta atttgaacca atcaggcttg caaacaaaga ttaacttggg 60
   accagttgga atagatttcc aaatttccat tgttagaact agtgccccaa ctacaatcac 120
   cttgaaggaa tattaaggct ttgttctcat taacttaatt aaattcagga caactgaaag 180
   gagcttacaa ctaacaagtt 200
<210> 107
   <211> 200
   <212> DNA
   <213> Amaranthus palmeri
<400> 107
   caaattaagg ttattgtatt cttgtatgaa ttcgactcaa tgtaaatttg tttaatggag 60
   tatcaatttt taatagtttt caaccaagta gtattagata tattcgggat tgaaataatg 120
   catctatgag tgtacaaaac caaatggcac atttgattag aataaaagag agtataaggc 180
   taatttcgtt tacctaatat 200
<210> 108
   <211> 200
   <212> DNA
   <213> Amaranthus palmeri
<400> 108
   atattaggta aacgaaatta gccttatact ctcttttatt ctaatcaaat gtgccatttg 60
   gttttgtaca ctcatagatg cattatttca atcccgaata tatctaatac tacttggttg 120
   aaaactatta aaaattgata ctccattaaa caaatttaca ttgagtcgaa ttcatacaag 180
   aatacaataa ccttaatttg 200
<210> 109
   <211> 200
   <212> DNA
   <213> Amaranthus palmeri
<400> 109
   aaggctaatt tcgtttacct aatatttaaa gcgaccccta aaattcaatt ggcctaaacc 60
   cataaagttc aaaagcagag aagaacatag aatagtgcag gtccattggt aatgcactag 120
   gagttggagc ttttatgggt acaagtgtgt ggctagttgg ggatgactgt ctagcattgt 180
   ctaggtgaaa agctgaagcc 200
<210> 110
   <211> 200
   <212> DNA
   <213> Amaranthus palmeri
<400> 110
   ggcttcagct tttcacctag acaatgctag acagtcatcc ccaactagcc acacacttgt 60
   acccataaaa gctccaactc ctagtgcatt accaatggac ctgcactatt ctatgttctt 120
   ctctgctttt gaactttatg ggtttaggcc aattgaattt taggggtcgc tttaaatatt 180
   aggtaaacga aattagcctt 200
<210> 111
   <211> 200
   <212> DNA
   <213> Amaranthus palmeri
<400> 111
   ggaggaattt ttttgtgcat gtaaaatgtt ttctctctat tttttgattt atgctatttt 60
   ttctctttta tttttatcca aaaatatcat gggatgtcta gattggaata attagggagt 120
   aaaaagtacc ccttgattat gcagagcaaa aataaattgt ctgaatttga aataaatatc 180
   tacaagagta aatttttcca 200
<210> 112
   <211> 200
   <212> DNA
   <213> Amaranthus palmeri
<400> 112
   tggaaaaatt tactcttgta gatatttatt tcaaattcag acaatttatt tttgctctgc 60
   ataatcaagg ggtacttttt actccctaat tattccaatc tagacatccc atgatatttt 120
   tggataaaaa taaaagagaa aaaatagcat aaatcaaaaa atagagagaa aacattttac 180
   atgcacaaaa aaattcctcc 200
<210> 113
   <211> 200
   <212> DNA
   <213> Amaranthus palmeri
<400> 113
   atatctacaa gagtaaattt ttccatctta ttcaaaggta aatgtttgat ccacctacct    60
   ctatagatat atttcaggga actaaaattg tcttcaccat tatatttagt tacttggtct   120
   ttaaaaccca aatcatagaa attaacgata aaatcaaaat ataaagatat ttaaattcca   180
   attcaaacta actaatttta                                               200
<210> 114
   <211> 200
   <212> DNA
   <213> Amaranthus palmeri
<400> 114
   taaaattagt tagtttgaat tggaatttaa atatctttat attttgattt tatcgttaat    60
   ttctatgatt tgggttttaa agaccaagta actaaatata atggtgaaga caattttagt   120
   tccctgaaat atatctatag aggtaggtgg atcaaacatt tacctttgaa taagatggaa   180
   aaatttactc ttgtagatat                                               200
<210> 115
   <211> 200
   <212> DNA
   <213> Amaranthus palmeri
<400> 115
   ttccaattca aactaactaa ttttaaatta caaaatgaat atctgtaatt tacaaaagaa    60
   agtatcaaaa acatatgaaa atctcaacat ctgaaaatta aaaaacaagt attttgtttc   120
   ttcatttttt tctttttcgc tatttccttt caaaaataaa agtaaataaa aatattcaaa   180
   agcaattcca taaacaaaat                                               200
<210> 116
   <211> 200
   <212> DNA
   <213> Amaranthus palmeri
<400> 116
   attttgttta tggaattgct tttgaatatt tttatttact tttatttttg aaaggaaata    60
   gcgaaaaaga aaaaaatgaa gaaacaaaat acttgttttt taattttcag atgttgagat   120
   tttcatatgt tttgatactt ttcttttgta aattacagat attcattttg taatttaaaa   180
   ttagttagtt tgaattggaa                                               200
<210> 117
   <211> 200
   <212> DNA
   <213> Amaranthus palmeri
<400> 117
   tcaaaagcaa ttccataaac aaaatcttag atatgtaaat cacaaaaaca ttagatctag   60
   aaaaaaaaaa tttcttccat tgcaaaccct ttttcaacct tcataacttc cactaccata  120
   atgaggccag taaagagaca aaagtcattg agttgttgtt gtgcagttga tgataaatga  180
   tgatagaagg gtttattttt                                              200
<210> 118
   <211> 200
   <212> DNA
   <213> Amaranthus palmeri
<400> 118
   aaaaataaac ccttctatca tcatttatca tcaactgcac aacaacaact caatgacttt   60
   tgtctcttta ctggcctcat tatggtagtg gaagttatga aggttgaaaa agggtttgca  120
   atggaagaaa tttttttttt ctagatctaa tgtttttgtg atttacatat ctaagatttt  180
   gtttatggaa ttgcttttga                                              200
<210> 119
   <211> 200
   <212> DNA
   <213> Amaranthus palmeri
<400> 119
   aatgatgata gaagggttta tttttttttt gaaatgaatg gttagatttt ctgacttttt   60
   atttacccta tatagaatat caaacaatta actctataaa ttatttaata cattaaaatg  120
   tttcatgtaa tatgtctcct atattattta ccctttaatt tttaagtggg aaccaagtat  180
   gtcttaatta tctttatttt                                              200
<210> 120
   <211> 200
   <212> DNA
   <213> Amaranthus palmeri
<400> 120
   aaaataaaga taattaagac atacttggtt cccacttaaa aattaaaggg taaataatat 60
   aggagacata ttacatgaaa cattttaatg tattaaataa tttatagagt taattgtttg 120
   atattctata tagggtaaat aaaaagtcag aaaatctaac cattcatttc aaaaaaaaaa 180
   taaacccttc tatcatcatt 200
<210> 121
   <211> 200
   <212> DNA
   <213> Amaranthus palmeri
<400> 121
   agtatgtctt aattatcttt attttaatca aatacgcggt atacatgaaa taatcaacaa 60
   atgcaattac tatgctcgga cgagagtaaa tataatggga ggaagttgta catacaatta 120
   cgaaatagtc taaataaata acgataattt gtaatataaa caaacaaatc acacttatat 180
   aaacagattt tatagggtgg 200
<210> 122
   <211> 200
   <212> DNA
   <213> Amaranthus palmeri
<400> 122
   ccaccctata aaatctgttt atataagtgt gatttgtttg tttatattac aaattatcgt 60
   tatttattta gactatttcg taattgtatg tacaacttcc tcccattata tttactctcg 120
   tccgagcata gtaattgcat ttgttgatta tttcatgtat accgcgtatt tgattaaaat 180
   aaagataatt aagacatact 200
<210> 123
   <211> 200
   <212> DNA
   <213> Amaranthus palmeri
<400> 123
   tatataaaca gattttatag ggtggaatca ttaggattct aatttatctt ttttcttctt 60
   ttgtttactt tgctgatatt tattttgtat ttttcctatt tctcaaaagg aagactaaca 120
   ctcaaataaa atgatattga aatacaaagc atcaccagcc aagccgagat gacaaaacta 180
   ttggctaagt gataactgat 200
<210> 124
   <211> 200
   <212> DNA
   <213> Amaranthus palmeri
<400> 124
   atcagttatc acttagccaa tagttttgtc atctcggctt ggctggtgat gctttgtatt    60
   tcaatatcat tttatttgag tgttagtctt ccttttgaga aataggaaaa atacaaaata   120
   aatatcagca aagtaaacaa aagaagaaaa aagataaatt agaatcctaa tgattccacc   180
   ctataaaatc tgtttatata                                               200
<210> 125
   <211> 200
   <212> DNA
   <213> Amaranthus palmeri
<400> 125
   aactattggc taagtgataa ctgataagtg acgtatttcc gttactctca agtcttaaca    60
   gctttttgtc acgcaaggaa aagaagaccg tggacgtcaa cggtgacgtt gaatgttcat   120
   ctttacagtc gcagtcaatc aatctctttt tagatcgatc ttccacctca attctccgtt   180
   acaaatcaaa ttccatctag                                               200
<210> 126
   <211> 200
   <212> DNA
   <213> Amaranthus palmeri
<400> 126
   ctagatggaa tttgatttgt aacggagaat tgaggtggaa gatcgatcta aaaagagatt    60
   gattgactgc gactgtaaag atgaacattc aacgtcaccg ttgacgtcca cggtcttctt   120
   ttccttgcgt gacaaaaagc tgttaagact tgagagtaac ggaaatacgt cacttatcag   180
   ttatcactta gccaatagtt                                               200
<210> 127
   <211> 200
   <212> DNA
   <213> Amaranthus palmeri
<400> 127
   ccgttacaaa tcaaattcca tctagaactt ctttttaatt attttgactc ataaattccc    60
   ccaaaaatac ttctatttta ttataaataa attccaattt ctatgttctc cattcatcac   120
   cacccattac tccgttttcc aaaccaccat tttctctctc ctccattacc cctaacacaa   180
   ctaccatttt cgcttcttcc                                               200
<210> 128
   <211> 200
   <212> DNA
   <213> Amaranthus palmeri
<400> 128
   ggaagaagcg aaaatggtag ttgtgttagg ggtaatggag gagagagaaa atggtggttt   60
   ggaaaacgga gtaatgggtg gtgatgaatg gagaacatag aaattggaat ttatttataa  120
   taaaatagaa gtatttttgg gggaatttat gagtcaaaat aattaaaaag aagttctaga  180
   tggaatttga tttgtaacgg                                              200
<210> 129
   <211> 200
   <212> DNA
   <213> Amaranthus palmeri
<400> 129
   cacaactacc attttcgctt cttccccgtc tgagtcttat tacatcgctt tctctctcat   60
   catctgacat gggaactttg aaacccgaaa ctcaacccga ttccgaattc aaactcgtgg  120
   gttactccaa cttcgttcgg gttaacccca aatctgaccg ttttgctgtt aagcgtttcc  180
   accatataga gttttggtgt                                              200
<210> 130
   <211> 200
   <212> DNA
   <213> Amaranthus palmeri
<400> 130
   acaccaaaac tctatatggt ggaaacgctt aacagcaaaa cggtcagatt tggggttaac   60
   ccgaacgaag ttggagtaac ccacgagttt gaattcggaa tcgggttgag tttcgggttt  120
   caaagttccc atgtcagatg atgagagaga aagcgatgta ataagactca gacggggaag  180
   aagcgaaaat ggtagttgtg                                              200
<210> 131
   <211> 200
   <212> DNA
   <213> Amaranthus palmeri
<400> 131
   tttccaccat atagagtttt ggtgtggcga tgcaaccaat gttagcagac gattttcttg   60
   gggtcttgga atgcctatcg tcgctaaatc tgacctgtct acaggaaact ctgttcacgc  120
   ttcttttctt cttcgttccg gtgacctttc ttttctcttt acttcaccgt attctcctac  180
   catgtccatc ccttcttctg                                              200
<210> 132
   <211> 200
   <212> DNA
   <213> Amaranthus palmeri
<400> 132
   cagaagaagg gatggacatg gtaggagaat acggtgaagt aaagagaaaa gaaaggtcac 60
   cggaacgaag aagaaaagaa gcgtgaacag agtttcctgt agacaggtca gatttagcga 120
   cgataggcat tccaagaccc caagaaaatc gtctgctaac attggttgca tcgccacacc 180
   aaaactctat atggtggaaa 200
<210> 133
   <211> 200
   <212> DNA
   <213> Amaranthus palmeri
<400> 133
   cctaccatgt ccatcccttc ttctgctgca atcccctcgt ttgatttcaa tcattttacc 60
   aaattcctta catcgcacgg tcttggcgtg cgtgctgttg ccgtcgaagt agaagacgcc 120
   gaagctgctt ttaatatcag cgtttcgcac ggggctatcc cctgtgtttc tcctattcac 180
   ttggaaaacg gtgtcgtttt 200
<210> 134
   <211> 200
   <212> DNA
   <213> Amaranthus palmeri
<400> 134
   aaaacgacac cgttttccaa gtgaatagga gaaacacagg ggatagcccc gtgcgaaacg 60
   ctgatattaa aagcagcttc ggcgtcttct acttcgacgg caacagcacg cacgccaaga 120
   ccgtgcgatg taaggaattt ggtaaaatga ttgaaatcaa acgaggggat tgcagcagaa 180
   gaagggatgg acatggtagg 200
<210> 135
   <211> 200
   <212> DNA
   <213> Amaranthus palmeri
<400> 135
   ttcacttgga aaacggtgtc gttttatctg aggttcattt atatggggat gttgtgcttc 60
   ggtatgtaag ctacggaaat gaatgtgggg atgtgttttt tcttcctggg tttgaggaaa 120
   tgccggagga atcatcgttt cgaggacttg attttggcat tcgaaggttg gatcatgctg 180
   tagggaatgt ccctgagttg 200
<210> 136
   <211> 200
   <212> DNA
   <213> Amaranthus palmeri
<400> 136
   caactcaggg acattcccta cagcatgatc caaccttcga atgccaaaat caagtcctcg 60
   aaacgatgat tcctccggca tttcctcaaa cccaggaaga aaaaacacat ccccacattc 120
   atttccgtag cttacatacc gaagcacaac atccccatat aaatgaacct cagataaaac 180
   gacaccgttt tccaagtgaa 200
<210> 137
   <211> 200
   <212> DNA
   <213> Amaranthus palmeri
<400> 137
   tgctgtaggg aatgtccctg agttggctcc tgcaattgct tatttgaaga agtttactgg 60
   gtttcatgag tttgctgagt ttacagctga agatgttggg acgagtgaaa gtggattgaa 120
   ttcagccgta ttggcaaaca atgatgaaat ggtgttgttt ccaatgaatg aacctgtgta 180
   tgggacaaaa aggaagagcc 200
<210> 138
   <211> 200
   <212> DNA
   <213> Amaranthus palmeri
<400> 138
   ggctcttcct ttttgtccca tacacaggtt cattcattgg aaacaacacc atttcatcat 60
   tgtttgccaa tacggctgaa ttcaatccac tttcactcgt cccaacatct tcagctgtaa 120
   actcagcaaa ctcatgaaac ccagtaaact tcttcaaata agcaattgca ggagccaact 180
   cagggacatt ccctacagca 200
<210> 139
   <211> 200
   <212> DNA
   <213> Amaranthus palmeri
<400> 139
   gtgtatggga caaaaaggaa gagccaaatt caaacttatt tggagcataa tgaaggagct 60
   ggtgtacagc atttggcttt gatgagtgaa gacatatttt ggactttaag ggagatgagg 120
   aagagaagtg gtcttggtgg gtttgaattt atgccgtcgc cgcctccgac ttattaccgg 180
   aatttgagga gcagagctgc 200
<210> 140
   <211> 200
   <212> DNA
   <213> Amaranthus palmeri
<400> 140
   gcagctctgc tcctcaaatt ccggtaataa gtcggaggcg gcgacggcat aaattcaaac 60
   ccaccaagac cacttctctt cctcatctcc cttaaagtcc aaaatatgtc ttcactcatc 120
   aaagccaaat gctgtacacc agctccttca ttatgctcca aataagtttg aatttggctc 180
   ttcctttttg tcccatacac 200
<210> 141
   <211> 200
   <212> DNA
   <213> Amaranthus palmeri
<400> 141
   accggaattt gaggagcaga gctgctgatg tattgagtga ggagcagatg aaggagtgtg 60
   aagagttggg gattttggtg gataaagatg atcagggcac tttgcttcaa atcttcacca 120
   aacctattgg agacaggtag attttaatct tgctttcaat tgcttttgct tgattgattg 180
   actagccaat tttgattgca 200
<210> 142
   <211> 200
   <212> DNA
   <213> Amaranthus palmeri
<400> 142
   tgcaatcaaa attggctagt caatcaatca agcaaaagca attgaaagca agattaaaat 60
   ctacctgtct ccaataggtt tggtgaagat ttgaagcaaa gtgccctgat catctttatc 120
   caccaaaatc cccaactctt cacactcctt catctgctcc tcactcaata catcagcagc 180
   tctgctcctc aaattccggt 200
<210> 143
   <211> 200
   <212> DNA
   <213> Amaranthus palmeri
<400> 143
   gattgactag ccaattttga ttgcattttg ttgcttatat gacttgatga taatagatgg 60
   tttacctttc tcagctgttc atttgtagcc agtatagatt cgttctaaaa tatttgcaac 120
   tgattatgac atgtagtagc agaaaatgtc cctatattgg atgtttggca taaattaagc 180
   ttggttttgc acttatcctc 200
<210> 144
   <211> 200
   <212> DNA
   <213> Amaranthus palmeri
<400> 144
   gaggataagt gcaaaaccaa gcttaattta tgccaaacat ccaatatagg gacattttct 60
   gctactacat gtcataatca gttgcaaata ttttagaacg aatctatact ggctacaaat 120
   gaacagctga gaaaggtaaa ccatctatta tcatcaagtc atataagcaa caaaatgcaa 180
   tcaaaattgg ctagtcaatc 200
<210> 145
   <211> 200
   <212> DNA
   <213> Amaranthus palmeri
<400> 145
   taagcttggt tttgcactta tcctcattta tttataaatt ctaaaacttg ttagttgtaa 60
   ttaagttaat gagaacaaag ccttaatatt ccttcaaggt gattgtagtt ggggcactag 120
   ttctaacaat ggaaatttgg aaatctattc caactggtcc caagttaatc tttgtttgca 180
   agcctgattg gttcaaatta 200
<210> 146
   <211> 200
   <212> DNA
   <213> Amaranthus palmeri
<400> 146
   taatttgaac caatcaggct tgcaaacaaa gattaacttg ggaccagttg gaatagattt 60
   ccaaatttcc attgttagaa ctagtgcccc aactacaatc accttgaagg aatattaagg 120
   ctttgttctc attaacttaa ttacaactaa caagttttag aatttataaa taaatgagga 180
   taagtgcaaa accaagctta 200
<210> 147
   <211> 200
   <212> DNA
   <213> Amaranthus palmeri
<400> 147
   ttgcaagcct gattggttca aattaaggtt attgtattct tgtatgaatt cgactcaatg   60
   taaatttgtt taatggagca tcaattttta atagttttcg accaagcagt attagatata  120
   ttcgggattg aaataatgca tctatgagtg tataaaacca aatggcacat ttgattagaa  180
   taaaagagag tataaggcta                                               200
<210> 148
   <211> 200
   <212> DNA
   <213> Amaranthus palmeri
<400> 148
   tagccttata ctctctttta ttctaatcaa atgtgccatt tggttttata cactcataga   60
   tgcattattt caatcccgaa tatatctaat actgcttggt cgaaaactat taaaaattga  120
   tgctccatta aacaaattta cattgagtcg aattcataca agaatacaat aaccttaatt  180
   tgaaccaatc aggcttgcaa                                               200
<210> 149
   <211> 200
   <212> DNA
   <213> Amaranthus palmeri
<400> 149
   ttttctcttt tatttttatc caaaaatatc atgggatgtc tagattggaa taattaggga   60
   gtaaaaagta ccccttgatt atgcagagca aaaataaatt gtctgaattt gaaataaata  120
   tctacaagag taaatttttc catcttattc aaaggtaaat gtttgatcca cctacctcta  180
   tagatatatt tcagggaact                                               200
<210> 150
   <211> 200
   <212> DNA
   <213> Amaranthus palmeri
<400> 150
   agttccctga aatatatcta tagaggtagg tggatcaaac atttaccttt gaataagatg   60
   gaaaaattta ctcttgtaga tatttatttc aaattcagac aatttatttt tgctctgcat  120
   aatcaagggg tactttttac tccctaatta ttccaatcta gacatcccat gatatttttg  180
   gataaaaata aaagagaaaa                                               200
<210> 151
   <211> 200
   <212> DNA
   <213> Amaranthus palmeri
<400> 151
   ctctatagat atatttcagg gaactaaatt gtcttcacca ttaaatttgg ttacttggtc 60
   tttaaaaccc aaatcataga aattaatgat aaaatcaaaa taaaaaagat atttaaattc 120
   aaattcaaac taactaattt taaattacaa aatgaatatc tgtaatttac aaaagaaagt 180
   atcaaaaaca tatgaaaatc 200
<210> 152
   <211> 200
   <212> DNA
   <213> Amaranthus palmeri
<400> 152
   gattttcata tgtttttgat actttctttt gtaaattaca gatattcatt ttgtaattta 60
   aaattagtta gtttgaattt gaatttaaat atctttttta ttttgatttt atcattaatt 120
   tctatgattt gggttttaaa gaccaagtaa ccaaatttaa tggtgaagac aatttagttc 180
   cctgaaatat atctatagag 200
<210> 153
   <211> 200
   <212> DNA
   <213> Amaranthus palmeri
<400> 153
   aaagtatcaa aaacatatga aaatctcaac atctgaaaat tacaaacaag tattctgttt 60
   tttcattttt tttttctttt tcgctatttc ctttcaaaaa taaaagtaaa taaaaatatt 120
   caaaagcaat tccataaaca aaatcttaga tatgtaaatc acaaaaacat tagatctaga 180
   aaaaaaaaat ttcttccatt 200
<210> 154
   <211> 200
   <212> DNA
   <213> Amaranthus palmeri
<400> 154
   aatggaagaa attttttttt tctagatcta atgtttttgt gatttacata tctaagattt 60
   tgtttatgga attgcttttg aatattttta tttactttta tttttgaaag gaaatagcga 120
   aaaagaaaaa aaaaatgaaa aaacagaata cttgtttgta attttcagat gttgagattt 180
   tcatatgttt ttgatacttt 200
<210> 155
   <211> 200
   <212> DNA
   <213> Amaranthus palmeri
<400> 155
   ctagaaaaaa aaaatttctt ccattgcaaa ccctttttca accttcataa cttccactac 60
   cataatgagg ccagtaaaga gacaaaagtc attgagttgt tgttgtgcag ttgatgataa 120
   atgatgatag aagggtttat tttttttttg aaatgaatgg ttagattttc tgacttttta 180
   tttaccctat aatgaatatc 200
<210> 156
   <211> 200
   <212> DNA
   <213> Amaranthus palmeri
<400> 156
   gatattcatt atagggtaaa taaaaagtca gaaaatctaa ccattcattt caaaaaaaaa 60
   ataaaccctt ctatcatcat ttatcatcaa ctgcacaaca acaactcaat gacttttgtc 120
   tctttactgg cctcattatg gtagtggaag ttatgaaggt tgaaaaaggg tttgcaatgg 180
   aagaaatttt ttttttctag 200
<210> 157
   <211> 200
   <212> DNA
   <213> Amaranthus palmeri
<400> 157
   ttttatttac cctataatga atatcaaaca attaactcta taaattattt aatacattaa 60
   aatgtttcat gtaatatgtc tcctatatta tttacccttt aatttttaag tgggaaccaa 120
   gtatgtctta attatcttta ttttaatcaa atacgcggta tacatgaaat aatcaacaaa 180
   tgcaattact atgctcggac 200
<210> 158
   <211> 200
   <212> DNA
   <213> Amaranthus palmeri
<400> 158
   gtccgagcat agtaattgca tttgttgatt atttcatgta taccgcgtat ttgattaaaa 60
   taaagataat taagacatac ttggttccca cttaaaaatt aaagggtaaa taatatagga 120
   gacatattac atgaaacatt ttaatgtatt aaataattta tagagttaat tgtttgatat 180
   tcattatagg gtaaataaaa 200
<210> 159
   <211> 200
   <212> DNA
   <213> Amaranthus palmeri
<400> 159
   acaaatgcaa ttactatgct cggacgagag taaatataat gggaggaagt tgtacataca 60
   attacgaaat agtctaaata aataacgata atttgtaata taaacaaaca aatcacactt 120
   atataaacag attttatagg gtggaatcat taggattcta atttatcttt tttcttcttt 180
   tgtttacttt gctgatattt 200
<210> 160
   <211> 200
   <212> DNA
   <213> Amaranthus palmeri
<400> 160
   aaatatcagc aaagtaaaca aaagaagaaa aaagataaat tagaatccta atgattccac 60
   cctataaaat ctgtttatat aagtgtgatt tgtttgttta tattacaaat tatcgttatt 120
   tatttagact atttcgtaat tgtatgtaca acttcctccc attatattta ctctcgtccg 180
   agcatagtaa ttgcatttgt 200
<210> 161
   <211> 200
   <212> DNA
   <213> Amaranthus palmeri
<400> 161
   tcttttgttt actttgctga tatttatttt gtatttttcc tatttctcaa aaggaagact 60
   aacactcaaa taaaatgata ttgaaataca aagcatcacc ggccaagccg agatgacgaa 120
   actattttga ataattatga tgatttacaa ctccaaatag aagtaattga tcaagacttt 180
   aggacttgga agtgttgggc 200
<210> 162
   <211> 200
   <212> DNA
   <213> Amaranthus palmeri
<400> 162
   gcccaacact tccaagtcct aaagtcttga tcaattactt ctatttggag ttgtaaatca 60
   tcataattat tcaaaatagt ttcgtcatct cggcttggcc ggtgatgctt tgtatttcaa 120
   tatcatttta tttgagtgtt agtcttcctt ttgagaaata ggaaaaatac aaaataaata 180
   tcagcaaagt aaacaaaaga 200
<210> 163
   <211> 200
   <212> DNA
   <213> Amaranthus palmeri
<400> 163
   actttaggac ttggaagtgt tgggcaaaat cttccagagt ccaggataag tgataagtga 60
   cgtatttccg ttactcttaa gtgttaacag ctttttgtca cgcaaggaaa agaagaccgt 120
   ggacgtcaac gatgacgttg aatgttcatc tttacagtcg cagtcaatca atctcttttt 180
   agatcgatct tccacctcaa 200
<210> 164
   <211> 200
   <212> DNA
   <213> Amaranthus palmeri
<400> 164
   ttgaggtgga agatcgatct aaaaagagat tgattgactg cgactgtaaa gatgaacatt 60
   caacgtcatc gttgacgtcc acggtcttct tttccttgcg tgacaaaaag ctgttaacac 120
   ttaagagtaa cggaaatacg tcacttatca cttatcctgg actctggaag attttgccca 180
   acacttccaa gtcctaaagt 200
<210> 165
   <211> 200
   <212> DNA
   <213> Amaranthus palmeri
<400> 165
   tttttagatc gatcttccac ctcaattctc cgttacaaat caaattccat ctagaacttc 60
   ttttttatta ttttgactca taaattcccc caaaaatact tctattttat tataaataaa 120
   ttccaatttc tatgttctcc attcattacc acccattact ccgttttcca aaccaccatt 180
   ttctctctcc tcctttaccg 200
<210> 166
   <211> 200
   <212> DNA
   <213> Amaranthus palmeri
<400> 166
   cggtaaagga ggagagagaa aatggtggtt tggaaaacgg agtaatgggt ggtaatgaat   60
   ggagaacata gaaattggaa tttatttata ataaaataga agtatttttg ggggaattta  120
   tgagtcaaaa taataaaaaa gaagttctag atggaatttg atttgtaacg gagaattgag  180
   gtggaagatc gatctaaaaa                                              200
<210> 167
   <211> 200
   <212> DNA
   <213> Amaranthus palmeri
<400> 167
   ccattttctc tctcctcctt taccgctaac gctaccacca ttttcgcttc ttccccgtct   60
   gaattttatt acttcgcttt ctctatcatc atctgacatg ggtactttga aacccgaaac  120
   tcaacccgat tccgaattca aactcgtggg ttactccaac ttcgttcggg ttaaccccaa  180
   atctgaccgt tttgctgtta                                              200
<210> 168
   <211> 200
   <212> DNA
   <213> Amaranthus palmeri
<400> 168
   taacagcaaa acggtcagat ttggggttaa cccgaacgaa gttggagtaa cccacgagtt   60
   tgaattcgga atcgggttga gtttcgggtt tcaaagtacc catgtcagat gatgatagag  120
   aaagcgaagt aataaaattc agacggggaa gaagcgaaaa tggtggtagc gttagcggta  180
   aaggaggaga gagaaaatgg                                              200
<210> 169
   <211> 200
   <212> DNA
   <213> Amaranthus palmeri
<400> 169
   cccaaatctg accgttttgc tgttaagcgt ttccaccata tagagttttg gtgtggcgat   60
   gcaaccaatg ttagcagacg attttcttgg ggtcttggaa tgcctaccgt tgctaaatct  120
   gacctttcta caggaaactc tgttcacgct tcttttcttc ttcgttccgg tgacctttct  180
   tttctcttta cttcacctta                                              200
<210> 170
   <211> 200
   <212> DNA
   <213> Amaranthus palmeri
<400> 170
   taaggtgaag taaagagaaa agaaaggtca ccggaacgaa gaagaaaaga agcgtgaaca 60
   gagtttcctg tagaaaggtc agatttagca acggtaggca ttccaagacc ccaagaaaat 120
   cgtctgctaa cattggttgc atcgccacac caaaactcta tatggtggaa acgcttaaca 180
   gcaaaacggt cagatttggg 200
<210> 171
   <211> 200
   <212> DNA
   <213> Amaranthus palmeri
<400> 171
   tttcttttct ctttacttca ccttactctc ctaccatgtc catcccttct tctgctgcaa 60
   tcccctcgtt tgatttcaat cattttacca aattccttac atcgcacggt cttggcgtgc 120
   gtgctgttgc cgtcgaagta gaagacgcgg aagctgcttt taacatcagc gtttcgcatg 180
   gggctattcc ctgtgtttct 200
<210> 172
   <211> 200
   <212> DNA
   <213> Amaranthus palmeri
<400> 172
   agaaacacag ggaatagccc catgcgaaac gctgatgtta aaagcagctt ccgcgtcttc 60
   tacttcgacg gcaacagcac gcacgccaag accgtgcgat gtaaggaatt tggtaaaatg 120
   attgaaatca aacgagggga ttgcagcaga agaagggatg gacatggtag gagagtaagg 180
   tgaagtaaag agaaaagaaa 200
<210> 173
   <211> 200
   <212> DNA
   <213> Amaranthus palmeri
<400> 173
   gcatggggct attccctgtg tttctcctat tcaattggaa aacggtgtcg ttttatctga 60
   ggttcattta tatggggatg ttgtgcttcg gtatgtaagc tacggaaatg aatgtgggga 120
   tgtgtttttt cttcctgggt ttgaggaaat gccggaggaa tcatcgtttc gaggacttga 180
   ttttggcatt cgaaggttgg 200
<210> 174
   <211> 200
   <212> DNA
   <213> Amaranthus palmeri
<400> 174
   ccaaccttcg aatgccaaaa tcaagtcctc gaaacgatga ttcctccggc atttcctcaa 60
   acccaggaag aaaaaacaca tccccacatt catttccgta gcttacatac cgaagcacaa 120
   catccccata taaatgaacc tcagataaaa cgacaccgtt ttccaattga ataggagaaa 180
   cacagggaat agccccatgc 200
<210> 175
   <211> 200
   <212> DNA
   <213> Amaranthus palmeri
<400> 175
   cttgattttg gcattcgaag gttggatcat gctgtaggga atgtccctga gttggctcct 60
   gcaattgctt atttgaagaa gtttactggg tttcatgagt ttgctgagtt tacagctgaa 120
   gatgttggga cgagtgaaag tggattgaat tcagccgtat tggcaaacaa tgatgaaatg 180
   gtgttgtttc caatgaatga 200
<210> 176
   <211> 200
   <212> DNA
   <213> Amaranthus palmeri
<400> 176
   tcattcattg gaaacaacac catttcatca ttgtttgcca atacggctga attcaatcca 60
   ctttcactcg tcccaacatc ttcagctgta aactcagcaa actcatgaaa cccagtaaac 120
   ttcttcaaat aagcaattgc aggagccaac tcagggacat tccctacagc atgatccaac 180
   cttcgaatgc caaaatcaag 200
<210> 177
   <211> 200
   <212> DNA
   <213> Amaranthus palmeri
<400> 177
   aaatggtgtt gtttccaatg aatgaacctg tgtatgggac aaaaaggaag agccaaattc   60
   aaacttattt ggagcataat gaaggagctg gtgtacagca tttggctttg atgagtgaag  120
   acatattttg gactttaagg gagatgagga agagaagtgg tcttggtggg tttgaattta  180
   tgccgtcgcc gcctccgact                                              200
<210> 178
   <211> 200
   <212> DNA
   <213> Amaranthus palmeri
<400> 178
   agtcggaggc ggcgacggca taaattcaaa cccaccaaga ccacttctct tcctcatctc   60
   ccttaaagtc caaaatatgt cttcactcat caaagccaaa tgctgtacac cagctccttc  120
   attatgctcc aaataagttt gaatttggct cttccttttt gtcccataca caggttcatt  180
   cattggaaac aacaccattt                                              200
<210> 179
   <211> 200
   <212> DNA
   <213> Amaranthus palmeri
<400> 179
   atttatgccg tcgccgcctc cgacttatta ccggaatttg aggagcagag ctgctgatgt   60
   attgagtgag gagcagatga aggagtgtga agagttgggg attttggtgg ataaagatga  120
   tcagggcact ttgcttcaaa tcttcaccaa acctattgga gacaggccaa ccatattcat  180
   cgagattatc caaagaattg                                              200
<210> 180
   <211> 200
   <212> DNA
   <213> Amaranthus palmeri
<400> 180
   caattctttg gataatctcg atgaatatgg ttggcctgtc tccaataggt ttggtgaaga   60
   tttgaagcaa agtgccctga tcatctttat ccaccaaaat ccccaactct tcacactcct  120
   tcatctgctc ctcactcaat acatcagcag ctctgctcct caaattccgg taataagtcg  180
   gaggcggcga cggcataaat                                              200
<210> 181
   <211> 200
   <212> DNA
   <213> Amaranthus palmeri
<400> 181
   ttcatcgaga ttatccaaag aattggttgc atgatgaaag atgaagacgg caagatgtac 60
   caaaagggtg gttgcggagg atttggaaag ggaaactttt cagagttgtt caaatcaatt 120
   gaggagtacg agaagactct tgaacgtaaa caggttccag atacagctgc tgcatgatga 180
   gcagactgaa atattgctgt 200
<210> 182
   <211> 200
   <212> DNA
   <213> Amaranthus palmeri
<400> 182
   acagcaatat ttcagtctgc tcatcatgca gcagctgtat ctggaacctg tttacgttca 60
   agagtcttct cgtactcctc aattgatttg aacaactctg aaaagtttcc ctttccaaat 120
   cctccgcaac cacccttttg gtacatcttg ccgtcttcat ctttcatcat gcaaccaatt 180
   ctttggataa tctcgatgaa 200
<210> 183
   <211> 200
   <212> DNA
   <213> Amaranthus palmeri
<400> 183
   gatgagcaga ctgaaatatt gctgtcttgc tggtggaagc catataatgg taatatgata 60
   gaaaaggttt gttgctcaaa attttcttta ttaaataatg aagtgtaaac ttatacaaac 120
   tgtgtcatat atggtgattg atgatcatgc atggttatgt agaatgtata acataattga 180
   taatctgtgt atatgctgaa 200
<210> 184
   <211> 200
   <212> DNA
   <213> Amaranthus palmeri
<400> 184
   ttcagcatat acacagatta tcaattatgt tatacattct acataaccat gcatgatcat 60
   caatcaccat atatgacaca gtttgtataa gtttacactt cattatttaa taaagaaaat 120
   tttgagcaac aaaccttttc tatcatatta ccattatatg gcttccacca gcaagacagc 180
   aatatttcag tctgctcatc 200
<210> 185
   <211> 200
   <212> DNA
   <213> Amaranthus rudis
<400> 185
   aaatgaatgt ggggatgtgt tttttcttcc tgggtttgag gaaatgccgg aggaatcatc 60
   gtttcgagga cttgatttcg gccttcgaag gttggatcat gctgtaggga atgtccctga 120
   gttggctcct gcaattgctt atttgaagaa gtttactggg tttcatgagt ttgctgagtt 180
   tacagctgaa gatgttggga 200
<210> 186
   <211> 200
   <212> DNA
   <213> Amaranthus rudis
<400> 186
   tcccaacatc ttcagctgta aactcagcaa actcatgaaa cccagtaaac ttcttcaaat 60
   aagcaattgc aggagccaac tcagggacat tccctacagc atgatccaac cttcgaaggc 120
   cgaaatcaag tcctcgaaac gatgattcct ccggcatttc ctcaaaccca ggaagaaaaa 180
   acacatcccc acattcattt 200
<210> 187
   <211> 200
   <212> DNA
   <213> Amaranthus rudis
<400> 187
   gagtttacag ctgaagatgt tgggacgagt gaaagtgggt tgaattcagc cgtattggca 60
   aataatgatg aaatggtgtt gtttccaatg aatgaaccag tgtatgggac aaaaaggaag 120
   agtcaaattc aaacttattt ggagcataat gaaggagctg gtgtacaaca tttggctttg 180
   atgagtgaag atatattctg 200
<210> 188
   <211> 200
   <212> DNA
   <213> Amaranthus rudis
<400> 188
   cagaatatat cttcactcat caaagccaaa tgttgtacac cagctccttc attatgctcc   60
   aaataagttt gaatttgact cttccttttt gtcccataca ctggttcatt cattggaaac  120
   aacaccattt catcattatt tgccaatacg gctgaattca acccactttc actcgtccca  180
   acatcttcag ctgtaaactc                                              200
<210> 189
   <211> 200
   <212> DNA
   <213> Amaranthus rudis
<400> 189
   ctttgatgag tgaagatata ttctggactt taagggagat gaggaagaga agtggtcttg   60
   gtgggtttga gtttatgccg tcgccgcctc cgacttatta ccggaatttg aggaacagag  120
   ctgctgatgt attgagtgag gagcagatga aggagtgtga agagttgggg attttggtgg  180
   ataaagatga tcagggtact                                              200
<210> 190
   <211> 200
   <212> DNA
   <213> Amaranthus rudis
<400> 190
   agtaccctga tcatctttat ccaccaaaat ccccaactct tcacactcct tcatctgctc   60
   ctcactcaat acatcagcag ctctgttcct caaattccgg taataagtcg gaggcggcga  120
   cggcataaac tcaaacccac caagaccact tctcttcctc atctccctta aagtccagaa  180
   tatatcttca ctcatcaaag                                              200
<210> 191
   <211> 200
   <212> DNA
   <213> Amaranthus rudis
<400> 191
   ggtggataaa gatgatcagg gtactttgct tcaaatcttc accaaaccta ttggagacag   60
   gccaactata tttatcgaga tcatccaaag aattggttgc atgatgaaag atgaagacgg  120
   caagatgtac caaaagggtg gttgcggagg atttggaaag ggaaactttt cggagctttt  180
   caaatcaatt gaggagtacg                                              200
<210> 192
   <211> 200
   <212> DNA
   <213> Amaranthus rudis
<400> 192
   cgtactcctc aattgatttg aaaagctccg aaaagtttcc ctttccaaat cctccgcaac    60
   cacccttttg gtacatcttg ccgtcttcat ctttcatcat gcaaccaatt ctttggatga   120
   tctcgataaa tatagttggc ctgtctccaa taggtttggt gaagatttga agcaaagtac   180
   cctgatcatc tttatccacc                                                200
<210> 193
   <211> 200
   <212> DNA
   <213> Amaranthus rudis
<400> 193
   aatcatttta ccaaattcct tacatcgcac ggtcttggcg tgcgtgctgt tgctgtcgaa    60
   gtagaagacg cagaagctgc ttttaatatc agcgtttcca acggggctat tccctgtgtt   120
   tctcctattc acttggaaaa cggtgtcgtt ttatctgagg ttcatttata tggggatgtt   180
   gtgcttcggt atgtaagcta                                                200
<210> 194
   <211> 200
   <212> DNA
   <213> Amaranthus rudis
<400> 194
   tagcttacat accgaagcac aacatcccca tataaatgaa cctcagataa aacgacaccg    60
   ttttccaagt gaataggaga aacacaggga atagccccgt tggaaacgct gatattaaaa   120
   gcagcttctg cgtcttctac ttcgacagca acagcacgca cgccaagacc gtgcgatgta   180
   aggaatttgg taaaatgatt                                                200
<210> 195
   <211> 200
   <212> DNA
   <213> Amaranthus rudis
<400> 195
   atgttgtgct tcggtatgta agctacggaa atgaatgtgg ggatgtgttt tttcttcctg    60
   ggtttgagga aatgccggag gaatcatcgt ttcgaggact agatttcggc cttcgaaggt   120
   tggatcatgc tgtagggaat gtccctgagt tggctcctgc aattgcttat ttgaagaagt   180
   ttactgggtt tcatgagttt                                                200
<210> 196
   <211> 200
   <212> DNA
   <213> Amaranthus rudis
<400> 196
   aaactcatga aacccagtaa acttcttcaa ataagcaatt gcaggagcca actcagggac   60
   attccctaca gcatgatcca accttcgaag gccgaaatct agtcctcgaa acgatgattc  120
   ctccggcatt tcctcaaacc caggaagaaa aaacacatcc ccacattcat ttccgtagct  180
   tacataccga agcacaacat                                              200
<210> 197
   <211> 200
   <212> DNA
   <213> Amaranthus rudis
<400> 197
   gaagtttact gggtttcatg agtttgctga gtttacagct gaagatgttg ggacgagtga   60
   aagtgggttg aattcagccg tattggcaaa taatgatgaa atggtgttgt ttccaatgaa  120
   tgaaccagtg tatgggacaa aaaggaagag tcaaattcaa acttatttgg agcataatga  180
   aggagctggt gtacaacatt                                              200
<210> 198
   <211> 200
   <212> DNA
   <213> Amaranthus rudis
<400> 198
   aatgttgtac accagctcct tcattatgct ccaaataagt ttgaatttga ctcttccttt   60
   ttgtcccata cactggttca ttcattggaa acaacaccat ttcatcatta tttgccaata  120
   cggctgaatt caacccactt tcactcgtcc caacatcttc agctgtaaac tcagcaaact  180
   catgaaaccc agtaaacttc                                              200
<210> 199
   <211> 200
   <212> DNA
   <213> Amaranthus rudis
<400> 199
   aatgaaggag ctggtgtaca acatttggct ttgatgagtg aagatatatt ctggacttta   60
   agggagatga ggaagagaag tggtcttggt gggtttgagt ttatgccgtc gccgcctccg  120
   acttattacc ggaatttgag gaacagagct gctgatgtat tgagtgagga gcagatgaag  180
   gagtgtgaag agttggggat                                              200
<210> 200
   <211> 200
   <212> DNA
   <213> Amaranthus rudis
<400> 200
   atccccaact cttcacactc cttcatctgc tcctcactca atacatcagc agctctgttc 60
   ctcaaattcc ggtaataagt cggaggcggc gacggcataa actcaaaccc accaagacca 120
   cttctcttcc tcatctccct taaagtccag aatatatctt cactcatcaa agccaaatgt 180
   tgtacaccag ctccttcatt 200
<210> 201
   <211> 200
   <212> DNA
   <213> Amaranthus rudis
<400> 201
   accaccattt tcgtttcttc cccgtctgag ttttattact tcactttctc tctcatcatc 60
   tgacatggga actttgaaac ccgaaaatca acccgattcc gaattcaaac tcgtgggtta 120
   ctccaacttt gttcgggtta accccaaatc tgaccgtttt actgttaagc gtttccatca 180
   tatagagttt tggtgtggcg 200
<210> 202
   <211> 200
   <212> DNA
   <213> Amaranthus rudis
<400> 202
   cgccacacca aaactctata tgatggaaac gcttaacagt aaaacggtca gatttggggt 60
   taacccgaac aaagttggag taacccacga gtttgaattc ggaatcgggt tgattttcgg 120
   gtttcaaagt tcccatgtca gatgatgaga gagaaagtga agtaataaaa ctcagacggg 180
   gaagaaacga aaatggtggt 200
<210> 203
   <211> 200
   <212> DNA
   <213> Amaranthus rudis
<400> 203
   catcatatag agttttggtg tggcgacgca accaatgtta gcagacgatt ttcttggggt 60
   cttggaatgc ctaccgtcgc taaatctgac ctttctacgg gaaactctgt tcacgcttct 120
   tttcttcttc gttccggtga cctttctttc cttacttcac cttactcccc taccatgtcc 180
   atcccttctt ctgctgcaat 200
<210> 204
   <211> 200
   <212> DNA
   <213> Amaranthus rudis
<400> 204
   attgcagcag aagaagggat ggacatggta ggggagtaag gtgaagtaag gaaagaaagg 60
   tcaccggaac gaagaagaaa agaagcgtga acagagtttc ccgtagaaag gtcagattta 120
   gcgacggtag gcattccaag accccaagaa aatcgtctgc taacattggt tgcgtcgcca 180
   caccaaaact ctatatgatg 200
<210> 205
   <211> 200
   <212> DNA
   <213> Amaranthus rudis
<400> 205
   tgtccatccc ttcttctgct gcaatcccct cgtttgattt caatcatttt accaaattcc 60
   ttacatcgca cggtcttggc gtgcgtgctg ttgctgtcga agtagaagac gcagaagctg 120
   cttttaatat cagcgtttcc aacggggcta ttccctgtgt ttctcctatt cacttggaaa 180
   acggtgtcgt tttatctgag 200
<210> 206
   <211> 200
   <212> DNA
   <213> Amaranthus rudis
<400> 206
   ctcagataaa acgacaccgt tttccaagtg aataggagaa acacagggaa tagccccgtt 60
   ggaaacgctg atattaaaag cagcttctgc gtcttctact tcgacagcaa cagcacgcac 120
   gccaagaccg tgcgatgtaa ggaatttggt aaaatgattg aaatcaaacg aggggattgc 180
   agcagaagaa gggatggaca 200
<210> 207
   <211> 200
   <212> DNA
   <213> Amaranthus rudis
<400> 207
   ggaaaacggt gtcgttttat ctgaggttca tttatatggg gatgttgtgc ttcggtatgt 60
   aagctacgga aatgaatgtg gggatgtgtt ttttcttcct gggtttgagg aaatgccgga 120
   ggaatcttcg tttcgaggac ttgatttcgg ccttcgaagg ttggatcatg ctgtagggaa 180
   tgtccctgag ttggctcctg 200
<210> 208
   <211> 200
   <212> DNA
   <213> Amaranthus rudis
<400> 208
   caggagccaa ctcagggaca ttccctacag catgatccaa ccttcgaagg ccgaaatcaa 60
   gtcctcgaaa cgaagattcc tccggcattt cctcaaaccc aggaagaaaa aacacatccc 120
   cacattcatt tccgtagctt acataccgaa gcacaacatc cccatataaa tgaacctcag 180
   ataaaacgac accgttttcc 200
<210> 209
   <211> 200
   <212> DNA
   <213> Amaranthus rudis
<400> 209
   gggaatgtcc ctgagttggc tcctgcaatt gcttatttga agaagtttac tgggtttcat 60
   gagtttgctg agtttacagc tgaagatgtt gggacgagtg aaagtgggtt gaattcagcc 120
   gtattggcaa ataatgatga aatggtgttg tttccaatga atgaaccagt gtatgggaca 180
   aaaaggaaga gtcaaattca 200
<210> 210
   <211> 200
   <212> DNA
   <213> Amaranthus rudis
<400> 210
   tgaatttgac tcttcctttt tgtcccatac actggttcat tcattggaaa caacaccatt 60
   tcatcattat ttgccaatac ggctgaattc aacccacttt cactcgtccc aacatcttca 120
   gctgtaaact cagcaaactc atgaaaccca gtaaacttct tcaaataagc aattgcagga 180
   gccaactcag ggacattccc 200
<210> 211
   <211> 200
   <212> DNA
   <213> Amaranthus rudis
<400> 211
   ggacaaaaag gaagagtcaa attcaaactt atttggagca taatgaagga gctggtgtac   60
   aacatttggc tttgatgagt gaagatatat tctggacttt aagggagatg aggaagagaa  120
   gtggtcttgg tgggtttgag tttatgccgt cgccgcctcc gacttattac cggaatttga  180
   ggaacagagc tgctgatgta                                              200
<210> 212
   <211> 200
   <212> DNA
   <213> Amaranthus rudis
<400> 212
   tacatcagca gctctgttcc tcaaattccg gtaataagtc ggaggcggcg acggcataaa   60
   ctcaaaccca ccaagaccac ttctcttcct catctccctt aaagtccaga atatatcttc  120
   actcatcaaa gccaaatgtt gtacaccagc tccttcatta tgctccaaat aagtttgaat  180
   ttgactcttc ctttttgtcc                                              200
<210> 213
   <211> 200
   <212> DNA
   <213> Amaranthus rudis
<400> 213
   tttgaggaac agagctgctg atgtattgag tgaggagcag atgaaggagt gtgaagagtt   60
   ggggattttg gtggataaag atgatcaggg tactttgctt caaatcttca ccaaacctat  120
   tggagacagg tagtttttaa tcttgctttc aattgctttt gattaattga ttgattagcc  180
   aatttgatga ttgcattttg                                              200
<210> 214
   <211> 200
   <212> DNA
   <213> Amaranthus rudis
<400> 214
   caaaatgcaa tcatcaaatt ggctaatcaa tcaattaatc aaaagcaatt gaaagcaaga   60
   ttaaaaacta cctgtctcca ataggtttgg tgaagatttg aagcaaagta ccctgatcat  120
   ctttatccac caaaatcccc aactcttcac actccttcat ctgctcctca ctcaatacat  180
   cagcagctct gttcctcaaa                                              200
<210> 215
   <211> 200
   <212> DNA
   <213> Amaranthus rudis
<400> 215
   cttacatcgc acggtcttgg tgtgcgtgct gttgctgtcg aagtagagga cgcagaagct 60
   gcttttaata tcagcgtttc caacggggct attccctgtg tttctcctat tcaattggaa 120
   aacggtgtcg ttttatctga ggttcattta tatggggatg ttgtgcttcg ctatgtaagc 180
   tacggaaatg aacgtgggga 200
<210> 216
   <211> 200
   <212> DNA
   <213> Amaranthus rudis
<400> 216
   tccccacgtt catttccgta gcttacatag cgaagcacaa catccccata taaatgaacc 60
   tcagataaaa cgacaccgtt ttccaattga ataggagaaa cacagggaat agccccgttg 120
   gaaacgctga tattaaaagc agcttctgcg tcctctactt cgacagcaac agcacgcaca 180
   ccaagaccgt gcgatgtaag 200
<210> 217
   <211> 200
   <212> DNA
   <213> Amaranthus rudis
<400> 217
   taagctacgg aaatgaacgt ggggatgtgt tttttcttcc tgggtttgag gaaatgccgg 60
   aggaatcgtc gtttcgagga cttgatttcg gccttcgaag gttggatcat gctgtaggga 120
   atgttcccga gttggctcct gcaattgctt atttgaagaa gtttactggg tttcatgagt 180
   ttgctgagtt tacagctgaa 200
<210> 218
   <211> 200
   <212> DNA
   <213> Amaranthus rudis
<400> 218
   ttcagctgta aactcagcaa actcatgaaa cccagtaaac ttcttcaaat aagcaattgc 60
   aggagccaac tcgggaacat tccctacagc atgatccaac cttcgaaggc cgaaatcaag 120
   tcctcgaaac gacgattcct ccggcatttc ctcaaaccca ggaagaaaaa acacatcccc 180
   acgttcattt ccgtagctta 200
<210> 219
   <211> 200
   <212> DNA
   <213> Amaranthus rudis
<400> 219
   tgagtttgct gagtttacag ctgaagatgt tgggacgagt gaaagtgggt tgaattcagc 60
   cgtattggca aataatgatg aaatggtgtt gtttccaatg aatgaacctg tgtatgggac 120
   aaaaaggaag agtcaaattc aaacttattt ggagcataat gaaggagctg gtgtacaaca 180
   tttggctttg atgagtgaag 200
<210> 220
   <211> 200
   <212> DNA
   <213> Amaranthus rudis
<400> 220
   cttcactcat caaagccaaa tgttgtacac cagctccttc attatgctcc aaataagttt 60
   gaatttgact cttccttttt gtcccataca caggttcatt cattggaaac aacaccattt 120
   catcattatt tgccaatacg gctgaattca acccactttc actcgtccca acatcttcag 180
   ctgtaaactc agcaaactca 200
<210> 221
   <211> 200
   <212> DNA
   <213> Amaranthus rudis
<400> 221
   caacatttgg ctttgatgag tgaagatata ttttggactt taagggagat gaggaagaga 60
   agtggtcttg gtgggtttga gtttatgccg tcgccgcctc cgacttatta ccggaatttg 120
   aggaatagag ctgctgatgt attgagtgag gagcagatga aggagtgtga agagttgggg 180
   attttggtgg ataaagatga 200
<210> 222
   <211> 200
   <212> DNA
   <213> Amaranthus rudis
<400> 222
   tcatctttat ccaccaaaat ccccaactct tcacactcct tcatctgctc ctcactcaat 60
   acatcagcag ctctattcct caaattccgg taataagtcg gaggcggcga cggcataaac 120
   tcaaacccac caagaccact tctcttcctc atctccctta aagtccaaaa tatatcttca 180
   ctcatcaaag ccaaatgttg 200
<210> 223
   <211> 200
   <212> DNA
   <213> Amaranthus rudis
<400> 223
   tggggatttt ggtggataaa gatgatcagg gtactttgct tcaaatcttc actaagccta 60
   ttggtgacag gccaactata tttatcgaga tcatccaaag aattggttgc atgatgaaag 120
   atgaagacgg caagatgtac caaaagggtg gttgcggagg atttggaaag ggaaactttt 180
   cggagctttt caaatcaatt 200
<210> 224
   <211> 200
   <212> DNA
   <213> Amaranthus rudis
<400> 224
   aattgatttg aaaagctccg aaaagtttcc ctttccaaat cctccgcaac cacccttttg 60
   gtacatcttg ccgtcttcat ctttcatcat gcaaccaatt ctttggatga tctcgataaa 120
   tatagttggc ctgtcaccaa taggcttagt gaagatttga agcaaagtac cctgatcatc 180
   tttatccacc aaaatcccca 200
<210> 225
   <211> 200
   <212> DNA
   <213> Amaranthus rudis
<400> 225
   cttttcggag cttttcaaat caattgagga gtatgagaag actcttgaac gtaaacaggt 60
   tccagataca gctgctgcat gagcagacta aaatattgct gaaacgcagg ctgcagccat 120
   atgttagaac agtcattctg atggaaacac tcaagcggtg agtagctgag gttggtgatg 180
   ctgaagtcga gtcggtattt 200
<210> 226
   <211> 200
   <212> DNA
   <213> Amaranthus rudis
<400> 226
   aaataccgac tcgacttcag catcaccaac ctcagctact caccgcttga gtgtttccat 60
   cagaatgact gttctaacat atggctgcag cctgcgtttc agcaatattt tagtctgctc 120
   atgcagcagc tgtatctgga acctgtttac gttcaagagt cttctcatac tcctcaattg 180
   atttgaaaag ctccgaaaag 200
<210> 227
   <211> 200
   <212> DNA
   <213> Amaranthus rudis
<400> 227
   tcatcaacaa caaaagagtt gaaattctaa taataatccc agtaagcagt gagtaagatc 60
   aaatggagag tcagttagta gctaatcata caaaaccatt aaagctacaa agttactcta 120
   atttcttaag atcaaaccca aaatctgatc gtttcaaagt gaaaaggttc caccacattg 180
   agttctggtg tggtgatgca 200
<210> 228
   <211> 200
   <212> DNA
   <213> Amaranthus rudis
<400> 228
   tgcatcacca caccagaact caatgtggtg gaaccttttc actttgaaac gatcagattt 60
   tgggtttgat cttaagaaat tagagtaact ttgtagcttt aatggttttg tatgattagc 120
   tactaactga ctctccattt gatcttactc actgcttact gggattatta ttagaatttc 180
   aactcttttg ttgttgatga 200
<210> 229
   <211> 200
   <212> DNA
   <213> Amaranthus rudis
<400> 229
   cattgagttc tggtgtggtg atgcaaccaa cactagcctt ttattttcgt tagggcttgg 60
   catgcctatg gttgccaaat ccgatctctc cacaggcaac cttatacatg cctcctacgt 120
   cttacgtagt ggcgaacttt gtttcgtatt cacggctcct tactctccgt catccatgct 180
   cacaactgca tcaattcctt 200
<210> 230
   <211> 200
   <212> DNA
   <213> Amaranthus rudis
<400> 230
   aaggaattga tgcagttgtg agcatggatg acggagagta aggagccgtg aatacgaaac    60
   aaagttcgcc actacgtaag acgtaggagg catgtataag gttgcctgtg gagagatcgg   120
   atttggcaac cataggcatg ccaagcccta acgaaaataa aaggctagtg ttggttgcat   180
   caccacacca gaactcaatg                                               200
<210> 231
   <211> 200
   <212> DNA
   <213> Amaranthus thunbergii
<400> 231
   cccgagttgg ctcctgcaat tgcttatttg aagaagttta ctgggtttca tgagtttgct    60
   gagtttacag ctgaagatgt tgggacgagt gaaagtgggt tgaattcagc cgtattggca   120
   aataatgatg aaatggtgtt gtttccaatg aatgaacctg tgtatgggac aaaaaggaag   180
   agccaaattc aaacttattt                                               200
<210> 232
   <211> 200
   <212> DNA
   <213> Amaranthus thunbergii
<400> 232
   aaataagttt gaatttggct cttccttttt gtcccataca caggttcatt cattggaaac    60
   aacaccattt catcattatt tgccaatacg gctgaattca acccactttc actcgtccca   120
   acatcttcag ctgtaaactc agcaaactca tgaaacccag taaacttctt caaataagca   180
   attgcaggag ccaactcggg                                               200
<210> 233
   <211> 200
   <212> DNA
   <213> Amaranthus thunbergii
<400> 233
   ggaagagcca aattcaaact tatttggagc ataatgaagg agctggtgta caacatttgg    60
   ctttgatgag tgaagatata ttttggactt taagggagat gaggaagaga agtggtcttg   120
   gtgggtttga gtttatgccg tcgccgcctc cgacttatta ccggaatttg aggaatagag 180
   ctgctgatgt attgagtgag 200
<210> 234
   <211> 200
   <212> DNA
   <213> Amaranthus thunbergii
<400> 234
   ctcactcaat acatcagcag ctctattcct caaattccgg taataagtcg gaggcggcga 60
   cggcataaac tcaaacccac caagaccact tctcttcctc atctccctta aagtccaaaa 120
   tatatcttca ctcatcaaag ccaaatgttg tacaccagct ccttcattat gctccaaata 180
   agtttgaatt tggctcttcc 200
<210> 235
   <211> 200
   <212> DNA
   <213> Amaranthus thunbergii
<400> 235
   tagagctgct gatgtattga gtgaggagca gatgaaggag tgtgaagagt tggggatttt 60
   ggtggataaa gatgatcagg gtactttgct tcaaatcttc actaagccta ttggtgacag 120
   gccaaccata tttatcgaga ttatccaaag aattggatgc atgatgaaag atgaggacgg 180
   caagatgtac caaaagggtg 200
<210> 236
   <211> 200
   <212> DNA
   <213> Amaranthus thunbergii
<400> 236
   cacccttttg gtacatcttg ccgtcctcat ctttcatcat gcatccaatt ctttggataa 60
   tctcgataaa tatggttggc ctgtcaccaa taggcttagt gaagatttga agcaaagtac 120
   cctgatcatc tttatccacc aaaatcccca actcttcaca ctccttcatc tgctcctcac 180
   tcaatacatc agcagctcta 200
<210> 237
   <211> 200
   <212> DNA
   <213> Amaranthus viridis
<400> 237
   aaccaccatt ttctctctcc tccattacca ctaacactcc caccattttc gtttcttccc 60
   cgtctgagtc ttattacttc gctttctctc acatcatctg acatgggaac tttgaaaccc 120
   gaaactcagc ccgattccga attcaaactc gtgggatact ccaacttcgt tcgggttaac 180
   cccaaatctg accgttttac 200
<210> 238
   <211> 200
   <212> DNA
   <213> Amaranthus viridis
<400> 238
   gtaaaacggt cagatttggg gttaacccga acgaagttgg agtatcccac gagtttgaat 60
   tcggaatcgg gctgagtttc gggtttcaaa gttcccatgt cagatgatgt gagagaaagc 120
   gaagtaataa gactcagacg gggaagaaac gaaaatggtg ggagtgttag tggtaatgga 180
   ggagagagaa aatggtggtt 200
<210> 239
   <211> 200
   <212> DNA
   <213> Amaranthus viridis
<400> 239
   ttaaccccaa atctgaccgt tttactgtta agcgttttca tcatatagag ttttggtgtg 60
   gcgatgcaac caatgttagc agacgatttt cttggggtct tggaatgcct accgttgcta 120
   aatctgacct ttctactgga aactctgttc acgcttcttt tcttcttcgt tccggtgacc 180
   tttcttttct ctttacttca 200
<210> 240
   <211> 200
   <212> DNA
   <213> Amaranthus viridis
<400> 240
   tgaagtaaag agaaaagaaa ggtcaccgga acgaagaaga aaagaagcgt gaacagagtt 60
   tccagtagaa aggtcagatt tagcaacggt aggcattcca agaccccaag aaaatcgtct 120
   gctaacattg gttgcatcgc cacaccaaaa ctctatatga tgaaaacgct taacagtaaa 180
   acggtcagat ttggggttaa 200
<210> 241
   <211> 200
   <212> DNA
   <213> Amaranthus viridis
<400> 241
   tgacctttct tttctcttta cttcacctta ctctcctacc atgtccgtcc cttcttctgc   60
   tgcaatcccc tcgtttgatt tcaatcattt taccaaattc cttacatcgc acggtcttgg  120
   tgtgcgtgct gttgctgtcg aagtagaaga cgcagaagct gcttttaata tcagcgtttc  180
   caacggggct attccctgtg                                              200
<210> 242
   <211> 200
   <212> DNA
   <213> Amaranthus viridis
<400> 242
   cacagggaat agccccgttg gaaacgctga tattaaaagc agcttctgcg tcttctactt   60
   cgacagcaac agcacgcaca ccaagaccgt gcgatgtaag gaatttggta aaatgattga  120
   aatcaaacga ggggattgca gcagaagaag ggacggacat ggtaggagag taaggtgaag  180
   taaagagaaa agaaaggtca                                              200
<210> 243
   <211> 200
   <212> DNA
   <213> Amaranthus viridis
<400> 243
   gtttccaacg gggctattcc ctgtgtttct cctattcaat tggaaaacgg tgtcgtttta   60
   tctgaggttc atttatatgg ggatgttgtg cttcggtatg taagctacgg aaatgaatgt  120
   ggggatgtgt ttttcttcct gggtttgagg aaatgccgga ggaatcgtcg tttcgaggac  180
   ttgatttcgg ccttcgaagg                                              200
<210> 244
   <211> 200
   <212> DNA
   <213> Amaranthus viridis
<400> 244
   ccttcgaagg ccgaaatcaa gtcctcgaaa cgacgattcc tccggcattt cctcaaaccc   60
   aggaagaaaa acacatcccc acattcattt ccgtagctta cataccgaag cacaacatcc  120
   ccatataaat gaacctcaga taaaacgaca ccgttttcca attgaatagg agaaacacag  180
   ggaatagccc cgttggaaac                                              200
<210> 245
   <211> 200
   <212> DNA
   <213> Amaranthus viridis
<400> 245
   aggacttgat ttcggccttc gaaggttgga tcatgctgta gggaatgttc ccgagttggc 60
   tcctgcaatt gcttatttga aaaagtttac tgggtttcat gagtttgctg agtttacagc 120
   tgaagatgtt gggacgagtg aaagtgggtt gaattcagcc gtattggcaa ataatgatga 180
   aatggtgttg tttccaatga 200
<210> 246
   <211> 200
   <212> DNA
   <213> Amaranthus viridis
<400> 246
   tcattggaaa caacaccatt tcatcattat ttgccaatac ggctgaattc aacccacttt 60
   cactcgtccc aacatcttca gctgtaaact cagcaaactc atgaaaccca gtaaactttt 120
   tcaaataagc aattgcagga gccaactcgg gaacattccc tacagcatga tccaaccttc 180
   gaaggccgaa atcaagtcct 200
<210> 247
   <211> 200
   <212> DNA
   <213> Amaranthus viridis
<400> 247
   gatgaaatgg tgttgtttcc aatgaatgaa cctgtgtatg ggacaaaaag gaagagccaa 60
   attcaaactt atttggagca taatgaagga gctggtgtac aacatttggc tttgatgagt 120
   gaagatatat tttggacttt aagggagatg aggaagagaa gtggtcttgg tgggtttgag 180
   tttatgccgt cgccgcctcc 200
<210> 248
   <211> 200
   <212> DNA
   <213> Amaranthus viridis
<400> 248
   ggaggcggcg acggcataaa ctcaaaccca ccaagaccac ttctcttcct catctccctt 60
   aaagtccaaa atatatcttc actcatcaaa gccaaatgtt gtacaccagc tccttcatta 120
   tgctccaaat aagtttgaat ttggctcttc ctttttgtcc catacacagg ttcattcatt 180
   ggaaacaaca ccatttcatc 200
<210> 249
   <211> 200
   <212> DNA
   <213> Amaranthus viridis
<400> 249
   ttgagtttat gccgtcgccg cctccgactt attaccggaa tttgaggaat agagctgctg 60
   atgtattgag tgaggagcag atgaaggagt gtgaagagtt ggggattttg gttgacagag 120
   atgatcaagg cactctgctt caaattttca ctaagcccat tggagatagg cctacgatat 180
   tcatagaaat aatacagaga 200
<210> 250
   <211> 200
   <212> DNA
   <213> Amaranthus viridis
<400> 250
   tctctgtatt atttctatga atatcgtagg cctatctcca atgggcttag tgaaaatttg 60
   aagcagagtg ccttgatcat ctctgtcaac caaaatcccc aactcttcac actccttcat 120
   ctgctcctca ctcaatacat cagcagctct attcctcaaa ttccggtaat aagtcggagg 180
   cggcgacggc ataaactcaa 200
<210> 251
   <211> 200
   <212> DNA
   <213> Ambrosia trifida
<400> 251
   ccggtttacg ggacgaagag gaagagtcag atacagacat atttggaaca taatgaaggg 60
   gcaggggtgc agcatttggc gttggcgagt gaggatatat ttaggacatt gagggagatg 120
   aggaaaagga gtggggtggg tgggtttgag tttatgccat ctccgccgcc tacttattat 180
   cggaatttga agaacagggc 200
<210> 252
   <211> 200
   <212> DNA
   <213> Ambrosia trifida
<400> 252
   gccctgttct tcaaattccg ataataagta ggcggcggag atggcataaa ctcaaaccca   60
   cccaccccac tccttttcct catctccctc aatgtcctaa atatatcctc actcgccaac  120
   gccaaatgct gcacccctgc cccttcatta tgttccaaat atgtctgtat ctgactcttc  180
   ctcttcgtcc cgtaaaccgg                                              200
<210> 253
   <211> 200
   <212> DNA
   <213> Ambrosia trifida
<400> 253
   attatcggaa tttgaagaac agggcgggcg atgtgttgtc ggatgagcag attaaggagt   60
   gtgaggagtt ggggatattg gtggataagg atgatcaggg gactttgctt cagattttta  120
   ccaagcctgt tggtgatagg ccgacgatat tcatagagat aattcagaga gtaggatgta  180
   tgatgaagga tgaagaagga                                              200
<210> 254
   <211> 200
   <212> DNA
   <213> Ambrosia trifida
<400> 254
   tccttcttca tccttcatca tacatcctac tctctgaatt atctctatga atatcgtcgg   60
   cctatcacca acaggcttgg taaaaatctg aagcaaagtc ccctgatcat ccttatccac  120
   caatatcccc aactcctcac actccttaat ctgctcatcc gacaacacat cgcccgccct  180
   gttcttcaaa ttccgataat                                              200
<210> 255
   <211> 200
   <212> DNA
   <213> Ambrosia trifida
<400> 255
   atgtatgatg aaggatgaag aaggaaaggt gcagcagaag gcgggctgtg gaggatttgg   60
   taaagggaac ttttcggagc tttttaaatc gattgaggaa tatgaaaaga cactggaagc  120
   aagaacctga agccacatga aaaccacaca caaataatct ttcatgagat tttaaaatct  180
   aatgattatg catctgtgga                                              200
<210> 256
   <211> 200
   <212> DNA
   <213> Ambrosia trifida
<400> 256
   tccacagatg cataatcatt agattttaaa atctcatgaa agattatttg tgtgtggttt 60
   tcatgtggct tcaggttctt gcttccagtg tcttttcata ttcctcaatc gatttaaaaa 120
   gctccgaaaa gttcccttta ccaaatcctc cacagcccgc cttctgctgc acctttcctt 180
   cttcatcctt catcatacat 200
<210> 257
   <211> 54
   <212> DNA
   <213> Ambrosia trifida
<400> 257
   ctccgaggcg ttttgggtcc aaaatccgaa gcgcggggct taaagcgcga ggcg 54
<210> 258
   <211> 54
   <212> DNA
   <213> Ambrosia trifida
<400> 258
   cgcctcgcgc tttaagcccc gcgcttcgga ttttggaccc aaaacgcctc ggag 54
<210> 259
   <211> 200
   <212> DNA
   <213> Ambrosia trifida
<400> 259
   cttcatgtag ttcagcgctt tttggcaaaa aaagcgtttt tcttggagcc ttgtgcctca 60
   aacgcttaaa gtgcgcccca ggcgagcttt tttaaaccaa ggaacacgca caagcactcc 120
   accaatagtt caatgccgtg ctaaccagtg ggattaacta gtggaagctg gattcaaaca 180
   tgctagagtg gaaatatatt 200
<210> 260
   <211> 200
   <212> DNA
   <213> Ambrosia trifida
<400> 260
   aatatatttc cactctagca tgtttgaatc cagcttccac tagttaatcc cactggttag 60
   cacggcattg aactattggt ggagtgcttg tgcgtgttcc ttggtttaaa aaagctcgcc 120
   tggggcgcac tttaagcgtt tgaggcacaa ggctccaaga aaaacgcttt ttttgccaaa 180
   aagcgctgaa ctacatgaag 200
<210> 261
   <211> 200
   <212> DNA
   <213> Ambrosia trifida
<400> 261
   aaacatgcta gagtggaaat atattttttt taatgtttgg atatgcatat ttaaccatta   60
   tagcataata ttaatgacaa gggttgtaaa ttggtatgca aattgtttga gcaggccgac  120
   gatattcata gagataattc agagagtagg atgtatgatg aaggatgatg aaggaaaggt  180
   gcagcagaag gcgggttgtg                                              200
<210> 262
   <211> 200
   <212> DNA
   <213> Ambrosia trifida
<400> 262
   cacaacccgc cttctgctgc acctttcctt catcatcctt catcatacat cctactctct   60
   gaattatctc tatgaatatc gtcggcctgc tcaaacaatt tgcataccaa tttacaaccc  120
   ttgtcattaa tattatgcta taatggttaa atatgcatat ccaaacatta aaaaaaatat  180
   atttccactc tagcatgttt                                              200
<210> 263
   <211> 200
   <212> DNA
   <213> Ambrosia trifida
<400> 263
   aaggtgcagc agaaggcggg ttgtggagga tttggtaaag ggaacttttc ggagcttttt   60
   aaatcgattg aggaatatga aaagacactg gaagcaagaa cctgaagcca catgaaaacc  120
   acacaaataa tctttaatga gatcttaaaa tctaatgatt atgcatctgt ggattctata  180
   caacaaacag atcctgaaaa                                              200
<210> 264
   <211> 200
   <212> DNA
   <213> Ambrosia trifida
<400> 264
   ttttcaggat ctgtttgttg tatagaatcc acagatgcat aatcattaga ttttaagatc 60
   tcattaaaga ttatttgtgt ggttttcatg tggcttcagg ttcttgcttc cagtgtcttt 120
   tcatattcct caatcgattt aaaaagctcc gaaaagttcc ctttaccaaa tcctccacaa 180
   cccgccttct gctgcacctt 200
<210> 265
   <211> 200
   <212> DNA
   <213> Ambrosia trifida
<400> 265
   gcagttgagc tctacggctc actgaaacag acctttaaac catgaacaag gtttatgtgt 60
   aggtaaacta actttggacc aaaaagctgt ttttttaacc ttcacaattt gtttcatgga 120
   aggttttcct ttttgttcag ttgacatgtt tctaaacggg acaaataaac atacatctat 180
   gcctattgca catatagcat 200
<210> 266
   <211> 200
   <212> DNA
   <213> Ambrosia trifida
<400> 266
   atgctatatg tgcaataggc atagatgtat gtttatttgt cccgtttaga aacatgtcaa 60
   ctgaacaaaa aggaaaacct tccatgaaac aaattgtgaa ggttaaaaaa acagcttttt 120
   ggtccaaagt tagtttacct acacataaac cttgttcatg gtttaaaggt ctgtttcagt 180
   gagccgtaga gctcaactgc 200
<210> 267
   <211> 200
   <212> DNA
   <213> Ambrosia trifida
<400> 267
   tctatgccta ttgcacatat agcatcaacc acctcactcc catgtggccc tcacaacaag 60
   gttgtaaaaa tcgggactag tcgccgacta gtccccgata aatcggtgat cggaggtcta 120
   ctgattaatt tttcattaat cactcaatta gtcggaatcg gcccaagccg gtccaagccg 180
   gccaaagtcg gccgaagtcg 200
<210> 268
   <211> 200
   <212> DNA
   <213> Ambrosia trifida
<400> 268
   cgacttcggc cgactttggc cggcttggac cggcttgggc cgattccgac taattgagtg 60
   attaatgaaa aattaatcag tagacctccg atcaccgatt tatcggggac tagtcggcga 120
   ctagtcccga tttttacaac cttgttgtga gggccacatg ggagtgaggt ggttgatgct 180
   atatgtgcaa taggcataga 200
<210> 269
   <211> 200
   <212> DNA
   <213> Ambrosia trifida
<400> 269
   agccggccaa agtcggccga agtcggagct agtcgccaaa gaattccgtt tgaagttggc 60
   ttaaaaacat gctttcatcc cacaaactga tccattaccc caaaaaacca aagttaaaaa 120
   cctgacctgg atccctaaat gcttcataat ccggcaaacc tttctgttcc ggcgaacctt 180
   tctgtttccg gcgacccatt 200
<210> 270
   <211> 200
   <212> DNA
   <213> Ambrosia trifida
<400> 270
   aatgggtcgc cggaaacaga aaggttcgcc ggaacagaaa ggtttgccgg attatgaagc 60
   atttagggat ccaggtcagg tttttaactt tggttttttg gggtaatgga tcagtttgtg 120
   ggatgaaagc atgtttttaa gccaacttca aacggaattc tttggcgact agctccgact 180
   tcggccgact ttggccggct 200
<210> 271
   <211> 200
   <212> DNA
   <213> Ambrosia trifida
<400> 271
   acctttctgt ttccggcgac ccatttggag caggtggagt tggttttgtt cagtcttttg 60
   cggcgtataa tgaaagtggt agtgagtagg gtttttaagt ttggatcttt ttatatttaa 120
   ccccttatag ttttactagt ttatattttg tcccttaact tatatattta tacataataa 180
   gcataaagtt aatgttatat 200
<210> 272
   <211> 200
   <212> DNA
   <213> Ambrosia trifida
<400> 272
   atataacatt aactttatgc ttattatgta taaatatata agttaaggga caaaatataa    60
   actagtaaaa ctataagggg ttaaatataa aaagatccaa acttaaaaac cctactcact   120
   accactttca ttatacgccg caaaagactg aacaaaacca actccacctg ctccaaatgg   180
   gtcgccggaa acagaaaggt                                               200
<210> 273
   <211> 200
   <212> DNA
   <213> Ambrosia trifida
<400> 273
   tacctcacaa acccaatcct ccatacttcc gtgttctcta ctactgttcc actttattaa    60
   tcttacccaa ccccttctca tctatttccc ccctacctca caaacataac ccaaataaaa   120
   aataaaatta aaataaaaaa taaaatggaa ctgaacccac cctcgttgac cccgtcgtcg   180
   ccggagacaa ctccgaccac                                               200
<210> 274
   <211> 200
   <212> DNA
   <213> Ambrosia trifida
<400> 274
   gtggtcggag ttgtctccgg cgacgacggg gtcaacgagg gtgggttcag ttccatttta    60
   ttttttattt taattttatt ttttatttgg gttatgtttg tgaggtaggg gggaaataga   120
   tgagaagggg ttgggtaaga ttaataaagt ggaacagtag tagagaacac ggaagtatgg   180
   aggattgggt ttgtgaggta                                               200
<210> 275
   <211> 200
   <212> DNA
   <213> Ambrosia trifida
<400> 275
   cgtcgccgga gacaactccg accactccaa ccaccccatc tccgccttca agctagtcgg    60
   cttcaaaaac ttcatccgta acaaccctaa gtccgacaaa ttctccgtca aatccttcca   120
   ccacatcgag ttctggtgct ccgacgccac caacgccgcc cgtcgcttct catggggtct   180
   cggcatgcct attacttaca                                               200
<210> 276
   <211> 200
   <212> DNA
   <213> Ambrosia trifida
<400> 276
   tgtaagtaat aggcatgccg agaccccatg agaagcgacg ggcggcgttg gtggcgtcgg 60
   agcaccagaa ctcgatgtgg tggaaggatt tgacggagaa tttgtcggac ttagggttgt 120
   tacggatgaa gtttttgaag ccgactagct tgaaggcgga gatggggtgg ttggagtggt 180
   cggagttgtc tccggcgacg 200
<210> 277
   <211> 200
   <212> DNA
   <213> Ambrosia trifida
<400> 277
   ggtctcggca tgcctattac ttacaaatcc gacctttcta ccggtaacca tacccacgcc 60
   tcttatctcc ttaactccgg ccaccttaac ttcctcttca ccgcccctta ttcccccacc 120
   atctccacca ccaccaccac cgcctccatc ccgtcgttct cccactccgc ctgccgtcac 180
   ttcaccgcct cccacggcct 200
<210> 278
   <211> 200
   <212> DNA
   <213> Ambrosia trifida
<400> 278
   aggccgtggg aggcggtgaa gtgacggcag gcggagtggg agaacgacgg gatggaggcg 60
   gtggtggtgg tggtggagat ggtgggggaa taaggggcgg tgaagaggaa gttaaggtgg 120
   ccggagttaa ggagataaga ggcgtgggta tggttaccgg tagaaaggtc ggatttgtaa 180
   gtaataggca tgccgagacc 200
<210> 279
   <211> 200
   <212> DNA
   <213> Ambrosia trifida
<400> 279
   tcgtcgtttc aggagctgga ttacggtatc cggcggctag atcacgctgt ggggaacgta 60
   ccggagttag caccagcagt ggaatatata aaatcgttta ccgggtttca cgagtttgct 120
   gagtttacag cagaggatgt gggaacgagt gagagtggac tcaactcggt ggtgttggct 180
   tgcaatagtg agatggtatt 200
<210> 280
   <211> 200
   <212> DNA
   <213> Ambrosia trifida
<400> 280
   aataccatct cactattgca agccaacacc accgagttga gtccactctc actcgttccc 60
   acatcctctg ctgtaaactc agcaaactcg tgaaacccgg taaacgattt tatatattcc 120
   actgctggtg ctaactccgg tacgttcccc acagcgtgat ctagccgccg gataccgtaa 180
   tccagctcct gaaacgacga 200
<210> 281
   <211> 200
   <212> DNA
   <213> Ambrosia trifida
<400> 281
   tggcttgcaa tagtgagatg gtattaatac ctctgaatga gccggtttac gggacgaaga 60
   ggaagagtca gatacagaca tatttggaac ataatgaagg ggcaggggtg cagcatttgg 120
   cgttggcgag tgaggatata tttaggacat tgagggagat gaggaaaagg agtggggtgg 180
   gtgggtttga gtttatgccg 200
<210> 282
   <211> 200
   <212> DNA
   <213> Ambrosia trifida
<400> 282
   cggcataaac tcaaacccac ccaccccact ccttttcctc atctccctca atgtcctaaa 60
   tatatcctca ctcgccaacg ccaaatgctg cacccctgcc ccttcattat gttccaaata 120
   tgtctgtatc tgactcttcc tcttcgtccc gtaaaccggc tcattcagag gtattaatac 180
   catctcacta ttgcaagcca 200
<210> 283
   <211> 200
   <212> DNA
   <213> Ambrosia trifida
<400> 283
   ggtgggtggg tttgagttta tgccgtctcc gccgcctact tattatcgga atttgaagaa    60
   tagggcgggc gatgtgttgt cggatgagca gattaaggag tgtgaggagt tggggatatt   120
   ggtggataag gatgatcagg ggactttgct tcagattttt accaagcctg ttggtgatag   180
   gtattgtttt tcgttaatgt                                                200
<210> 284
   <211> 200
   <212> DNA
   <213> Ambrosia trifida
<400> 284
   acattaacga aaaacaatac ctatcaccaa caggcttggt aaaaatctga agcaaagtcc    60
   cctgatcatc cttatccacc aatatcccca actcctcaca ctccttaatc tgctcatccg   120
   acaacacatc gcccgcccta ttcttcaaat tccgataata agtaggcggc ggagacggca   180
   taaactcaaa cccacccacc                                                200
<210> 285
   <211> 200
   <212> DNA
   <213> Ambrosia trifida
<400> 285
   gataggtatt gtttttcgtt aatgtagcgt tacttgaatt cgtaattatg tgtttaaact    60
   ttaaactaga tagtattaag gcttatgcaa tatgtattgt tcccctagag gacgagggtt   120
   ctagactcta gttccgtaat gtagtgataa aggtgtagat taagagtagg attagcgggt   180
   gtgttagtta gcggttttaa                                                200
<210> 286
   <211> 200
   <212> DNA
   <213> Ambrosia trifida
<400> 286
   ttaaaaccgc taactaacac acccgctaat cctactctta atctacacct ttatcactac    60
   attacggaac tagagtctag aaccctcgtc ctctagggga acaatacata ttgcataagc   120
   cttaatacta tctagtttaa agtttaaaca cataattacg aattcaagta acgctacatt   180
   aacgaaaaac aatacctatc                                                200
<210> 287
   <211> 200
   <212> DNA
   <213> Ambrosia trifida
<400> 287
   cgggtgtgtt agttagcggt tttaacaaga aaaacacata atatgttgaa agaacaataa 60
   caagaatggc aaacataaaa aaggaaaagc cataaaggtt gtcattctat aatttgcttg 120
   cattcaagat tcttactagg attgtattat tatgtggtta ctaaactcta ttcaagtata 180
   aaaacatgaa accccttcac 200
<210> 288
   <211> 200
   <212> DNA
   <213> Ambrosia trifida
<400> 288
   gtgaaggggt ttcatgtttt tatacttgaa tagagtttag taaccacata ataatacaat 60
   cctagtaaga atcttgaatg caagcaaatt atagaatgac aacctttatg gcttttcctt 120
   ttttatgttt gccattcttg ttattgttct ttcaacatat tatgtgtttt tcttgttaaa 180
   accgctaact aacacacccg 200
<210> 289
   <211> 200
   <212> DNA
   <213> Conyza canadensis
<400> 289
   aacacgaatg ggctagctag ctgatgaatt taatgtatat atgtctttaa aaaccacttt 60
   catgggctac ttgtgttttt gatgagtttt tcaacatcat tctcatctta ccttcttaca 120
   atggttcacc atattgcttc gtttgtcgtc tcctgcatgg ttatcgaatt tattttttca 180
   aattatatta ctattatctt 200
<210> 290
   <211> 200
   <212> DNA
   <213> Conyza canadensis
<400> 290
   aagataatag taatataatt tgaaaaaata aattcgataa ccatgcagga gacgacaaac 60
   gaagcaatat ggtgaaccat tgtaagaagg taagatgaga atgatgttga aaaactcatc 120
   aaaaacacaa gtagcccatg aaagtggttt ttaaagacat atatacatta aattcatcag 180
   ctagctagcc cattcgtgtt 200
<210> 291
   <211> 200
   <212> DNA
   <213> Conyza canadensis
<400> 291
   tttcaaatta tattactatt atctttttgt tttctgattt ctcacatgta atttacatta 60
   tgagtttcac catccgtatg taaattgtaa aataaatcac aaatttggtt tttaaccatc 120
   aactaaattc attgaaatcc ttaagttata acccatttga ggttatagat tatgagagta 180
   gattcctatt ttctctttgg 200
<210> 292
   <211> 200
   <212> DNA
   <213> Conyza canadensis
<400> 292
   ccaaagagaa aataggaatc tactctcata atctataacc tcaaatgggt tataacttaa 60
   ggatttcaat gaatttagtt gatggttaaa aaccaaattt gtgatttatt ttacaattta 120
   catacggatg gtgaaactca taatgtaaat tacatgtgag aaatcagaaa acaaaaagat 180
   aatagtaata taatttgaaa 200
<210> 293
   <211> 200
   <212> DNA
   <213> Conyza canadensis
<400> 293
   gagtagattc ctattttctc tttggcttat gtatccttaa ttatgtttga attgattcta 60
   actttattat attttaggaa ttacttaact tcaaaaatct caacacgtca tataatgctt 120
   tttgtggatg gatgcttttg acccatttcg ttgaaaactt atgtcgacac acatatctga 180
   caaacttgta ctcgtatatc 200
<210> 294
   <211> 200
   <212> DNA
   <213> Conyza canadensis
<400> 294
   gatatacgag tacaagtttg tcagatatgt gtgtcgacat aagttttcaa cgaaatgggt   60
   caaaagcatc catccacaaa aagcattata tgacgtgttg agatttttga agttaagtaa  120
   ttcctaaaat ataataaagt tagaatcaat tcaaacataa ttaaggatac ataagccaaa  180
   gagaaaatag gaatctactc                                              200
<210> 295
   <211> 200
   <212> DNA
   <213> Conyza canadensis
<400> 295
   tctgacaaac ttgtactcgt atatccttta tcgcatatgc tatagtataa tattcgaaat   60
   ttaatctcaa gataattttc ccaaaaggtc atattaattg aattgagatt aattcctacc  120
   taacatatat agatatattg tgttggtgtt atcctctttg gagttttagt cgcgcatgta  180
   ttctaagaaa atggtaattt                                              200
<210> 296
   <211> 200
   <212> DNA
   <213> Conyza canadensis
<400> 296
   aaattaccat tttcttagaa tacatgcgcg actaaaactc caaagaggat aacaccaaca   60
   caatatatct atatatgtta ggtaggaatt aatctcaatt caattaatat gaccttttgg  120
   gaaaattatc ttgagattaa atttcgaata ttatactata gcatatgcga taaaggatat  180
   acgagtacaa gtttgtcaga                                              200
<210> 297
   <211> 200
   <212> DNA
   <213> Conyza canadensis
<400> 297
   atgtattcta agaaaatggt aattttgaac ttatattgat ggtctaaatt ttatcctatc   60
   actaaaattt cagtcatgtt tcctagctaa ttaatttggg ttcaatccag gtattctaaa  120
   taagttttgt aaatacatgt ctattatcat aatgaaacat agaaaaaccc gtaaagtaaa  180
   aatgtctata tttttagtgg                                              200
<210> 298
   <211> 200
   <212> DNA
   <213> Conyza canadensis
<400> 298
   ccactaaaaa tatagacatt tttactttac gggtttttct atgtttcatt atgataatag   60
   acatgtattt acaaaactta tttagaatac ctggattgaa cccaaattaa ttagctagga  120
   aacatgactg aaattttagt gataggataa aatttagacc atcaatataa gttcaaaatt  180
   accattttct tagaatacat                                              200
<210> 299
   <211> 200
   <212> DNA
   <213> Conyza canadensis
<400> 299
   gtaaaaatgt ctatattttt agtggcaaat tattaatgtt gctataatga aaagagaaga   60
   ttcaaccttt tgtacatcat aaaaaatgtt attctacatt atgctaattt tacattagac  120
   ttcatagttt tgaaaacttt caaatgaagg gacttaaatt ttaaatttaa agatgatgtc  180
   ataaattagt tagataaaag                                              200
<210> 300
   <211> 200
   <212> DNA
   <213> Conyza canadensis
<400> 300
   cttttatcta actaatttat gacatcatct ttaaatttaa aatttaagtc ccttcatttg   60
   aaagttttca aaactatgaa gtctaatgta aaattagcat aatgtagaat aacatttttt  120
   atgatgtaca aaaggttgaa tcttctcttt tcattatagc aacattaata atttgccact  180
   aaaaatatag acatttttac                                              200
<210> 301
   <211> 200
   <212> DNA
   <213> Conyza canadensis
<400> 301
   atgtcataaa ttagttagat aaaagttaaa taattaataa gaaaaggtct aatgatacca   60
   aataaaattt tcttaaaaaa gtattaatgt cattacaatt ttattttatt tatataagag  120
   aagataatag attttcttgt ataaaaaact tacctgtaaa cttctatata aactttttag  180
   aaaaataatt taaaacggtc                                              200
<210> 302
   <211> 200
   <212> DNA
   <213> Conyza canadensis
<400> 302
   gaccgtttta aattattttt ctaaaaagtt tatatagaag tttacaggta agttttttat 60
   acaagaaaat ctattatctt ctcttatata aataaaataa aattgtaatg acattaatac 120
   ttttttaaga aaattttatt tggtatcatt agaccttttc ttattaatta tttaactttt 180
   atctaactaa tttatgacat 200
<210> 303
   <211> 200
   <212> DNA
   <213> Conyza canadensis
<400> 303
   tttagaaaaa taatttaaaa cggtctcaaa tatcgttatt tccgaacata caaaaaccca 60
   aaacctaatc catttaatta tattgtaaga cctcgattag cttaatttat tttgaaacct 120
   atttacaaat gagtttggca tgatatgaac ttttttgcat ttacatatgg ctgtaaacga 180
   actgaacagt tcacgaacaa 200
<210> 304
   <211> 200
   <212> DNA
   <213> Conyza canadensis
<400> 304
   ttgttcgtga actgttcagt tcgtttacag ccatatgtaa atgcaaaaaa gttcatatca 60
   tgccaaactc atttgtaaat aggtttcaaa ataaattaag ctaatcgagg tcttacaata 120
   taattaaatg gattaggttt tgggtttttg tatgttcgga aataacgata tttgagaccg 180
   ttttaaatta tttttctaaa 200
<210> 305
   <211> 200
   <212> DNA
   <213> Conyza canadensis
<400> 305
   aacgaactga acagttcacg aacaattcat gaaccgttcg gcgggaggtt cgttcgtgtt 60
   agttcgttta gttaaatgaa tgaacatgaa caaagctctc gttcgttcat ttacgttcat 120
   gaacgttcga tagtctgttc gtgaacttta gttcgtctat gttcatgaac tgtcgttcgt 180
   gaacaataat ttgattatgt 200
<210> 306
   <211> 200
   <212> DNA
   <213> Conyza canadensis
<400> 306
   acataatcaa attattgttc acgaacgaca gttcatgaac atagacgaac taaagttcac 60
   gaacagacta tcgaacgttc atgaacgtaa atgaacgaac gagagctttg ttcatgttca 120
   ttcatttaac taaacgaact aacacgaacg aacctcccgc cgaacggttc atgaattgtt 180
   cgtgaactgt tcagttcgtt 200
<210> 307
   <211> 200
   <212> DNA
   <213> Conyza canadensis
<400> 307
   ttcgtgaaca ataatttgat tatgttcatt tacacacaca tacataatca actgtactat 60
   aaaaatagtt taaatataaa tattttttta tataatatat attattaata tctaattatt 120
   taagaaaaag tttaaataaa atacttaatt tataaatatt tcgttcgttt gtgttcgtgt 180
   acatttgttc atgaacacaa 200
<210> 308
   <211> 200
   <212> DNA
   <213> Conyza canadensis
<400> 308
   ttgtgttcat gaacaaatgt acacgaacac aaacgaacga aatatttata aattaagtat 60
   tttatttaaa ctttttctta aataattaga tattaataat atatattata taaaaaaata 120
   tttatattta aactattttt atagtacagt tgattatgta tgtgtgtgta aatgaacata 180
   atcaaattat tgttcacgaa 200
<210> 309
   <211> 200
   <212> DNA
   <213> Conyza canadensis
<400> 309
   cgtgtacatt tgttcatgaa cacaaatgaa cgaacatgaa caagtcatta tgttcgttta 60
   tctgttcgtt aagttaaatg aacgaacgaa cacgaacaag gtctcgtttg tgttcgttcg 120
   gttcgtttac ggcccttaat tacatgtgaa gttaataaat aaaaataaag ttcatcaaaa 180
   ttaaacctat ggttggagat 200
<210> 310
   <211> 200
   <212> DNA
   <213> Conyza canadensis
<400> 310
   atctccaacc ataggtttaa ttttgatgaa ctttattttt atttattaac ttcacatgta 60
   attaagggcc gtaaacgaac cgaacgaaca caaacgagac cttgttcgtg ttcgttcgtt 120
   catttaactt aacgaacaga taaacgaaca taatgacttg ttcatgttcg ttcatttgtg 180
   ttcatgaaca aatgtacacg 200
<210> 311
   <211> 200
   <212> DNA
   <213> Conyza canadensis
<400> 311
   caaaattaaa cctatggttg gagataaaac cagtcagcct tacgtgtgga aactatccta 60
   ctctacaatt aatagcaccc aaagaaaata aaccacttcc gtgttcttct tcatctccat 120
   catccaaccc ctcaactact tgtactgtat ctatatctat cattctatat atctacttca 180
   tatttctatc tatacattca 200
<210> 312
   <211> 200
   <212> DNA
   <213> Conyza canadensis
<400> 312
   tgaatgtata gatagaaata tgaagtagat atatagaatg atagatatag atacagtaca 60
   agtagttgag gggttggatg atggagatga agaagaacac ggaagtggtt tattttcttt 120
   gggtgctatt aattgtagag taggatagtt tccacacgta aggctgactg gttttatctc 180
   caaccatagg tttaattttg 200
<210> 313
   <211> 200
   <212> **DNA**
   <213> Conyza canadensis
<400> 313
   cttcatattt ctatctatac attcaaatta ataaaaacaa aatggtaact gaagcagcaa 60
   acggaaacac actcaccgga caacaacaaa ccactaccac cacctccgcc acttacaaac 120
   tcgtcggctt caaaaacttc atccgtcaaa accctaaatc cgacaaattc accgtcaaaa 180
   aattccacca cgtcgaattc 200
<210> 314
   <211> 200
   <212> DNA
   <213> Conyza canadensis
<400> 314
   gaattcgacg tggtggaatt ttttgacggt gaatttgtcg gatttagggt tttgacggat 60
   gaagtttttg aagccgacga gtttgtaagt ggcggaggtg gtggtagtgg tttgttgttg 120
   tccggtgagt gtgtttccgt ttgctgcttc agttaccatt ttgtttttat taatttgaat 180
   gtatagatag aaatatgaag 200
<210> 315
   <211> 200
   <212> DNA
   <213> Conyza canadensis
<400> 315
   caaaaaattc caccacgtcg aattctggtg ttccgacgcc accaacaccg cccgccgctt 60
   ctcttggggt ctcggcatgc cgcttcttta caaatccgat ctctccaccg gtaacaacac 120
   tcacgcttct taccttatta actccggcca gctcaacttc ctcttcaccg caccttattc 180
   caccaccatc tccaccaccg 200
<210> 316
   <211> 200
   <212> DNA
   <213> Conyza canadensis
<400> 316
   cggtggtgga gatggtggtg gaataaggtg cggtgaagag gaagttgagc tggccggagt 60
   taataaggta agaagcgtga gtgttgttac cggtggagag atcggatttg taaagaagcg 120
   gcatgccgag accccaagag aagcggcggg cggtgttggt ggcgtcggaa caccagaatt 180
   cgacgtggtg gaattttttg 200
<210> 317
   <211> 200
   <212> **DNA**
   <213> Conyza canadensis
<400> 317
   tattccacca ccatctccac caccggttcc acatcgtcta ttcctacttt ctctcacact   60
   gcttgccgga acttcaccgc cactcacggc ctcgctgtac ggtctatcgc cgttgaagtt  120
   gaagacgctg aaatcgctta ttctgttagt atcgctcacg gtgctaaacc gtcgtcagct  180
   ccggtcactc taggaaataa                                               200
<210> 318
   <211> 200
   <212> DNA
   <213> Conyza canadensis
<400> 318
   ttatttccta gagtgaccgg agctgacgac ggtttagcac cgtgagcgat actaacagaa   60
   taagcgattt cagcgtcttc aacttcaacg gcgatagacc gtacagcgag gccgtgagtg  120
   gcggtgaagt tccggcaagc agtgtgagag aaagtaggaa tagacgatgt ggaaccggtg  180
   gtggagatgg tggtggaata                                               200
<210> 319
   <211> 200
   <212> DNA
   <213> Conyza canadensis
<400> 319
   cagctccggt cactctagga aataacgacg tcgttttggc tgaagttaaa ctgtacggcg   60
   atgtcgtttt gagatatgtt agttttaaaa acactaatta tgatgttact actaatttcc  120
   taccgggttt tgagcctgtg gacgcaacgt cgtcgtttca tgaacttaat tacggaattc  180
   ataagttaga tcacgccgta                                               200
<210> 320
   <211> 200
   <212> DNA
   <213> Conyza canadensis
<400> 320
   tacggcgtga tctaacttat gaattccgta attaagttca tgaaacgacg acgttgcgtc   60
   cacaggctca aaacccggta ggaaattagt agtaacatca taattagtgt ttttaaaact  120
   aacatatctc aaaacgacat cgccgtacag tttaacttca gccaaaacga cgtcgttatt  180
   tcctagagtg accggagctg                                               200
<210> 321
   <211> 200
   <212> DNA
   <213> Conyza canadensis
<400> 321
   aattcataag ttagatcacg ccgtaggaaa cgtgccggag ctagctccgg cagtagacta 60
   cgtgaagtcc ttcactggat ttcacgaatt tgctgaattt acggcagagg atgtaggaac 120
   gagtgagagc gggcttaatt cggttgtatt agcgtgtaat agtgaaacgg ttttgatacc 180
   gatgaacgag ccagtttatg 200
<210> 322
   <211> 200
   <212> DNA
   <213> Conyza canadensis
<400> 322
   cataaactgg ctcgttcatc ggtatcaaaa ccgtttcact attacacgct aatacaaccg 60
   aattaagccc gctctcactc gttcctacat cctctgccgt aaattcagca aattcgtgaa 120
   atccagtgaa ggacttcacg tagtctactg ccggagctag ctccggcacg tttcctacgg 180
   cgtgatctaa cttatgaatt 200
<210> 323
   <211> 200
   <212> DNA
   <213> Conyza canadensis
<400> 323
   ataccgatga acgagccagt ttatggaacg aaaaggaaga gccagataca gacgtatttg 60
   gaacataatg agggagctgg tgtgcagcat ttggcgttgg cgagtgagga tatttttagg 120
   actttgagag agatgagaaa acggagtagt atcggtggtt ttgagtttat gccgtctcca 180
   ccgcctactt attataagaa 200
<210> 324
   <211> 200
   <212> DNA
   <213> Conyza canadensis
<400> 324
   ttcttataat aagtaggcgg tggagacggc ataaactcaa aaccaccgat actactccgt 60
   tttctcatct ctctcaaagt cctaaaaata tcctcactcg ccaacgccaa atgctgcaca 120
   ccagctccct cattatgttc caaatacgtc tgtatctggc tcttcctttt cgttccataa 180
   actggctcgt tcatcggtat 200
<210> 325
   <211> 200
   <212> DNA
   <213> Conyza canadensis
<400> 325
   ctccaccgcc tacttattat aagaatttga agaatagggt cggggacgtg ttgagtgacg 60
   aacagattaa ggaatgtgag gaattgggga ttttggttga tagagatgat cagggaactt 120
   tgcttcagat tttcaccaag cctgttggtg ataggtatgt ctatgttaac tttatcagtg 180
   attgtgcatt tgtccatttt 200
<210> 326
   <211> 200
   <212> DNA
   <213> Conyza canadensis
<400> 326
   aaaatggaca aatgcacaat cactgataaa gttaacatag acatacctat caccaacagg 60
   cttggtgaaa atctgaagca aagttccctg atcatctcta tcaaccaaaa tccccaattc 120
   ctcacattcc ttaatctgtt cgtcactcaa cacgtccccg accctattct tcaaattctt 180
   ataataagta ggcggtggag 200
<210> 327
   <211> 200
   <212> DNA
   <213> Conyza canadensis
<400> 327
   cagtgattgt gcatttgtcc attttgttga attcgtaaaa catgagatta aatacctgca 60
   atatgttttg cttacattga atctaggcca ggttttcagt gaacaagtac cataaatgta 120
   taatgtaggt ctagtcttgt taatgatcag tgatcaatag tgcatacatt gaatgacgaa 180
   ttgacaacta gtttctaata 200
<210> 328
   <211> 200
   <212> DNA
   <213> Conyza canadensis
<400> 328
   tattagaaac tagttgtcaa ttcgtcattc aatgtatgca ctattgatca ctgatcatta 60
   acaagactag acctacatta tacatttatg gtacttgttc actgaaaacc tggcctagat 120
   tcaatgtaag caaaacatat tgcaggtatt taatctcatg ttttacgaat tcaacaaaat 180
   ggacaaatgc acaatcactg 200
<210> 329
   <211> 200
   <212> DNA
   <213> Conyza canadensis
<400> 329
   acgaattgac aactagtttc taatagaatg tgaaatataa agtgaatgtc acaaaagctg 60
   ttatcttatc tattttagtt gtaatggacc aatggttgga ctgtttgaaa gtgtgcttac 120
   aatcaagatt cgagattgtt actaggatgc tagcgctagg tatatatcat atggtgtctg 180
   agctctatgg tagtataaat 200
<210> 330
   <211> 200
   <212> DNA
   <213> Conyza canadensis
<400> 330
   atttatacta ccatagagct cagacaccat atgatatata cctagcgcta gcatcctagt 60
   aacaatctcg aatcttgatt gtaagcacac tttcaaacag tccaaccatt ggtccattac 120
   aactaaaata gataagataa cagcttttgt gacattcact ttatatttca cattctatta 180
   gaaactagtt gtcaattcgt 200
<210> 331
   <211> 200
   <212> DNA
   <213> Conyza canadensis
<400> 331
   gtctgagctc tatggtagta taaattgtga atacactaaa catctcccaa aaatatttga 60
   gtaaccgatc aatctactag ttattgcaat gccaaacatc tctttaaaca actacttatt 120
   gtaaagccat actacatcaa cacaccaaaa atctatctcc cacaaccccc gttttacttg 180
   ggttatgaac ttagtatagt 200
<210> 332
   <211> 200
   <212> DNA
   <213> Conyza canadensis
<400> 332
   actatactaa gttcataacc caagtaaaac gggggttgtg ggagatagat ttttggtgtg 60
   ttgatgtagt atggctttac aataagtagt tgtttaaaga gatgtttggc attgcaataa 120
   ctagtagatt gatcggttac tcaaatattt ttgggagatg tttagtgtat tcacaattta 180
   tactaccata gagctcagac 200
<210> 333
   <211> 200
   <212> DNA
   <213> Conyza canadensis
<400> 333
   acttgggtta tgaacttagt atagttattc aaaataatca ctttcaaaat ggatccaaca 60
   catctgaaac tggtcacatt tgagttctta tctttcaaat aatttgtaat tcatatcagg 120
   ctcagcttca tggggtctgg cctttgtctg tgtgtttgtg ttgtgtcttt tctggtgtga 180
   tctaattcgg tatgcatatt 200
<210> 334
   <211> 200
   <212> DNA
   <213> Conyza canadensis
<400> 334
   aatatgcata ccgaattaga tcacaccaga aaagacacaa cacaaacaca cagacaaagg 60
   ccagacccca tgaagctgag cctgatatga attacaaatt atttgaaaga taagaactca 120
   aatgtgacca gtttcagatg tgttggatcc attttgaaag tgattatttt gaataactat 180
   actaagttca taacccaagt 200
<210> 335
   <211> 200
   <212> DNA
   <213> Conyza canadensis
<400> 335
   tgtgatctaa ttcggtatgc atattgtttg agcaggccaa ctatattcat agagattatt 60
   cagagagtag ggtgtatgct gaaagatgat gaaggcaagg tgcaacagaa gccaggctgt 120
   ggaggatttg gcaagggcaa tttctcagag ctcttcaaat ctattgaaga atacgagaag 180
   acgctggaag cacgaaacac 200
<210> 336
   <211> 200
   <212> DNA
   <213> Conyza canadensis
<400> 336
   gtgtttcgtg cttccagcgt cttctcgtat tcttcaatag atttgaagag ctctgagaaa 60
   ttgcccttgc caaatcctcc acagcctggc ttctgttgca ccttgccttc atcatctttc 120
   agcatacacc ctactctctg aataatctct atgaatatag ttggcctgct caaacaatat 180
   gcataccgaa ttagatcaca 200
<210> 337
   <211> 200
   <212> DNA
   <213> Conyza canadensis
<400> 337
   agaagacgct ggaagcacga aacaccactg aaccaactgc cactgcatga aaactgccta 60
   tgtataatgt ttatatcttc ttgactcaaa gaacatgatt atcaccatat gtatttgtgg 120
   attatgtaca attgaatgaa acaatgcaat accatatgtg atgtgattta tatagaataa 180
   caatagatgt cattcatata 200
<210> 338
   <211> 200
   <212> DNA
   <213> Conyza canadensis
<400> 338
   tatatgaatg acatctattg ttattctata taaatcacat cacatatggt attgcattgt 60
   ttcattcaat tgtacataat ccacaaatac atatggtgat aatcatgttc tttgagtcaa 120
   gaagatataa acattataca taggcagttt tcatgcagtg gcagttggtt cagtggtgtt 180
   tcgtgcttcc agcgtcttct 200
<210> 339
   <211> 200
   <212> DNA
   <213> Conyza canadensis
<400> 339
   aataacaata gatgtcattc atatatgccg ttcctgtttt agtaattgtg tgttggtggt 60
   gtgttgcaag ttccaatcag ctgtatataa tgcctaaata ttcaatttga cctccattaa 120
   ggatccattg tgcagattct ttgtttctgt cttaaaagtg tgggagactt gaaacacatc 180
   tgtaaaactg aaccagatct 200
<210> 340
   <211> 200
   <212> DNA
   <213> Conyza canadensis
<400> 340
   agatctggtt cagttttaca gatgtgtttc aagtctccca cacttttaag acagaaacaa 60
   agaatctgca caatggatcc ttaatggagg tcaaattgaa tatttaggca ttatatacag 120
   ctgattggaa cttgcaacac accaccaaca cacaattact aaaacaggaa cggcatatat 180
   gaatgacatc tattgttatt 200
<210> 341
   <211> 200
   <212> DNA
   <213> Conyza canadensis
<400> 341
   acatctgtaa aactgaacca gatctgaatc gagctgaatg gaagcggcgc aggtctgctc 60
   tgtggttcca cttacaacaa acaattgttc atggtacgtc gcctcttcat agccagtgtt 120
   tattgtaaga aagcattgtt taggcgaaaa aggaaaaggt gatttatgta agaaaaggta 180
   gtgctattaa ggttatgttc 200
<210> 342
   <211> 200
   <212> DNA
   <213> Conyza canadensis
<400> 342
   gaacataacc ttaatagcac taccttttct tacataaatc accttttcct ttttcgccta 60
   aacaatgctt tcttacaata aacactggct atgaagaggc gacgtaccat gaacaattgt 120
   ttgttgtaag tggaaccaca gagcagacct gcgccgcttc cattcagctc gattcagatc 180
   tggttcagtt ttacagatgt 200
<210> 343
   <211> 200
   <212> DNA
   <213> Conyza canadensis
<400> 343
   aggtagtgct attaaggtta tgttccccat gaacaaacag ggtaaattca tacaggatca 60
   acttttaaat gatactatta aaggtcatct acttctgttt cccatgcttg tgccaagctc 120
   aaatttagac gaaaccaaaa caggatgaga aaactaatta acacatattt tagtaagcaa 180
   ttacagctat atatcatact 200
<210> 344
   <211> 200
   <212> DNA
   <213> Conyza canadensis
<400> 344
   agtatgatat atagctgtaa ttgcttacta aaatatgtgt taattagttt tctcatcctg 60
   ttttggtttc gtctaaattt gagcttggca caagcatggg aaacagaagt agatgacctt 120
   taatagtatc atttaaaagt tgatcctgta tgaatttacc ctgtttgttc atggggaaca 180
   taaccttaat agcactacct 200
<210> 345
   <211> 200
   <212> DNA
   <213> Conyza canadensis
<400> 345
   attaaaaaca cgaatgggct agctagctga tgaatttaat gtatatatgt ctttaaaaac 60
   cactttcatg ggctacttgt gtttttgatg agtttttcaa catcattctc atcttacctt 120
   cttacaatgg ttcaccatat tgcttcgttt gtcgtctcct gcatggttat cgaatttatt 180
   ttttcaaatt atattactat 200
<210> 346
   <211> 200
   <212> DNA
   <213> Conyza canadensis
<400> 346
   atagtaatat aatttgaaaa aataaattcg ataaccatgc aggagacgac aaacgaagca 60
   atatggtgaa ccattgtaag aaggtaagat gagaatgatg ttgaaaaact catcaaaaac 120
   acaagtagcc catgaaagtg gtttttaaag acatatatac attaaattca tcagctagct 180
   agcccattcg tgtttttaat 200
<210> 347
   <211> 200
   <212> DNA
   <213> Conyza canadensis
<400> 347
   ttattttttc aaattatatt actattatct ttttgttttc tgatttctca catgtaattt   60
   acattatgag tttcaccatc cgtatgtaaa ttgtaaaata aatcacaaat ttggttttta  120
   accatcaact aaattcattg aaatccttaa gttataaccc atttgaggtt atagattatg  180
   agagtagatt cctattttct                                              200
<210> 348
   <211> 200
   <212> DNA
   <213> Conyza canadensis
<400> 348
   agaaaatagg aatctactct cataatctat aacctcaaat gggttataac ttaaggattt   60
   caatgaattt agttgatggt taaaaaccaa atttgtgatt tattttacaa tttacatacg  120
   gatggtgaaa ctcataatgt aaattacatg tgagaaatca gaaaacaaaa agataatagt  180
   aatataattt gaaaaaataa                                              200
<210> 349
   <211> 200
   <212> DNA
   <213> Conyza canadensis
<400> 349
   ttatgagagt agattcctat tttctctttg gcttatgtat ccttaattat gtttgaattg   60
   attctaactt tattatattt taggaattac ttaacttcaa aaatctcaac acgtcatata  120
   atgctttttg tggatggatg cttttgaccc atttcgttga aaacttatgt cgacacacat  180
   atctgacaaa cttgtactcg                                              200
<210> 350
   <211> 200
   <212> DNA
   <213> Conyza canadensis
<400> 350
   cgagtacaag tttgtcagat atgtgtgtcg acataagttt tcaacgaaat gggtcaaaag   60
   catccatcca caaaaagcat tatatgacgt gttgagattt ttgaagttaa gtaattccta  120
   aaatataata aagttagaat caattcaaac ataattaagg atacataagc caaagagaaa  180
   ataggaatct actctcataa                                              200
<210> 351
   <211> 200
   <212> DNA
   <213> Conyza canadensis
<400> 351
   cacatatctg acaaacttgt actcgtatat cctttatcgc atatgctata gtataatatt 60
   cgaaatttaa tctcaagata attttcccaa aaggtcatat taattgaatt gagattaatt 120
   cctacctaac atatatagat atattgtgtt ggtgttatcc tctttggagt tttagtcgcg 180
   catgtattct aagaaaatgg 200
<210> 352
   <211> 200
   <212> DNA
   <213> Conyza canadensis
<400> 352
   ccattttctt agaatacatg cgcgactaaa actccaaaga ggataacacc aacacaatat 60
   atctatatat gttaggtagg aattaatctc aattcaatta atatgacctt ttgggaaaat 120
   tatcttgaga ttaaatttcg aatattatac tatagcatat gcgataaagg atatacgagt 180
   acaagtttgt cagatatgtg 200
<210> 353
   <211> 200
   <212> DNA
   <213> Conyza canadensis
<400> 353
   tcgcgcatgt attctaagaa aatggtaatt ttgaacttat attgatggtc taaattttat 60
   cctatcacta aaatttcagt catgtttcct agctaattaa tttgggttca atccaggtat 120
   tctaaataag ttttgtaaat acatgtctat tatcataatg aaacatagaa aaacccgtaa 180
   agtaaaaatg tctatatttt 200
<210> 354
   <211> 200
   <212> DNA
   <213> Conyza canadensis
<400> 354
   aaaatataga catttttact ttacgggttt ttctatgttt cattatgata atagacatgt 60
   atttacaaaa cttatttaga atacctggat tgaacccaaa ttaattagct aggaaacatg 120
   actgaaattt tagtgatagg ataaaattta gaccatcaat ataagttcaa aattaccatt 180
   ttcttagaat acatgcgcga 200
<210> 355
   <211> 200
   <212> DNA
   <213> Conyza canadensis
<400> 355
   cgtaaagtaa aaatgtctat atttttagtg gcaaattatt aatgttgcta taatgaaaag 60
   agaagattca accttttgta catcataaaa aatgttattc tacattatgc taattttaca 120
   ttagacttca tagttttgaa aactttcaaa tgaagggact taaattttaa atttaaagat 180
   gatgtcataa attagttaga 200
<210> 356
   <211> 200
   <212> DNA
   <213> Conyza canadensis
<400> 356
   tctaactaat ttatgacatc atctttaaat ttaaaattta agtcccttca tttgaaagtt 60
   ttcaaaacta tgaagtctaa tgtaaaatta gcataatgta gaataacatt ttttatgatg 120
   tacaaaaggt tgaatcttct cttttcatta tagcaacatt aataatttgc cactaaaaat 180
   atagacattt ttactttacg 200
<210> 357
   <211> 200
   <212> DNA
   <213> Conyza canadensis
<400> 357
   aagatgatgt cataaattag ttagataaaa gttaaataat taataagaaa aggtctaatg 60
   ataccaaata aaattttctt aaaaaagtat taatgtcatt acaattttat tttatttata 120
   taagagaaga taatagattt tcttgtataa aaaacttacc tgtaaacttc tatataaact 180
   ttttagaaaa ataatttaaa 200
<210> 358
   <211> 200
   <212> DNA
   <213> Conyza canadensis
<400> 358
   tttaaattat ttttctaaaa agtttatata gaagtttaca ggtaagtttt ttatacaaga   60
   aaatctatta tcttctctta tataaataaa ataaaattgt aatgacatta atactttttt  120
   aagaaaattt tatttggtat cattagacct tttcttatta attatttaac ttttatctaa  180
   ctaatttatg acatcatctt                                              200
<210> 359
   <211> 200
   <212> DNA
   <213> Conyza canadensis
<400> 359
   aaacttttta gaaaaataat ttaaaacggt ctcaaatatc gttatttccg aacatacaaa   60
   aacccaaaac ctaatccatt taattatatt gtaagacctc gattagctta atttattttg  120
   aaacctattt acaaatgagt ttggcatgat atgaactttt ttgcatttac atatggctgt  180
   aaacgaactg aacagttcac                                              200
<210> 360
   <211> 200
   <212> DNA
   <213> Conyza canadensis
<400> 360
   gtgaactgtt cagttcgttt acagccatat gtaaatgcaa aaaagttcat atcatgccaa   60
   actcatttgt aaataggttt caaaataaat taagctaatc gaggtcttac aatataatta  120
   aatggattag gttttgggtt tttgtatgtt cggaaataac gatatttgag accgttttaa  180
   attatttttc taaaaagttt                                              200
<210> 361
   <211> 200
   <212> DNA
   <213> Conyza canadensis
<400> 361
   gctgtaaacg aactgaacag ttcacgaaca attcatgaac cgttcggcgg gaggttcgtt   60
   cgtgttagtt cgtttagtta aatgaatgaa catgaacaaa gctctcgttc gttcatttac  120
   gttcatgaac gttcgatagt ctgttcgtga actttagttc gtctatgttc atgaactgtc  180
   gttcgtgaac aataatttga                                              200
<210> 362
   <211> 200
   <212> DNA
   <213> Conyza canadensis
<400> 362
   tcaaattatt gttcacgaac gacagttcat gaacatagac gaactaaagt tcacgaacag    60
   actatcgaac gttcatgaac gtaaatgaac gaacgagagc tttgttcatg ttcattcatt   120
   taactaaacg aactaacacg aacgaacctc ccgccgaacg gttcatgaat tgttcgtgaa   180
   ctgttcagtt cgtttacagc                                                200
<210> 363
   <211> 200
   <212> DNA
   <213> Conyza canadensis
<400> 363
   ctgtcgttcg tgaacaataa tttgattatg ttcatttaca cacacataca taatcaactg    60
   tactataaaa atagtttaaa tataaatatt tttttatata atatatatta ttaatatcta   120
   attatttaag aaaaagttta aataaaatac ttaacttata aatatttcgt tcgtttgtgt   180
   tcgtgtacat ttgttcatga                                                200
<210> 364
   <211> 200
   <212> DNA
   <213> Conyza canadensis
<400> 364
   tcatgaacaa atgtacacga acacaaacga acgaaatatt tataagttaa gtattttatt    60
   taaacttttt cttaaataat tagatattaa taatatatat tatataaaaa aatatttata   120
   tttaaactat ttttatagta cagttgatta tgtatgtgtg tgtaaatgaa cataatcaaa   180
   ttattgttca cgaacgacag                                                200
<210> 365
   <211> 200
   <212> DNA
   <213> Conyza canadensis
<400> 365
   tgtgttcgtg tacatttgtt catgaacaca aatgaacgaa catgaacaag tcattatgtt    60
   cgtttatctg tttgttaagt taaatgaacg aacgaacacg aacaaggtct cgtttgtgtt   120
   cgttcggttc gtttacggcc cttaattaca tgtgaagtta ataaataaaa ataaagttca   180
   tcaaaattaa acctatggtt                                                200
<210> 366
   <211> 200
   <212> DNA
   <213> Conyza canadensis
<400> 366
   aaccataggt ttaattttga tgaactttat ttttatttat taacttcaca tgtaattaag 60
   ggccgtaaac gaaccgaacg aacacaaacg agaccttgtt cgtgttcgtt cgttcattta 120
   acttaacaaa cagataaacg aacataatga cttgttcatg ttcgttcatt tgtgttcatg 180
   aacaaatgta cacgaacaca 200
<210> 367
   <211> 200
   <212> DNA
   <213> Conyza canadensis
<400> 367
   gttcatcaaa attaaaccta tggttggaga taaaaccagt cagccttacg tgtggaaact 60
   atcctactct acaattaata gcacccaaag aaaataaacc acttccgtgt tcttcttcat 120
   ctccatcatc caacccctca actacttgta ctgtatctat atctatcatt ctatatatct 180
   acttcatatt tctatctata 200
<210> 368
   <211> 200
   <212> DNA
   <213> Conyza canadensis
<400> 368
   tatagataga aatatgaagt agatatatag aatgatagat atagatacag tacaagtagt 60
   tgaggggttg gatgatggag atgaagaaga acacggaagt ggtttatttt ctttgggtgc 120
   tattaattgt agagtaggat agtttccaca cgtaaggctg actggtttta tctccaacca 180
   taggtttaat tttgatgaac 200
<210> 369
   <211> 200
   <212> DNA
   <213> Conyza canadensis
<400> 369
   tatctacttc atatttctat ctatacattc aaattaataa aaacaaaatg gtaactgaag 60
   cagcaaacgg aaacacactc accggacaac aacaaaccac taccaccacc tccgccactt 120
   acaaactcgt cggcttcaaa aacttcatcc gtcaaaaccc taaatccgac aaattcaccg 180
   tcaaaaaatt ccaccacgtc 200
<210> 370
   <211> 200
   <212> DNA
   <213> Conyza canadensis
<400> 370
   gacgtggtgg aattttttga cggtgaattt gtcggattta gggttttgac ggatgaagtt   60
   tttgaagccg acgagtttgt aagtggcgga ggtggtggta gtggtttgtt gttgtccggt  120
   gagtgtgttt ccgtttgctg cttcagttac cattttgttt ttattaattt gaatgtatag  180
   atagaaatat gaagtagata                                              200
<210> 371
   <211> 200
   <212> DNA
   <213> Conyza canadensis
<400> 371
   caccgtcaaa aaattccacc acgtcgaatt ctggtgttcc gacgccacca acaccgcccg   60
   ccgcttctct tggggtctcg gcatgccgct tctttacaaa tccgatctct ccaccggtaa  120
   caacactcac gcttcttacc ttattaactc cggccagctc aacttcctct tcaccgcacc  180
   ttattccacc accatctcca                                              200
<210> 372
   <211> 200
   <212> DNA
   <213> Conyza canadensis
<400> 372
   tggagatggt ggtggaataa ggtgcggtga agaggaagtt gagctggccg gagttaataa   60
   ggtaagaagc gtgagtgttg ttaccggtgg agagatcgga tttgtaaaga agcggcatgc  120
   cgagacccca agagaagcgg cgggcggtgt tggtggcgtc ggaacaccag aattcgacgt  180
   ggtggaattt tttgacggtg                                              200
<210> 373
   <211> 200
   <212> DNA
   <213> Conyza canadensis
<400> 373
   gcaccttatt ccaccaccat ctccaccacc ggttccacat cgtctattcc tactttctct 60
   cacactgctt gccggaactt caccgccact cacggcctcg ctgtacggtc tatcgccgtt 120
   gaagttgaag acgctgaaat cgcttattct gttagtatcg ctcacggtgc taaaccgtcg 180
   tcagctccgg tcactctagg 200
<210> 374
   <211> 200
   <212> DNA
   <213> Conyza canadensis
<400> 374
   cctagagtga ccggagctga cgacggttta gcaccgtgag cgatactaac agaataagcg 60
   atttcagcgt cttcaacttc aacggcgata gaccgtacag cgaggccgtg agtggcggtg 120
   aagttccggc aagcagtgtg agagaaagta ggaatagacg atgtggaacc ggtggtggag 180
   atggtggtgg aataaggtgc 200
<210> 375
   <211> 200
   <212> DNA
   <213> Conyza canadensis
<400> 375
   cgtcgtcagc tccggtcact ctaggaaata acgacgtcgt tttggctgaa gttaaactgt 60
   acggcgatgt cgttttgaga tatgttagtt ttaaaaacac taattatgat gttactacta 120
   atttcctacc gggttttgag cctgtggacg caacgtcgtc gtttcatgaa cttaattacg 180
   gaattcataa gttagatcac 200
<210> 376
   <211> 200
   <212> DNA
   <213> Conyza canadensis
<400> 376
   gtgatctaac ttatgaattc cgtaattaag ttcatgaaac gacgacgttg cgtccacagg 60
   ctcaaaaccc ggtaggaaat tagtagtaac atcataatta gtgtttttaa aactaacata 120
   tctcaaaacg acatcgccgt acagtttaac ttcagccaaa acgacgtcgt tatttcctag 180
   agtgaccgga gctgacgacg 200
<210> 377
   <211> 200
   <212> DNA
   <213> Conyza canadensis
<400> 377
   ttacggaatt cataagttag atcacgccgt aggaaacgtg ccggagctag ctccggcagt 60
   agactacgtg aagtccttca ctggatttca cgaatttgct gaatttacgg cagaggatgt 120
   aggaacgagt gagagcgggc ttaattcggt tgtattagcg tgtaatagtg aaacggtttt 180
   gataccgatg aacgagccag 200
<210> 378
   <211> 200
   <212> DNA
   <213> Conyza canadensis
<400> 378
   ctggctcgtt catcggtatc aaaaccgttt cactattaca cgctaataca accgaattaa 60
   gcccgctctc actcgttcct acatcctctg ccgtaaattc agcaaattcg tgaaatccag 120
   tgaaggactt cacgtagtct actgccggag ctagctccgg cacgtttcct acggcgtgat 180
   ctaacttatg aattccgtaa 200
<210> 379
   <211> 200
   <212> DNA
   <213> Conyza canadensis
<400> 379
   gttttgatac cgatgaacga gccagtttat ggaacgaaaa ggaagagcca gatacagacg 60
   tatttggaac ataatgaggg agctggtgtg cagcatttgg cgttggcgag tgaggatatt 120
   tttaggactt tgagagagat gagaaaacgg agtagtatcg gtggttttga gtttatgccg 180
   tctccaccgc ctacttatta 200
<210> 380
   <211> 200
   <212> DNA
   <213> Conyza canadensis
<400> 380
   taataagtag gcggtggaga cggcataaac tcaaaaccac cgatactact ccgttttctc 60
   atctctctca aagtcctaaa aatatcctca ctcgccaacg ccaaatgctg cacaccagct 120
   ccctcattat gttccaaata cgtctgtatc tggctcttcc ttttcgttcc ataaactggc 180
   tcgttcatcg gtatcaaaac 200
<210> 381
   <211> 200
   <212> DNA
   <213> Conyza canadensis
<400> 381
   tgccgtctcc accgcctact tattataaga atttgaagaa tagggtcggg gacgtgttga 60
   gtgacgaaca gattaaggaa tgtgaggaat tggggatttt ggttgataga gatgatcagg 120
   gaactttgct tcagattttc accaagcctg ttggtgatag gaggccaact atattcatag 180
   agattattca gagagtaggg 200
<210> 382
   <211> 200
   <212> DNA
   <213> Conyza canadensis
<400> 382
   ccctactctc tgaataatct ctatgaatat agttggcctc ctatcaccaa caggcttggt 60
   gaaaatctga agcaaagttc cctgatcatc tctatcaacc aaaatcccca attcctcaca 120
   ttccttaatc tgttcgtcac tcaacacgtc cccgacccta ttcttcaaat tcttataata 180
   agtaggcggt ggagacggca 200
<210> 383
   <211> 200
   <212> DNA
   <213> Conyza canadensis
<400> 383
   catagagatt attcagagag tagggtgtat gctgaaagat gatgaaggca aggtgcaaca 60
   gaagccaggc tgtggaggat ttggcaaggg caatttctca gagctcttca aatctattga 120
   agaatacgag aagacgctgg aagcacgaaa caccactgaa ccaactgcca ctgcatgaaa 180
   actgcctatg tataatgttt 200
<210> 384
   <211> 200
   <212> DNA
   <213> Conyza canadensis
<400> 384
   aaacattata cataggcagt tttcatgcag tggcagttgg ttcagtggtg tttcgtgctt 60
   ccagcgtctt ctcgtattct tcaatagatt tgaagagctc tgagaaattg cccttgccaa 120
   atcctccaca gcctggcttc tgttgcacct tgccttcatc atctttcagc atacacccta 180
   ctctctgaat aatctctatg 200
<210> 385
   <211> 200
   <212> DNA
   <213> Conyza canadensis
<400> 385
   tgaaaactgc ctatgtataa tgtttatatc ttcttgactc aaagaacatg attatcacca 60
   tatgtatttg tggattatgt acaattgaat gaaacaatgc aataccatat gtgatgtgat 120
   ttatatagaa taacaataga tgtcattcat atatgccgtt cctgttttag taattgtgtg 180
   ttggtggtgt gttgcaagtt 200
<210> 386
   <211> 200
   <212> DNA
   <213> Conyza canadensis
<400> 386
   aacttgcaac acaccaccaa cacacaatta ctaaaacagg aacggcatat atgaatgaca 60
   tctattgtta ttctatataa atcacatcac atatggtatt gcattgtttc attcaattgt 120
   acataatcca caaatacata tggtgataat catgttcttt gagtcaagaa gatataaaca 180
   ttatacatag gcagttttca 200
<210> 387
   <211> 200
   <212> DNA
   <213> Conyza canadensis
<400> 387
   gtgtgttggt ggtgtgttgc aagttccaat cagctgtata taatgcctaa atattcaatt 60
   tgacctccat taaggatcca ttgtgcagat tctttgtttc tgtcttaaaa gtgtgggaga 120
   cttgaaacac atctgtaaaa ctgaaccaga tctgaatcga gctgaatgga agcggcgcag 180
   gtctgctctt tggttccact 200
<210> 388
   <211> 200
   <212> DNA
   <213> Conyza canadensis
<400> 388
   agtggaacca aagagcagac ctgcgccgct tccattcagc tcgattcaga tctggttcag 60
   ttttacagat gtgtttcaag tctcccacac ttttaagaca gaaacaaaga atctgcacaa 120
   tggatcctta atggaggtca aattgaatat ttaggcatta tatacagctg attggaactt 180
   gcaacacacc accaacacac 200
<210> 389
   <211> 200
   <212> DNA
   <213> Conyza canadensis
<400> 389
   cgcaggtctg ctctttggtt ccacttacaa caaacaattg ttcatggtac gtcgcctctt   60
   catggccagt gtttattgta agaaagcatt gtttaggcga aaaaggaaaa ggtgatttat  120
   gtaagaaaag gtagtgctat taaggttatg ttccccatga acaaacaggg taaattcata  180
   caggatcaac ttttaaatga                                               200
<210> 390
   <211> 200
   <212> DNA
   <213> Conyza canadensis
<400> 390
   tcatttaaaa gttgatcctg tatgaattta ccctgtttgt tcatggggaa cataacctta   60
   atagcactac cttttcttac ataaatcacc ttttcctttt tcgcctaaac aatgctttct  120
   tacaataaac actggccatg aagaggcgac gtaccatgaa caattgtttg ttgtaagtgg  180
   aaccaaagag cagacctgcg                                               200
<210> 391
   <211> 200
   <212> DNA
   <213> Conyza canadensis
<400> 391
   tcatacagga tcaactttta aatgatacta ttaaaggtca tctacttctg tttcccatgc   60
   ttgtgccaag ctcaaattta gacgaaacca aaacaggatg aaaaaactaa ttaacacata  120
   ttttagtaag caattacagc tatatatcat actatcatca acaatctgaa aagttgcata  180
   caatttatcg tttattcctg                                               200
<210> 392
   <211> 200
   <212> DNA
   <213> Conyza canadensis
<400> 392
   caggaataaa cgataaattg tatgcaactt ttcagattgt tgatgatagt atgatatata 60
   gctgtaattg cttactaaaa tatgtgttaa ttagtttttt catcctgttt tggtttcgtc 120
   taaatttgag cttggcacaa gcatgggaaa cagaagtaga tgacctttaa tagtatcatt 180
   taaaagttga tcctgtatga 200
<210> 393
   <211> 200
   <212> DNA
   <213> Conyza canadensis
<400> 393
   gcatacaatt tatcgtttat tcctgtcaag tgatgacatg aaaaatacca tttagtttac 60
   acgtactcag atatgccaat aatagaggga ctttttctat atgagaacat acatgatgtt 120
   cataattcat aatttacttg atacggcaag gtacttggta tggcgtcctt gtgcaattgt 180
   ttttccggtc tttttgttcc 200
<210> 394
   <211> 200
   <212> DNA
   <213> Conyza canadensis
<400> 394
   ggaacaaaaa gaccggaaaa acaattgcac aaggacgcca taccaagtac cttgccgtat 60
   caagtaaatt atgaattatg aacatcatgt atgttctcat atagaaaaag tccctctatt 120
   attggcatat ctgagtacgt gtaaactaaa tggtattttt catgtcatca cttgacagga 180
   ataaacgata aattgtatgc 200
<210> 395
   <211> 200
   <212> DNA
   <213> Conyza canadensis
<400> 395
   attgtttttc cggtcttttt gttccttaat tcaacggtca caacagcaat agccttccca 60
   acacgcaatg tttttgcctc tatctcaatc tcgtcctgta agaaaattgc atccttaaca 120
   aaattttact caaagacgca tctatggtac gaagtacaaa ccaaagattt tcagtaagtc 180
   gtgcaaaacc tttatgcata 200
<210> 396
   <211> 200
   <212> DNA
   <213> Conyza canadensis
<400> 396
   tatgcataaa ggttttgcac gacttactga aaatctttgg tttgtacttc gtaccataga 60
   tgcgtctttg agtaaaattt tgttaaggat gcaattttct tacaggacga gattgagata 120
   gaggcaaaaa cattgcgtgt tgggaaggct attgctgttg tgaccgttga attaaggaac 180
   aaaaagaccg gaaaaacaat 200
<210> 397
   <211> 200
   <212> DNA
   <213> Conyza canadensis
<400> 397
   aagtcgtgca aaacctttat gcatactgca actactcaaa cttcacatct tatgcctaaa 60
   atcaaagacc ccaaagaagc cttgagttca aaatccaaga atttctgtca gctacagggg 120
   acaggttcaa cccatttaca tacaaatggg tcaaaatggg ttatgttatt ctgtgtttct 180
   gccaaaccag acctggcacg 200
<210> 398
   <211> 200
   <212> DNA
   <213> Conyza canadensis
<400> 398
   cgtgccaggt ctggtttggc agaaacacag aataacataa cccattttga cccatttgta 60
   tgtaaatggg ttgaacctgt cccctgtagc tgacagaaat tcttggattt tgaactcaag 120
   gcttctttgg ggtctttgat tttaggcata agatgtgaag tttgagtagt tgcagtatgc 180
   ataaaggttt tgcacgactt 200
<210> 399
   <211> 200
   <212> DNA
   <213> Conyza canadensis
<400> 399
   tttctgccaa accagacctg gcacgtacta aaactcaccc attacccaac ccacctaccc 60
   agcccccctt tttaccaaca tgcattttgc ttttgcaacc ctgttgcttc aagcattcaa 120
   ctattcaagt agcaagatcg aaagaactta tacaaaaagt tgccatttat gattaagtga 180
   agccttaatg acaccaaatc 200
<210> 400
   <211> 200
   <212> DNA
   <213> Conyza canadensis
<400> 400
   gatttggtgt cattaaggct tcacttaatc ataaatggca actttttgta taagttcttt 60
   cgatcttgct acttgaatag ttgaatgctt gaagcaacag ggttgcaaaa gcaaaatgca 120
   tgttggtaaa aaggggggct gggtaggtgg gttgggtaat gggtgagttt tagtacgtgc 180
   caggtctggt ttggcagaaa 200
<210> 401
   <211> 200
   <212> DNA
   <213> Conyza canadensis
<400> 401
   agtgaagcct taatgacacc aaatcagtta ttatagcatt acaatgggag agcttcaaca 60
   gaaaagttat caagtcttct ccaacaagat catgagctca ttttcaattt tatatcaaac 120
   ggtagcaata acgaaagtaa atctggtggc tcaaggtaaa acctcataac ctttgatggc 180
   ataataactt ccagaccaac 200
<210> 402
   <211> 200
   <212> DNA
   <213> Conyza canadensis
<400> 402
   gttggtctgg aagttattat gccatcaaag gttatgaggt tttaccttga gccaccagat 60
   ttactttcgt tattgctacc gtttgatata aaattgaaaa tgagctcatg atcttgttgg 120
   agaagacttg ataacttttc tgttgaagct ctcccattgt aatgctataa taactgattt 180
   ggtgtcatta aggcttcact 200
<210> 403
   <211> 200
   <212> DNA
   <213> Conyza canadensis
<400> 403
   atggcataat aacttccaga ccaactaaac accaaggtgg aagttattac tttgtgctaa 60
   tgatagcagt tacaaacata aaaaatctgg tcggcttcgt tcagttttct gaaataccaa 120
   tatcgtctga caaggtgcca tcttcatatt cagagtcact tcatgatcaa ctcttgtggg 180
   gtttcaaact caagaacgtt 200
<210> 404
   <211> 200
   <212> DNA
   <213> Conyza canadensis
<400> 404
   aacgttcttg agtttgaaac cccacaagag ttgatcatga agtgactctg aatatgaaga   60
   tggcaccttg tcagacgata ttggtatttc agaaaactga acgaagccga ccagattttt  120
   tatgtttgta actgctatca ttagcacaaa gtaataactt ccaccttggt gtttagttgg  180
   tctggaagtt attatgccat                                              200
<210> 405
   <211> 200
   <212> DNA
   <213> Conyza canadensis
<400> 405
   gtggggtttc aaactcaaga acgttttaaa tacattcatt taatattttt atctccagaa   60
   tactactcaa cactttcctg taaggtcttc aatttcaggg ggaaaaaaac ccaaaattca  120
   aggaatcctg ctattagtgt atcgtttctt gatagcaaag ctttcattta caatctctaa  180
   caacattttt acttcaaaat                                              200
<210> 406
   <211> 200
   <212> DNA
   <213> Conyza canadensis
<400> 406
   attttgaagt aaaaatgttg ttagagattg taaatgaaag ctttgctatc aagaaacgat   60
   acactaatag caggattcct tgaattttgg gtttttttcc ccctgaaatt gaagacctta  120
   caggaaagtg ttgagtagta ttctggagat aaaaatatta aatgaatgta tttaaaacgt  180
   tcttgagttt gaaaccccac                                              200
<210> 407
   <211> 200
   <212> DNA
   <213> Conyza canadensis
<400> 407
   tatggtattg cattgtttca ttcaattgta cataatccac aaatacatat ggtgataatc   60
   atgttctttg agtcaagaag atataaacat tatacatagg cagttttcat gcagtggcag  120
   ttggttcagt ggtgtttcgt gcttccagcg tcttctcgta ttcttcaata gatttgaaga 180
   gctctgagaa attgcccttg 200
<210> 408
   <211> 200
   <212> DNA
   <213> Conyza canadensis
<400> 408
   caagggcaat ttctcagagc tcttcaaatc tattgaagaa tacgagaaga cgctggaagc 60
   acgaaacacc actgaaccaa ctgccactgc atgaaaactg cctatgtata atgtttatat 120
   cttcttgact caaagaacat gattatcacc atatgtattt gtggattatg tacaattgaa 180
   tgaaacaatg caataccata 200
<210> 409
   <211> 200
   <212> DNA
   <213> Conyza canadensis
<400> 409
   gaagagctct gagaaattgc ccttgccaaa tcctccacag cctggcttct gttgcacctt 60
   gccttcatca tctttcagca tacaccctac tctctgaata atctctatga atatagttgg 120
   cctatcacca acaggcttgg tgaaaatctg aagcaaagtt ccctgatcat ctctatcaac 180
   caaaatcccc aattcctcac 200
<210> 410
   <211> 200
   <212> DNA
   <213> Conyza canadensis
<400> 410
   gtgaggaatt ggggattttg gttgatagag atgatcaggg aactttgctt cagattttca 60
   ccaagcctgt tggtgatagg ccaactatat tcatagagat tattcagaga gtagggtgta 120
   tgctgaaaga tgatgaaggc aaggtgcaac agaagccagg ctgtggagga tttggcaagg 180
   gcaatttctc agagctcttc 200
<210> 411
   <211> 200
   <212> DNA
   <213> Conyza canadensis
<400> 411
   tcaaccaaaa tccccaattc ctcacattcc ttaatctgtt cgtcactcaa cacgtccccg 60
   accctattct tcaaattctt ataataagta ggcggtggag acggcataaa ctcaaaacca 120
   ccgacactac tccgttttct catctctctc aaagtcctaa aaatatcctc actcgccaac 180
   gccaaatgct gcacaccagc 200
<210> 412
   <211> 200
   <212> DNA
   <213> Conyza canadensis
<400> 412
   gctggtgtgc agcatttggc gttggcgagt gaggatattt ttaggacttt gagagagatg 60
   agaaaacgga gtagtgtcgg tggttttgag tttatgccgt ctccaccgcc tacttattat 120
   aagaatttga agaatagggt cggggacgtg ttgagtgacg aacagattaa ggaatgtgag 180
   gaattgggga ttttggttga 200
<210> 413
   <211> 200
   <212> DNA
   <213> Conyza canadensis
<400> 413
   ccaacgccaa atgctgcaca ccagctccct cattatgttc caaatacgtc tgtatctggc 60
   tcttcctttt cgttccataa actggctcgt tcatcggtat caaaaccgtt tcactattac 120
   acgctaatac aaccgaatta agcccgctct cactcgttcc tacatcctct gccgtaaatt 180
   cagcaaattc gtgaaatcca 200
<210> 414
   <211> 200
   <212> DNA
   <213> Conyza canadensis
<400> 414
   tggatttcac gaatttgctg aatttacggc agaggatgta ggaacgagtg agagcgggct 60
   taattcggtt gtattagcgt gtaatagtga aacggttttg ataccgatga acgagccagt 120
   ttatggaacg aaaaggaaga gccagataca gacgtatttg gaacataatg agggagctgg 180
   tgtgcagcat ttggcgttgg 200
<210> 415
   <211> 200
   <212> DNA
   <213> Conyza canadensis
<400> 415
   aaattcagca aattcgtgaa atccagtgaa ggacttcacg tagtctactg ccggagctag 60
   ctccggcacg tttcctacgg cgtgatctaa cttatgaatt ccgtaattaa gttcatgaaa 120
   cgacgacgtt gcgtccacag gctcaaaacc cggtaggaaa ttagtagtaa catcataatt 180
   agtgttttta aaactaacat 200
<210> 416
   <211> 200
   <212> DNA
   <213> Conyza canadensis
<400> 416
   atgttagttt taaaaacact aattatgatg ttactactaa tttcctaccg ggttttgagc 60
   ctgtggacgc aacgtcgtcg tttcatgaac ttaattacgg aattcataag ttagatcacg 120
   ccgtaggaaa cgtgccggag ctagctccgg cagtagacta cgtgaagtcc ttcactggat 180
   ttcacgaatt tgctgaattt 200
<210> 417
   <211> 200
   <212> DNA
   <213> Conyza canadensis
<400> 417
   taattagtgt ttttaaaact aacatatctc aaaacgacat cgccgtacag tttaacttca 60
   gccaaaacga cgtcgttatt tcctagagtg accggagctg acgacggttt agcaccgtga 120
   gcgatactaa cagaataagc gatttcagcg tcttcaactt caacggcgat agaccgtaca 180
   gcgaggccgt gagtggcggt 200
<210> 418
   <211> 200
   <212> DNA
   <213> Conyza canadensis
<400> 418
   accgccactc acggcctcgc tgtacggtct atcgccgttg aagttgaaga cgctgaaatc 60
   gcttattctg ttagtatcgc tcacggtgct aaaccgtcgt cagctccggt cactctagga 120
   aataacgacg tcgttttggc tgaagttaaa ctgtacggcg atgtcgtttt gagatatgtt 180
   agttttaaaa acactaatta 200
<210> 419
   <211> 200
   <212> DNA
   <213> Conyza canadensis
<400> 419
   gtacagcgag gccgtgagtg gcggtgaagt tccggcaagc agtgtgagag aaagtaggaa 60
   tagacgatgt ggaaccggtg gtggagatgg tggtggaata aggtgcggtg aagaggaagt 120
   tgagctggcc ggagttaata aggtaagaag cgtgagtgtt gttaccggtg gagagatcgg 180
   atttgtaaag aagcggcatg 200
<210> 420
   <211> 200
   <212> DNA
   <213> Conyza canadensis
<400> 420
   catgccgctt ctttacaaat ccgatctctc caccggtaac aacactcacg cttcttacct 60
   tattaactcc ggccagctca acttcctctt caccgcacct tattccacca ccatctccac 120
   caccggttcc acatcgtcta ttcctacttt ctctcacact gcttgccgga acttcaccgc 180
   cactcacggc ctcgctgtac 200
<210> 421
   <211> 200
   <212> DNA
   <213> Conyza canadensis
<400> 421
   atcggatttg taaagaagcg gcatgccgag accccaagag aagcggcggg cggtgttggt 60
   ggcgtcggaa caccagaatt cgacgtggtg gaattttttg acggtgaatt tgtcggattt 120
   agggttttga cggatgaagt ttttgaagcc gacgagtttg taagtggcgg aggtggtggt 180
   agtggtttgt tgttgtccgg 200
<210> 422
   <211> 200
   <212> DNA
   <213> Conyza canadensis
<400> 422
   ccggacaaca acaaaccact accaccacct ccgccactta caaactcgtc ggcttcaaaa   60
   acttcatccg tcaaaaccct aaatccgaca aattcaccgt caaaaaattc caccacgtcg  120
   aattctggtg ttccgacgcc accaacaccg cccgccgctt ctcttggggt ctcggcatgc  180
   cgcttcttta caaatccgat                                              200
<210> 423
   <211> 200
   <212> DNA
   <213> Conyza canadensis
<400> 423
   gtggtagtgg tttgttgttg tccggtgagt gtgtttccgt ttgctgcttc agttaccatt   60
   ttgtttttat taatttgaat gtatagatag aaatatgaag tagatatata gaatgataga  120
   tatagataca gtacaagtag ttgaggggtt ggatgatgga gatgaagaag aacacggaag  180
   tggtttattt tctttgggtg                                              200
<210> 424
   <211> 200
   <212> DNA
   <213> Conyza canadensis
<400> 424
   cacccaaaga aaataaacca cttccgtgtt cttcttcatc tccatcatcc aacccctcaa   60
   ctacttgtac tgtatctata tctatcattc tatatatcta cttcatattt ctatctatac  120
   attcaaatta ataaaaacaa aatggtaact gaagcagcaa acggaaacac actcaccgga  180
   caacaacaaa ccactaccac                                              200
<210> 425
   <211> 200
   <212> DNA
   <213> Digitaria sanguinalis
<400> 425
   gccgccaccg cctccctccc ctccttctcc gcccccgccg cgcgccgctt cgcctccgac   60
   cacggcctcg ccgtgcgcgc cgtagcgctc cgcgtcgccg acgccgagga cgccttccgc  120
   gccagcgtcg ccaacggggc gcgcccggcg ttcgagcccg tcgagctcgg cctcggcttc  180
   cgcctcgccg aggtcgagct                                              200
<210> 426
   <211> 200
   <212> DNA
   <213> Digitaria sanguinalis
<400> 426
   agctcgacct cggcgaggcg gaagccgagg ccgagctcga cgggctcgaa cgccgggcgc 60
   gccccgttgg cgacgctggc gcggaaggcg tcctcggcgt cggcgacgcg gagcgctacg 120
   gcgcgcacgg cgaggccgtg gtcggaggcg aagcggcgcg cggcgggggc ggagaaggag 180
   gggagggagg cggtggcggc 200
<210> 427
   <211> 200
   <212> DNA
   <213> Digitaria sanguinalis
<400> 427
   gcttccgcct cgccgaggtc gagctctacg gcgacgtcgt gctccgctac gtcagctacc 60
   cggacgccgc ggatttgccc ttcctgccgg gcttcgagga cgtcgtcatc agcaacccag 120
   gggcggtgga ctacgggctg aggcgcttcg accacatcgt cggcaacgtc tcggagctgg 180
   cgccggtggc cgcgtacgtc 200
<210> 428
   <211> 200
   <212> DNA
   <213> Digitaria sanguinalis
<400> 428
   gacgtacgcg gccaccggcg ccagctccga gacgttgccg acgatgtggt cgaagcgcct 60
   cagcccgtag tccaccgccc ctgggttgct gatgacgacg tcctcgaagc ccggcaggaa 120
   gggcaaatcc gcggcgtccg ggtagctgac gtagcggagc acgacgtcgc cgtagagctc 180
   gacctcggcg aggcggaagc 200
<210> 429
   <211> 200
   <212> DNA
   <213> Digitaria sanguinalis
<400> 429
   gctggcgccg gtggccgcgt acgtcgccgg attcacgggg ttccacgagt tcgccgagtt 60
   cacggcggag gacgtgggca cggcggagag cgggctcaat tccgtggtgc tcgccaacaa 120
   ctccgagaac gtgctgctcc cgctcaacga gccggtgcac ggcaccaagc gccgcagcca 180
   gatacagaca tacctggacc 200
<210> 430
   <211> 200
   <212> DNA
   <213> Digitaria sanguinalis
<400> 430
   ggtccaggta tgtctgtatc tggctgcggc gcttggtgcc gtgcaccggc tcgttgagcg 60
   ggagcagcac gttctcggag ttgttggcga gcaccacgga attgagcccg ctctccgccg 120
   tgcccacgtc ctccgccgtg aactcggcga actcgtggaa ccccgtgaat ccggcgacgt 180
   acgcggccac cggcgccagc 200
<210> 431
   <211> 200
   <212> DNA
   <213> Digitaria sanguinalis
<400> 431
   agccagatac agacatacct ggaccaccac ggcggccctg gagtgcagca catcgcgctg 60
   gctagcgacg acgtgctcag gacactgcgg gagatgcagg cgcgctccgc catgggtggg 120
   tttgagttca tggaggctcc gccacccact tactatgagg gtgtgaggcg gcgcgccggg 180
   gacgtgctct cggaggagca 200
<210> 432
   <211> 200
   <212> DNA
   <213> Digitaria sanguinalis
<400> 432
   tgctcctccg agagcacgtc cccggcgcgc cgcctcacac cctcatagta agtgggtggc 60
   ggagcctcca tgaactcaaa cccacccatg gcggagcgcg cctgcatctc ccgcagtgtc 120
   ctgagcacgt cgtcgctagc cagcgcgatg tgctgcactc cagggccgcc gtggtggtcc 180
   aggtatgtct gtatctggct 200
<210> 433
   <211> 200
   <212> DNA
   <213> Digitaria sanguinalis
<400> 433
   ccggggacgt gctctcggag gagcagataa aggagtgcca gaaactgggg gtgctggtgg 60
   acagggatga ccagggagtg ttgctccaaa tcttcaccaa gccagtgggg gacccaacgt 120
   ttttcttgga gataatccaa aggattgggt gcatggagaa ggatgagcag ggaaaggact 180
   accagaaggg cggctgtggc 200
<210> 434
   <211> 200
   <212> DNA
   <213> Digitaria sanguinalis
<400> 434
   gccacagccg cccttctggt agtcctttcc ctgctcatcc ttctccatgc acccaatcct 60
   ttggattatc tccaagaaaa acgttgggtc ccccactggc ttggtgaaga tttggagcaa 120
   cactccctgg tcatccctgt ccaccagcac ccccagtttc tggcactcct ttatctgctc 180
   ctccgagagc acgtccccgg 200
<210> 435
   <211> 200
   <212> DNA
   <213> Digitaria sanguinalis
<400> 435
   ttgtgaagtt ggtgtcatat atttcaagat ctgttcgtat agaatccaca gatacataat 60
   cattagatta taaaatctca tgaagattat tcatgggtgg ttttcatgca gcagcagtgg 120
   cttcagtggt ggctcttgct tccaatgtct tctcatattc ctcaatggat ttaaaaagct 180
   ctgagaagtt ccctttgcca 200
<210> 436
   <211> 200
   <212> DNA
   <213> Digitaria sanguinalis
<400> 436
   tggcaaaggg aacttctcag agctttttaa atccattgag gaatatgaga agacattgga 60
   agcaagagcc accactgaag ccactgctgc tgcatgaaaa ccacccatga ataatcttca 120
   tgagatttta taatctaatg attatgtatc tgtggattct atacgaacag atcttgaaat 180
   atatgacacc aacttcacaa 200
<210> 437
   <211> 200
   <212> DNA
   <213> Digitaria sanguinalis
<400> 437
   aagctctgag aagttccctt tgccaaaccc tccacaccct gccttctgct gctcctttcc 60
   ttcattatcc ttcaccatac accctactct ctgaattatc tctatgaata tcgtcggcct 120
   gtcaccaaca ggcttggtaa aaatctgaag caaagtcccc tgatcatctc tatcaaccaa 180
   tatccccaat tcttcacact 200
<210> 438
   <211> 200
   <212> DNA
   <213> Digitaria sanguinalis
<400> 438
   agtgtgaaga attggggata ttggttgata gagatgatca ggggactttg cttcagattt 60
   ttaccaagcc tgttggtgac aggccgacga tattcataga gataattcag agagtagggt 120
   gtatggtgaa ggataatgaa ggaaaggagc agcagaaggc agggtgtgga gggtttggca 180
   aagggaactt ctcagagctt 200
<210> 439
   <211> 200
   <212> DNA
   <213> Digitaria sanguinalis
<400> 439
   accaatatcc ccaattcttc acactcctta atctgttcat cagacaacac atcgcccacc 60
   ctattcttca aattcctata ataagtagga gggggagacg gcataaactc aaagccacca 120
   accccactcc ttttcctcat ctcccttaac gtcctaaata tatcctcact agccaacgcc 180
   aaatgctgaa cccccgcccc 200
<210> 440
   <211> 200
   <212> DNA
   <213> Digitaria sanguinalis
<400> 440
   ggggcggggg ttcagcattt ggcgttggct agtgaggata tatttaggac gttaagggag 60
   atgaggaaaa ggagtggggt tggtggcttt gagtttatgc cgtctccccc tcctacttat 120
   tataggaatt tgaagaatag ggtgggcgat gtgttgtctg atgaacagat taaggagtgt 180
   gaagaattgg ggatattggt 200
<210> 441
   <211> 200
   <212> DNA
   <213> Digitaria sanguinalis
<400> 441
   acgccaaatg ctgaaccccc gccccttcat tatgttccaa atacgtctgt atctgactct 60
   tcctcttcgt cccgtaaacc ggttcattca tcggtatcaa taccatctca ctattgcaag 120
   ccaaaaccac cgagtttagt ccactctcac tcgttcccac atcctccgcc gtaaactcag 180
   caaactcgtg aaatccagta 200
<210> 442
   <211> 200
   <212> DNA
   <213> Digitaria sanguinalis
<400> 442
   tactggattt cacgagtttg ctgagtttac ggcggaggat gtgggaacga gtgagagtgg 60
   actaaactcg gtggttttgg cttgcaatag tgagatggta ttgataccga tgaatgaacc 120
   ggtttacggg acgaagagga agagtcagat acagacgtat ttggaacata atgaaggggc 180
   gggggttcag catttggcgt 200
<210> 443
   <211> 200
   <212> DNA
   <213> Euphorbia heterophylla
<400> 443
   gacaattaat aaaaaaaacg taaagactac ctttaattgg aggagaggaa aacaccacgt 60
   ttaaaaatcc cgttgttatc cgattgatga aaaaaaagat taacacgtta cgacttctcc 120
   attcaataat ccattttctt tatcttataa ataatttgaa atcccatcct cctcgttctc 180
   cgttcaccag aaaaaacaga 200
<210> 444
   <211> 200
   <212> DNA
   <213> Euphorbia heterophylla
<400> 444
   tctgtttttt ctggtgaacg gagaacgagg aggatgggat ttcaaattat ttataagata 60
   aagaaaatgg attattgaat ggagaagtcg taacgtgtta atcttttttt tcatcaatcg 120
   gataacaacg ggatttttaa acgtggtgtt ttcctctcct ccaattaaag gtagtcttta 180
   cgtttttttt attaattgtc 200
<210> 445
   <211> 200
   <212> DNA
   <213> Euphorbia heterophylla
<400> 445
   ttctccgttc accagaaaaa acagaaatgg gaaaagacac atcagctgcc gccgaagcat 60
   tcaagctcgt cggattttcc aatttcgtca gaatcaatcc ccggtccgac cgtttcccgg 120
   tcaagcgctt ccaccacgtc gagttctggt gctccgacgc aaccaacacc gctcgccgct 180
   tctcatgggg cctcgggatg 200
<210> 446
   <211> 200
   <212> DNA
   <213> Euphorbia heterophylla
<400> 446
   catcccgagg ccccatgaga agcggcgagc ggtgttggtt gcgtcggagc accagaactc 60
   gacgtggtgg aagcgcttga ccgggaaacg gtcggaccgg ggattgattc tgacgaaatt 120
   ggaaaatccg acgagcttga atgcttcggc ggcagctgat gtgtcttttc ccatttctgt 180
   tttttctggt gaacggagaa 200
<210> 447
   <211> 200
   <212> DNA
   <213> Euphorbia heterophylla
<400> 447
   ccgcttctca tggggcctcg ggatgccgtt cgtcgccaaa tcggatctct ccaccggcaa 60
   cgtcacccac gcctcctacc tcctccgctc cggcgacctc aatttcctct tcacagctcc 120
   ctactctccc tccatagccg ccatggagaa tctctccgat actgctaccg catcaatccc 180
   tactttctcc cgcgacgttt 200
<210> 448
   <211> 200
   <212> DNA
   <213> Euphorbia heterophylla
<400> 448
   aaacgtcgcg ggagaaagta gggattgatg cggtagcagt atcggagaga ttctccatgg 60
   cggctatgga gggagagtag ggagctgtga agaggaaatt gaggtcgccg gagcggagga 120
   ggtaggaggc gtgggtgacg ttgccggtgg agagatccga tttggcgacg aacggcatcc 180
   cgaggcccca tgagaagcgg 200
<210> 449
   <211> 200
   <212> DNA
   <213> Euphorbia heterophylla
<400> 449
   atccctactt tctcccgcga cgttttcctc gatttctccg ccaaacacgg cctcgccgtc 60
   cgagctatag caatcgaagt ggaggacgct gcagttgcct tcaatactag tgttgctcaa 120
   ggcgcggttc cggtggccgg acctgtagtg ctcgataatc gcgcttcggt agcggaggtt 180
   cacttgtacg gcgacgtcgt 200
<210> 450
   <211> 200
   <212> DNA
   <213> Euphorbia heterophylla
<400> 450
   acgacgtcgc cgtacaagtg aacctccgct accgaagcgc gattatcgag cactacaggt 60
   ccggccaccg gaaccgcgcc ttgagcaaca ctagtattga aggcaactgc agcgtcctcc 120
   acttcgattg ctatagctcg gacggcgagg ccgtgtttgg cggagaaatc gaggaaaacg 180
   tcgcgggaga aagtagggat 200
<210> 451
   <211> 200
   <212> DNA
   <213> Euphorbia heterophylla
<400> 451
   aggttcactt gtacggcgac gtcgttttgc ggtacgtcag ttacctaaac tctgatgact 60
   gtttgtttct gccgaaattt gaggcggtag atgaggaggc gtcgtttccg ttggattacg 120
   ggatccggcg gctagatcac gcggttggaa atgtaccgga tcttgctccg gcggtttcgt 180
   atgtgaagaa gttcaccgga 200
<210> 452
   <211> 200
   <212> DNA
   <213> Euphorbia heterophylla
<400> 452
   tccggtgaac ttcttcacat acgaaaccgc cggagcaaga tccggtacat ttccaaccgc 60
   gtgatctagc cgccggatcc cgtaatccaa cggaaacgac gcctcctcat ctaccgcctc 120
   aaatttcggc agaaacaaac agtcatcaga gtttaggtaa ctgacgtacc gcaaaacgac 180
   gtcgccgtac aagtgaacct 200
<210> 453
   <211> 200
   <212> DNA
   <213> Euphorbia heterophylla
<400> 453
   ttcgtatgtg aagaagttca ccggattcca cgagttcgct gagttcacgg cggaggatgt 60
   agggacgagt gagagcggat tgaactcggt ggtgttggct aacaacgaag aaacggtact 120
   gctgccgatg aatgaaccgg tgtttgggac gaagaggaaa agtcagatac agacgtattt 180
   ggagcacaat gaaggagctg 200
<210> 454
   <211> 200
   <212> DNA
   <213> Euphorbia heterophylla
<400> 454
   cagctccttc attgtgctcc aaatacgtct gtatctgact tttcctcttc gtcccaaaca 60
   ccggttcatt catcggcagc agtaccgttt cttcgttgtt agccaacacc accgagttca 120
   atccgctctc actcgtccct acatcctccg ccgtgaactc agcgaactcg tggaatccgg 180
   tgaacttctt cacatacgaa 200
<210> 455
   <211> 200
   <212> DNA
   <213> Euphorbia heterophylla
<400> 455
   tatttggagc acaatgaagg agctggagta cagcatttgg cgcttgtgag tgctgatatt 60
   ttcaatactt tgagagagat gaggaggagg agtgcgattg ggggatttga gtttatgccg 120
   tctcctccgc cgacatatta ccggaatttg aagaagagag ttggagatat tttgagtgat 180
   gaacagatta aggagtgtga 200
<210> 456
   <211> 200
   <212> DNA
   <213> Euphorbia heterophylla
<400> 456
   tcacactcct taatctgttc atcactcaaa atatctccaa ctctcttctt caaattccgg 60
   taatatgtcg gcggaggaga cggcataaac tcaaatcccc caatcgcact cctcctcctc 120
   atctctctca aagtattgaa aatatcagca ctcacaagcg ccaaatgctg tactccagct 180
   ccttcattgt gctccaaata 200
<210> 457
   <211> 200
   <212> DNA
   <213> Euphorbia heterophylla
<400> 457
   gtgatgaaca gattaaggag tgtgaagaat tagggattct ggtggacagg gatgatcaag   60
   ggaccttgct tcagattttc actaaacctg tgggagatag gtatttctat cttcttcttc  120
   attgttttca cttacaatac ctttttccat tgaaaattct ttgtttcttg tgtttctctt  180
   atatgtgttg gctgctatac                                              200
<210> 458
   <211> 200
   <212> DNA
   <213> Euphorbia heterophylla
<400> 458
   gtatagcagc caacacatat aagagaaaca caagaaacaa agaattttca atggaaaaag   60
   gtattgtaag tgaaaacaat gaagaagaag atagaaatac ctatctccca caggtttagt  120
   gaaaatctga agcaaggtcc cttgatcatc cctgtccacc agaatcccta attcttcaca  180
   ctccttaatc tgttcatcac                                              200
<210> 459
   <211> 200
   <212> DNA
   <213> Euphorbia heterophylla
<400> 459
   ctcttatatg tgttggctgc tatactatgg ttgatagaga attagaattt agcgtgttca   60
   aaactcaatt ctttatcttt atctttagcc ttaggtcatt ttgggattgc tttcaaactg  120
   atttgattat gttactaatt aattagtacg aaggactaac atgtgttgtt ctaagctaaa  180
   atatattcta taaagctaaa                                              200
<210> 460
   <211> 200
   <212> DNA
   <213> Euphorbia heterophylla
<400> 460
   tttagcttta tagaatatat tttagcttag aacaacacat gttagtcctt cgtactaatt 60
   aattagtaac ataatcaaat cagtttgaaa gcaatcccaa aatgacctaa ggctaaagat 120
   aaagataaag aattgagttt tgaacacgct aaattctaat tctctatcaa ccatagtata 180
   gcagccaaca catataagag 200
<210> 461
   <211> 200
   <212> DNA
   <213> Euphorbia heterophylla
<400> 461
   ctaaaatata ttctataaag ctaaaatatc ttaataagca ataccaaact agcccttaaa 60
   tgcatagtta gaccctaaat ttgacaactt ttataacttg gcgcctctaa tttacaaatg 120
   tcttcgccaa cctcttgaac tggcaaaagt tttcactgca acccctaggt gccacgtgtc 180
   accttgtgat tgatttgact 200
<210> 462
   <211> 200
   <212> DNA
   <213> Euphorbia heterophylla
<400> 462
   agtcaaatca atcacaaggt gacacgtggc acctaggggt tgcagtgaaa acttttgcca 60
   gttcaagagg ttggcgaaga catttgtaaa ttagaggcgc caagttataa aagttgtcaa 120
   atttagggtc taactatgca tttaagggct agtttggtat tgcttattaa gatattttag 180
   ctttatagaa tatattttag 200
<210> 463
   <211> 200
   <212> DNA
   <213> Euphorbia heterophylla
<400> 463
   gtgtcacctt gtgattgatt tgacttttca aagatgtcaa aattttttat tttcctccct 60
   ctaatcaaaa ggtgacaagt gatattgagt aggggtctgt agtgaatact ttccccaagt 120
   tgagacatcc agaaatcgga ggcgtcatgt gataaaagtt gcttagttaa gggagcttaa 180
   tatgtattaa gccttttcgc 200
<210> 464
   <211> 200
   <212> DNA
   <213> Euphorbia heterophylla
<400> 464
   gcgaaaaggc ttaatacata ttaagctccc ttaactaagc aacttttatc acatgacgcc 60
   tccgatttct ggatgtctca acttggggaa agtattcact acagacccct actcaatatc 120
   acttgtcacc ttttgattag agggaggaaa ataaaaaatt ttgacatctt tgaaaagtca 180
   aatcaatcac aaggtgacac 200
<210> 465
   <211> 200
   <212> DNA
   <213> Euphorbia heterophylla
<400> 465
   cttaatatgt attaagcctt ttcgctacct tgccatttaa atccttagtc caagcaaaat 60
   atacaaaaat tagttagaac tttatgattc agaacctacg taactttaat ttgaccagta 120
   gttctatctt atgcatgcca gccatgttga ctggtgaact tatattcaat tttgctattc 180
   aagtaaatcc ttatccatca 200
<210> 466
   <211> 200
   <212> DNA
   <213> Euphorbia heterophylla
<400> 466
   tgatggataa ggatttactt gaatagcaaa attgaatata agttcaccag tcaacatggc 60
   tggcatgcat aagatagaac tactggtcaa attaaagtta cgtaggttct gaatcataaa 120
   gttctaacta atttttgtat attttgcttg gactaaggat ttaaatggca aggtagcgaa 180
   aaggcttaat acatattaag 200
<210> 467
   <211> 200
   <212> DNA
   <213> Euphorbia heterophylla
<400> 467
   tattcaagta aatccttatc catcaatccg aatcaaaaga ttttaatgtt aggttaaact 60
   tgtacgattc aaaacttagg ttgtactttt tgactagtag ttctattatg tatgtgtata 120
   tataatgggg tttttttctt gtaggccaac gattttcgta gagataatac agagagtagg 180
   gtgtatgcta aaggacgaag 200
<210> 468
   <211> 200
   <212> DNA
   <213> Euphorbia heterophylla
<400> 468
   cttcgtcctt tagcatacac cctactctct gtattatctc tacgaaaatc gttggcctac   60
   agaaaaaaaa ccccattata tatacacata cataatagaa ctactagtca aaaagtacaa  120
   cctaagtttt gaatcgtaca agtttaacct aacattaaaa tcttttgatt cggattgatg  180
   gataaggatt tacttgaata                                              200
<210> 469
   <211> 200
   <212> DNA
   <213> Euphorbia heterophylla
<400> 469
   gtagggtgta tgctaaagga cgaagcaggg agagaatacc agaagggtgg atgtggcggt   60
   tttggtaagg gaaacttttc ggagttattc aaatccattg aagaatacga gaaaacactg  120
   gaagccaaac gaactgcaca agcatagagg ttagtaggga atttgatgat acccataaat  180
   aatggttgtt acttgttgcc                                              200
<210> 470
   <211> 200
   <212> DNA
   <213> Euphorbia heterophylla
<400> 470
   ggcaacaagt aacaaccatt atttatgggt atcatcaaat tccctactaa cctctatgct   60
   tgtgcagttc gtttggcttc cagtgttttc tcgtattctt caatggattt gaataactcc  120
   gaaaagtttc ccttaccaaa accgccacat ccacccttct ggtattctct ccctgcttcg  180
   tcctttagca tacaccctac                                              200
<210> 471
   <211> 200
   <212> DNA
   <213> Euphorbia heterophylla
<400> 471
   tgactgaaat taaattttaa ttatttgcaa agcttacggt gaaattaaac tctaaccatt 60
   aaaattttaa tgagtcaaaa ttgaccgaaa tgttcatgtc ataaatttag ataatccttt 120
   cagatatgta cgtgaaacca tgtacctaaa ttgtagtcag attgctaaac ccccttcgaa 180
   aatgaatgaa atgatatgat 200
<210> 472
   <211> 200
   <212> DNA
   <213> Euphorbia heterophylla
<400> 472
   atcatatcat ttcattcatt ttcgaagggg gtttagcaat ctgactacaa tttaggtaca 60
   tggtttcacg tacatatctg aaaggattat ctaaatttat gacatgaaca tttcggtcaa 120
   ttttgactca ttaaaatttt aatggttaga gtttaatttc accgtaagct ttgcaaataa 180
   ttaaaattta atttcagtca 200
<210> 473
   <211> 200
   <212> DNA
   <213> Euphorbia heterophylla
<400> 473
   tcgaaaatga atgaaatgat atgatatgct atgctatgta ttttaaggat tattttatag 60
   taactttgtt tataatttac tttatatgat catcatccaa ttaactttca cctcactaat 120
   tcaatgattg aaatggacta agtaatttta cttaataaaa aaataaaatc actataacct 180
   acacatattt ttaaaaataa 200
<210> 474
   <211> 200
   <212> DNA
   <213> Euphorbia heterophylla
<400> 474
   ttatttttaa aaatatgtgt aggttatagt gattttattt ttttattaag taaaattact 60
   tagtccattt caatcattga attagtgagg tgaaagttaa ttggatgatg atcatataaa 120
   gtaaattata aacaaagtta ctataaaata atccttaaaa tacatagcat agcatatcat 180
   atcatttcat tcattttcga 200
<210> 475
   <211> 200
   <212> DNA
   <213> Euphorbia heterophylla
<400> 475
   aacctacaca tatttttaaa aataacacca taatttatta aaggacattt aattgaagta    60
   gaaaattata taattactcg ctaataaatt ttcaattgga ggagaggaaa acaccacgtt   120
   taaaaatccc gttgttatcc gattgacgaa aaaaaagatt aacacgtcac gacttctcca   180
   ttcaataatc cattttcttt                                               200
<210> 476
   <211> 200
   <212> DNA
   <213> Euphorbia heterophylla
<400> 476
   aaagaaaatg gattattgaa tggagaagtc gtgacgtgtt aatctttttt ttcgtcaatc    60
   ggataacaac gggattttta aacgtggtgt tttcctctcc tccaattgaa aatttattag   120
   cgagtaatta tataattttc tacttcaatt aaatgtcctt taataaatta tggtgttatt   180
   tttaaaaata tgtgtaggtt                                               200
<210> 477
   <211> 200
   <212> DNA
   <213> Euphorbia heterophylla
<400> 477
   ctccattcaa taatccattt tctttatctt ataaataatt tgaaatccca tcctcctcgt    60
   tctccgttca ccagaaaaaa cagaaatggg aaaagacacg tcagccgctg ccgaagcatt   120
   caagctcgtc ggattttcca atttcgtcag aatcaatccc cggtccgacc gtttcccggt   180
   caagcgcttc caccacgtcg                                               200
<210> 478
   <211> 200
   <212> DNA
   <213> Euphorbia heterophylla
<400> 478
   cgacgtggtg gaagcgcttg accgggaaac ggtcggaccg gggattgatt ctgacgaaat    60
   tggaaaatcc gacgagcttg aatgcttcgg cagcggctga cgtgtctttt cccatttctg   120
   ttttttctgg tgaacggaga acgaggagga tgggatttca aattatttat aagataaaga   180
   aaatggatta ttgaatggag                                               200
<210> 479
   <211> 200
   <212> DNA
   <213> Euphorbia heterophylla
<400> 479
   ccggtcaagc gcttccacca cgtcgagttc aggtgctccg acgcaaccaa caccgctcgc 60
   cgcttctcat ggggcctcgg gatgccgttc gtcgccaaat cggatctctc caccggcaac 120
   gtcacccacg cctcctacct cctccgctcc ggcgacctca atttcctctt cacagctccc 180
   tactctccct ccatagccgc 200
<210> 480
   <211> 200
   <212> DNA
   <213> Euphorbia heterophylla
<400> 480
   gcggctatgg agggagagta gggagctgtg aagaggaaat tgaggtcgcc ggagcggagg 60
   aggtaggagg cgtgggtgac gttgccggtg gagagatccg atttggcgac gaacggcatc 120
   ccgaggcccc atgagaagcg gcgagcggtg ttggttgcgt cggagcacct gaactcgacg 180
   tggtggaagc gcttgaccgg 200
<210> 481
   <211> 200
   <212> DNA
   <213> Euphorbia heterophylla
<400> 481
   ctccctactc tccctccata gccgccatgg agaatctctc cgatactgcc accgcatcaa 60
   tccctacttt ctcccgcgac gttttcctcg atttctccgc caaacacggc ctcgccgtcc 120
   gagctatagc cattgaagtg gaagatgctg cgattgcttt cactaccagc gttgctcaag 180
   gcgcgattcc ggtggccgga 200
<210> 482
   <211> 200
   <212> DNA
   <213> Euphorbia heterophylla
<400> 482
   tccggccacc ggaatcgcgc cttgagcaac gctggtagtg aaagcaatcg cagcatcttc 60
   cacttcaatg gctatagctc ggacggcgag gccgtgtttg gcggagaaat cgaggaaaac 120
   gtcgcgggag aaagtaggga ttgatgcggt ggcagtatcg gagagattct ccatggcggc 180
   tatggaggga gagtagggag 200
<210> 483
   <211> 200
   <212> DNA
   <213> Euphorbia heterophylla
<400> 483
   tcaaggcgcg attccggtgg ccggacctat tgtgctcgat aatcgtgctt cagttgcgga 60
   ggtccacttg tacggcgacg tcgttttgcg gtacgtcagt tatctaaact ccgatagttg 120
   cttgtttctg ccgaaattcg agccggtaga tgaggcgtcg tctttcccgt tggattacgg 180
   gattcgacgg ctagatcacg 200
<210> 484
   <211> 200
   <212> DNA
   <213> Euphorbia heterophylla
<400> 484
   cgtgatctag ccgtcgaatc ccgtaatcca acgggaaaga cgacgcctca tctaccggct 60
   cgaatttcgg cagaaacaag caactatcgg agtttagata actgacgtac cgcaaaacga 120
   cgtcgccgta caagtggacc tccgcaactg aagcacgatt atcgagcaca ataggtccgg 180
   ccaccggaat cgcgccttga 200
<210> 485
   <211> 200
   <212> DNA
   <213> Euphorbia heterophylla
<400> 485
   tacgggattc gacggctaga tcacgcggtt ggaaatgtgc cggaattatc tccggcggtt 60
   tcgtatgtga aacagttcac cggattccat gagttcgccg agttcacggc ggaggatgtg 120
   gggacgagtg agagcggatt gaactcggtg gtgttggcta ataacgaaga aacggtttta 180
   ctaccgttga atgaaccggt 200
<210> 486
   <211> 200
   <212> DNA
   <213> Euphorbia heterophylla
<400> 486
   accggttcat tcaacggtag taaaaccgtt tcttcgttat tagccaacac caccgagttc 60
   aatccgctct cactcgtccc cacatcctcc gccgtgaact cggcgaactc atggaatccg 120
   gtgaactgtt tcacatacga aaccgccgga gataattccg gcacatttcc aaccgcgtga 180
   tctagccgtc gaatcccgta 200
<210> 487
   <211> 200
   <212> DNA
   <213> Euphorbia heterophylla
<400> 487
   ttttactacc gttgaatgaa ccggtgtatg gcacaaagag gaaaagtcag atacaaacgt 60
   atttggagca caacgaaggg gctggagtac agcatttagc acttgtgagt gcggatatat 120
   ttaacacttt gagagagatg aggaagagga gtggcgttgg gggatttgag tttatgccgt 180
   ctcctccgcc cacatattac 200
<210> 488
   <211> 200
   <212> DNA
   <213> Euphorbia heterophylla
<400> 488
   gtaatatgtg ggcggaggag acggcataaa ctcaaatccc ccaacgccac tcctcttcct 60
   catctctctc aaagtgttaa atatatccgc actcacaagt gctaaatgct gtactccagc 120
   cccttcgttg tgctccaaat acgtttgtat ctgacttttc ctctttgtgc catacaccgg 180
   ttcattcaac ggtagtaaaa 200
<210> 489
   <211> 200
   <212> DNA
   <213> Euphorbia heterophylla
<400> 489
   gccgtctcct ccgcccacat attaccggaa tttgaagaag agagtcgggg atattttgag 60
   cgatgaacag attaaggaat gtgaagaatt agggattttg gtggataggg atgatcaagg 120
   gaccttgctt cagattttca ctaaacctgt gggagatagg tatgtcaatc aatcttgtga 180
   aatgattagt tgttctataa 200
<210> 490
   <211> 200
   <212> DNA
   <213> Euphorbia heterophylla
<400> 490
   ttatagaaca actaatcatt tcacaagatt gattgacata cctatctccc acaggtttag 60
   tgaaaatctg aagcaaggtc ccttgatcat ccctatccac caaaatccct aattcttcac 120
   attccttaat ctgttcatcg ctcaaaatat ccccgactct cttcttcaaa ttccggtaat 180
   atgtgggcgg aggagacggc 200
<210> 491
   <211> 200
   <212> DNA
   <213> Euphorbia heterophylla
<400> 491
   tgtgaaatga ttagttgttc tataattact tatgtgattt acattcttag agtctgtaaa 60
   ttcttgtgat cataaatttg ttggcttgga taccatatta atattatcct taatttagaa 120
   tgtcaaagtt atgttctgtc tttacaatgc cgttgaaatc gttatttatc aagtccaagc 180
   aaacggtcct aaaatgtcct 200
<210> 492
   <211> 200
   <212> DNA
   <213> Euphorbia heterophylla
<400> 492
   aggacatttt aggaccgttt gcttggactt gataaataac gatttcaacg gcattgtaaa 60
   gacagaacat aactttgaca ttctaaatta aggataatat taatatggta tccaagccaa 120
   caaatttatg atcacaagaa tttacagact ctaagaatgt aaatcacata agtaattata 180
   gaacaactaa tcatttcaca 200
<210> 493
   <211> 200
   <212> DNA
   <213> Euphorbia heterophylla
<400> 493
   caagcaaacg gtcctaaaat gtcctttaac ttactggttc ggaatctcat ctgttcaatt 60
   tgattagtag ttccatcttt tagtatatac gccaggcgcc agacatgttg atttgtgagt 120
   ttttgttatt tggaatctca aagttgagtt ttttctatct ataaaaagta gctcaaatcc 180
   ttagttatca agtcaaaacc 200
<210> 494
   <211> 200
   <212> DNA
   <213> Euphorbia heterophylla
<400> 494
   ggttttgact tgataactaa ggatttgagc tactttttat agatagaaaa aactcaactt    60
   tgagattcca aataacaaaa actcacaaat caacatgtct ggcgcctggc gtatatacta   120
   aaagatggaa ctactaatca aattgaacag atgagattcc gaaccagtaa gttaaaggac   180
   attttaggac cgtttgcttg                                               200
<210> 495
   <211> 200
   <212> DNA
   <213> Euphorbia heterophylla
<400> 495
   aatccttagt tatcaagtca aaacccaaag atcctattat taacttggat ctgaaattat    60
   atcgctttct ggtttatttc taaatttggt ggtcacttga tttctcatct tgacaagtga   120
   attcatctgg acatagttgg tcttttatgg gatgaatgga tgtgctgttt ttgcaggcca   180
   accattttcg ttgaaataat                                               200
<210> 496
   <211> 200
   <212> DNA
   <213> Euphorbia heterophylla
<400> 496
   attatttcaa cgaaaatggt tggcctgcaa aaacagcaca tccattcatc ccataaaaga    60
   ccaactatgt ccagatgaat tcacttgtca agatgagaaa tcaagtgacc accaaattta   120
   gaaataaacc agaaagcgat ataatttcag atccaagtta ataataggat ctttgggttt   180
   tgacttgata actaaggatt                                               200
<210> 497
   <211> 200
   <212> DNA
   <213> Euphorbia heterophylla
<400> 497
   ggccaaccat tttcgttgaa ataattcagc gagtcgggtg tatggtcaag gatgaagcag    60
   ggagagaata ccagaagggt gggtgtggcg gtttcggtaa gggaaacttc tcggagttat   120
   tcaaatcaat cgaggaatat gagaaaacat tggaggccaa acgaactgca gaagcagctc   180
   gagcataaaa taaaggttag                                               200
<210> 498
   <211> 200
   <212> DNA
   <213> Euphorbia heterophylla
<400> 498
   ctaaccttta ttttatgctc gagctgcttc tgcagttcgt ttggcctcca atgttttctc 60
   atattcctcg attgatttga ataactccga gaagtttccc ttaccgaaac cgccacaccc 120
   acccttctgg tattctctcc ctgcttcatc cttgaccata cacccgactc gctgaattat 180
   ttcaacgaaa atggttggcc 200
<210> 499
   <211> 200
   <212> DNA
   <213> Euphorbia heterophylla
<400> 499
   agctcgagca taaaataaag gttagtatgg aagatgatat gcatacataa ataaaggttg 60
   gctgttgttt attacgttgt aatattctac tccaatgatg ctgttattgt ctgaaaatac 120
   cagtgcctgc gaattggaat gttctctaat tgttaggaac aactggaatc attgtatatc 180
   gtaaactatg gttcattaaa 200
<210> 500
   <211> 200
   <212> DNA
   <213> Euphorbia heterophylla
<400> 500
   tttaatgaac catagtttac gatatacaat gattccagtt gttcctaaca attagagaac 60
   attccaattc gcaggcactg gtattttcag acaataacag catcattgga gtagaatatt 120
   acaacgtaat aaacaacagc caacctttat ttatgtatgc atatcatctt ccatactaac 180
   ctttatttta tgctcgagct 200
<210> 501
   <211> 200
   <212> DNA
   <213> Euphorbia heterophylla
<400> 501
   atatcgtaaa ctatggttca ttaaaagtta aaaacatata tatgatatga ttaatataga 60
   attactccct ttatttggaa tctaaagatt catttctcag tttctttctg ggcgattttg 120
   agcatttctg tacttaaatt ctgcgcgaat acacccacca agccctagct agcttggtgg 180
   ttacctttta agggtgttgg 200
<210> 502
   <211> 200
   <212> DNA
   <213> Euphorbia heterophylla
<400> 502
   ccaacaccct taaaaggtaa ccaccaagct agctagggct tggtgggtgt attcgcgcag 60
   aatttaagta cagaaatgct caaaatcgcc cagaaagaaa ctgagaaatg aatctttaga 120
   ttccaaataa agggagtaat tctatattaa tcatatcata tatatgtttt taacttttaa 180
   tgaaccatag tttacgatat 200
<210> 503
   <211> 200
   <212> DNA
   <213> Euphorbia heterophylla
<400> 503
   ggtggttacc ttttaagggt gttggaaacc taaattagaa aagatttcag tttaatttat 60
   gtcattttat atataaaagt ttgaaacttt tttattcaaa ataagttgat tgataaattg 120
   aaatcaatta gctttgtagc agatgttcac ccaaatctaa tataaatata ttataaatct 180
   aatatatttc tgttaacaaa 200
<210> 504
   <211> 200
   <212> DNA
   <213> Euphorbia heterophylla
<400> 504
   tttgttaaca gaaatatatt agatttataa tatatttata ttagatttgg gtgaacatct 60
   gctacaaagc taattgattt caatttatca atcaacttat tttgaataaa aaagtttcaa 120
   acttttatat ataaaatgac ataaattaaa ctgaaatctt ttctaattta ggtttccaac 180
   acccttaaaa ggtaaccacc 200
<210> 505
   <211> 200
   <212> DNA
   <213> Euphorbia heterophylla
<400> 505
   aatctaatat atttctgtta acaaacttat aatatattaa taaatacaat tgcaaagaca 60
   aagagactta tattagaatt ataacataat tttttataaa tatattagat acgtatatct 120
   tataattgta acatataaaa gattttatag taaatatgtg ttataagtct aaaataagcc 180
   tttttaagtt taatacaagt 200
<210> 506
   <211> 200
   <212> DNA
   <213> Euphorbia heterophylla
<400> 506
   acttgtatta aacttaaaaa ggcttatttt agacttataa cacatattta ctataaaatc 60
   ttttatatgt tacaattata agatatacgt atctaatata tttataaaaa attatgttat 120
   aattctaata taagtctctt tgtctttgca attgtattta ttaatatatt ataagtttgt 180
   taacagaaat atattagatt 200
<210> 507
   <211> 200
   <212> DNA
   <213> Euphorbia heterophylla
<400> 507
   aagccttttt aagtttaata caagtctctt tgtgtaacac cccgggttcg accgattgac 60
   ccccacaaac caacacaagt ctttctagta agctttgcct tgagattgtt ccaagtcaag 120
   gacgctcaac tttggagttc tcccatataa gctcctgaaa agaaggtgta acttgttggt 180
   ataggcagca gtaccaatca 200
<210> 508
   <211> 200
   <212> DNA
   <213> Euphorbia heterophylla
<400> 508
   tgattggtac tgctgcctat accaacaagt tacaccttct tttcaggagc ttatatggga 60
   gaactccaaa gttgagcgtc cttgacttgg aacaatctca aggcaaagct tactagaaag 120
   acttgtgttg gtttgtgggg gtcaatcggt cgaacccggg gtgttacaca aagagacttg 180
   tattaaactt aaaaaggctt 200
<210> 509
   <211> 200
   <212> DNA
   <213> Euphorbia heterophylla
<400> 509
   ttggtatagg cagcagtacc aatcaaacct ttttaagtcc ctttccactt taatgtccct 60
   ttctattgat ataattttat tgactttatg taatcaaatg tttattcatc ccgtgttttt 120
   ttattcatta tgttttaatt ttttaattag tcaaaatata atatctaaaa aatctatcaa 180
   atttgggctc ggctcggctt 200
<210> 510
   <211> 200
   <212> DNA
   <213> Euphorbia heterophylla
<400> 510
   aagccgagcc gagcccaaat ttgatagatt ttttagatat tatattttga ctaattaaaa 60
   aattaaaaca taatgaataa aaaaacacgg gatgaataaa catttgatta cataaagtca 120
   ataaaattat atcaatagaa agggacatta aagtggaaag ggacttaaaa aggtttgatt 180
   ggtactgctg cctataccaa 200
<210> 511
   <211> 200
   <212> DNA
   <213> Euphorbia heterophylla
<400> 511
   atcaaatttg ggctcggctc ggcttgactc attaacatcc gactctctac ctcttacttt 60
   tattaaatac atttgtgttt ttcagttgta cgttccttct catatttaca tttccctttt 120
   ttcttttctt tttaatttat tttttattta tttcgcaaat cgttgatttt ctcggtatgc 180
   tgctttataa ttttttgttc 200
<210> 512
   <211> 200
   <212> DNA
   <213> Euphorbia heterophylla
<400> 512
   gaacaaaaaa ttataaagca gcataccgag aaaatcaacg atttgcgaaa taaataaaaa 60
   ataaattaaa aagaaaagaa aaaagggaaa tgtaaatatg agaaggaacg tacaactgaa 120
   aaacacaaat gtatttaata aaagtaagag gtagagagtc ggatgttaat gagtcaagcc 180
   gagccgagcc caaatttgat 200
<210> 513
   <211> 200
   <212> DNA
   <213> Euphorbia heterophylla
<400> 513
   tatgctgctt tataattttt tgttcgtctc gtttttatct tcgaaagtat attcatgttg 60
   atatactttt tcaagttctt attttcaaag atatgtaaca aatgacataa acattatcca 120
   gcaaaaatca gtaaattact agcatttcac caacaaaata ctagcatttt acccgtgacg 180
   aaaagtttga aaaaaatata 200
<210> 514
   <211> 200
   <212> DNA
   <213> Euphorbia heterophylla
<400> 514
   tatatttttt tcaaactttt cgtcacgggt aaaatgctag tattttgttg gtgaaatgct 60
   agtaatttac tgatttttgc tggataatgt ttatgtcatt tgttacatat ctttgaaaat 120
   aagaacttga aaaagtatat caacatgaat atactttcga agataaaaac gagacgaaca 180
   aaaaattata aagcagcata 200
<210> 515
   <211> 200
   <212> DNA
   <213> Euphorbia heterophylla
<400> 515
   tgacgaaaag tttgaaaaaa atatataaaa aaaatcaaac aaaaatcatt tttataccaa 60
   cattatacca tgtatgaaaa aataaaactg atcataaaca taaagaaatc aaacaaaatt 120
   caccttatct taatatttta ccaagagtga aggaccaaac ataaacatgt tttacgaaaa 180
   aaatcaaaca aaaatcattt 200
<210> 516
   <211> 200
   <212> DNA
   <213> Euphorbia heterophylla
<400> 516
   aaatgatttt tgtttgattt ttttcgtaaa acatgtttat gtttggtcct tcactcttgg 60
   taaaatatta agataaggtg aattttgttt gatttcttta tgtttatgat cagttttatt 120
   ttttcataca tggtataatg ttggtataaa aatgattttt gtttgatttt ttttatatat 180
   ttttttcaaa cttttcgtca 200
<210> 517
   <211> 200
   <212> DNA
   <213> Euphorbia heterophylla
<400> 517
   gaaaaaaatc aaacaaaaat catttcacac tagcatttta ttaacaaaat ctcgatatat 60
   tatcagtaaa ataccaacat tttatcaaca aaataccatc atttgagagg aggttcgagt 120
   gcgccttcgc cgttctgcgg cggcttcttt gacggtggag aaagcatagc tgcgtgatgg 180
   aggcagaaga ggggttgatg 200
<210> 518
   <211> 200
   <212> DNA
   <213> Euphorbia heterophylla
<400> 518
   catcaacccc tcttctgcct ccatcacgca gctatgcttt ctccaccgtc aaagaagccg 60
   ccgcagaacg gcgaaggcgc actcgaacct cctctcaaat gatggtattt tgttgataaa 120
   atgttggtat tttactgata atatatcgag attttgttaa taaaatgcta gtgtgaaatg 180
   atttttgttt gatttttttc 200
<210> 519
   <211> 200
   <212> DNA
   <213> Euphorbia heterophylla
<400> 519
   gatggaggca gaagaggggt tgatgaaatt gggttggatt gaagtggaat tgagtcaatg 60
   aggctaggtg atagggtgag atgatgcatg actcgaattg gtggttgatc gcagaggtgg 120
   aggtggcgat tggccggcgg agaagacggc aaatagatgt ttttgggcgg agtacaaagc 180
   gccttaaatc ctagcaaagg 200
<210> 520
   <211> 200
   <212> DNA
   <213> Euphorbia heterophylla
<400> 520
   cctttgctag gatttaaggc gctttgtact ccgcccaaaa acatctattt gccgtcttct 60
   ccgccggcca atcgccacct ccacctctgc gatcaaccac caattcgagt catgcatcat 120
   ctcaccctat cacctagcct cattgactca attccacttc aatccaaccc aatttcatca 180
   acccctcttc tgcctccatc 200
<210> 521
   <211> 200
   <212> DNA
   <213> Euphorbia heterophylla
<400> 521
   aaagcgcctt aaatcctagc aaaggctgat gaaaataaaa aagggtaatg ctgcactttt   60
   atctactttg tgcaatatct atatacagta tttattatca tcttattcct tttatttatt  120
   tctttcatct tttatgggag ttgaaattct ttctagatat tcttttcttt ttgctaaatt  180
   tttctgctca ttgtcataca                                              200
<210> 522
   <211> 200
   <212> DNA
   <213> Euphorbia heterophylla
<400> 522
   tgtatgacaa tgagcagaaa aatttagcaa aaagaaaaga atatctagaa agaatttcaa   60
   ctcccataaa agatgaaaga aataaataaa aggaataaga tgataataaa tactgtatat  120
   agatattgca caaagtagat aaaagtgcag cattaccctt ttttattttc atcagccttt  180
   gctaggattt aaggcgcttt                                              200
<210> 523
   <211> 200
   <212> DNA
   <213> Euphorbia heterophylla
<400> 523
   aaatttttct gctcattgtc atacacttgc ttggtaaact gaatctcaaa ccaaataatg   60
   aaatctgaag tatgtatcca cagaatcgca atgctcatcg acggaagtag aaggggaaaa  120
   ctgctgttgg agttgaggta tataatctgc agttgaaatc gacgttgtgt atgtaatttt  180
   gaattgaaca agagcgaaac                                              200
<210> 524
   <211> 200
   <212> DNA
   <213> Euphorbia heterophylla
<400> 524
   gtttcgctct tgttcaattc aaaattacat acacaacgtc gatttcaact gcagattata 60
   tacctcaact ccaacagcag ttttcccctt ctacttccgt cgatgagcat tgcgattctg 120
   tggatacata cttcagattt cattatttgg tttgagattc agtttaccaa gcaagtgtat 180
   gacaatgagc agaaaaattt 200
<210> 525
   <211> 200
   <212> DNA
   <213> Euphorbia heterophylla
<400> 525
   attttgaatt gaacaagagc gaaacctagt tttctatgat ctgtacctgg cgtataagaa 60
   ttccatcaat atcggcaggg cgatgcgtta atattgcatt aacaccggtg gtgagaaggg 120
   agctaattgc ttgaaatccg ctggcatgcc gcaatgttga tatgtcacat aaatgcgact 180
   aagtgtgact tatcttgaag 200
<210> 526
   <211> 200
   <212> DNA
   <213> Euphorbia heterophylla
<400> 526
   cttcaagata agtcacactt agtcgcattt atgtgacata tcaacattgc ggcatgccag 60
   cggatttcaa gcaattagct cccttctcac caccggtgtt aatgcaatat taacgcatcg 120
   ccctgccgat attgatggaa ttcttatacg ccaggtacag atcatagaaa actaggtttc 180
   gctcttgttc aattcaaaat 200
<210> 527
   <211> 200
   <212> DNA
   <213> Euphorbia heterophylla
<400> 527
   cgactaagtg tgacttatct tgaagaggag tgaaattggg gaaacgacta agagtgactt 60
   atcaagaagt gtgattacca tgcaactaag tgcgacacgg tgaaacgact cgactcctgc 120
   agccaccacc aaatttcaca atttcatata ggtaaaaggg agacgatcag aaagagggga 180
   aacaaaacac aaagagaggg 200
<210> 528
   <211> 200
   <212> DNA
   <213> Euphorbia heterophylla
<400> 528
   ccctctcttt gtgttttgtt tcccctcttt ctgatcgtct cccttttacc tatatgaaat 60
   tgtgaaattt ggtggtggct gcaggagtcg agtcgtttca ccgtgtcgca cttagttgca 120
   tggtaatcac acttcttgat aagtcactct tagtcgtttc cccaatttca ctcctcttca 180
   agataagtca cacttagtcg 200
<210> 529
   <211> 200
   <212> DNA
   <213> Euphorbia heterophylla
<400> 529
   ggggaaacaa aacacaaaga gagggaaaag aaatgttgga gggactgaaa ttatagaagg 60
   ggtaaaatta gaaacaaaaa aaattataag gtgttgtaag tagaaattaa attacgagca 120
   aaagttggtt gaaaataaaa atgaagatat tgtgataaat tatttctttt tttttatatt 180
   tgaccaactt acattgtaaa 200
<210> 530
   <211> 200
   <212> DNA
   <213> Euphorbia heterophylla
<400> 530
   tttacaatgt aagttggtca aatataaaaa aaaagaaata atttatcaca atatcttcat 60
   ttttattttc aaccaacttt tgctcgtaat ttaatttcta cttacaacac cttataattt 120
   tttttgtttc taattttacc ccttctataa tttcagtccc tccaacattt cttttccctc 180
   tctttgtgtt ttgtttcccc 200
<210> 531
   <211> 200
   <212> DNA
   <213> Euphorbia heterophylla
<400> 531
   atatttgacc aacttacatt gtaaagcctg taatttttat taatttcgca ttatagccat 60
   aaaaagttat tccaatctat gaagttgtga ctttgcggaa catattttcc gaaaaactta 120
   tttttcaagt tttttaaata gtaagaaaaa ggaaatttta aattataata acataatagg 180
   tagtaaatat aaatagtata 200
<210> 532
   <211> 200
   <212> DNA
   <213> Euphorbia heterophylla
<400> 532
   tatactattt atatttacta cctattatgt tattataatt taaaatttcc tttttcttac   60
   tatttaaaaa acttgaaaaa taagtttttc ggaaaatatg ttccgcaaag tcacaacttc  120
   atagattgga ataacttttt atggctataa tgcgaaatta ataaaaatta caggctttac  180
   aatgtaagtt ggtcaaatat                                              200
<210> 533
   <211> 200
   <212> DNA
   <213> Euphorbia heterophylla
<400> 533
   ataggtagta aatataaata gtatatattt ataattcaaa taattatcag gaaatcttat   60
   ttattttatg aaatttctag gtaaaattaa tttagtcatg actaagtgtt gtttctcaca  120
   aaattgtaat cataatcaaa ctaaattttg atatcgagaa acacaaaatg agcatttgtt  180
   gaaagagact tataggtcga                                              200
<210> 534
   <211> 200
   <212> DNA
   <213> Euphorbia heterophylla
<400> 534
   tcgacctata agtctctttc aacaaatgct cattttgtgt ttctcgatat caaaatttag   60
   tttgattatg attacaattt tgtgagaaac aacacttagt catgactaaa ttaattttac  120
   ctagaaattt cataaaataa ataagatttc ctgataatta tttgaattat aaatatatac  180
   tatttatatt tactacctat                                              200
<210> 535
   <211> 200
   <212> DNA
   <213> Euphorbia heterophylla
<400> 535
   ttgttgaaag agacttatag gtcgagtctt aacaaacttg ataagattat aaaattgtct 60
   agcataaact cctcatgcag cgaaacaata atgtatgatt acaatcataa gccaatgttc 120
   atgttggata aaccacacga taactaattc tagaatctct caagagtcta aataatttct 180
   atgcacactt ttaaatttat 200
<210> 536
   <211> 200
   <212> DNA
   <213> Euphorbia heterophylla
<400> 536
   ataaatttaa aagtgtgcat agaaattatt tagactcttg agagattcta gaattagtta 60
   tcgtgtggtt tatccaacat gaacattggc ttatgattgt aatcatacat tattgtttcg 120
   ctgcatgagg agtttatgct agacaatttt ataatcttat caagtttgtt aagactcgac 180
   ctataagtct ctttcaacaa 200
<210> 537
   <211> 200
   <212> DNA
   <213> Euphorbia heterophylla
<400> 537
   tttctatgca cacttttaaa tttatttaag cttcatagtg aaatattagc ttgtgctaca 60
   ctaatttaca ttgtacgtta ttttcaatgg aatgacaact ttattttgtg cccatagtgc 120
   ataacagtaa aatttcagtt caatgatttc atatattatt ttttatatta cgaagtttta 180
   ggtttttgga ttttagatta 200
<210> 538
   <211> 200
   <212> DNA
   <213> Euphorbia heterophylla
<400> 538
   taatctaaaa tccaaaaacc taaaacttcg taatataaaa aataatatat gaaatcattg 60
   aactgaaatt ttactgttat gcactatggg cacaaaataa agttgtcatt ccattgaaaa 120
   taacgtacaa tgtaaattag tgtagcacaa gctaatattt cactatgaag cttaaataaa 180
   tttaaaagtg tgcatagaaa 200
<210> 539
   <211> 200
   <212> DNA
   <213> Euphorbia heterophylla
<400> 539
   ttttaggttt ttggatttta gattacaatc gagaccatag caagagttga aacatgcaaa   60
   tgagatttaa gcttaaaaaa atgttataaa attattaaca tttagcactt atagtagcta  120
   caaaaatttg aaggattgaa tatttctaaa tattacaaag tataaactta aatatcaaca  180
   aatatgattc atggagtttg                                              200
<210> 540
   <211> 200
   <212> DNA
   <213> Euphorbia heterophylla
<400> 540
   caaactccat gaatcatatt tgttgatatt taagtttata ctttgtaata tttagaaata   60
   ttcaatcctt caaatttttg tagctactat aagtgctaaa tgttaataat tttataacat  120
   ttttttaagc ttaaatctca tttgcatgtt tcaactcttg ctatggtctc gattgtaatc  180
   taaaatccaa aaacctaaaa                                              200
<210> 541
   <211> 200
   <212> DNA
   <213> Euphorbia heterophylla
<400> 541
   caacaaatat gattcatgga gtttgagtta agtataattt aacaatatat caatgatcga   60
   gttttttgca aaaatcataa atattagctc aatttgaaga acttaacaaa gcactcaaaa  120
   ctttaattga aaacaaaaat ttaaggttaa gggcttatat atataaaaaa aaattcaaag  180
   tatgaaagca atttcggact                                              200
<210> 542
   <211> 200
   <212> DNA
   <213> Euphorbia heterophylla
<400> 542
   agtccgaaat tgctttcata ctttgaattt ttttttatat atataagccc ttaaccttaa   60
   atttttgttt tcaattaaag ttttgagtgc tttgttaagt tcttcaaatt gagctaatat  120
   ttatgatttt tgcaaaaaac tcgatcattg atatattgtt aaattatact taactcaaac  180
   tccatgaatc atatttgttg                                              200
<210> 543
   <211> 200
   <212> DNA
   <213> Euphorbia heterophylla
<400> 543
   caaagtatga aagcaatttc ggacttcaat ttttataatt tttgattcat aacgaactat    60
   taaattattg ataaatatac ttctgcatag agtccaaaaa attataaggc cggcctaata   120
   aagcctatga tagaatccta tagaaacatt gttgacccaa gcacaggccg agtgaactcg   180
   tggctgagtt tctatgctaa                                               200
<210> 544
   <211> 200
   <212> DNA
   <213> Euphorbia heterophylla
<400> 544
   ttagcataga aactcagcca cgagttcact cggcctgtgc ttgggtcaac aatgtttcta    60
   taggattcta tcataggctt tattaggccg gccttataat tttttggact ctatgcagaa   120
   gtatatttat caataattta atagttcgtt atgaatcaaa aattataaaa attgaagtcc   180
   gaaattgctt tcatactttg                                               200
<210> 545
   <211> 200
   <212> DNA
   <213> Euphorbia heterophylla
<400> 545
   ttgtttctgc cgaaattcga gccggtagat gaggcgtcgt cgtttccgtt ggattacggg    60
   attcgacggc tagatcacgc ggttggaaat gtaccggatc ttgctccggc ggtttcgtat   120
   gtgaagaagt tcaccggatt ccacgagttc gctgagttca cggcggagga tgtagggacg   180
   agtgagagcg gattgaactc                                               200
<210> 546
   <211> 200
   <212> DNA
   <213> Euphorbia heterophylla
<400> 546
   gagttcaatc cgctctcact cgtccctaca tcctccgccg tgaactcagc gaactcgtgg 60
   aatccggtga acttcttcac atacgaaacc gccggagcaa gatccggtac atttccaacc 120
   gcgtgatcta gccgtcgaat cccgtaatcc aacggaaacg acgacgcctc atctaccggc 180
   tcgaatttcg gcagaaacaa 200
<210> 547
   <211> 200
   <212> DNA
   <213> Euphorbia heterophylla
<400> 547
   ggacgagtga gagcggattg aactcggtgg tgttggctaa caacgaagaa acggtactgc 60
   tgccgatgaa tgaaccggtg tttgggacga agaggaaaag tcagatacag acgtatttgg 120
   agcacaatga aggagctgga gtacagcatt tggcgcttgt gagtgctgat attttcaata 180
   ctttgagaga gatgaggagg 200
<210> 548
   <211> 200
   <212> DNA
   <213> Euphorbia heterophylla
<400> 548
   cctcctcatc tctctcaaag tattgaaaat atcagcactc acaagcgcca aatgctgtac 60
   tccagctcct tcattgtgct ccaaatacgt ctgtatctga cttttcctct tcgtcccaaa 120
   caccggttca ttcatcggca gcagtaccgt ttcttcgttg ttagccaaca ccaccgagtt 180
   caatccgctc tcactcgtcc 200
<210> 549
   <211> 200
   <212> DNA
   <213> Kochia scoparia
<400> 549
   accaccgcct caaccgagtt caagctggtg ggttactcca acttcgtccg agttaacccc 60
   atgtccgacc tcttctccgt caaaaaattc caccacatcg agttctggtg cggcgacgcg 120
   accaacgtca gccgtcgctt ctcatggggt ctaggcatgc cggcggtcgc taaatccgac 180
   ctctccaccg gaaactccgt 200
<210> 550
   <211> 200
   <212> DNA
   <213> Kochia scoparia
<400> 550
   acggagtttc cggtggagag gtcggattta gcgaccgccg gcatgcctag accccatgag   60
   aagcgacggc tgacgttggt cgcgtcgccg caccagaact cgatgtggtg gaattttttg  120
   acggagaaga ggtcggacat ggggttaact cggacgaagt tggagtaacc caccagcttg  180
   aactcggttg aggcggtggt                                               200
<210> 551
   <211> 200
   <212> DNA
   <213> Kochia scoparia
<400> 551
   ccgacctctc caccggaaac tccgtacacg cttcgtacct ccttcgctca ggcgacctct   60
   ccttcctctt cacctccccg tactcccctt ctctctcctc cccctcttcc gctgcaatac  120
   ccacgtttga tttctctctc ttctcctctt ttatcacctc ccacggcatc ggggttcgcg  180
   ccgttgcact cgaggtcgac                                               200
<210> 552
   <211> 200
   <212> DNA
   <213> Kochia scoparia
<400> 552
   gtcgacctcg agtgcaacgg cgcgaacccc gatgccgtgg gaggtgataa aagaggagaa   60
   gagagagaaa tcaaacgtgg gtattgcagc ggaagagggg gaggagagag aaggggagta  120
   cggggaggtg aagaggaagg agaggtcgcc tgagcgaagg aggtacgaag cgtgtacgga  180
   gtttccggtg gagaggtcgg                                               200
<210> 553
   <211> 200
   <212> DNA
   <213> Kochia scoparia
<400> 553
   tcgcgccgtt gcactcgagg tcgacgatgc cgaggttgct ttcaatacga gcgtctccca   60
   cggcgcaatc cctggttctc ccccgattaa gctcggaaac ggcgtcgttt tgtccgaggt  120
   cagcctctac ggcgacgtcg ttcttcgcta cgtgagctac ggaggtgaga cagagaaagc  180
   ggatacgaat tcgaattcat                                               200
<210> 554
   <211> 200
   <212> DNA
   <213> Kochia scoparia
<400> 554
   atgaattcga attcgtatcc gctttctctg tctcacctcc gtagctcacg tagcgaagaa    60
   cgacgtcgcc gtagaggctg acctcggaca aaacgacgcc gtttccgagc ttaatcgggg   120
   gagaaccagg gattgcgccg tgggagacgc tcgtattgaa agcaacctcg gcatcgtcga   180
   cctcgagtgc aacggcgcga                                               200
<210> 555
   <211> 200
   <212> DNA
   <213> Kochia scoparia
<400> 555
   aaagcggata cgaattcgaa ttcatggttt cttcctgggt tcgaggaaat gccggaggaa    60
   tcgtcgtttc gagggctcga tttcgggctc cggagactag accatgcggt ggggaatgtc   120
   ccggagctag ggaaagcaat cgagtatgtg aagaagttca ctgggtttca cgaatttgct   180
   gagtttacag ctgacgatgt                                               200
<210> 556
   <211> 200
   <212> DNA
   <213> Kochia scoparia
<400> 556
   acatcgtcag ctgtaaactc agcaaattcg tgaaacccag tgaacttctt cacatactcg    60
   attgctttcc ctagctccgg gacattcccc accgcatggt ctagtctccg gagcccgaaa   120
   tcgagccctc gaaacgacga ttcctccggc atttcctcga acccaggaag aaaccatgaa   180
   ttcgaattcg tatccgcttt                                               200
<210> 557
   <211> 200
   <212> DNA
   <213> Kochia scoparia
<400> 557
   ttgctgagtt tacagctgac gatgttggga cgagtgaaag tgggctgaat tcggctgtgc    60
   tggcgaacaa ctcggagacg gtgttgattc cgatgaacga gccggtttac gggacgaaga   120
   ggaagagtca aattcagaca tacttggagc acaatgaagg agctggggtt cagcatttgg   180
   cattgatgag tgaggatata                                               200
<210> 558
   <211> 200
   <212> DNA
   <213> Kochia scoparia
<400> 558
   tatatcctca ctcatcaatg ccaaatgctg aaccccagct ccttcattgt gctccaagta    60
   tgtctgaatt tgactcttcc tcttcgtccc gtaaaccggc tcgttcatcg gaatcaacac   120
   cgtctccgag ttgttcgcca gcacagccga attcagccca ctttcactcg tcccaacatc   180
   gtcagctgta aactcagcaa                                                200
<210> 559
   <211> 200
   <212> DNA
   <213> Kochia scoparia
<400> 559
   tttggcattg atgagtgagg atatattctg gactcttagg gagatgagga agcggagcgg    60
   gctcggtggg ttcgagttta tgccagcacc gccgcctacg tattatcgga acctgaggaa   120
   tcgtgtcggg gatgtgttga gtgaagaaca gatgaaggag tgtgaagagt tggggatatt   180
   ggttgataag gatgatcagg                                                200
<210> 560
   <211> 200
   <212> DNA
   <213> Kochia scoparia
<400> 560
   cctgatcatc cttatcaacc aatatcccca actcttcaca ctccttcatc tgttcttcac    60
   tcaacacatc cccgacacga ttcctcaggt tccgataata cgtaggcggc ggtgctggca   120
   taaactcgaa cccaccgagc ccgctccgct tcctcatctc cctaagagtc cagaatatat   180
   cctcactcat caatgccaaa                                                200
<210> 561
   <211> 200
   <212> DNA
   <213> Kochia scoparia
<400> 561
   atattggttg ataaggatga tcagggcact ctgctccaga tttttactaa gcatattggt    60
   gacccaacta tgttcattga gattatccaa agaattggct gcatgatgaa agatgaagaa   120
   ggcaaggtgt accaaaaggg aggctgtgga ggatttggga agggaaactt ttcagagctt   180
   ttcaaatcta tcgaagagta                                                200
<210> 562
   <211> 200
   <212> DNA
   <213> Kochia scoparia
<400> 562
   tactcttcga tagatttgaa aagctctgaa aagtttccct tcccaaatcc tccacagcct   60
   cccttttggt acaccttgcc ttcttcatct ttcatcatgc agccaattct ttggataatc  120
   tcaatgaaca tagttgggtc accaatatgc ttagtaaaaa tctggagcag agtgccctga  180
   tcatccttat caaccaatat                                               200
<210> 563
   <211> 200
   <212> DNA
   <213> Lolium multiflorum
<400> 563
   accgatcgct tccacgtgat ggatttccac cacgtcgagt tttggtgcgc cgatgccgcc   60
   tcggccgccg cacggttctc cttcgggctc ggcgtgccac tcgccgcgca gtccgacctc  120
   tccacgggga acactgcgca cgcctcacgc ctactacgcg cacgctcggg ctctctctcg  180
   ttcctcttca ccgcgccgta                                               200
<210> 564
   <211> 200
   <212> DNA
   <213> Lolium multiflorum
<400> 564
   tacggcgcgg tgaagaggaa cgagagagag cccgagcgtg cgcgtagtag gcgtgaggcg   60
   tgcgcagtgt tccccgtgga gaggtcggac tgcgcggcga gtggcacgcc gagcccgaag  120
   gagaaccgtg cggcggccga ggcggcatcg gcgcaccaaa actcgacgtg gtggaaatcc  180
   atcacgtgga agcgatcggt                                               200
<210> 565
   <211> 200
   <212> DNA
   <213> Lolium multiflorum
<400> 565
   tctcgttcct cttcaccgcg ccgtacgcgc cgcacgtcgc cgactcggcg accaccgcgt 60
   ccctgccctc cttctcggcg gacgccgcgc ggcgcttcac gggcacccac ggcggcctgg 120
   ccgtgcgtgc cgtggccgtc cgcgtcgctg acgcggccga ggccttcgtc gcgagcgtgg 180
   acgccggagc gcggccagcc 200
<210> 566
   <211> 200
   <212> DNA
   <213> Lolium multiflorum
<400> 566
   ggctggccgc gctccggcgt ccacgctcgc gacgaaggcc tcggccgcgt cagcgacgcg 60
   gacggccacg gcacgcacgg ccaggccgcc gtgggtgccc gtgaagcgcc gcgcggcgtc 120
   cgccgagaag gagggcaggg acgcggtggt cgccgagtcg gcgacgtgcg gcgcgtacgg 180
   cgcggtgaag aggaacgaga 200
<210> 567
   <211> 200
   <212> DNA
   <213> Lolium multiflorum
<400> 567
   cgtggacgcc ggagcgcggc cagcctgcgc cccgactgat ctcggccacg ggtttggctt 60
   cgcggaggtg gagctagccg gggacagcgt tctccgcttc gtgagctacc cggacggcac 120
   cgacgtgtcc ttcctgccgg ggttccagga cgtggcgagc gccggcgggg cgccggactt 180
   cgggctcacc cggtttgacc 200
<210> 568
   <211> 200
   <212> DNA
   <213> Lolium multiflorum
<400> 568
   ggtcaaaccg ggtgagcccg aagtccggcg ccccgccggc gctcgccacg tcctggaacc 60
   ccggcaggaa ggacacgtcg gtgccgtccg ggtagctcac gaagcggaga acgctgtccc 120
   cggctagctc cacctccgcg aagccaaacc cgtggccgag atcagtcggg gcgcaggctg 180
   gccgcgctcc ggcgtccacg 200
<210> 569
   <211> 200
   <212> DNA
   <213> Lolium multiflorum
<400> 569
   gacttcgggc tcacccggtt tgaccacgtc gacgttaaca tcccggagct ggcacccgtc 60
   gccgccaatg ttgccggctt caccgggttc cacaaattct gggagttcac cgcggacgac 120
   gtgtgcccgg aagagagcgg ggtgaacggc gtggtgatcg ccaacaactc agagaacgtg 180
   ctgctcagta tcttggagcc 200
<210> 570
   <211> 200
   <212> DNA
   <213> Lolium multiflorum
<400> 570
   ggctccaaga tactgagcag cacgttctct gagttgttgg cgatcaccac gccgttcacc 60
   ccgctctctt ccgggcacac gtcgtccgcg gtgaactccc agaatttgtg gaacccggtg 120
   aagccggcaa cattggcggc gacgggtgcc agctccggga tgttaacgtc gacgtggtca 180
   aaccgggtga gcccgaagtc 200
<210> 571
   <211> 200
   <212> DNA
   <213> Lolium multiflorum
<400> 571
   acgtgctgct cagtatcttg gagccggtgt tcggcaccaa gctgcggagc catgtcgaga 60
   cgttcctgga ccaccacggt ggcccgggca tacagcacct ggcaatgacc agccacgaca 120
   tccttggcgc gctcaggaaa atccgagctc ggtcctccat gggcgggttt gagctcctgc 180
   cgccgccgcc ggccagctac 200
<210> 572
   <211> 200
   <212> DNA
   <213> Lolium multiflorum
<400> 572
   gtagctggcc ggcggcggcg gcaggagctc aaacccgccc atggaggacc gagctcggat 60
   tttcctgagc gcgccaagga tgtcgtggct ggtcattgcc aggtgctgta tgcccgggcc 120
   accgtggtgg tccaggaacg tctcgacatg gctccgcagc ttggtgccga acaccggctc 180
   caagatactg agcagcacgt 200
<210> 573
   <211> 200
   <212> DNA
   <213> Lolium multiflorum
<400> 573
   ctcggccacg ggtttggctt cgcggaggtg gagctagccg gggacagcgt tctccgcttc 60
   gtgagctacc cggacggcac cgacgtgtcc ttcctgccgg ggttccagga cgtggcgagc 120
   gccggcgggg cgccggactt cgggctcacc cggtttgacc acgtcgacgt taacatcccg 180
   gagctggcac ccgtcgccgc 200
<210> 574
   <211> 200
   <212> DNA
   <213> Lolium multiflorum
<400> 574
   gcggcgacgg gtgccagctc cgggatgtta acgtcgacgt ggtcaaaccg ggtgagcccg 60
   aagtccggcg ccccgccggc gctcgccacg tcctggaacc ccggcaggaa ggacacgtcg 120
   gtgccgtccg ggtagctcac gaagcggaga acgctgtccc cggctagctc cacctccgcg 180
   aagccaaacc cgtggccgag 200
<210> 575
   <211> 200
   <212> DNA
   <213> Lolium multiflorum
<400> 575
   tcccggagct ggcacccgtc gccgccaatg ttgccggctt caccgggttc cacaaattct 60
   gggagttcac cgcggacgac gtgtgcccgg aagagagcgg ggtgaacggc gtggtgatcg 120
   ccaacaactc agagaacgtg ctgctcagta tcttggagcc ggtgttcggc accaagctgc 180
   ggagccatgt cgagacgttc 200
<210> 576
   <211> 200
   <212> DNA
   <213> Lolium multiflorum
<400> 576
   gaacgtctcg acatggctcc gcagcttggt gccgaacacc ggctccaaga tactgagcag 60
   cacgttctct gagttgttgg cgatcaccac gccgttcacc ccgctctctt ccgggcacac 120
   gtcgtccgcg gtgaactccc agaatttgtg gaacccggtg aagccggcaa cattggcggc 180
   gacgggtgcc agctccggga 200
<210> 577
   <211> 200
   <212> DNA
   <213> Lolium multiflorum
<400> 577
   gctgcggagc catgtcgaga cgttcctgga ccaccacggt ggcccgggca tacagcacct   60
   ggcaatgacc agccacgaca tccttggcgc gctcaggaaa atccgagctc ggtcctccat  120
   gggcgggttt gagctcctgc cgccgccgcc ggccagctac tatgacggtg taaggcagcg  180
   cgccggggac gtgctgtcgg                                              200
<210> 578
   <211> 200
   <212> DNA
   <213> Lolium multiflorum
<400> 578
   ccgacagcac gtccccggcg cgctgcctta caccgtcata gtagctggcc ggcggcggcg   60
   gcaggagctc aaacccgccc atggaggacc gagctcggat tttcctgagc gcgccaagga  120
   tgtcgtggct ggtcattgcc aggtgctgta tgcccgggcc accgtggtgg tccaggaacg  180
   tctcgacatg gctccgcagc                                              200
<210> 579
   <211> 200
   <212> DNA
   <213> Lolium multiflorum
<400> 579
   cagcgcgccg gggacgtgct gtcggaagaa cagatcaagg agtgccaaga gctgggcgtg   60
   cgggtggaca gagggtatga ggacggagtt gtgctccaag tcttcaccaa accggcggga  120
   gacccaacct tactgttaga gtttatccaa agaatcgggt gcatggtcaa ggacgagaac  180
   cagcaggaat accagagagg                                              200
<210> 580
   <211> 200
   <212> DNA
   <213> Lolium multiflorum
<400> 580
   cctctctggt attcctgctg gttctcgtcc ttgaccatgc acccgattct ttggataaac   60
   tctaacagta aggttgggtc tcccgccggt ttggtgaaga cttggagcac aactccgtcc  120
   tcataccctc tgtccacccg cacgcccagc tcttggcact ccttgatctg ttcttccgac  180
   agcacgtccc cggcgcgctg                                              200
<210> 581
   <211> 200
   <212> DNA
   <213> Xanthium strumarium
<400> 581
   agaaataaaa gacacatatg ttcgtgaggt tatgacacct ttggttgatg ttgtagccag 60
   tgattctagt gcaacactaa tcgatttcca caccttgtgg gtaagtcatc aatattcaag 120
   ggtacctgtt tttgaacaac gtgttgataa tatagtcggt attgcatatg caatggatct 180
   attagactat gttcaaaagg 200
<210> 582
   <211> 200
   <212> DNA
   <213> Xanthium strumarium
<400> 582
   ccttttgaac atagtctaat agatccattg catatgcaat accgactata ttatcaacac 60
   gttgttcaaa aacaggtacc cttgaatatt gatgacttac ccacaaggtg tggaaatcga 120
   ttagtgttgc actagaatca ctggctacaa catcaaccaa aggtgtcata acctcacgaa 180
   catatgtgtc ttttatttct 200
<210> 583
   <211> 200
   <212> DNA
   <213> Xanthium strumarium
<400> 583
   gatctattag actatgttca aaagggagac cttttagaaa gtaccacggt gggggatatg 60
   gctcataaac ctgcttattt tgtgcctgat tcaatgtcgg tatggaatct tcttagagaa 120
   tttcgcatta ggaaggtaca catggctgtt gtcctgaacg aatacggtgg gacagttgga 180
   attgtaacac tagaagacgt 200
<210> 584
   <211> 200
   <212> DNA
   <213> Xanthium strumarium
<400> 584
   acgtcttcta gtgttacaat tccaactgtc ccaccgtatt cgttcaggac aacagccatg 60
   tgtaccttcc taatgcgaaa ttctctaaga agattccata ccgacattga atcaggcaca 120
   aaataagcag gtttatgagc catatccccc accgtggtac tttctaaaag gtctcccttt 180
   tgaacatagt ctaatagatc 200
<210> 585
   <211> 200
   <212> DNA
   <213> Xanthium strumarium
<400> 585
   ttggaattgt aacactagaa gacgtggttg aagaaattgt tggtgaaatt tcttcaaccc 60
   acgcctctta tctccttaaa tccggccaac ttaacttcct cttcaccgcc ccttattccc 120
   cttccatctc atccaccaca accaccgcct ccatcccttc cttttctcac tccacctgtc 180
   gccacttcac ctcctcccac 200
<210> 586
   <211> 200
   <212> DNA
   <213> Xanthium strumarium
<400> 586
   gtgggaggag gtgaagtggc gacaggtgga gtgagaaaag gaagggatgg aggcggtggt 60
   tgtggtggat gagatggaag gggaataagg ggcggtgaag aggaagttaa gttggccgga 120
   tttaaggaga taagaggcgt gggttgaaga aatttcacca acaatttctt caaccacgtc 180
   ttctagtgtt acaattccaa 200
<210> 587
   <211> 200
   <212> DNA
   <213> Xanthium strumarium
<400> 587
   ctgtcgccac ttcacctcct cccacggcct cgctgttcgt gccatcgccg ttgaagtcca 60
   tgacgccgaa ctcgctttct ccgttagcgt cgctcacggc gctaaagcct ccggatacca 120
   taatccagct cttcaaacga cgacgtttct ccacagcttc aaacccaggc aaaacatatt 180
   gttattatta ttattgtttg 200
<210> 588
   <211> 200
   <212> DNA
   <213> Xanthium strumarium
<400> 588
   caaacaataa taataataac aatatgtttt gcctgggttt gaagctgtgg agaaacgtcg 60
   tcgtttgaag agctggatta tggtatccgg aggctttagc gccgtgagcg acgctaacgg 120
   agaaagcgag ttcggcgtca tggacttcaa cggcgatggc acgaacagcg aggccgtggg 180
   aggaggtgaa gtggcgacag 200
<210> 589
   <211> 200
   <212> DNA
   <213> Xanthium strumarium
<400> 589
   atattgttat tattattatt gtttgtaact aatataacgc aaaacgacgt cgccgtagag 60
   ttgaacttct gataagacga cgtcgttatg accgagggtg ataatccgga gggttgacca 120
   cgcagtgggg aacgtgccgg agttagcacc ggcagtggaa tatataaaat catttactgg 180
   atttcacgag tttgctgagt 200
<210> 590
   <211> 200
   <212> DNA
   <213> Xanthium strumarium
<400> 590
   actcagcaaa ctcgtgaaat ccagtaaatg attttatata ttccactgcc ggtgctaact 60
   ccggcacgtt ccccactgcg tggtcaaccc tccggattat caccctcggt cataacgacg 120
   tcgtcttatc agaagttcaa ctctacggcg acgtcgtttt gcgttatatt agttacaaac 180
   aataataata ataacaatat 200
<210> 591
   <211> 200
   <212> DNA
   <213> Xanthium strumarium
<400> 591
   actggatttc acgagtttgc tgagtttacg gcggaggatg tgggaacgag tgagagtgga 60
   ctaaactcgg tggttttggc ttgcaatagt gagatggtat tgataccgat gaatgaaccg 120
   gtttacggga cgaagaggaa gagtcagata cagacgtatt tggaacataa tgaaggggcg 180
   ggggttcagc atttggcgtt 200
<210> 592
   <211> 200
   <212> DNA
   <213> Xanthium strumarium
<400> 592
   aacgccaaat gctgaacccc cgccccttca ttatgttcca aatacgtctg tatctgactc   60
   ttcctcttcg tcccgtaaac cggttcattc atcggtatca ataccatctc actattgcaa  120
   gccaaaacca ccgagtttag tccactctca ctcgttccca catcctccgc cgtaaactca  180
   gcaaactcgt gaaatccagt                                              200
<210> 593
   <211> 200
   <212> DNA
   <213> Xanthium strumarium
<400> 593
   gggcgggggt tcagcatttg gcgttggcta gtgaggatat atttaggacg ttaagggaga   60
   tgaggaaaag gagtggggtt ggtggctttg agtttatgcc gtctccccct cctacttatt  120
   ataggaattt gaagaatagg gtgggcgatg tgttgtctga tgaacagatt aaggagtgtg  180
   aagaattggg gatattggtt                                              200
<210> 594
   <211> 200
   <212> DNA
   <213> Xanthium strumarium
<400> 594
   aaccaatatc cccaattctt cacactcctt aatctgttca tcagacaaca catcgcccac   60
   cctattcttc aaattcctat aataagtagg agggggagac ggcataaact caaagccacc  120
   aaccccactc cttttcctca tctcccttaa cgtcctaaat atatcctcac tagccaacgc  180
   caaatgctga acccccgccc                                              200
<210> 595
   <211> 200
   <212> DNA
   <213> Xanthium strumarium
<400> 595
   gtgtgaagaa ttggggatat tggttgatag agatgatcag gggactttgc ttcagatttt   60
   taccaagcct gttggtgaca ggccgacgat attcatagag ataattcaga gagtagggtg  120
   tatggtgaag gataatgaag gaaaggagca gcagaaggca gggtgtggag ggtttggcaa  180
   agggaacttc tcagagcttt                                              200
<210> 596
   <211> 200
   <212> DNA
   <213> Xanthium strumarium
<400> 596
   aaagctctga gaagttccct ttgccaaacc ctccacaccc tgccttctgc tgctcctttc 60
   cttcattatc cttcaccata caccctactc tctgaattat ctctatgaat atcgtcggcc 120
   tgtcaccaac aggcttggta aaaatctgaa gcaaagtccc ctgatcatct ctatcaacca 180
   atatccccaa ttcttcacac 200
<210> 597
   <211> 21
   <212> DNA
   <213> Unknown
<220>
   <223> Found in multiple species
<400> 597
   tccccaactc ttcacactcc t 21
<210> 598
   <211> 21
   <212> DNA
   <213> Unknown
<220>
   <223> Found in multiple species
<400> 598
   ggagtgtgaa gagttgggga t 21
<210> 599
   <211> 21
   <212> DNA
   <213> Unknown
<220>
   <223> Found in multiple species
<400> 599
   ccccaactct tcacactcct t 21
<210> 600
   <211> 21
   <212> DNA
   <213> Unknown
<220>
   <223> Found in multiple species
<400> 600
   atccccaact cttcacactc c 21
<210> 601
   <211> 21
   <212> DNA
   <213> Unknown
<220>
   <223> Found in multiple species
<400> 601
   aggagtgtga agagttgggg a 21
<210> 602
   <211> 21
   <212> DNA
   <213> Unknown
<220>
   <223> Found in multiple species
<400> 602
   aaggagtgtg aagagttggg g 21
<210> 603
   <211> 21
   <212> DNA
   <213> Unknown
<220>
   <223> Found in multiple species
<400> 603
   tgtgaagagt tggggatttt g 21
<210> 604
   <211> 21
   <212> DNA
   <213> Unknown
<220>
   <223> Found in multiple species
<400> 604
   tgggtttgag tttatgccgt c 21
<210> 605
   <211> 21
   <212> DNA
   <213> Unknown
<220>
   <223> Found in multiple species
<400> 605
   tgaaggagtg tgaagagttg g 21
<210> 606
   <211> 21
   <212> DNA
   <213> Unknown
<220>
   <223> Found in multiple species
<400> 606
   tgaagagttg gggattttgg t 21
<210> 607
   <211> 21
   <212> DNA
   <213> Unknown
<220>
   <223> Found in multiple species
<400> 607
   gtgtgaagag ttggggattt t 21
<210> 608
   <211> 21
   <212> DNA
   <213> Unknown
<220>
   <223> Found in multiple species
<400> 608
   gtgggtttga gtttatgccg t 21
<210> 609
   <211> 21
   <212> DNA
   <213> Unknown
<220>
   <223> Found in multiple species
<400> 609
   gtgaagagtt ggggattttg g 21
<210> 610
   <211> 21
   <212> DNA
   <213> Unknown
<220>
   <223> Found in multiple species
<400> 610
   ggtgggtttg agtttatgcc g 21
<210> 611
   <211> 21
   <212> DNA
   <213> Unknown
<220>
   <223> Found in multiple species
<400> 611
   gatgaaggag tgtgaagagt t 21
<210> 612
   <211> 21
   <212> DNA
   <213> Unknown
<220>
   <223> Found in multiple species
<400> 612
   gagtgtgaag agttggggat t 21
<210> 613
   <211> 21
   <212> DNA
   <213> Unknown
<220>
   <223> Found in multiple species
<400> 613
   gacggcataa actcaaaccc a 21
<210> 614
   <211> 21
   <212> DNA
   <213> Unknown
<220>
   <223> Found in multiple species
<400> 614
   gaaggagtgt gaagagttgg g 21
<210> 615
   <211> 21
   <212> DNA
   <213> Unknown
<220>
   <223> Found in multiple species
<400> 615
   ctcttcacac tccttcatct g 21
<210> 616
   <211> 21
   <212> DNA
   <213> Unknown
<220>
   <223> Found in multiple species
<400> 616
   cggcataaac tcaaacccac c 21
<210> 617
   <211> 21
   <212> DNA
   <213> Unknown
<220>
   <223> Found in multiple species
<400> 617
   cccaactctt cacactcctt c 21
<210> 618
   <211> 21
   <212> DNA
   <213> Unknown
<220>
   <223> Found in multiple species
<400> 618
   ccaactcttc acactccttc a 21
<210> 619
   <211> 21
   <212> DNA
   <213> Unknown
<220>
   <223> Found in multiple species
<400> 619
   ccaaaatccc caactcttca c 21
<210> 620
   <211> 21
   <212> DNA
   <213> Unknown
<220>
   <223> Found in multiple species
<400> 620
   cagatgaagg agtgtgaaga g 21
<210> 621
   <211> 21
   <212> DNA
   <213> Unknown
<220>
   <223> Found in multiple species
<400> 621
   caactcttca cactccttca t 21
<210> 622
   <211> 21
   <212> DNA
   <213> Unknown
<220>
   <223> Found in multiple species
<400> 622
   caaaatcccc aactcttcac a 21
<210> 623
   <211> 21
   <212> DNA
   <213> Unknown
<220>
   <223> Found in multiple species
<400> 623
   atgaaggagt gtgaagagtt g 21
<210> 624
   <211> 21
   <212> DNA
   <213> Unknown
<220>
   <223> Found in multiple species
<400> 624
   agtgtgaaga gttggggatt t 21
<210> 625
   <211> 21
   <212> DNA
   <213> Unknown
<220>
   <223> Found in multiple species
<400> 625
   agatgaagga gtgtgaagag t 21
<210> 626
   <211> 21
   <212> DNA
   <213> Unknown
<220>
   <223> Found in multiple species
<400> 626
   actcttcaca ctccttcatc t 21
<210> 627
   <211> 21
   <212> DNA
   <213> Unknown
<220>
   <223> Found in multiple species
<400> 627
   acggcataaa ctcaaaccca c 21
<210> 628
   <211> 21
   <212> DNA
   <213> Unknown
<220>
   <223> Found in multiple species
<400> 628
   accaaaatcc ccaactcttc a 21
<210> 629
   <211> 21
   <212> DNA
   <213> Unknown
<220>
   <223> Found in multiple species
<400> 629
   aatccccaac tcttcacact c 21
<210> 630
   <211> 21
   <212> DNA
   <213> Unknown
<220>
   <223> Found in multiple species
<400> 630
   aactcttcac actccttcat c 21
<210> 631
   <211> 21
   <212> DNA
   <213> Unknown
<220>
   <223> Found in multiple species
<400> 631
   aaatccccaa ctcttcacac t 21
<210> 632
   <211> 21
   <212> DNA
   <213> Unknown
<220>
   <223> Found in multiple species
<400> 632
   aaaatcccca actcttcaca c 21
<210> 633
   <211> 21
   <212> DNA
   <213> Unknown
<220>
   <223> Found in multiple species
<400> 633
   tttttgtccc atacacaggt t 21
<210> 634
   <211> 21
   <212> DNA
   <213> Unknown
<220>
   <223> Found in multiple species
<400> 634
   ttttgtccca tacacaggtt c 21
<210> 635
   <211> 21
   <212> DNA
   <213> Unknown
<220>
   <223> Found in multiple species
<400> 635
   ttttggactt taagggagat g 21
<210> 636
   <211> 21
   <212> DNA
   <213> Unknown
<220>
   <223> Found in multiple species
<400> 636
   tttgtcccat acacaggttc a 21
<210> 637
   <211> 21
   <212> DNA
   <213> Unknown
<220>
   <223> Found in multiple species
<400> 637
   tttggctttg atgagtgaag a 21
<210> 638
   <211> 21
   <212> DNA
   <213> Unknown
<220>
   <223> Found in multiple species
<400> 638
   tttggagcat aatgaaggag c 21
<210> 639
   <211> 21
   <212> DNA
   <213> Unknown
<220>
   <223> Found in multiple species
<400> 639
   tttggacttt aagggagatg a 21
<210> 640
   <211> 21
   <212> DNA
   <213> Unknown
<220>
   <223> Found in multiple species
<400> 640
   tttgctgagt ttacagctga a 21
<210> 641
   <211> 21
   <212> DNA
   <213> Unknown
<220>
   <223> Found in multiple species
<400> 641
   tttgccaata cggctgaatt c 21
<210> 642
   <211> 21
   <212> DNA
   <213> Unknown
<220>
   <223> Found in multiple species
<400> 642
   tttgagttta tgccgtcgcc g 21
<210> 643
   <211> 21
   <212> DNA
   <213> Unknown
<220>
   <223> Found in multiple species
<400> 643
   tttccaatga atgaacctgt g 21
<210> 644
   <211> 21
   <212> DNA
   <213> Unknown
<220>
   <223> Found in multiple species
<400> 644
   tttcatgagt ttgctgagtt t 21
<210> 645
   <211> 21
   <212> DNA
   <213> Unknown
<220>
   <223> Found in multiple species
<400> 645
   tttatgccgt cgccgcctcc g 21
<210> 646
   <211> 21
   <212> DNA
   <213> Unknown
<220>
   <223> Found in multiple species
<400> 646
   tttactgggt ttcatgagtt t 21
<210> 647
   <211> 21
   <212> DNA
   <213> Unknown
<220>
   <223> Found in multiple species
<400> 647
   tttaagggag atgaggaaga g 21
<210> 648
   <211> 21
   <212> DNA
   <213> Unknown
<220>
   <223> Found in multiple species
<400> 648
   ttgtttccaa tgaatgaacc t 21
<210> 649
   <211> 21
   <212> DNA
   <213> Unknown
<220>
   <223> Found in multiple species
<400> 649
   ttgtcccata cacaggttca t 21
<210> 650
   <211> 21
   <212> DNA
   <213> Unknown
<220>
   <223> Found in multiple species
<400> 650
   ttggtgggtt tgagtttatg c 21
<210> 651
   <211> 21
   <212> DNA
   <213> Unknown
<220>
   <223> Found in multiple species
<400> 651
   ttggctcctg caattgctta t 21
<210> 652
   <211> 21
   <212> DNA
   <213> Unknown
<220>
   <223> Found in multiple species
<400> 652
   ttggagcata atgaaggagc t 21
<210> 653
   <211> 21
   <212> DNA
   <213> Unknown
<220>
   <223> Found in multiple species
<400> 653
   ttggacttta agggagatga g 21
<210> 654
   <211> 21
   <212> DNA
   <213> Unknown
<220>
   <223> Found in multiple species
<400> 654
   ttggaaacaa caccatttca t 21
<210> 655
   <211> 21
   <212> DNA
   <213> Unknown
<220>
   <223> Found in multiple species
<400> 655
   ttgctgagtt tacagctgaa g 21
<210> 656
   <211> 21
   <212> DNA
   <213> Unknown
<220>
   <223> Found in multiple species
<400> 656
   ttgccaatac ggctgaattc a 21
<210> 657
   <211> 21
   <212> DNA
   <213> Unknown
<220>
   <223> Found in multiple species
<400> 657
   ttgagtttat gccgtcgccg c 21
<210> 658
   <211> 21
   <212> DNA
   <213> Unknown
<220>
   <223> Found in multiple species
<400> 658
   ttgagtgagg agcagatgaa g 21
<210> 659
   <211> 21
   <212> DNA
   <213> Unknown
<220>
   <223> Found in multiple species
<400> 659
   ttgaattcag ccgtattggc a 21
<210> 660
   <211> 21
   <212> DNA
   <213> Unknown
<220>
   <223> Found in multiple species
<400> 660
   ttctcttcct catctccctt a 21
<210> 661
   <211> 21
   <212> DNA
   <213> Unknown
<220>
   <223> Found in multiple species
<400> 661
   ttcctttttg tcccatacac a 21
<210> 662
   <211> 21
   <212> DNA
   <213> Unknown
<220>
   <223> Found in multiple species
<400> 662
   ttcctcatct cccttaaagt c 21
<210> 663
   <211> 21
   <212> DNA
   <213> Unknown
<220>
   <223> Found in multiple species
<400> 663
   ttcctcaaat tccggtaata a 21
<210> 664
   <211> 21
   <212> DNA
   <213> Unknown
<220>
   <223> Found in multiple species
<400> 664
   ttccggtaat aagtcggagg c 21
<210> 665
   <211> 21
   <212> DNA
   <213> Unknown
<220>
   <223> Found in multiple species
<400> 665
   ttccaatgaa tgaacctgtg t 21
<210> 666
   <211> 21
   <212> DNA
   <213> Unknown
<220>
   <223> Found in multiple species
<400> 666
   ttcattggaa acaacaccat t 21
<210> 667
   <211> 21
   <212> DNA
   <213> Unknown
<220>
   <223> Found in multiple species
<400> 667
   ttcattcatt ggaaacaaca c 21
<210> 668
   <211> 21
   <212> DNA
   <213> Unknown
<220>
   <223> Found in multiple species
<400> 668
   ttcattatgc tccaaataag t 21
<210> 669
   <211> 21
   <212> DNA
   <213> Unknown
<220>
   <223> Found in multiple species
<400> 669
   ttcatgagtt tgctgagttt a 21
<210> 670
   <211> 21
   <212> DNA
   <213> Unknown
<220>
   <223> Found in multiple species
<400> 670
   ttcatctgct cctcactcaa t 21
<210> 671
   <211> 21
   <212> DNA
   <213> Unknown
<220>
   <223> Found in multiple species
<400> 671
   ttcagctgta aactcagcaa a 21
<210> 672
   <211> 21
   <212> DNA
   <213> Unknown
<220>
   <223> Found in multiple species
<400> 672
   ttcactcatc aaagccaaat g 21
<210> 673
   <211> 21
   <212> DNA
   <213> Unknown
<220>
   <223> Found in multiple species
<400> 673
   ttcacactcc ttcatctgct c 21
<210> 674
   <211> 21
   <212> DNA
   <213> Unknown
<220>
   <223> Found in multiple species
<400> 674
   ttcaaataag caattgcagg a 21
<210> 675
   <211> 21
   <212> DNA
   <213> Unknown
<220>
   <223> Found in multiple species
<400> 675
   ttcaaactta tttggagcat a 21
<210> 676
   <211> 21
   <212> DNA
   <213> Unknown
<220>
   <223> Found in multiple species
<400> 676
   ttatttggag cataatgaag g 21
<210> 677
   <211> 21
   <212> DNA
   <213> Unknown
<220>
   <223> Found in multiple species
<400> 677
   ttattaccgg aatttgagga a 21
<210> 678
   <211> 21
   <212> DNA
   <213> Unknown
<220>
   <223> Found in multiple species
<400> 678
   ttatgctcca aataagtttg a 21
<210> 679
   <211> 21
   <212> DNA
   <213> Unknown
<220>
   <223> Found in multiple species
<400> 679
   ttatgccgtc gccgcctccg a 21
<210> 680
   <211> 21
   <212> DNA
   <213> Unknown
<220>
   <223> Found in multiple species
<400> 680
   ttactgggtt tcatgagttt g 21
<210> 681
   <211> 21
   <212> DNA
   <213> Unknown
<220>
   <223> Found in multiple species
<400> 681
   ttaagggaga tgaggaagag a 21
<210> 682
   <211> 21
   <212> DNA
   <213> Unknown
<220>
   <223> Found in multiple species
<400> 682
   tgtttccaat gaatgaacct g 21
<210> 683
   <211> 21
   <212> DNA
   <213> Unknown
<220>
   <223> Found in multiple species
<400> 683
   tgttgtttcc aatgaatgaa c 21
<210> 684
   <211> 21
   <212> DNA
   <213> Unknown
<220>
   <223> Found in multiple species
<400> 684
   tgtgtatggg acaaaaagga a 21
<210> 685
   <211> 21
   <212> DNA
   <213> Unknown
<220>
   <223> Found in multiple species
<400> 685
   tgtcccatac acaggttcat t 21
<210> 686
   <211> 21
   <212> DNA
   <213> Unknown
<220>
   <223> Found in multiple species
<400> 686
   tgtattgagt gaggagcaga t 21
<210> 687
   <211> 21
   <212> DNA
   <213> Unknown
<220>
   <223> Found in multiple species
<400> 687
   tgtatgggac aaaaaggaag a 21
<210> 688
   <211> 21
   <212> DNA
   <213> Unknown
<220>
   <223> Found in multiple species
<400> 688
   tgtacaccag ctccttcatt a 21
<210> 689
   <211> 21
   <212> DNA
   <213> Unknown
<220>
   <223> Found in multiple species
<400> 689
   tgtaaactca gcaaactcat g 21
<210> 690
   <211> 21
   <212> DNA
   <213> Unknown
<220>
   <223> Found in multiple species
<400> 690
   tggtgttgtt tccaatgaat g 21
<210> 691
   <211> 21
   <212> DNA
   <213> Unknown
<220>
   <223> Found in multiple species
<400> 691
   tggtgggttt gagtttatgc c 21
<210> 692
   <211> 21
   <212> DNA
   <213> Unknown
<220>
   <223> Found in multiple species
<400> 692
   tggtcttggt gggtttgagt t 21
<210> 693
   <211> 21
   <212> DNA
   <213> Unknown
<220>
   <223> Found in multiple species
<400> 693
   tgggtttcat gagtttgctg a 21
<210> 694
   <211> 21
   <212> DNA
   <213> Unknown
<220>
   <223> Found in multiple species
<400> 694
   tggctcctgc aattgcttat t 21
<210> 695
   <211> 21
   <212> DNA
   <213> Unknown
<220>
   <223> Found in multiple species
<400> 695
   tggagcataa tgaaggagct g 21
<210> 696
   <211> 21
   <212> DNA
   <213> Unknown
<220>
   <223> Found in multiple species
<400> 696
   tggactttaa gggagatgag g 21
<210> 697
   <211> 21
   <212> DNA
   <213> Unknown
<220>
   <223> Found in multiple species
<400> 697
   tggaaacaac accatttcat c 21
<210> 698
   <211> 21
   <212> DNA
   <213> Unknown
<220>
   <223> Found in multiple species
<400> 698
   tgctgatgta ttgagtgagg a 21
<210> 699
   <211> 21
   <212> DNA
   <213> Unknown
<220>
   <223> Found in multiple species
<400> 699
   tgctgagttt acagctgaag a 21
<210> 700
   <211> 21
   <212> DNA
   <213> Unknown
<220>
   <223> Found in multiple species
<400> 700
   tgctcctcac tcaatacatc a 21
<210> 701
   <211> 21
   <212> DNA
   <213> Unknown
<220>
   <223> Found in multiple species
<400> 701
   tgctccaaat aagtttgaat t 21
<210> 702
   <211> 21
   <212> DNA
   <213> Unknown
<220>
   <223> Found in multiple species
<400> 702
   tgccgtcgcc gcctccgact t 21
<210> 703
   <211> 21
   <212> DNA
   <213> Unknown
<220>
   <223> Found in multiple species
<400> 703
   tgccaatacg gctgaattca a 21
<210> 704
   <211> 21
   <212> DNA
   <213> Unknown
<220>
   <223> Found in multiple species
<400> 704
   tgatgtattg agtgaggagc a 21
<210> 705
   <211> 21
   <212> DNA
   <213> Unknown
<220>
   <223> Found in multiple species
<400> 705
   tgatgaaatg gtgttgtttc c 21
<210> 706
   <211> 21
   <212> DNA
   <213> Unknown
<220>
   <223> Found in multiple species
<400> 706
   tgagtttgct gagtttacag c 21
<210> 707
   <211> 21
   <212> DNA
   <213> Unknown
<220>
   <223> Found in multiple species
<400> 707
   tgagtttatg ccgtcgccgc c 21
<210> 708
   <211> 21
   <212> DNA
   <213> Unknown
<220>
   <223> Found in multiple species
<400> 708
   tgagtttaca gctgaagatg t 21
<210> 709
   <211> 21
   <212> DNA
   <213> Unknown
<220>
   <223> Found in multiple species
<400> 709
   tgagtgagga gcagatgaag g 21
<210> 710
   <211> 21
   <212> DNA
   <213> Unknown
<220>
   <223> Found in multiple species
<400> 710
   tgaggagcag atgaaggagt g 21
<210> 711
   <211> 21
   <212> DNA
   <213> Unknown
<220>
   <223> Found in multiple species
<400> 711
   tgaggaagag aagtggtctt g 21
<210> 712
   <211> 21
   <212> DNA
   <213> Unknown
<220>
   <223> Found in multiple species
<400> 712
   tgaattcagc cgtattggca a 21
<210> 713
   <211> 21
   <212> DNA
   <213> Unknown
<220>
   <223> Found in multiple species
<400> 713
   tgaatgaacc tgtgtatggg a 21
<210> 714
   <211> 21
   <212> DNA
   <213> Unknown
<220>
   <223> Found in multiple species
<400> 714
   tgaacctgtg tatgggacaa a 21
<210> 715
   <211> 21
   <212> DNA
   <213> Unknown
<220>
   <223> Found in multiple species
<400> 715
   tgaaatggtg ttgtttccaa t 21
<210> 716
   <211> 21
   <212> DNA
   <213> Unknown
<220>
   <223> Found in multiple species
<400> 716
   tcttggtggg tttgagttta t 21
<210> 717
   <211> 21
   <212> DNA
   <213> Unknown
<220>
   <223> Found in multiple species
<400> 717
   tcttcctttt tgtcccatac a 21
<210> 718
   <211> 21
   <212> DNA
   <213> Unknown
<220>
   <223> Found in multiple species
<400> 718
   tcttcctcat ctcccttaaa g 21
<210> 719
   <211> 21
   <212> DNA
   <213> Unknown
<220>
   <223> Found in multiple species
<400> 719
   tcttcagctg taaactcagc a 21
<210> 720
   <211> 21
   <212> DNA
   <213> Unknown
<220>
   <223> Found in multiple species
<400> 720
   tcttcactca tcaaagccaa a 21
<210> 721
   <211> 21
   <212> DNA
   <213> Unknown
<220>
   <223> Found in multiple species
<400> 721
   tcttcacact ccttcatctg c 21
<210> 722
   <211> 21
   <212> DNA
   <213> Unknown
<220>
   <223> Found in multiple species
<400> 722
   tctgctcctc actcaataca t 21
<210> 723
   <211> 21
   <212> DNA
   <213> Unknown
<220>
   <223> Found in multiple species
<400> 723
   tctcttcctc atctccctta a 21
<210> 724
   <211> 21
   <212> DNA
   <213> Unknown
<220>
   <223> Found in multiple species
<400> 724
   tctcccttaa agtccaaaat a 21
<210> 725
   <211> 21
   <212> DNA
   <213> Unknown
<220>
   <223> Found in multiple species
<400> 725
   tcggaggcgg cgacggcata a 21
<210> 726
   <211> 21
   <212> DNA
   <213> Unknown
<220>
   <223> Found in multiple species
<400> 726
   tcgccgcctc cgacttatta c 21
<210> 727
   <211> 21
   <212> DNA
   <213> Unknown
<220>
   <223> Found in multiple species
<400> 727
   tcctttttgt cccatacaca g 21
<210> 728
   <211> 21
   <212> DNA
   <213> Unknown
<220>
   <223> Found in multiple species
<400> 728
   tccttcatta tgctccaaat a 21
<210> 729
   <211> 21
   <212> DNA
   <213> Unknown
<220>
   <223> Found in multiple species
<400> 729
   tccttcatct gctcctcact c 21
<210> 730
   <211> 21
   <212> DNA
   <213> Unknown
<220>
   <223> Found in multiple species
<400> 730
   tcctgcaatt gcttatttga a 21
<210> 731
   <211> 21
   <212> DNA
   <213> Unknown
<220>
   <223> Found in multiple species
<400> 731
   tcctcatctc ccttaaagtc c 21
<210> 732
   <211> 21
   <212> DNA
   <213> Unknown
<220>
   <223> Found in multiple species
<400> 732
   tcctcactca atacatcagc a 21
<210> 733
   <211> 21
   <212> DNA
   <213> Unknown
<220>
   <223> Found in multiple species
<400> 733
   tcctcaaatt ccggtaataa g 21
<210> 734
   <211> 21
   <212> DNA
   <213> Unknown
<220>
   <223> Found in multiple species
<400> 734
   tccggtaata agtcggaggc g 21
<210> 735
   <211> 21
   <212> DNA
   <213> Unknown
<220>
   <223> Found in multiple species
<400> 735
   tccgacttat taccggaatt t 21
<210> 736
   <211> 21
   <212> DNA
   <213> Unknown
<220>
   <223> Found in multiple species
<400> 736
   tcccatacac aggttcattc a 21
<210> 737
   <211> 21
   <212> DNA
   <213> Unknown
<220>
   <223> Found in multiple species
<400> 737
   tccaatgaat gaacctgtgt a 21
<210> 738
   <211> 21
   <212> DNA
   <213> Unknown
<220>
   <223> Found in multiple species
<400> 738
   tcattggaaa caacaccatt t 21
<210> 739
   <211> 21
   <212> DNA
   <213> Unknown
<220>
   <223> Found in multiple species
<400> 739
   tcattcattg gaaacaacac c 21
<210> 740
   <211> 21
   <212> DNA
   <213> Unknown
<220>
   <223> Found in multiple species
<400> 740
   tcattatgct ccaaataagt t 21
<210> 741
   <211> 21
   <212> DNA
   <213> Unknown
<220>
   <223> Found in multiple species
<400> 741
   tcatgagttt gctgagttta c 21
<210> 742
   <211> 21
   <212> DNA
   <213> Unknown
<220>
   <223> Found in multiple species
<400> 742
   tcatctgctc ctcactcaat a 21
<210> 743
   <211> 21
   <212> DNA
   <213> Unknown
<220>
   <223> Found in multiple species
<400> 743
   tcatctccct taaagtccaa a 21
<210> 744
   <211> 21
   <212> DNA
   <213> Unknown
<220>
   <223> Found in multiple species
<400> 744
   tcagctgtaa actcagcaaa c 21
<210> 745
   <211> 21
   <212> DNA
   <213> Unknown
<220>
   <223> Found in multiple species
<400> 745
   tcagcaaact catgaaaccc a 21
<210> 746
   <211> 21
   <212> DNA
   <213> Unknown
<220>
   <223> Found in multiple species
<400> 746
   tcactcaata catcagcagc t 21
<210> 747
   <211> 21
   <212> DNA
   <213> Unknown
<220>
   <223> Found in multiple species
<400> 747
   tcacactcct tcatctgctc c 21
<210> 748
   <211> 21
   <212> DNA
   <213> Unknown
<220>
   <223> Found in multiple species
<400> 748
   tcaaattccg gtaataagtc g 21
<210> 749
   <211> 21
   <212> DNA
   <213> Unknown
<220>
   <223> Found in multiple species
<400> 749
   tcaaataagc aattgcagga g 21
<210> 750
   <211> 21
   <212> DNA
   <213> Unknown
<220>
   <223> Found in multiple species
<400> 750
   tcaaacttat ttggagcata a 21
<210> 751
   <211> 21
   <212> DNA
   <213> Unknown
<220>
   <223> Found in multiple species
<400> 751
   tcaaacccac caagaccact t 21
<210> 752
   <211> 21
   <212> DNA
   <213> Unknown
<220>
   <223> Found in multiple species
<400> 752
   tattttggac tttaagggag a 21
<210> 753
   <211> 21
   <212> DNA
   <213> Unknown
<220>
   <223> Found in multiple species
<400> 753
   tatttggagc ataatgaagg a 21
<210> 754
   <211> 21
   <212> DNA
   <213> Unknown
<220>
   <223> Found in multiple species
<400> 754
   tattgagtga ggagcagatg a 21
<210> 755
   <211> 21
   <212> DNA
   <213> Unknown
<220>
   <223> Found in multiple species
<400> 755
   tatgctccaa ataagtttga a 21
<210> 756
   <211> 21
   <212> DNA
   <213> Unknown
<220>
   <223> Found in multiple species
<400> 756
   tatgccgtcg ccgcctccga c 21
<210> 757
   <211> 21
   <212> DNA
   <213> Unknown
<220>
   <223> Found in multiple species
<400> 757
   tactgggttt catgagtttg c 21
<210> 758
   <211> 21
   <212> DNA
   <213> Unknown
<220>
   <223> Found in multiple species
<400> 758
   tacaccagct ccttcattat g 21
<210> 759
   <211> 21
   <212> DNA
   <213> Unknown
<220>
   <223> Found in multiple species
<400> 759
   tacacaggtt cattcattgg a 21
<210> 760
   <211> 21
   <212> DNA
   <213> Unknown
<220>
   <223> Found in multiple species
<400> 760
   taatgaagga gctggtgtac a 21
<210> 761
   <211> 21
   <212> DNA
   <213> Unknown
<220>
   <223> Found in multiple species
<400> 761
   taataagtcg gaggcggcga c 21
<210> 762
   <211> 21
   <212> DNA
   <213> Unknown
<220>
   <223> Found in multiple species
<400> 762
   taagtcggag gcggcgacgg c 21
<210> 763
   <211> 21
   <212> DNA
   <213> Unknown
<220>
   <223> Found in multiple species
<400> 763
   taagggagat gaggaagaga a 21
<210> 764
   <211> 21
   <212> DNA
   <213> Unknown
<220>
   <223> Found in multiple species
<400> 764
   taagcaattg caggagccaa c 21
<210> 765
   <211> 21
   <212> DNA
   <213> Unknown
<220>
   <223> Found in multiple species
<400> 765
   taaactcagc aaactcatga a 21
<210> 766
   <211> 21
   <212> DNA
   <213> Unknown
<220>
   <223> Found in multiple species
<400> 766
   taaactcaaa cccaccaaga c 21
<210> 767
   <211> 21
   <212> DNA
   <213> Unknown
<220>
   <223> Found in multiple species
<400> 767
   gtttgctgag tttacagctg a 21
<210> 768
   <211> 21
   <212> DNA
   <213> Unknown
<220>
   <223> Found in multiple species
<400> 768
   gtttgagttt atgccgtcgc c 21
<210> 769
   <211> 21
   <212> DNA
   <213> Unknown
<220>
   <223> Found in multiple species
<400> 769
   gtttccaatg aatgaacctg t 21
<210> 770
   <211> 21
   <212> DNA
   <213> Unknown
<220>
   <223> Found in multiple species
<400> 770
   gtttcatgag tttgctgagt t 21
<210> 771
   <211> 21
   <212> DNA
   <213> Unknown
<220>
   <223> Found in multiple species
<400> 771
   gtttatgccg tcgccgcctc c 21
<210> 772
   <211> 21
   <212> DNA
   <213> Unknown
<220>
   <223> Found in multiple species
<400> 772
   gtttactggg tttcatgagt t 21
<210> 773
   <211> 21
   <212> DNA
   <213> Unknown
<220>
   <223> Found in multiple species
<400> 773
   gtttacagct gaagatgttg g 21
<210> 774
   <211> 21
   <212> DNA
   <213> Unknown
<220>
   <223> Found in multiple species
<400> 774
   gttgtttcca atgaatgaac c 21
<210> 775
   <211> 21
   <212> DNA
   <213> Unknown
<220>
   <223> Found in multiple species
<400> 775
   gttggctcct gcaattgctt a 21
<210> 776
   <211> 21
   <212> DNA
   <213> Unknown
<220>
   <223> Found in multiple species
<400> 776
   gttcattcat tggaaacaac a 21
<210> 777
   <211> 21
   <212> DNA
   <213> Unknown
<220>
   <223> Found in multiple species
<400> 777
   gtgttgtttc caatgaatga a 21
<210> 778
   <211> 21
   <212> DNA
   <213> Unknown
<220>
   <223> Found in multiple species
<400> 778
   gtgtatggga caaaaaggaa g 21
<210> 779
   <211> 21
   <212> DNA
   <213> Unknown
<220>
   <223> Found in multiple species
<400> 779
   gtggtcttgg tgggtttgag t 21
<210> 780
   <211> 21
   <212> DNA
   <213> Unknown
<220>
   <223> Found in multiple species
<400> 780
   gtgaggagca gatgaaggag t 21
<210> 781
   <211> 21
   <212> DNA
   <213> Unknown
<220>
   <223> Found in multiple species
<400> 781
   gtcttggtgg gtttgagttt a 1
<210> 782
   <211> 21
   <212> DNA
   <213> Unknown
<220>
   <223> Found in multiple species
<400> 782
   gtcggaggcg gcgacggcat a 21
<210> 783
   <211> 21
   <212> DNA
   <213> Unknown
<220>
   <223> Found in multiple species
<400> 783
   gtcgccgcct ccgacttatt a 21
<210> 784
   <211> 21
   <212> DNA
   <213> Unknown
<220>
   <223> Found in multiple species
<400> 784
   gtcccataca caggttcatt c 21
<210> 785
   <211> 21
   <212> DNA
   <213> Unknown
<220>
   <223> Found in multiple species
<400> 785
   gtattgagtg aggagcagat g 21
<210> 786
   <211> 21
   <212> DNA
   <213> Unknown
<220>
   <223> Found in multiple species
<400> 786
   gtatgggaca aaaaggaaga g 21
<210> 787
   <211> 21
   <212> DNA
   <213> Unknown
<220>
   <223> Found in multiple species
<400> 787
   gtacaccagc tccttcatta t 21
<210> 788
   <211> 21
   <212> DNA
   <213> Unknown
<220>
   <223> Found in multiple species
<400> 788
   gtaataagtc ggaggcggcg a 21
<210> 789
   <211> 21
   <212> DNA
   <213> Unknown
<220>
   <223> Found in multiple species
<400> 789
   gtaaactcag caaactcatg a 21
<210> 790
   <211> 21
   <212> DNA
   <213> Unknown
<220>
   <223> Found in multiple species
<400> 790
   ggtttgagtt tatgccgtcg c 21
<210> 791
   <211> 21
   <212> DNA
   <213> Unknown
<220>
   <223> Found in multiple species
<400> 791
   ggtttcatga gtttgctgag t 21
<210> 792
   <211> 21
   <212> DNA
   <213> Unknown
<220>
   <223> Found in multiple species
<400> 792
   ggttcattca ttggaaacaa c 21
<210> 793
   <211> 21
   <212> DNA
   <213> Unknown
<220>
   <223> Found in multiple species
<400> 793
   ggtgttgttt ccaatgaatg a 21
<210> 794
   <211> 21
   <212> DNA
   <213> Unknown
<220>
   <223> Found in multiple species
<400> 794
   ggtcttggtg ggtttgagtt t 21
<210> 795
   <211> 21
   <212> DNA
   <213> Unknown
<220>
   <223> Found in multiple species
<400> 795
   ggtaataagt cggaggcggc g 21
<210> 796
   <211> 21
   <212> DNA
   <213> Unknown
<220>
   <223> Found in multiple species
<400> 796
   gggtttgagt ttatgccgtc g 21
<210> 797
   <211> 21
   <212> DNA
   <213> Unknown
<220>
   <223> Found in multiple species
<400> 797
   gggtttcatg agtttgctga g 21
<210> 798
   <211> 21
   <212> DNA
   <213> Unknown
<220>
   <223> Found in multiple species
<400> 798
   gggagatgag gaagagaagt g 21
<210> 799
   <211> 21
   <212> DNA
   <213> Unknown
<220>
   <223> Found in multiple species
<400> 799
   ggctcctgca attgcttatt t 21
<210> 800
   <211> 21
   <212> DNA
   <213> Unknown
<220>
   <223> Found in multiple species
<400> 800
   ggcggcgacg gcataaactc a 21
<210> 801
   <211> 21
   <212> DNA
   <213> Unknown
<220>
   <223> Found in multiple species
<400> 801
   ggcgacggca taaactcaaa c 21
<210> 802
   <211> 21
   <212> DNA
   <213> Unknown
<220>
   <223> Found in multiple species
<400> 802
   ggcataaact caaacccacc a 21
<210> 803
   <211> 21
   <212> DNA
   <213> Unknown
<220>
   <223> Found in multiple species
<400> 803
   ggaggcggcg acggcataaa c 21
<210> 804
   <211> 21
   <212> DNA
   <213> Unknown
<220>
   <223> Found in multiple species
<400> 804
   ggagcataat gaaggagctg g 21
<210> 805
   <211> 21
   <212> DNA
   <213> Unknown
<220>
   <223> Found in multiple species
<400> 805
   ggagcagatg aaggagtgtg a 21
<210> 806
   <211> 21
   <212> DNA
   <213> Unknown
<220>
   <223> Found in multiple species
<400> 806
   ggagatgagg aagagaagtg g 21
<210> 807
   <211> 21
   <212> DNA
   <213> Unknown
<220>
   <223> Found in multiple species
<400> 807
   ggactttaag ggagatgagg a 21
<210> 808
   <211> 21
   <212> DNA
   <213> Unknown
<220>
   <223> Found in multiple species
<400> 808
   ggaagagaag tggtcttggt g 21
<210> 809
   <211> 21
   <212> DNA
   <213> Unknown
<220>
   <223> Found in multiple species
<400> 809
   ggaaacaaca ccatttcatc a 21
<210> 810
   <211> 21
   <212> DNA
   <213> Unknown
<220>
   <223> Found in multiple species
<400> 810
   gctgtaaact cagcaaactc a 21
<210> 811
   <211> 21
   <212> DNA
   <213> Unknown
<220>
   <223> Found in multiple species
<400> 811
   gctgctgatg tattgagtga g 21
<210> 812
   <211> 21
   <212> DNA
   <213> Unknown
<220>
   <223> Found in multiple species
<400> 812
   gctgatgtat tgagtgagga g 21
<210> 813
   <211> 21
   <212> DNA
   <213> Unknown
<220>
   <223> Found in multiple species
<400> 813
   gctgagttta cagctgaaga t 21
<210> 814
   <211> 21
   <212> DNA
   <213> Unknown
<220>
   <223> Found in multiple species
<400> 814
   gctccttcat tatgctccaa a 21
<210> 815
   <211> 21
   <212> DNA
   <213> Unknown
<220>
   <223> Found in multiple species
<400> 815
   gctcctgcaa ttgcttattt g 21
<210> 816
   <211> 21
   <212> DNA
   <213> Unknown
<220>
   <223> Found in multiple species
<400> 816
   gctcctcact caatacatca g 21
<210> 817
   <211> 21
   <212> DNA
   <213> Unknown
<220>
   <223> Found in multiple species
<400> 817
   gctccaaata agtttgaatt t 21
<210> 818
   <211> 21
   <212> DNA
   <213> Unknown
<220>
   <223> Found in multiple species
<400> 818
   gcggcgacgg cataaactca a 21
<210> 819
   <211> 21
   <212> DNA
   <213> Unknown
<220>
   <223> Found in multiple species
<400> 819
   gcgacggcat aaactcaaac c 21
<210> 820
   <211> 21
   <212> DNA
   <213> Unknown
<220>
   <223> Found in multiple species
<400> 820
   gcctccgact tattaccgga a 21
<210> 821
   <211> 21
   <212> DNA
   <213> Unknown
<220>
   <223> Found in multiple species
<400> 821
   gccgtcgccg cctccgactt a 21
<210> 822
   <211> 21
   <212> DNA
   <213> Unknown
<220>
   <223> Found in multiple species
<400> 822
   gccgcctccg acttattacc g 21
<210> 823
   <211> 21
   <212> DNA
   <213> Unknown
<220>
   <223> Found in multiple species
<400> 823
   gcataatgaa ggagctggtg t 21
<210> 824
   <211> 21
   <212> DNA
   <213> Unknown
<220>
   <223> Found in multiple species
<400> 824
   gcataaactc aaacccacca a 21
<210> 825
   <211> 21
   <212> DNA
   <213> Unknown
<220>
   <223> Found in multiple species
<400> 825
   gcagatgaag gagtgtgaag a 21
<210> 826
   <211> 21
   <212> DNA
   <213> Unknown
<220>
   <223> Found in multiple species
<400> 826
   gcaaactcat gaaacccagt a 21
<210> 827
   <211> 21
   <212> DNA
   <213> Unknown
<220>
   <223> Found in multiple species
<400> 827
   gatgtattga gtgaggagca g 21
<210> 828
   <211> 21
   <212> DNA
   <213> Unknown
<220>
   <223> Found in multiple species
<400> 828
   gatgaggaag agaagtggtc t 21
<210> 829
   <211> 21
   <212> DNA
   <213> Unknown
<220>
   <223> Found in multiple species
<400> 829
   gatgaaatgg tgttgtttcc a 21
<210> 830
   <211> 21
   <212> DNA
   <213> Unknown
<220>
   <223> Found in multiple species
<400> 830
   gagtttgctg agtttacagc t 21
<210> 831
   <211> 21
   <212> DNA
   <213> Unknown
<220>
   <223> Found in multiple species
<400> 831
   gagtttatgc cgtcgccgcc t 21
<210> 832
   <211> 21
   <212> DNA
   <213> Unknown
<220>
   <223> Found in multiple species
<400> 832
   gagtttacag ctgaagatgt t 21
<210> 833
   <211> 21
   <212> DNA
   <213> Unknown
<220>
   <223> Found in multiple species
<400> 833
   gagttggctc ctgcaattgc t 21
<210> 834
   <211> 21
   <212> DNA
   <213> Unknown
<220>
   <223> Found in multiple species
<400> 834
   gagtgaggag cagatgaagg a 21
<210> 835
   <211> 21
   <212> DNA
   <213> Unknown
<220>
   <223> Found in multiple species
<400> 835
   gaggcggcga cggcataaac t 21
<210> 836
   <211> 21
   <212> DNA
   <213> Unknown
<220>
   <223> Found in multiple species
<400> 836
   gaggagcaga tgaaggagtg t 21
<210> 837
   <211> 21
   <212> DNA
   <213> Unknown
<220>
   <223> Found in multiple species
<400> 837
   gaggaagaga agtggtcttg g 21
<210> 838
   <211> 21
   <212> DNA
   <213> Unknown
<220>
   <223> Found in multiple species
<400> 838
   gagctgctga tgtattgagt g 21
<210> 839
   <211> 21
   <212> DNA
   <213> Unknown
<220>
   <223> Found in multiple species
<400> 839
   gagcataatg aaggagctgg t 21
<210> 840
   <211> 21
   <212> DNA
   <213> Unknown
<220>
   <223> Found in multiple species
<400> 840
   gagcagatga aggagtgtga a 21
<210> 841
   <211> 21
   <212> DNA
   <213> Unknown
<220>
   <223> Found in multiple species
<400> 841
   gagatgagga agagaagtgg t 21
<210> 842
   <211> 21
   <212> DNA
   <213> Unknown
<220>
   <223> Found in multiple species
<400> 842
   gagaagtggt cttggtgggt t 21
<210> 843
   <211> 21
   <212> DNA
   <213> Unknown
<220>
   <223> Found in multiple species
<400> 843
   gactttaagg gagatgagga a 21
<210> 844
   <211> 21
   <212> DNA
   <213> Unknown
<220>
   <223> Found in multiple species
<400> 844
   gacttattac cggaatttga g 21
<210> 845
   <211> 21
   <212> DNA
   <213> Unknown
<220>
   <223> Found in multiple species
<400> 845
   gaccacttct cttcctcatc t 21
<210> 846
   <211> 21
   <212> DNA
   <213> Unknown
<220>
   <223> Found in multiple species
<400> 846
   gaattcagcc gtattggcaa a 21
<210> 847
   <211> 21
   <212> DNA
   <213> Unknown
<220>
   <223> Found in multiple species
<400> 847
   gaatgaacct gtgtatggga c 1
<210> 848
   <211> 21
   <212> DNA
   <213> Unknown
<220>
   <223> Found in multiple species
<400> 848
   gaagtggtct tggtgggttt g 21
<210> 849
   <211> 21
   <212> DNA
   <213> Unknown
<220>
   <223> Found in multiple species
<400> 849
   gaagagaagt ggtcttggtg g 21
<210> 850
   <211> 21
   <212> DNA
   <213> Unknown
<220>
   <223> Found in multiple species
<400> 850
   gaacctgtgt atgggacaaa a 21
<210> 851
   <211> 21
   <212> DNA
   <213> Unknown
<220>
   <223> Found in multiple species
<400> 851
   gaaatggtgt tgtttccaat g 21
<210> 852
   <211> 21
   <212> DNA
   <213> Unknown
<220>
   <223> Found in multiple species
<400> 852
   gaaacaacac catttcatca t 21
<210> 853
   <211> 21
   <212> DNA
   <213> Unknown
<220>
   <223> Found in multiple species
<400> 853
   ctttttgtcc catacacagg t 21
<210> 854
   <211> 21
   <212> DNA
   <213> Unknown
<220>
   <223> Found in multiple species
<400> 854
   ctttaaggga gatgaggaag a 21
<210> 855
   <211> 21
   <212> DNA
   <213> Unknown
<220>
   <223> Found in multiple species
<400> 855
   cttggtgggt ttgagtttat g 21
<210> 856
   <211> 21
   <212> DNA
   <213> Unknown
<220>
   <223> Found in multiple species
<400> 856
   cttctcttcc tcatctccct t 21
<210> 857
   <211> 21
   <212> DNA
   <213> Unknown
<220>
   <223> Found in multiple species
<400> 857
   cttccttttt gtcccataca c 21
<210> 858
   <211> 21
   <212> DNA
   <213> Unknown
<220>
   <223> Found in multiple species
<400> 858
   cttcctcatc tcccttaaag t 21
<210> 859
   <211> 21
   <212> DNA
   <213> Unknown
<220>
   <223> Found in multiple species
<400> 859
   cttcattatg ctccaaataa g 21
<210> 860
   <211> 21
   <212> DNA
   <213> Unknown
<220>
   <223> Found in multiple species
<400> 860
   cttcatctgc tcctcactca a 21
<210> 861
   <211> 21
   <212> DNA
   <213> Unknown
<220>
   <223> Found in multiple species
<400> 861
   cttcagctgt aaactcagca a 21
<210> 862
   <211> 21
   <212> DNA
   <213> Unknown
<220>
   <223> Found in multiple species
<400> 862
   cttcactcat caaagccaaa t 21
<210> 863
   <211> 21
   <212> DNA
   <213> Unknown
<220>
   <223> Found in multiple species
<400> 863
   cttcacactc cttcatctgc t 21
<210> 864
   <211> 21
   <212> DNA
   <213> Unknown
<220>
   <223> Found in multiple species
<400> 864
   cttatttgga gcataatgaa g 21
<210> 865
   <211> 21
   <212> DNA
   <213> Unknown
<220>
   <223> Found in multiple species
<400> 865
   cttattaccg gaatttgagg a 21
<210> 866
   <211> 21
   <212> DNA
   <213> Unknown
<220>
   <223> Found in multiple species
<400> 866
   ctgtgtatgg gacaaaaagg a 21
<210> 867
   <211> 21
   <212> DNA
   <213> Unknown
<220>
   <223> Found in multiple species
<400> 867
   ctgtaaactc agcaaactca t 21
<210> 868
   <211> 21
   <212> DNA
   <213> Unknown
<220>
   <223> Found in multiple species
<400> 868
   ctgggtttca tgagtttgct g 21
<210> 869
   <211> 21
   <212> DNA
   <213> Unknown
<220>
   <223> Found in multiple species
<400> 869
   ctgctgatgt attgagtgag g 21
<210> 870
   <211> 21
   <212> DNA
   <213> Unknown
<220>
   <223> Found in multiple species
<400> 870
   ctgctcctca ctcaatacat c 21
<210> 871
   <211> 21
   <212> DNA
   <213> Unknown
<220>
   <223> Found in multiple species
<400> 871
   ctgatgtatt gagtgaggag c 21
<210> 872
   <211> 21
   <212> DNA
   <213> Unknown
<220>
   <223> Found in multiple species
<400> 872
   ctgagtttac agctgaagat g 21
<210> 873
   <211> 21
   <212> DNA
   <213> Unknown
<220>
   <223> Found in multiple species
<400> 873
   ctcttccttt ttgtcccata c 21
<210> 874
   <211> 21
   <212> DNA
   <213> Unknown
<220>
   <223> Found in multiple species
<400> 874
   ctcttcctca tctcccttaa a 21
<210> 875
   <211> 21
   <212> DNA
   <213> Unknown
<220>
   <223> Found in multiple species
<400> 875
   ctccttcatt atgctccaaa t 21
<210> 876
   <211> 21
   <212> DNA
   <213> Unknown
<220>
   <223> Found in multiple species
<400> 876
   ctccttcatc tgctcctcac t 21
<210> 877
   <211> 21
   <212> DNA
   <213> Unknown
<220>
   <223> Found in multiple species
<400> 877
   ctcctgcaat tgcttatttg a 21
<210> 878
   <211> 21
   <212> DNA
   <213> Unknown
<220>
   <223> Found in multiple species
<400> 878
   ctcctcactc aatacatcag c 21
<210> 879
   <211> 21
   <212> DNA
   <213> Unknown
<220>
   <223> Found in multiple species
<400> 879
   ctccgactta ttaccggaat t 21
<210> 880
   <211> 21
   <212> DNA
   <213> Unknown
<220>
   <223> Found in multiple species
<400> 880
   ctcccttaaa gtccaaaata t 21
<210> 881
   <211> 21
   <212> DNA
   <213> Unknown
<220>
   <223> Found in multiple species
<400> 881
   ctccaaataa gtttgaattt g 21
<210> 882
   <211> 21
   <212> DNA
   <213> Unknown
<220>
   <223> Found in multiple species
<400> 882
   ctcatgaaac ccagtaaact t 21
<210> 883
   <211> 21
   <212> DNA
   <213> Unknown
<220>
   <223> Found in multiple species
<400> 883
   ctcatctccc ttaaagtcca a 21
<210> 884
   <211> 21
   <212> DNA
   <213> Unknown
<220>
   <223> Found in multiple species
<400> 884
   ctcagcaaac tcatgaaacc c 21
<210> 885
   <211> 21
   <212> DNA
   <213> Unknown
<220>
   <223> Found in multiple species
<400> 885
   ctcactcaat acatcagcag c 21
<210> 886
   <211> 21
   <212> DNA
   <213> Unknown
<220>
   <223> Found in multiple species
<400> 886
   ctcaaattcc ggtaataagt c 21
<210> 887
   <211> 21
   <212> DNA
   <213> Unknown
<220>
   <223> Found in multiple species
<400> 887
   ctcaaaccca ccaagaccac t 21
<210> 888
   <211> 21
   <212> DNA
   <213> Unknown
<220>
   <223> Found in multiple species
<400> 888
   cgtcgccgcc tccgacttat t 21
<210> 889
   <211> 21
   <212> DNA
   <213> Unknown
<220>
   <223> Found in multiple species
<400> 889
   cggtaataag tcggaggcgg c 21
<210> 890
   <211> 21
   <212> DNA
   <213> Unknown
<220>
   <223> Found in multiple species
<400> 890
   cggcgacggc ataaactcaa a 21
<210> 891
   <211> 21
   <212> DNA
   <213> Unknown
<220>
   <223> Found in multiple species
<400> 891
   cggaggcggc gacggcataa a 21
<210> 892
   <211> 21
   <212> DNA
   <213> Unknown
<220>
   <223> Found in multiple species
<400> 892
   cgcctccgac ttattaccgg a 21
<210> 893
   <211> 21
   <212> DNA
   <213> Unknown
<220>
   <223> Found in multiple species
<400> 893
   cgccgcctcc gacttattac c 21
<210> 894
   <211> 21
   <212> DNA
   <213> Unknown
<220>
   <223> Found in multiple species
<400> 894
   cgacttatta ccggaatttg a 21
<210> 895
   <211> 21
   <212> DNA
   <213> Unknown
<220>
   <223> Found in multiple species
<400> 895
   cgacggcata aactcaaacc c 21
<210> 896
   <211> 21
   <212> DNA
   <213> Unknown
<220>
   <223> Found in multiple species
<400> 896
   cctttttgtc ccatacacag g 21
<210> 897
   <211> 21
   <212> DNA
   <213> Unknown
<220>
   <223> Found in multiple species
<400> 897
   ccttcattat gctccaaata a 21
<210> 898
   <211> 21
   <212> DNA
   <213> Unknown
<220>
   <223> Found in multiple species
<400> 898
   ccttcatctg ctcctcactc a 21
<210> 899
   <211> 21
   <212> DNA
   <213> Unknown
<220>
   <223> Found in multiple species
<400> 899
   cctgtgtatg ggacaaaaag g 21
<210> 900
   <211> 21
   <212> DNA
   <213> Unknown
<220>
   <223> Found in multiple species
<400> 900
   cctccgactt attaccggaa t 21
<210> 901
   <211> 21
   <212> DNA
   <213> Unknown
<220>
   <223> Found in multiple species
<400> 901
   cctcatctcc cttaaagtcc a 21
<210> 902
   <211> 21
   <212> DNA
   <213> Unknown
<220>
   <223> Found in multiple species
<400> 902
   cctcactcaa tacatcagca g 21
<210> 903
   <211> 21
   <212> DNA
   <213> Unknown
<220>
   <223> Found in multiple species
<400> 903
   cctcaaattc cggtaataag t 21
<210> 904
   <211> 21
   <212> DNA
   <213> Unknown
<220>
   <223> Found in multiple species
<400> 904
   ccgtcgccgc ctccgactta t 21
<210> 905
   <211> 21
   <212> DNA
   <213> Unknown
<220>
   <223> Found in multiple species
<400> 905
   ccggtaataa gtcggaggcg g 21
<210> 906
   <211> 21
   <212> DNA
   <213> Unknown
<220>
   <223> Found in multiple species
<400> 906
   ccgcctccga cttattaccg g 21
<210> 907
   <211> 21
   <212> DNA
   <213> Unknown
<220>
   <223> Found in multiple species
<400> 907
   ccgacttatt accggaattt g 1
<210> 908
   <211> 21
   <212> DNA
   <213> Unknown
<220>
   <223> Found in multiple species
<400> 908
   cccatacaca ggttcattca t 21
<210> 909
   <211> 21
   <212> DNA
   <213> Unknown
<220>
   <223> Found in multiple species
<400> 909
   cccaccaaga ccacttctct t 21
<210> 910
   <211> 21
   <212> DNA
   <213> Unknown
<220>
   <223> Found in multiple species
<400> 910
   ccatacacag gttcattcat t 21
<210> 911
   <211> 21
   <212> DNA
   <213> Unknown
<220>
   <223> Found in multiple species
<400> 911
   ccagctcctt cattatgctc c 21
<210> 912
   <211> 21
   <212> DNA
   <213> Unknown
<220>
   <223> Found in multiple species
<400> 912
   ccacttctct tcctcatctc c 21
<210> 913
   <211> 21
   <212> DNA
   <213> Unknown
<220>
   <223> Found in multiple species
<400> 913
   ccaccaagac cacttctctt c 21
<210> 914
   <211> 21
   <212> DNA
   <213> Unknown
<220>
   <223> Found in multiple species
<400> 914
   ccaatgaatg aacctgtgta t 21
<210> 915
   <211> 21
   <212> DNA
   <213> Unknown
<220>
   <223> Found in multiple species
<400> 915
   ccaagaccac ttctcttcct c 21
<210> 916
   <211> 21
   <212> DNA
   <213> Unknown
<220>
   <223> Found in multiple species
<400> 916
   ccaacatctt cagctgtaaa c 21
<210> 917
   <211> 21
   <212> DNA
   <213> Unknown
<220>
   <223> Found in multiple species
<400> 917
   catttggctt tgatgagtga a 21
<210> 918
   <211> 21
   <212> DNA
   <213> Unknown
<220>
   <223> Found in multiple species
<400> 918
   cattggaaac aacaccattt c 21
<210> 919
   <211> 21
   <212> DNA
   <213> Unknown
<220>
   <223> Found in multiple species
<400> 919
   cattcattgg aaacaacacc a 21
<210> 920
   <211> 21
   <212> DNA
   <213> Unknown
<220>
   <223> Found in multiple species
<400> 920
   cattatgctc caaataagtt t 21
<210> 921
   <211> 21
   <212> DNA
   <213> Unknown
<220>
   <223> Found in multiple species
<400> 921
   catgagtttg ctgagtttac a 21
<210> 922
   <211> 21
   <212> DNA
   <213> Unknown
<220>
   <223> Found in multiple species
<400> 922
   catcttcagc tgtaaactca g 21
<210> 923
   <211> 21
   <212> DNA
   <213> Unknown
<220>
   <223> Found in multiple species
<400> 923
   catctgctcc tcactcaata c 21
<210> 924
   <211> 21
   <212> DNA
   <213> Unknown
<220>
   <223> Found in multiple species
<400> 924
   catctccctt aaagtccaaa a 1
<210> 925
   <211> 21
   <212> DNA
   <213> Unknown
<220>
   <223> Found in multiple species
<400> 925
   catacacagg ttcattcatt g 21
<210> 926
   <211> 21
   <212> DNA
   <213> Unknown
<220>
   <223> Found in multiple species
<400> 926
   cataatgaag gagctggtgt a 21
<210> 927
   <211> 21
   <212> DNA
   <213> Unknown
<220>
   <223> Found in multiple species
<400> 927
   cataaactca aacccaccaa g 21
<210> 928
   <211> 21
   <212> DNA
   <213> Unknown
<220>
   <223> Found in multiple species
<400> 928
   caggttcatt cattggaaac a 21
<210> 929
   <211> 21
   <212> DNA
   <213> Unknown
<220>
   <223> Found in multiple species
<400> 929
   cagctgtaaa ctcagcaaac t 21
<210> 930
   <211> 21
   <212> DNA
   <213> Unknown
<220>
   <223> Found in multiple species
<400> 930
   cagctccttc attatgctcc a 21
<210> 931
   <211> 21
   <212> DNA
   <213> Unknown
<220>
   <223> Found in multiple species
<400> 931
   cagcaaactc atgaaaccca g 21
<210> 932
   <211> 21
   <212> DNA
   <213> Unknown
<220>
   <223> Found in multiple species
<400> 932
   cacttctctt cctcatctcc c 21
<210> 933
   <211> 21
   <212> DNA
   <213> Unknown
<220>
   <223> Found in multiple species
<400> 933
   cactccttca tctgctcctc a 21
<210> 934
   <211> 21
   <212> DNA
   <213> Unknown
<220>
   <223> Found in multiple species
<400> 934
   cactcaatac atcagcagct c 21
<210> 935
   <211> 21
   <212> DNA
   <213> Unknown
<220>
   <223> Found in multiple species
<400> 935
   caccagctcc ttcattatgc t 21
<210> 936
   <211> 21
   <212> DNA
   <213> Unknown
<220>
   <223> Found in multiple species
<400> 936
   caccaagacc acttctcttc c 21
<210> 937
   <211> 21
   <212> DNA
   <213> Unknown
<220>
   <223> Found in multiple species
<400> 937
   cacaggttca ttcattggaa a 21
<210> 938
   <211> 21
   <212> DNA
   <213> Unknown
<220>
   <223> Found in multiple species
<400> 938
   cacactcctt catctgctcc t 21
<210> 939
   <211> 21
   <212> DNA
   <213> Unknown
<220>
   <223> Found in multiple species
<400> 939
   caatgaatga acctgtgtat g 21
<210> 940
   <211> 21
   <212> DNA
   <213> Unknown
<220>
   <223> Found in multiple species
<400> 940
   caagaccact tctcttcctc a 21
<210> 941
   <211> 21
   <212> DNA
   <213> Unknown
<220>
   <223> Found in multiple species
<400> 941
   caacatcttc agctgtaaac t 21
<210> 942
   <211> 21
   <212> DNA
   <213> Unknown
<220>
   <223> Found in multiple species
<400> 942
   caaattccgg taataagtcg g 21
<210> 943
   <211> 21
   <212> DNA
   <213> Unknown
<220>
   <223> Found in multiple species
<400> 943
   caaattcaaa cttatttgga g 21
<210> 944
   <211> 21
   <212> DNA
   <213> Unknown
<220>
   <223> Found in multiple species
<400> 944
   caaataagca attgcaggag c 21
<210> 945
   <211> 21
   <212> DNA
   <213> Unknown
<220>
   <223> Found in multiple species
<400> 945
   caaacttatt tggagcataa t 21
<210> 946
   <211> 21
   <212> DNA
   <213> Unknown
<220>
   <223> Found in multiple species
<400> 946
   caaactcatg aaacccagta a 21
<210> 947
   <211> 21
   <212> DNA
   <213> Unknown
<220>
   <223> Found in multiple species
<400> 947
   caaacccacc aagaccactt c 21
<210> 948
   <211> 21
   <212> DNA
   <213> Unknown
<220>
   <223> Found in multiple species
<400> 948
   attttggact ttaagggaga t 21
<210> 949
   <211> 21
   <212> DNA
   <213> Unknown
<220>
   <223> Found in multiple species
<400> 949
   atttggcttt gatgagtgaa g 21
<210> 950
   <211> 21
   <212> DNA
   <213> Unknown
<220>
   <223> Found in multiple species
<400> 950
   atttggagca taatgaagga g 21
<210> 951
   <211> 21
   <212> DNA
   <213> Unknown
<220>
   <223> Found in multiple species
<400> 951
   attggaaaca acaccatttc a 21
<211> 21
   <212> DNA
   <213> Unknown
<220>
   <223> Found in multiple species
<400> 952
   attgagtgag gagcagatga a 21
<210> 953
   <211> 21
   <212> DNA
   <213> Unknown
<220>
   <223> Found in multiple species
<400> 953
   attccggtaa taagtcggag g 21
<210> 954
   <211> 21
   <212> DNA
   <213> Unknown
<220>
   <223> Found in multiple species
<400> 954
   attcattgga aacaacacca t 21
<210> 955
   <211> 21
   <212> DNA
   <213> Unknown
<220>
   <223> Found in multiple species
<400> 955
   attcaaactt atttggagca t 21
<210> 956
   <211> 21
   <212> DNA
   <213> Unknown
<220>
   <223> Found in multiple species
<400> 956
   attatgctcc aaataagttt g 21
<210> 957
   <211> 21
   <212> DNA
   <213> Unknown
<220>
   <223> Found in multiple species
<400> 957
   atgtattgag tgaggagcag a 21
<210> 958
   <211> 21
   <212> DNA
   <213> Unknown
<220>
   <223> Found in multiple species
<400> 958
   atggtgttgt ttccaatgaa t 21
<210> 959
   <211> 21
   <212> DNA
   <213> Unknown
<220>
   <223> Found in multiple species
<400> 959
   atgctccaaa taagtttgaa t 21
<210> 960
   <211> 21
   <212> DNA
   <213> Unknown
<220>
   <223> Found in multiple species
<400> 960
   atgccgtcgc cgcctccgac t 21
<210> 961
   <211> 21
   <212> DNA
   <213> Unknown
<220>
   <223> Found in multiple species
<400> 961
   atgatgaaat ggtgttgttt c 21
<211> 21
   <212> DNA
   <213> Unknown
<220>
   <223> Found in multiple species
<400> 962
   atgagtttgc tgagtttaca g 21
<210> 963
   <211> 21
   <212> DNA
   <213> Unknown
<220>
   <223> Found in multiple species
<400> 963
   atgaggaaga gaagtggtct t 21
<210> 964
   <211> 21
   <212> DNA
   <213> Unknown
<220>
   <223> Found in multiple species
<400> 964
   atgaatgaac ctgtgtatgg g 21
<210> 965
   <211> 21
   <212> DNA
   <213> Unknown
<220>
   <223> Found in multiple species
<400> 965
   atgaacctgt gtatgggaca a 21
<210> 966
   <211> 21
   <212> DNA
   <213> Unknown
<220>
   <223> Found in multiple species
<400> 966
   atgaaatggt gttgtttcca a 21
<210> 967
   <211> 21
   <212> DNA
   <213> Unknown
<220>
   <223> Found in multiple species
<400> 967
   atcttcagct gtaaactcag c 21
<210> 968
   <211> 21
   <212> DNA
   <213> Unknown
<220>
   <223> Found in multiple species
<400> 968
   atctgctcct cactcaatac a 21
<210> 969
   <211> 21
   <212> DNA
   <213> Unknown
<220>
   <223> Found in multiple species
<400> 969
   atctccctta aagtccaaaa t 21
<210> 970
   <211> 21
   <212> DNA
   <213> Unknown
<220>
   <223> Found in multiple species
<400> 970
   atattttgga ctttaaggga g 21
<210> 971
   <211> 21
   <212> DNA
   <213> Unknown
<220>
   <223> Found in multiple species
<400> 971
   atacacaggt tcattcattg g 21
<210> 972
   <211> 21
   <212> DNA
   <213> Unknown
<220>
   <223> Found in multiple species
<400> 972
   ataatgaagg agctggtgta c 21
<210> 973
   <211> 21
   <212> DNA
   <213> Unknown
<220>
   <223> Found in multiple species
<400> 973
   ataagtcgga ggcggcgacg g 21
<210> 974
   <211> 21
   <212> DNA
   <213> Unknown
<220>
   <223> Found in multiple species
<400> 974
   ataagcaatt gcaggagcca a 21
<210> 975
   <211> 21
   <212> DNA
   <213> Unknown
<220>
   <223> Found in multiple species
<400> 975
   ataaactcaa acccaccaag a 21
<210> 976
   <211> 21
   <212> DNA
   <213> Unknown
<220>
   <223> Found in multiple species
<400> 976
   agtttgctga gtttacagct g 21
<210> 977
   <211> 21
   <212> DNA
   <213> Unknown
<220>
   <223> Found in multiple species
<400> 977
   agtttatgcc gtcgccgcct c 21
<210> 978
   <211> 21
   <212> DNA
   <213> Unknown
<220>
   <223> Found in multiple species
<400> 978
   agtttactgg gtttcatgag t 21
<210> 979
   <211> 21
   <212> DNA
   <213> Unknown
<220>
   <223> Found in multiple species
<400> 979
   agtttacagc tgaagatgtt g 21
<210> 980
   <211> 21
   <212> DNA
   <213> Unknown
<220>
   <223> Found in multiple species
<400> 980
   agttggctcc tgcaattgct t 21
<210> 981
   <211> 21
   <212> DNA
   <213> Unknown
<220>
   <223> Found in multiple species
<400> 981
   agtggtcttg gtgggtttga g 21
<210> 982
   <211> 21
   <212> DNA
   <213> Unknown
<220>
   <223> Found in multiple species
<400> 982
   agtgaggagc agatgaagga g 21
<210> 983
   <211> 21
   <212> DNA
   <213> Unknown
<220>
   <223> Found in multiple species
<400> 983
   agtcggaggc ggcgacggca t 1
<210> 984
   <211> 21
   <212> DNA
   <213> Unknown
<220>
   <223> Found in multiple species
<400> 984
   aggttcattc attggaaaca a 21
<210> 985
   <211> 21
   <212> DNA
   <213> Unknown
<220>
   <223> Found in multiple species
<400> 985
   agggagatga ggaagagaag t 21
<210> 986
   <211> 21
   <212> DNA
   <213> Unknown
<220>
   <223> Found in multiple species
<400> 986
   aggcggcgac ggcataaact c 21
<210> 987
   <211> 21
   <212> DNA
   <213> Unknown
<220>
   <223> Found in multiple species
<400> 987
   aggagcagat gaaggagtgt g 21
<210> 988
   <211> 21
   <212> DNA
   <213> Unknown
<220>
   <223> Found in multiple species
<400> 988
   aggaagagaa gtggtcttgg t 21
<210> 989
   <211> 21
   <212> DNA
   <213> Unknown
<220>
   <223> Found in multiple species
<400> 989
   agctgtaaac tcagcaaact c 21
<210> 990
   <211> 21
   <212> DNA
   <213> Unknown
<220>
   <223> Found in multiple species
<400> 990
   agctgctgat gtattgagtg a 21
<210> 991
   <211> 21
   <212> DNA
   <213> Unknown
<220>
   <223> Found in multiple species
<400> 991
   agctccttca ttatgctcca a 21
<210> 992
   <211> 21
   <212> DNA
   <213> Unknown
<220>
   <223> Found in multiple species
<400> 992
   agcataatga aggagctggt g 21
<210> 993
   <211> 21
   <212> DNA
   <213> Unknown
<220>
   <223> Found in multiple species
<400> 993
   agcagatgaa ggagtgtgaa g 21
<210> 994
   <211> 21
   <212> DNA
   <213> Unknown
<220>
   <223> Found in multiple species
<400> 994
   agcaattgca ggagccaact c 21
<210> 995
   <211> 21
   <212> DNA
   <213> Unknown
<220>
   <223> Found in multiple species
<400> 995
   agcaaactca tgaaacccag t 21
<210> 996
   <211> 21
   <212> DNA
   <213> Unknown
<220>
   <223> Found in multiple species
<400> 996
   agatgaggaa gagaagtggt c 21
<210> 997
   <211> 21
   <212> DNA
   <213> Unknown
<220>
   <223> Found in multiple species
<400> 997
   agagctgctg atgtattgag t 21
<210> 998
   <211> 21
   <212> DNA
   <213> Unknown
<220>
   <223> Found in multiple species
<400> 998
   agagaagtgg tcttggtggg t 21
<210> 999
   <211> 21
   <212> DNA
   <213> Unknown
<220>
   <223> Found in multiple species
<400> 999
   agaccacttc tcttcctcat c 21
<210> 1000
   <211> 21
   <212> DNA
   <213> Unknown
<220>
   <223> Found in multiple species
<400> 1000
   agaagtggtc ttggtgggtt t 21
<210> 1001
   <211> 21
   <212> DNA
   <213> Unknown
<220>
   <223> Found in multiple species
<400> 1001
   actttaaggg agatgaggaa g 21
<210> 1002
   <211> 21
   <212> DNA
   <213> Unknown
<220>
   <223> Found in multiple species
<400> 1002
   acttctcttc ctcatctccc t 21
<210> 1003
   <211> 21
   <212> DNA
   <213> Unknown
<220>
   <223> Found in multiple species
<400> 1003
   acttatttgg agcataatga a 21
<210> 1004
   <211> 21
   <212> DNA
   <213> Unknown
<220>
   <223> Found in multiple species
<400> 1004
   acttattacc ggaatttgag g 21
<210> 1005
   <211> 21
   <212> DNA
   <213> Unknown
<220>
   <223> Found in multiple species
<400> 1005
   actgggtttc atgagtttgc t 21
<210> 1006
   <211> 21
   <212> DNA
   <213> Unknown
<220>
   <223> Found in multiple species
<400> 1006
   actccttcat ctgctcctca c 21
<210> 1007
   <211> 21
   <212> DNA
   <213> Unknown
<220>
   <223> Found in multiple species
<400> 1007
   actcatgaaa cccagtaaac t 21
<210> 1008
   <211> 21
   <212> DNA
   <213> Unknown
<220>
   <223> Found in multiple species
<400> 1008
   actcagcaaa ctcatgaaac c 21
<210> 1009
   <211> 21
   <212> DNA
   <213> Unknown
<220>
   <223> Found in multiple species
<400> 1009
   actcaataca tcagcagctc t 21
<210> 1010
   <211> 21
   <212> DNA
   <213> Unknown
<220>
   <223> Found in multiple species
<400> 1010
   actcaaaccc accaagacca c 21
<210> 1011
   <211> 21
   <212> DNA
   <213> Unknown
<220>
   <223> Found in multiple species
<400> 1011
   acctgtgtat gggacaaaaa g 21
<210> 1012
   <211> 21
   <212> DNA
   <213> Unknown
<220>
   <223> Found in multiple species
<400> 1012
   acccaccaag accacttctc t 21
<210> 1013
   <211> 21
   <212> DNA
   <213> Unknown
<220>
   <223> Found in multiple species
<400> 1013
   accagctcct tcattatgct c 21
<210> 1014
   <211> 21
   <212> DNA
   <213> Unknown
<220>
   <223> Found in multiple species
<400> 1014
   accacttctc ttcctcatct c 21
<210> 1015
   <211> 21
   <212> DNA
   <213> Unknown
<220>
   <223> Found in multiple species
<400> 1015
   accaagacca cttctcttcc t 21
<210> 1016
   <211> 21
   <212> DNA
   <213> Unknown
<220>
   <223> Found in multiple species
<400> 1016
   acatcttcag ctgtaaactc a 21
<210> 1017
   <211> 21
   <212> DNA
   <213> Unknown
<220>
   <223> Found in multiple species
<400> 1017
   acaggttcat tcattggaaa c 21
<210> 1018
   <211> 21
   <212> DNA
   <213> Unknown
<220>
   <223> Found in multiple species
<400> 1018
   acactccttc atctgctcct c 21
<210> 1019
   <211> 21
   <212> DNA
   <213> Unknown
<220>
   <223> Found in multiple species
<400> 1019
   acaccagctc cttcattatg c 21
<210> 1020
   <211> 21
   <212> DNA
   <213> Unknown
<220>
   <223> Found in multiple species
<400> 1020
   acacaggttc attcattgga a 21
<210> 1021
   <211> 21
   <212> DNA
   <213> Unknown
<220>
   <223> Found in multiple species
<400> 1021
   aattccggta ataagtcgga g 21
<210> 1022
   <211> 21
   <212> DNA
   <213> Unknown
<220>
   <223> Found in multiple species
<400> 1022
   aattcaaact tatttggagc a 21
<210> 1023
   <211> 21
   <212> DNA
   <213> Unknown
<220>
   <223> Found in multiple species
<400> 1023
   aatggtgttg tttccaatga a 21
<210> 1024
   <211> 21
   <212> DNA
   <213> Unknown
<220>
   <223> Found in multiple species
<400> 1024
   aatgatgaaa tggtgttgtt t 21
<210> 1025
   <211> 21
   <212> DNA
   <213> Unknown
<220>
   <223> Found in multiple species
<400> 1025
   aatgaatgaa cctgtgtatg g 21
<210> 1026
   <211> 21
   <212> DNA
   <213> Unknown
<220>
   <223> Found in multiple species
<400> 1026
   aatgaacctg tgtatgggac a 21
<210> 1027
   <211> 21
   <212> DNA
   <213> Unknown
<220>
   <223> Found in multiple species
<400> 1027
   aataagtcgg aggcggcgac g 21
<210> 1028
   <211> 21
   <212> DNA
   <213> Unknown
<220>
   <223> Found in multiple species
<400> 1028
   aataagcaat tgcaggagcc a 21
<210> 1029
   <211> 21
   <212> DNA
   <213> Unknown
<220>
   <223> Found in multiple species
<400> 1029
   aagtttactg ggtttcatga g 21
<210> 1030
   <211> 21
   <212> DNA
   <213> Unknown
<220>
   <223> Found in multiple species
<400> 1030
   aagtggtctt ggtgggtttg a 21
<210> 1031
   <211> 21
   <212> DNA
   <213> Unknown
<220>
   <223> Found in multiple species
<400> 1031
   aagtcggagg cggcgacggc a 21
<210> 1032
   <211> 21
   <212> DNA
   <213> Unknown
<220>
   <223> Found in multiple species
<400> 1032
   aagggagatg aggaagagaa g 21
<210> 1033
   <211> 21
   <212> DNA
   <213> Unknown
<220>
   <223> Found in multiple species
<400> 1033
   aagcaattgc aggagccaac t 21
<210> 1034
   <211> 21
   <212> DNA
   <213> Unknown
<220>
   <223> Found in multiple species
<400> 1034
   aagagaagtg gtcttggtgg g 21
<210> 1035
   <211> 21
   <212> DNA
   <213> Unknown
<220>
   <223> Found in multiple species
<400> 1035
   aagaccactt ctcttcctca t 21
<210> 1036
   <211> 21
   <212> DNA
   <213> Unknown
<220>
   <223> Found in multiple species
<400> 1036
   aacttatttg gagcataatg a 21
<210> 1037
   <211> 21
   <212> DNA
   <213> Unknown
<220>
   <223> Found in multiple species
<400> 1037
   aactcatgaa acccagtaaa c 21
<210> 1038
   <211> 21
   <212> DNA
   <213> Unknown
<220>
   <223> Found in multiple species
<400> 1038
   aactcagcaa actcatgaaa c 21
<210> 1039
   <211> 21
   <212> DNA
   <213> Unknown
<220>
   <223> Found in multiple species
<400> 1039
   aactcaaacc caccaagacc a 21
<210> 1040
   <211> 21
   <212> DNA
   <213> Unknown
<220>
   <223> Found in multiple species
<400> 1040
   aacctgtgta tgggacaaaa a 21
<210> 1041
   <211> 21
   <212> DNA
   <213> Unknown
<220>
   <223> Found in multiple species
<400> 1041
   aacccaccaa gaccacttct c 21
<210> 1042
   <211> 21
   <212> DNA
   <213> Unknown
<220>
   <223> Found in multiple species
<400> 1042
   aacatcttca gctgtaaact c 21
<210> 1043
   <211> 21
   <212> DNA
   <213> Unknown
<220>
   <223> Found in multiple species
<400> 1043
   aaattccggt aataagtcgg a 21
<210> 1044
   <211> 21
   <212> DNA
   <213> Unknown
<220>
   <223> Found in multiple species
<400> 1044
   aaattcaaac ttatttggag c 21
<210> 1045
   <211> 21
   <212> DNA
   <213> Unknown
<220>
   <223> Found in multiple species
<400> 1045
   aaatggtgtt gtttccaatg a 21
<210> 1046
   <211> 21
   <212> DNA
   <213> Unknown
<220>
   <223> Found in multiple species
<400> 1046
   aaataagcaa ttgcaggagc c 21
<210> 1047
   <211> 21
   <212> DNA
   <213> Unknown
<220>
   <223> Found in multiple species
<400> 1047
   aaacttattt ggagcataat g 21
<210> 1048
   <211> 21
   <212> DNA
   <213> Unknown
<220>
   <223> Found in multiple species
<400> 1048
   aaactcatga aacccagtaa a 21
<210> 1049
   <211> 21
   <212> DNA
   <213> Unknown
<220>
   <223> Found in multiple species
<400> 1049
   aaactcagca aactcatgaa a 21
<210> 1050
   <211> 21
   <212> DNA
   <213> Unknown
<220>
   <223> Found in multiple species
<400> 1050
   aaactcaaac ccaccaagac c 21
<210> 1051
   <211> 21
   <212> DNA
   <213> Unknown
<220>
   <223> Found in multiple species
<400> 1051
   aaacccacca agaccacttc t 21
<210> 1052
   <211> 21
   <212> DNA
   <213> Unknown
<220>
   <223> Found in multiple species
<400> 1052
   aaacaacacc atttcatcat t 21
<210> 1053
   <211> 21
   <212> DNA
   <213> Unknown
<220>
   <223> Found in multiple species
<400> 1053
   tgggttgaat tcagccgtat t 21
<210> 1054
   <211> 21
   <212> DNA
   <213> Unknown
<220>
   <223> Found in multiple species
<400> 1054
   tgaattcaac ccactttcac t 21
<210> 1055
   <211> 21
   <212> DNA
   <213> Unknown
<220>
   <223> Found in multiple species
<400> 1055
   tgaaagtggg ttgaattcag c 1
<210> 1056
   <211> 21
   <212> DNA
   <213> Unknown
<220>
   <223> Found in multiple species
<400> 1056
   tacggctgaa ttcaacccac t 21
<210> 1057
   <211> 21
   <212> DNA
   <213> Unknown
<220>
   <223> Found in multiple species
<400> 1057
   gttgaattca gccgtattgg c 21
<210> 1058
   <211> 21
   <212> DNA
   <213> Unknown
<220>
   <223> Found in multiple species
<400> 1058
   gtgggttgaa ttcagccgta t 21
<210> 1059
   <211> 21
   <212> DNA
   <213> Unknown
<220>
   <223> Found in multiple species
<400> 1059
   gtgaaagtgg gttgaattca g 21
<210> 1060
   <211> 21
   <212> DNA
   <213> Unknown
<220>
   <223> Found in multiple species
<400> 1060
   ggttgaattc agccgtattg g 21
<210> 1061
   <211> 21
   <212> DNA
   <213> Unknown
<220>
   <223> Found in multiple species
<400> 1061
   gggttgaatt cagccgtatt g 21
<210> 1062
   <211> 21
   <212> DNA
   <213> Unknown
<220>
   <223> Found in multiple species
<400> 1062
   ggctgaattc aacccacttt c 21
<210> 1063
   <211> 21
   <212> DNA
   <213> Unknown
<220>
   <223> Found in multiple species
<400> 1063
   gctgaattca acccactttc a 21
<210> 1064
   <211> 21
   <212> DNA
   <213> Unknown
<220>
   <223> Found in multiple species
<400> 1064
   gccaatacgg ctgaattcaa c 21
<210> 1065
   <211> 21
   <212> DNA
   <213> Unknown
<220>
   <223> Found in multiple species
<400> 1065
   gagtgaaagt gggttgaatt c 21
<210> 1066
   <211> 21
   <212> DNA
   <213> Unknown
<220>
   <223> Found in multiple species
<400> 1066
   gaattcaacc cactttcact c 21
<210> 1067
   <211> 21
   <212> DNA
   <213> Unknown
<220>
   <223> Found in multiple species
<400> 1067
   gaaagtgggt tgaattcagc c 21
<210> 1068
   <211> 21
   <212> DNA
   <213> Unknown
<220>
   <223> Found in multiple species
<400> 1068
   ctgaattcaa cccactttca c 21
<210> 1069
   <211> 21
   <212> DNA
   <213> Unknown
<220>
   <223> Found in multiple species
<400> 1069
   cggctgaatt caacccactt t 21
<210> 1070
   <211> 21
   <212> DNA
   <213> Unknown
<220>
   <223> Found in multiple species
<400> 1070
   cgagtgaaag tgggttgaat t 21
<210> 1071
   <211> 21
   <212> DNA
   <213> Unknown
<220>
   <223> Found in multiple species
<400> 1071
   ccaatacggc tgaattcaac c 21
<210> 1072
   <211> 21
   <212> DNA
   <213> Unknown
<220>
   <223> Found in multiple species
<400> 1072
   caatacggct gaattcaacc c 21
<210> 1073
   <211> 21
   <212> DNA
   <213> Unknown
<220>
   <223> Found in multiple species
<400> 1073
   attcaaccca ctttcactcg t 21
<210> 1074
   <211> 21
   <212> DNA
   <213> Unknown
<220>
   <223> Found in multiple species
<400> 1074
   atacggctga attcaaccca c 21
<210> 1075
   <211> 21
   <212> DNA
   <213> Unknown
<220>
   <223> Found in multiple species
<400> 1075
   agtgggttga attcagccgt a 21
<210> 1076
   <211> 21
   <212> DNA
   <213> Unknown
<220>
   <223> Found in multiple species
<400> 1076
   agtgaaagtg ggttgaattc a 21
<210> 1077
   <211> 21
   <212> DNA
   <213> Unknown
<220>
   <223> Found in multiple species
<400> 1077
   acggctgaat tcaacccact t 21
<210> 1078
   <211> 21
   <212> DNA
   <213> Unknown
<220>
   <223> Found in multiple species
<400> 1078
   acgagtgaaa gtgggttgaa t 21
<210> 1079
   <211> 21
   <212> DNA
   <213> Unknown
<220>
   <223> Found in multiple species
<400> 1079
   aattcaaccc actttcactc g 21
<210> 1080
   <211> 21
   <212> DNA
   <213> Unknown
<220>
   <223> Found in multiple species
<400> 1080
   aatacggctg aattcaaccc a 21
<210> 1081
   <211> 21
   <212> DNA
   <213> Unknown
<220>
   <223> Found in multiple species
<400> 1081
   aagtgggttg aattcagccg t 21
<210> 1082
   <211> 21
   <212> DNA
   <213> Unknown
<220>
   <223> Found in multiple species
<400> 1082
   aaagtgggtt gaattcagcc g 21
<210> 1083
   <211> 21
   <212> DNA
   <213> Amaranthus palmeri
<400> 1083
   tccgtagctt acataccgaa g 21
<210> 1084
   <211> 21
   <212> DNA
   <213> Amaranthus palmeri
<400> 1084
   tccaagtgaa taggagaaac a 21
<210> 1085
   <211> 21
   <212> DNA
   <213> Amaranthus palmeri
<400> 1085
   agcagcttct gcgtcttcta c 21
<210> 1086
   <211> 21
   <212> DNA
   <213> Amaranthus palmeri
<400> 1086
   acagcacgca cgccaagacc g 21
<210> 1087
   <211> 21
   <212> DNA
   <213> Amaranthus palmeri
<400> 1087
   cgatgtaagg aatttggtaa a 21
<210> 1088
   <211> 21
   <212> DNA
   <213> Amaranthus palmeri
<400> 1088
   cgaggggatt gcagcagaag a 21
<210> 1089
   <211> 21
   <212> DNA
   <213> Amaranthus palmeri
<400> 1089
   gtaggagaat acggtgaagt a 21
<210> 1090
   <211> 21
   <212> DNA
   <213> Amaranthus palmeri
<400> 1090
   gaccccaaga aaatcgtctg c 21
<210> 1091
   <211> 21
   <212> DNA
   <213> Amaranthus palmeri
<400> 1091
   attgaggagt acgagaagac t 21
<210> 1092
   <211> 21
   <212> DNA
   <213> Amaranthus palmeri
<400> 1092
   cttgaacgta aacaggttcc a 21